# EUROPÄISCHE PATENTSCHRIFT

(11) **EP 0 628 026 B1**
(45) Veröffentlichungstag und Bekanntmachung des Hinweises auf die Patenterteilung: **22.01.1997**
(21) Anmeldenummer: 93903979.8
(22) Anmeldetag: 17.02.1993
(51) Int. Cl.: C07C 255/64, C07D 261/08, C07D 263/32, A01N 37/34, A01N 37/52, A01N 43/74

(54) **CYANOOXIMETHER, VERFAHREN ZU IHRER HERSTELLUNG, SIE ENTHALTENDE MITTEL UND DEREN VERWENDUNG**
CYANO-OXIME ETHERS, PROCESS FOR PRODUCING THEM, AGENTS CONTAINING THEM AND THEIR USE
CYANO-OXIME-ETHERS, LEURS PROCEDE DE FABRICATION, PRODUITS LES RENFERMANT ET LEUR APPLICATION

(30) Priorität: 29.02.1992 DE 4206353
(43) Veröffentlichungstag der Anmeldung: 14.12.1994
(73) Patentinhaber: BASF Aktiengesellschaft, 67063 Ludwigshafen (DE)
(72) Erfinder: OBERDORF, Klaus, D-6900 Heidelberg (DE); KARDORFF, Uwe, D-6800 Mannheim 1 (DE); THEOBALD, Hans, D-6703 Limburgerhof (DE); HARREUS, Albrecht, D-6700 Ludwigshafen (DE); KOENIG, Hartmann, D-6703 Limburgerhof (DE); HARRIES, Volker, D-6710 Frankenthal (DE)
(86) Internationale Anmeldenummer: EP9300371
(87) Internationale Veröffentlichungsnummer: WO9316985

(56) Entgegenhaltungen:
- EP-A- 0 011 047
- EP-A- 0 303 934
- EP-A- 0 463 488
- DE-A- 2 837 857

## Beschreibung

Die vorliegende Erfindung betrifft Cyanooximether der Formel I

R¹R²CH-ON=C(CN)-R³ I

in der die Substituenten die folgende Bedeutung haben:
- R¹: Wasserstoff oder C₁-C₄-Alkyl;
- R²: ein über ein Kohlenstoffatom gebundenes ein- bis dreikerniges aliphatisches oder aromatisches Ringsystem, das neben Kohlenstoffatomen ein bis vier Stickstoffatome oder ein bis drei Heteroatome aus der Gruppe Sauerstoff, Schwefel und Stickstoff enthalten kann, wobei dieses Ringsystem ein bis fünf Halogenatome und/oder ein bis vier der folgenden Reste tragen kann:
- Cyano, Cyanato, Thiocyanato, Nitro, Amino, Hydroxy, Carboxyl, C₁-C₆-Alkyl, C₁-C₄-Halogenalkyl, C₁-C₆-Alkoxy, C₁-C₄-Halogenalkoxy, C₁-C₄-Alkoxy-C₁-C₄-alkyl, C₁-C₆-Alkylthio, C₁-C₄-Halogenalkylthio, C₁-C₄-Alkylthio-C₁-C₄-alkyl, C₃-C₁₅-Alkenyl, C₃-C₁₅-Alkenyloxy, C₃-C₈-Cycloalkyl, C₅-C₈-Cycloalkenyl, C₃-C₈-Cycloalkoxy, C₅-C₈-Cycloalkenyloxy, C₁-C₆-Alkylamino, Di-C₁-C₆-alkylamino, C₁-C₆-Alkylcarbonyl, C₁-C₆-Alkoxycarbonyl, C₁-C₆-Alkylaminocarbonyl, Di-C₁-C₆-alkylaminocarbonyl, C₁-C₆-Alkylcarboxyl,
- Phenyl, Phenoxy, Phenylthio, Phenylamino, Phenyl-C₁-C₄-alkyl, Phenoxy-C₁-C₄-alkyl, Phenylthio-C₁-C₄-alkyl, Phenylamino-C₁-C₄-alkyl, wobei die aromatischen Ringe ihrerseits ein bis fünf Halogenatome und/oder ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio,
- ein über ein Kohlenstoffatom gebundenes fünf- oder sechsgliedriges aromatisches Ringsystem, das neben Kohlenstoffatomen ein bis drei Stickstoffatome oder ein oder zwei Stickstoffatome und ein Sauerstoff- oder ein Schwefelatom oder ein Sauerstoff- oder ein Schwefelatom enthalten kann, wobei dieses Ringsystem ein bis vier Halogenatome und/oder ein bis drei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
- R³: ggf. subst. Alkyl oder ein ggf. subst., über ein Kohlenstoffatom gebundenes, aliphatisches Ringsystem, das neben Kohlenstoffatomen ein oder zwei Heteroatome aus der Gruppe Sauerstoff, Schwefel und Stickstoff enthalten kann.

Des weiteren betrifft die Erfindung Verfahren zur Herstellung dieser Verbindungen, die Verbindungen I enthaltende Mittel zur Bekämpfung von Schädlingen und deren Verwendung.

Aus der Literatur sind Cyanooximether als Wirkstoffe zum Schutz von Pflanzen und als Pharmaka bekannt (Fungizide: EP-A 370 629; Wachstumsregulatoren: US-A 4,451,286; Antidots: EP-A 12 158, EP-A 122 231; Insektizide: EP-A 303 934; Herbizide: DE-A 28 37 857; Pharmaka: DE-A 28 00 316).

Aus EP-A-463 488 sind weiter Phenylessigsäure-Derivate mit Oximether-Gruppen bekannt, die zur Schädlingsbekämpfung geeignet sind und die sich durch ihre fungizide Wirkung hervortun.

Der vorliegenden Erfindung lagen neue, zur Schädlingsbekämpfung geeignete Verbindungen als Aufgabe zugrunde.

Demgemäß wurden die eingangs definierten Cyanooximether der Formel I gefunden. Außerdem wurden Verfahren zur Herstellung dieser Verbindungen, diese Verbindungen enthaltende Mittel zur Bekämpfung von Schädlingen und deren Verwendung gefunden.

Man erhält die erfindungsgemäßen Verbindungen im allgemeinen dadurch, daß man ein Cyanoxim der Formel II in an sich bekannter Weise in einem inerten organischen Lösungsmittel mit einer Verbindung III verethert.

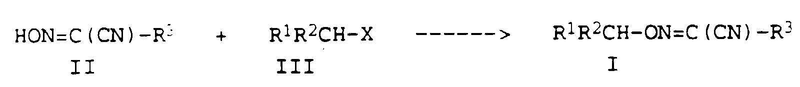

Der Substituent X in der Formel III steht für eine nukleofuge Abgangsgruppe wie Halogen, beispielsweise Chlor, Brom und Jod, aromatische und aliphatische Sulfonate, beispielsweise p-Toluolsulfonat, Methansulfonat und Triflat sowie Carboxylate wie Acetat, insbesondere Chlor und Brom.

Diese Umsetzung wird üblicherweise bei Temperaturen von -20°C bis 100°C, vorzugsweise 20°C bis 80°C durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Dimethylformamid, N-Methylpyrrolidon, Pyridin, Acetonitril und Tetrahydrofuran.

Es können auch Gemische der genannten Lösungsmittel verwendet werden.

Desweiteren kann die Umsetzung auch in einem Zweiphasen-Lösungsmittelsystem, beispielsweise Methylenchlorid/Wasser, unter Zugabe eines geeigneten Phasentransferkatalysators durchgeführt werden. Als Phasentransferkatalysatoren eignen sich beispielsweise Trimethylammonium-Salze wie Benzyltrimethylammoniumchlorid und Kronenether wie 18-Krone-6.

Die Edukte werden im allgemeinen in äquimolaren Mengen miteinander umgesetzt. Es kann für die Ausbeute vorteilhaft sein, einen der Ausgangsstoffe in einem überschuß von 0,1 bis 10 mol-äq., vorzugsweise 0,2 bis 2 mol-äq., bezogen auf den Reaktionspartner einzusetzen.

In der Regel ist es von Vorteil, die Umsetzung in Gegenwart einer Base durchzuführen.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Kaliumcarbonat, Natriummethanolat, Natriumethanolat und Natriumhydrid verwendet.

Die Basen werden im allgemeinen äquimolarer eingesetzt, sie können aber auch im Überschuß oder gegebenenfalls als Lösungsmittel verwendet werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die neuen Verbindungen der allgemeinen Formel I können bei der Herstellung aufgrund der C=N-Doppelbindungen als E/Z-Isomerengemische anfallen. Diese können in der üblichen Weise, z.B. durch Kristallisation oder Chromatographie in die einzelnen Komponenten aufgetrennt werden. Sowohl die einzelnen isomeren Verbindungen als auch ihre Gemische werden von der Erfindung umfaßt und sind als Schädlingsbekämpfungsmittel brauchbar.

Die für die Herstellung der Verbindungen I benötigten Cyanoxime II sind in der Literatur bekannt oder lassen sich in Analogie zu bekannten Verfahren herstellen (J. Org. Chem., 25(9), 1471 (1960); Rec. Trav. Chim. Pays-Bas, 91, 711 (1972); EP-A 031,754; EP-A 074,047; EP-A 115,318; EP-A 129,889; EP-A 150,822; EP-A 201,764; EP-A 201,807).

Die zur Herstellung der Verbindungen der allgemeinen Formel I benötigten Verbindungen der allgemeinen Formel III sind entweder in der Literatur beschrieben oder können in Analogie zu bekannten Verfahren hergestellt werden.

Die zur Herstellung der Verbindungen der allgemeinen Formel I benötigten Verbindungen der allgemeinen Formel III, in denen R² 4-Oxazolyl bedeutet, wobei der Ring wie vorstehend beschrieben substituiert sein kann, sind entweder in der Literatur beschrieben oder können nach den dort beschriebenen Methoden hergestellt werden (z.B.: J. Simiti und E. Chindris, Arch. Pharm. 304, 425-429 (1971).

Die zur Herstellung der Verbindungen der allgemeinen Formel I benötigten Verbindungen der allgemeinen Formel III, in denen R² 5-Isoxazolyl bedeutet, wobei der Ring wie vorstehend beschrieben substituiert sein kann, sind entweder in der Literatur beschrieben oder können nach den dort beschriebenen Methoden hergestellt werden (z.B. G.A. Lec, Synthesis 1982, 508-509).

Im Hinblick auf ihre Verwendung zur Bekämpfung von Schädlingen werden Verbindungen der Formel I bevorzugt, in denen die Substituenten die folgende Bedeutung haben:
R¹
Wasserstoff oder
C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Wasserstoff oder Methyl, insbesondere Wasserstoff;
ein über ein Kohlenstoffatom gebundenes ein- bis drei-kerniges aliphatisches oder aromatisches Ringsystem, das neben Kohlenstoffatomen ein bis vier Stickstoffatome oder ein bis drei Heteroatome aus der Gruppe Sauerstoff, Schwefel und Stickstoff enthalten kann, beispielsweise C₃-C₈-Cycloalkyl wie Cyclopropyl, Cyclobutyl, Cyclopentyl, Cyclohexyl, Cycloheptyl und Cyclooctyl, vorzugsweise Cyclopropyl, Cyclopentyl und Cyclohexyl, insbesondere Cyclopropyl und Cyclohexyl;
C₅-C₈-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-1-enyl, vorzugsweise Cyclopent-1-enyl, Cyclopent-3-enyl und Cyclohex-1-enyl, insbesondere Cyclopent-3-enyl;
C₅-C₈-Cycloalkdienyl wie Cyclopenta-1,3-dien-1-yl, Cyclopenta-1,3-dien-2-yl, Cyclopenta-1,3-dien-5-yl, Cyclohexa-1,3-dien-1-yl, Cyclohexa-1,3-dien-2-yl, Cyclohexa-1,3-dien-5-yl, Cyclohexa-1,4-dien-1-yl, Cyclohexa-1,4-dien-3-yl, Cyclohepta-1,3-dien-1-yl, Cyclohepta-1,3-dien-2-yl, Cyclohepta-1,3-dien-5-yl, Cyclohepta-1,3-dien-6-yl, Cyclohepta-1,4-dien-1-yl, Cyclohepta-1,4-dien-2-yl, Cyclohepta-1,4-dien-3-yl, Cyclohepta-1,4-dien-6-yl, Cycloocta-1,3-dien-1-yl, Cycloocta-1,3-dien-2-yl, Cycloocta-1,3-dien-5-yl, Cycloocta-1,3-dien-6-yl, Cycloocta-1,4-dien-1-yl, Cycloocta-1,4-dien-2-yl, Cycloocta-1,4-dien-3-yl, Cycloocta-1,4-dien-6-yl, Cycloocta-1,4-dien-7-yl, Cycloocta-1,4-dien-1-yl und Cycloocta-1,4-dien-3-yl;
3- bis 6-gliedrige, gesättigte oder ungesättigte Heterocyclen, enthaltend ein bis drei Stickstoffatome und/oder ein Sauerstoff- oder Schwefelatom wie 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl,1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,5-Dihydrofur-2-yl, 2,5-Dihydrofur-3-yl, 2,3-Dihydrothien-2-yl, 2,3-Dihydrothien-3-yl, 2, 5-Dihydrothien-2-yl, 2,5-Dihydrothien-3-yl, 2,3-Pyrrolin-2-yl, 2,3-Pyrrolin-3-yl, 2,5-Pyrrolin-2-yl, 2,5-Pyrrolin-3-yl, 2,3-Isoxazolin-3-yl, 2,5-Isoxazolin-3-yl, 4,5-Isoxazolin-3-yl, 2,3-Isoxazolin-4-yl, 2,5-Isoxazolin-4-yl, 4,5-Isoxazolin-4-yl, 2,3-Isoxazolin-5-yl, 2,5-Isoxazolin-5-yl, 4,5-Isoxazolin-5-yl, 2,3-Isothiazolin-3-yl, 2,5-Isothiazolin-3-yl, 4,5-Isothiazolin-3-yl, 2,3-Isothiazolin-4-yl, 2,5-Isothiazolin-4-yl, 4,5-Isothiazolin-4-yl, 2,3-Isothiazolin-4-yl, 2,5-Isothiazolin-5-yl, 4, 5-Isothiazolin-5-yl, 2,3-Dihydropyrazol-3-yl, 2,3-Dihydropyrazol-4-yl, 2,3-Dihydropyrazol-5-yl, 3,4-Dihydropyrazol-3-yl, 3,4-Dihydropyrazol-4-yl, 3,4-Dihydropyrazol-5-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-4-yl, 2,3-Dihydrooxazol-5-yl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, Thiazol-2-in-2-yl, 3,4,5,6-Tetrahydropyridin-2-yl, 4H-1,3-Thiazin-2-yl, Oxiranyl und 1,3-Dithian-2-yl;
fünfring Heteroaromaten enthaltend ein bis drei Stickstoffatome und/oder ein Sauerstoff- oder Schwefelatom wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, 1,2,3-Oxadiazol-4-yl, 1,2,3-Oxadiazol-5-yl, 1,2,5-Triazol-3-yl, 1,2,3-Triazol-4-yl, 1,2,3-Triazol-5-yl, 5-Tetrazolyl, 1,2,3,4-Thiatriazol-5-yl und 1,2,3,4-Oxatriazol-5-yl, insbesondere 3-Isoxazolyl, 5-Isoxazolyl, 4-Oxazolyl und 4-Thiazolyl;
oder sechsring Heteroaromaten enthaltend ein bis drei Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, insbesondere 2-Pyridinyl, 3-Pyridinyl, 2-Pyrimidinyl, 3-Pyrimidinyl und 1,3,5-Triazin-2-yl, sowie Phenyl, 1-Naphthyl, 2-Naphthyl, 1-Anthracenyl, 2-Anthracenyl, 9-Anthracenyl, 1-Phenanthrenyl, 2-Phenanthrenyl, 3-Phenanthrenyl, 4-Phenanthrenyl und 9-Phenanthrenyl, insbesondere Phenyl;
wobei diese vorstehend genannten Ringsysteme ein bis fünf Halogenatome wie Fluor, Chlor, Brom und Jod, insbesondere Fluor und Chlor insbesondere und/oder ein bis vier der folgenden Reste tragen können:
Cyano, Cyanato, Thiocyanato, Nitro, Amino, Hydroxy, Carboxyl, C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl,3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, 1-Methylethyl, 1-Methylpropyl und 1,1-Dimethylethyl;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;
C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy, insbesondere Methoxy, Ethoxy und 1-Methylethyloxy;
C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
C₁-C₄-Alkoxy-C₁-C₄-alkyl wie Methoxymethyl, Ethoxymethyl, Propyloxymethyl, 1-Methylethoxymethyl, Butyloxymethyl, 1-Methyl-propyloxymethyl, 2-Methylpropyloxymethyl, 1,1-Dimethylethoxymethyl, 1-Methoxyethyl, 1-Ethoxyethyl, 1-Propyloxyethyl, 1-(1-Methylethoxy)ethyl, 1-Butyloxyethyl, 1-(1-Methyl-propyloxy)ethyl, 1-(2-Methylpropyloxy)ethyl, 1-(1,1-Dimethylethoxy)ethyl, 2-Methoxyethyl, 2-Ethoxyethyl, 2-Propyloxyethyl, 2-(1-Methylethoxy)ethyl, 2-Butyloxyethyl, 2-(1-Methyl-propyloxy)ethyl, 2-(2-Methylpropyloxy)ethyl, 2-(1,1-Dimethylethoxy)ethyl, 1-Methoxypropyl, 1-Ethoxypropyl, 1-Propyloxypropyl, 1-(1-Methylethoxy)propyl, 1-Butyloxypropyl, 1-(1-Methyl-propyloxy)propyl, 1-(2-Methylpropyloxy)propyl, 1-(1,1-Dimethylethoxy)propyl, 2-Methoxypropyl, 2-Ethoxypropyl, 2-Propyloxypropyl, 2-(1-Methylethoxy)propyl, 2-Butyloxypropyl, 2-(1-Methyl-propyloxy)propyl, 2-(2-Methylpropyloxy)propyl, 2-(1,1-Dimethylethoxy)propyl, 3-Methoxypropyl, 3-Ethoxypropyl, 3-Propyloxypropyl, 3-(1-Methylethoxy)propyl, 3-Butyloxypropyl, 3-(1-Methyl-propyloxy)propyl, 3-(2-Methylpropyloxy)propyl, 3-(1,1-Dimethylethoxy)propyl, 1-Methoxy-(1-methyl)-ethyl, 1-Ethoxy-(1-methyl)-ethyl, 1-Propyloxy-(1-methyl)-ethyl, 1-(1-Methylethoxy)-(1-methyl)-ethyl, 1-Butyloxy-(1-methyl)-ethyl, 1-(1-Methyl-propyloxy)-(1-methyl)-ethyl, 1-(2-Methylpropyloxy)-(1-methyl)-ethyl, 1-(1,1-Dimethylethoxy)-(1-methyl)-ethyl, 2-Methoxy-(1-methyl)-ethyl, 2-Ethoxy-(1-methyl)-ethyl, 2-Propyloxy-(1-methyl)-ethyl, 2-(1-Methylethoxy)-(1-methyl)-ethyl, 2-Butyloxy-(1-methyl)-ethyl, 2-(1-Methyl-propyloxy)-(1-methyl)-ethyl, 2-(2-Methylpropyloxy)-(1-methyl)-ethyl, 2-(1,1-Dimethylethoxy)-(1-methyl)-ethyl, 1-Methoxybutyl, 1-Ethoxybutyl, 1-Propyloxybutyl, 1-(1-Methylethoxy)butyl, 1-Butyloxybutyl, 1-(1-Methyl-propyloxy)butyl, 1-(2-Methylpropyloxy)butyl, 1-(1,1-Dimethylethoxy)butyl, 2-Methoxybutyl, 2-Ethoxybutyl, 2-Propyloxybutyl, 2-(1-Methylethoxy)butyl, 2-Butyloxybutyl, 2-(1-Methyl-propyloxy)butyl, 2-(2-Methylpropyloxy)butyl, 2-(1,1-Dimethylethoxy)butyl, 3-Methoxybutyl, 3-Ethoxybutyl, 3-Propyloxybutyl, 3-(1-Methylethoxy)butyl, 3-Butyloxybutyl, 3-(1-Methyl-propyloxy)butyl, 3-(2-Methylpropyloxy)butyl, 3-(1,1-Dimethylethoxy)butyl, 4-Methoxybutyl, 4-Ethoxybutyl, 4-Propyloxybutyl, 4-(1-Methylethoxy)butyl, 4-Butyloxybutyl, 4-(1-Methyl-propyloxy)butyl, 4-(2-Methylpropyloxy)butyl, 4-(1,1-Dimethylethoxy)butyl, 1-Methoxy-(1-methyl)-propyl, 1-Ethoxy-(1-methyl)-propyl, 1-Propyloxy-(1-methyl)-propyl, 1-(1-Methylethoxy)-(1-methyl)-propyl, 1-Butyloxy-(1-methyl)-propyl, 1-(1-Methyl-propyloxy)-(1-methyl)-propyl, 1-(2-Methylpropyloxy)-(1-methyl)-propyl, 1-(1,1-Dimethylethoxy)-(1-methyl)-propyl, 2-Methoxy-(1-methyl)-propyl, 2-Ethoxy-(1-methyl)-propyl, 2-Propyloxy-(1-methyl)-propyl, 2-(1-Methylethoxy)-(1-methyl)-propyl, 2 -Butyloxy-(1-methyl)-propyl, 2-(1-Methyl-propyloxy)-(1-methyl)-propyl, 2-(2-Methylpropyloxy)-(1-methyl)-propyl, 2-(1,1-Dimethylethoxy)-(1-methyl)-propyl, 3-Methoxy-(1-methyl)-propyl, 3-Ethoxy-(1-methyl)-propyl, 3-Propyloxy-(1-methyl)-propyl, 3-(1-Methylethoxy)-(1-methyl)-propyl, 3-Butyloxy-(1-methyl)-propyl, 3-(1-Methyl-propyloxy)-(1-methyl)-propyl, 3-(2-Methylpropyloxy)-(1-methyl)-propyl, 3-(1,1-Dimethylethoxy)-(1-methyl)-propyl, 1-Methoxy-(2-methyl)-propyl, 1-Ethoxy-(2-methyl)-propyl, 1-Propyloxy-(2-methyl)-propyl, 1-(1-Methylethoxy)-(2-methyl)-propyl, 1-Butyloxy-(2-methyl)-propyl, 1-(1-Methyl-propyloxy)-(2-methyl)-propyl, 1-(2-Methylpropyloxy)-(2-methyl)-propyl, 1-(1,1-Dimethylethoxy)-(2-methyl)-propyl, 2-Methoxy-(2-methyl)-propyl, 2-Ethoxy-(2-methyl)-propyl, 2-Propyloxy-(2-methyl)-propyl, 2-(1-Methylethoxy)-(2-methyl)-propyl, 2-Butyloxy-(2-methyl)-propyl, 2-(1-Methyl-propyloxy)-(2-methyl)-propyl, 2-(2-Methylpropyloxy)-(2-methyl)-propyl, 2-(1,1-Dimethylethoxy)-(2-methyl)-propyl, 3-Methoxy-(2-methyl)-propyl, 3-Ethoxy-(2-methyl)-propyl, 3-Propyloxy-(2-methyl)-propyl, 3-(1-Methylethoxy)-(2-methyl)-propyl, 3-Butyloxy-(2-methyl)-propyl, 3-(1-Methyl-propyloxy)-(2-methyl)-propyl, 3-(2-Methylpropyloxy)-(2-methyl)-propyl, 3-(1,1-Dimethylethoxy)-(2-methyl)-propyl, 2-Methoxy-(1,1-dimethyl)-ethyl, 2-Ethoxy-(1,1-dimethyl)-ethyl, 2-Propyloxy-(1,1-dimethyl)-ethyl, 2-(1-Methylethoxy)-(1,1-dimethyl)-ethyl, 2-Butyloxy-(1,1-dimethyl)-ethyl, 2-(1-Methyl-propyloxy)-(1,1-dimethyl)-ethyl, 2-(2-Methylpropyloxy)-(1,1-dimethyl)-ethyl, 2-(1,1-Dimethylethoxy)-(1,1-dimethyl)-ethyl, insbesondere Methoxymethyl;
C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethyl-butylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-l-methylpropylthio und 1-Ethyl-2-methylpropylthio, insbesondere Methylthio, Ethylthio und 1-Methylethylthio;
C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio;
C₁-C₄-Alkylthio-C₁-C₄-alkyl, wie Methylthiomethyl, Ethylthiomethyl, Propylthiomethyl, 1-Methylethylthiomethyl, Butylthiomethyl, 1-Methyl-propylthiomethyl, 2-Methylpropylthiomethyl, 1,1-Dimethylethylthiomethyl, 1-Methylthioethyl, 1-Ethylthioethyl, 1-Propylthioethyl, 1-(1-Methylethylthio)ethyl, 1-Butylthioethyl, 1-(1-Methyl-propylthio)ethyl, 1-(2-Methylpropylthio)ethyl, 1-(1,1-Dimethylethylthio)ethyl, 2-Methylthioethyl, 2-Ethylthioethyl, 2-Propylthioethyl, 2-(1-Methylethylthio)ethyl, 2-Butylthioethyl, 2-(1-Methylpropylthio)ethyl, 2-(2-Methylpropylthio)ethyl, 2-(1,1-Dimethylethylthio)ethyl, 1-Methylthiopropyl, 1-Ethylthiopropyl, 1-Propylthiopropyl, 1-(1-Methylethylthio)propyl, 1-Butylthiopropyl, 1-(1-Methyl-propylthio)propyl, 1-(2-Methylpropylthio)propyl, 1-(1,1-Dimethylethylthio)propyl, 2-Methylthiopropyl, 2-Ethylthiopropyl, 2-Propylthiopropyl, 2-(1-Methylethylthio)propyl, 2-Butylthiopropyl, 2-(1-Methyl-propylthio)propyl, 2-(2-Methylpropylthio)propyl, 2-(1,1-Dimethylethylthio)propyl, 3-Methylthiopropyl, 3-Ethylthiopropyl, 3-Propylthiopropyl, 3-(1-Methylethylthio)propyl, 3-Butylthiopropyl, 3-(1-Methylpropylthio)propyl, 3-(2-Methylpropylthio)propyl, 3-(1,1-Dimethylethylthio)propyl, 1-Methylthio-(1-methyl)-ethyl, 1-Ethylthio-(1-methyl)-ethyl, 1-Propylthio-(1-methyl)-ethyl, 1-(1-Methylethylthio)-(1-methyl)-ethyl, 1-Butylthio-(1-methyl)-ethyl, 1-(1-Methyl-propylthio)-(1-methyl)-ethyl, 1-(2-Methylpropylthio)-(1-methyl)-ethyl, 1-(1,1-Dimethylethylthio)-(1-methyl)-ethyl, 2-Methylthio-(1-methyl)-ethyl, 2-Ethylthio-(1-methyl)-ethyl, 2-Propylthio-(1-methyl)-ethyl, 2-(1-Methylethylthio)-(l-methyl)-ethyl, 2-Butylthio-(1-methyl)-ethyl, 2-(1-Methyl-propylthio)-(1-methyl)-ethyl, 2-(2-Methylpropylthio)-(1-methyl)-ethyl, 2-(1,1-Dimethylethylthio)-(1-methyl)-ethyl, 1-Methylthiobutyl, 1-Ethylthiobutyl, 1-Propylthiobutyl, 1-(1-Methylethylthio)butyl, 1-Butylthiobutyl, 1-(1-Methyl-propylthio)butyl, 1-(2-Methylpropylthio)butyl, 1-(1,1-Dimethylethylthio)butyl, 2-Methylthiobutyl, 2-Ethylthiobutyl, 2-Propylthiobutyl, 2-(1-Methylethylthio)butyl, 2-Butylthiobutyl, 2-(1-Methyl-propylthio)butyl, 2-(2-Methylpropylthio)butyl, 2-(1,1-Dimethylethylthio)butyl, 3-Methylthiobutyl, 3-Ethylthiobutyl, 3-Propylthiobutyl, 3-(1-Methylethylthio)butyl, 3-Butylthiobutyl, 3-(1-Methyl-propylthio)butyl, 3-(2-Methylpropylthio)butyl, 3-(1,1-Dimethylethylthio)butyl, 4-Methylthiobutyl, 4-Ethylthiobutyl, 4-Propylthiobutyl, 4-(1-Methylethylthio)butyl, 4-Butylthiobutyl, 4-(1-Methyl-propylthio)butyl, 4-(2-Methylpropylthio)butyl, 4-(1,1-Dimethylethylthio)butyl, 1-Methylthio-(1-methyl)-propyl, 1-Ethylthio-(1-methyl)-propyl, 1-Propylthio-(1-methyl)-propyl, 1-(1-Methylethylthio)-(1-methyl)-propyl, 1-Butylthio-(1-methyl)-propyl, 1-(1-Methyl-propylthio)-(1-methyl)-propyl, 1-(2-Methylpropylthio)-(1-methyl)-propyl, 1-(1,1-Dimethylethylthio)-(1-methyl)-propyl, 2-Methylthio-(1-methyl)-propyl, 2-Ethylthio-(1-methyl)-propyl, 2-Propylthio-(1-methyl)-propyl, 2-(1-Methylethylthio)-(1-methyl)-propyl, 2-Butylthio-(1-methyl)-propyl, 2-(1-Methylpropylthio)-(1-methyl)-propyl, 2-(2-Methylpropylthio)-(1-methyl)-propyl, 2-(1,1-Dimethylethylthio)-(1-methyl)-propyl, 3-Methylthio-(1-methyl)-propyl, 3-Ethylthio-(1-methyl)-propyl, 3-Propylthio-(1-methyl)-propyl, 3-(1-Methylethylthio)-(1-methyl)-propyl, 3-Butylthio-(1-methyl)-propyl, 3-(1-Methyl-propylthio)-(1-methyl)-propyl, 3-(2-Methylpropylthio)-(1-methyl)-propyl, 3-(1,1-Dimethylethylthio)-(1-methyl)-propyl, 1-Methylthio-(2-methyl)-propyl, 1-Ethylthio-(2-methyl)-propyl, 1-Propylthio-(2-methyl)-propyl, 1-(1-Methylethylthio)-(2-methyl)-propyl, 1-Butylthio-(2-methyl)-propyl, 1-(1-Methyl-propylthio)-(2-methyl)-propyl, 1-(2-Methylpropylthio)-(2-methyl)-propyl, 1-(1,1-Dimethylethylthio)-(2-methyl)-propyl, 2-Methylthio-(2-methyl)-propyl, 2-Ethylthio-(2-methyl)-propyl, 2-Propylthio-(2-methyl)-propyl, 2-(1-Methylethylthio)-(2-methyl)-propyl, 2-Butylthio-(2-methyl)-propyl, 2-(1-Methyl-propylthio)-(2-methyl)-propyl, 2-(2-Methylpropylthio)-(2-methyl)-propyl, 2-(1,1-Dimethylethylthio)-(2-methyl)-propyl, 3-Methylthio-(2-methyl)-propyl, 3-Ethylthio-(2-methyl)-propyl, 3-Propylthio-(2-methyl)-propyl, 3-(1-Methylethylthio)-(2-methyl)-propyl, 3-Butylthio-(2-methyl)-propyl, 3-(1-Methylpropylthio)-(2-methyl)-propyl, 3-(2-Methylpropylthio)-(2-methyl)-propyl, 3-(1,1-Dimethylethylthio)-(2-methyl)-propyl, 2-Methylthio-(1,1-dimethyl)-ethyl, 2-Ethylthio-(1,1-dimethyl)-ethyl, 2-Propylthio-(1,1-dimethyl)-ethyl, 2-(1-Methylethylthio)-(1,1-dimethyl)-ethyl, 2-Butylthio-(1,1-dimethyl)-ethyl, 2-(1-Methyl-propylthio)-(1,1-dimethyl)-ethyl, 2-(2-Methylpropylthio)-(1,1-dimethyl)-ethyl, 2-(1,1-Dimethylethylthio)-(1,1-dimethyl)-ethyl;
C₃-C₁₅-Alkenyl, besonders C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl;
C₃-C₁₅-Alkenyloxy, besonders C₃-C₆-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Di-methyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 3,3-Dimethyl-2-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy;
C₃-C₈-Cycloalkyl wie Cyclopropyl, Cyclobutyl, Cyclopentyl, Cyclohexyl, Cycloheptyl und Cyclooctyl, insbesondere Cyclopropyl und Cyclohexyl;
C₅-C₈-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-1-enyl;
C₃-C₈-Cycloalkoxy wie Cyclopropyloxy, Cyclobutyloxy, Cyclopentyloxy, Cyclohexyloxy, Cycloheptyloxy und Cyclooctyloxy;
C₅-C₈-Cycloalkenyloxy wie Cyclopent-1-enyloxy, Cyclopent-2-enyloxy, Cyclopent-3-enyloxy, Cyclohex-1-enyloxy, Cyclohex-2-enyloxy, Cyclohex-3-enyloxy, Cyclohept-1-enyloxy, Cyclohept-2-enyloxy, Cyclohept-3-enyloxy, Cyclohept-4-enyloxy, Cyclooct-1-enyloxy, Cyclooct-2-enyloxy, Cyclooct-3-enyloxy und Cyclooct-1-enyloxy;
C₁-C₆-Alkylamino wie Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino und 1-Ethyl-2-methylpropylamino, insbesondere Methylamino und Ethylamino;
Di-C₁-C₆-alkylamino, besonders Di-C₁-C₄-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methyl-ethyl)amino, N-Butyl-N-ethylamino, N-Ethyl-N-(1-methyl-propyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methyl-propyl)amino, N-Butyl-N-(2-methylpropyl)amino, N-Butyl-N-(1,1-dimethylethyl)amino, N-(1-Methylpropyl)-N-(2-methyl-propyl)amino, N-(1,1-Dimethylethyl)-N-(1-methylpropyl)amino und N- (1,1-Dimethylethyl)-N-(2-methylpropyl)amino, insbesondere Dimethylamino und Diethylamino;
C₁-C₆-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl,2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbony1,1-Ethyl-1-methylpropylcarbonyl und 1-Ethyl-2-methylpropylcarbonyl, insbesondere Methylcarbonyl und Ethylcarbonyl;
C₁-C₆-Alkoxycarbonyl wie Methoxycarbonyl, Ethoxycarbonyl, Propyloxycarbonyl, 1-Methyl-ethoxycarbonyl, Butyloxycarbonyl, 1-Methylpropyloxycarbonyl, 2-Methylpropyloxycarbonyl,1,1-Dimethylethoxycarbonyl, Pentyloxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethylpropoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyloxycarbonyl, 1-Ethyl-1-methylpropyloxycarbonyl und 1-Ethyl-2-methylpropyloxycarbonyl, insbesondere Methoxycarbonyl und Ethoxycarbonyl;
C₁-C₆-Alkylaminocarbonyl wie Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl, 1,1-Dimethylethylaminocarbonyl, Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl,3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl,3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methyl-propylaminocarbonyl und 1-Ethyl-2-methylpropylaminocarbonyl, insbesondere Methylaminocarbonyl;
Di-C₁-C₆-alkylaminocarbonyl, besonders Di-C₁-C₄-alkylaminocarbonyl wie N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)- aminocarbonyl, N-Ethyl-N-methylaminocarbonyl, N-Methyl-N-propylaminocarbonyl, N-Methyl-N-(1-methylethyl)aminocarbonyl, N-Butyl-N-methylaminocarbonyl, N-Methyl-N-(1-methylpropyl)amino- carbonyl, N-Methyl-N-(2-methylpropyl)aminocarbonyl, N-(1,1-Dimethylethyl)-N-methylaminocarbonyl, N-Ethyl-N-propyl- aminocarbonyl, N-Ethyl-N-(1-methylethyl)aminocarbonyl, N-Butyl-N-ethylaminocarbonyl, N-Ethyl-N-(1-methylpropyl)amino- carbonyl, N-Ethyl-N-(2-methylpropyl)aminocarbonyl, N-Ethyl-N-(1,1-dimethylethyl)aminocarbonyl, N-(1-Methylethyl)- -N-propylaminocarbonyl, N-Butyl-N-propylaminocarbonyl, N-(1-Methylpropyl)-N-propylaminocarbonyl, N-(2-Methyl- propyl)-N-propylaminocarbonyl, N-(1,1-Dimethylethyl)- -N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Di-methylethyl)-N-(1-methylethyl)aminocarbonyl, N-Butyl- N-(1-methylpropyl)aminocarbonyl, N-Butyl-N-(2-methylpropyl)- aminocarbonyl, N-Butyl-N-(1,1-dimethylethyl)aminocarbonyl, N-(1-Methylpropyl)-N-(2-methyl-propyl)aminocarbonyl, N-(1,1-Dimethylethyl)-N-(1-methylpropyl)aminocarbonyl und N-(1,1-Dimethylethyl)-N-(2-methylpropyl)aminocarbonyl, insbesondere Dimethylaminocarbonyl;
C₁-C₆-Alkylcarboxyl wie Methylcarboxyl, Ethylcarboxyl, Propylcarboxyl, 1-Methylethyl-carboxyl, Butylcarboxyl, 1-Methylpropylcarboxyl, 2-Methylpropylcarboxyl, 1,1-Dimethylethylcarboxyl, Pentylcarboxyl, 1-Methylbutylcarboxyl, 2-Methylbutylcarboxyl, 3-Methylbutylcarboxyl,1,1-Dimethylpropylcarboxyl, 1,2-Dimethylpropylcarboxyl,2,2-Dimethylpropylcarboxyl, 1-Ethylpropylcarboxyl, Hexylcarboxyl, 1-Methylpentylcarboxyl, 2-Methylpentylcarboxyl, 3-Methylpentylcarboxyl, 4-Methylpentylcarboxyl, 1,1-Dimethylbutylcarboxyl, 1,2-Dimethylbutylcarboxyl, 1,3-Dimethylbutylcarboxyl, 2,2-Dimethylbutylcarboxyl, 2,3-Dimethylbutylcarboxyl, 3,3-Dimethylbutylcarboxyl, 1-Ethylbutylcarboxyl, 2-Ethylbutylcarboxyl, 1,1,2-Trimethylpropylcarboxyl, 1,2,2-Trimethylpropylcarboxyl,1-Ethyl-1-methylpropylcarboxyl und 1-Ethyl-2-methylpropylcarboxyl, insbesondere Methylcarboxyl;
Phenyl, Phenoxy, Phenylthio, Phenylamino;
Phenyl-C₁-C₄-alkyl wie Benzyl, 1-Phenylethyl, 2-Phenylethyl, 1-Phenylpropyl, 2-Phenylpropyl, 3-Phenylpropyl, 1-Methyl-1-phenylethyl, 1-Methyl-2-phenylethyl, 1-Phenylbutyl, 2-Phenylbutyl, 3-Phenylbutyl, 4-Phenylbutyl, 1-Methyl-1-phenylpropyl, 1-Methyl-2-phenylpropyl, 1-Methyl-3-phenylpropyl, 2-Methyl-1-phenylpropyl, 2-Methyl-2-phenylpropyl, 2-Methyl-3-phenylpropyl und 1,1-Dimethyl-2-phenylethyl, vorzugsweise Benzyl;
Phenoxy-C₁-C₄-alkyl wie Phenoxymethyl, 1-Phenoxyethyl, 2-Phenoxyethyl, 1-Phenoxypropyl, 2-Phenoxypropyl, 3-Phenoxypropyl, 1-Methyl-1-phenoxyethyl, 1-Methyl-2-phenoxyethyl, 1-Phenoxybutyl, 2-Phenoxybutyl, 3-Phenoxybutyl, 4-Phenoxybutyl, 1-Methyl-1-phenoxypropyl, 1-Methyl-2-phenoxypropyl, 1-Methyl-3-phenoxypropyl, 2-Methyl-1-phenoxypropyl, 2-Methyl-2-phenoxypropyl, 2-Methyl-3-phenoxypropyl und 1,1-Dimethyl-2-phenoxyethyl;
Phenylthio-C₁-C₄-alkyl wie Phenylthiomethyl, 1-Phenylthioethyl, 2-Phenylthioethyl, 1-Phenylthiopropyl, 2-Phenylthiopropyl, 3-Phenylthiopropyl, 1-Methyl-1-phenylthioethyl, 1-Methyl-2-phenylthioethyl, 1-Phenylthiobutyl, 2-Phenylthiobutyl, 3-Phenylthiobutyl, 4-Phenylthiobutyl, 1-Methyl-1-phenylthiopropyl, 1-Methyl-2-phenylthiopropyl, 1-Methyl-3-phenylthiopropyl, 2-Methyl-1-phenylthiopropyl, 2-Methyl-2-phenylthiopropyl, 2-Methyl-3-phenylthiopropyl und 1,1-Dimethyl-2-phenylthioethyl;
Phenylamino-C₁-C₄-alkyl, wie Phenylaminomethyl, 1-Phenylaminoethyl, 2-Phenylaminoethyl, 1-Phenylaminopropyl, 2-Phenylaminopropyl, 3-Phenylaminopropyl, 1-Methyl-1-phenylaminoethyl, 1-Methyl-2-phenylaminoethyl, 1-Phenylaminobutyl, 2-Phenylaminobutyl, 3-Phenylaminobutyl, 4-Phenylaminobutyl, 1-Methyl-1-phenylaminopropyl, 1-Methyl-2-phenylaminopropyl, 1-Methyl-3-phenylaminopropyl, 2-Methyl-1-phenylaminopropyl, 2-Methyl-2-phenylaminopropyl, 2-Methyl-3-phenylaminopropyl und 1,1-Dimethyl-2-phenylaminoethyl;
wobei die aromatischen Ringe ihrerseits ein bis fünf Halogenatome wie Fluor, Chlor, Brom und Jod, insbesondere Fluor und Chlor;
und/oder ein bis drei der folgenden Reste tragen können: Cyano, Nitro, C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Cyano, Methyl und 1-Methylethyl, insbesondere Methyl;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;
C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy, Ethoxy und 1-Methylethoxy, insbesondere Methoxy;
C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, insbesondere Methylthio;
und C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio;
ein über ein Kohlenstoffatom gebundenes fünf- oder sechsgliedriges aromatisches Ringsystem, das neben Kohlenstoffatomen ein bis drei Stickstoffatome oder ein oder zwei Stickstoffatome und ein Sauerstoff- oder ein Schwefelatom oder ein Sauerstoff- oder ein Schwefelatom enthalten kann, beispielsweise
fünfring Heteroaromaten enthaltend ein bis drei Stickstoffatome und/oder ein Sauerstoff- oder Schwefelatom wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4- Triazol-3-yl, 1,3,4-Oxadiazol-2-yl, 1,3,4-Thiadiazol-2-yl und 1,3,4-Triazol-2-yl;
oder sechsring Heteroaromaten enthaltend ein bis drei Stickstoffatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
wobei diese Ringsysteme ein bis vier Halogenatome wie Fluor, Chlor, Brom und Jod, vorzugsweise Fluor, Chlor und Brom und/oder ein bis drei der folgenden Reste tragen können:
C₁-C₄-Alkyl wie Methyl, Ethyl, Propyl, 1-Methylethyl, Butyl, 1-Methylpropyl, 2-Methylpropyl und 1,1-Dimethylethyl, insbesondere Methyl;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2- Chlor-2,2-difluorethyl, 2,2-Dichlor-2-fluorethyl, 2,2,2-Trichlorethyl und Pentafluorethyl, insbesondere Trifluormethyl;
C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere Methoxy;
C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2- Chlor-2-fluorethyloxy, 2-Chlor-2,2-difluorethyloxy, 2,2- Dichlor-2-fluorethyloxy, 2,2,2-Trichlorethyloxy und Pentafluorethyloxy, insbesondere Trifluotmethoxy; C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, insbesondere Methylthio;
und C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1- Fluorethylthio, 2-Fluorethylthio, 2,2-Difluorethylthio, 2,2,2-Trifluorethylthio, 2-Chlor-2-fluorethylthio, 2- Chlor-2,2-difluorethylthio, 2,2-Dichlor-2-fluorethylthio, 2,2,2- Trichlorethylthio und Pentafluorethylthio, insbesondere Trifluormethylthio;
R³
   ggf. subst. Alkyl mit ein bis zehn Kohlenstoffatomen, besonders C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl,3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl und 1-Methylethyl;
wobei diese Gruppen ein bis neun Halogenatome wie Fluor, Chlor, Brom und Jod, vorzugsweise Fluor, Chlor und Brom, insbesondere Chlor und Brom;
und/oder einen der bei R² im allgemeinen und im besonderen genannten Reste oder einen der folgenden Reste tragen können:
C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy, insbesondere C₁-C₄-Alkoxy;
C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Trifluormethoxy;
C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio, vorzugsweise C₁-C₃-Alkylthio, insbesondere Methylthio;
C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, vorzugsweise Trifluormethylthio;
C₃-C₈-Cycloalkoxy wie Cyclopropyloxy, Cyclobutyloxy, Cyclopentyloxy, Cyclohexyloxy, Cycloheptyloxy und Cyclooctyloxy, insbesondere Cyclopropyloxy, Cyclohexyloxy;
Di-C₁-C₆-alkylamino, besonders Di-C₁-C₄-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methyl-ethyl)amino, N-Butyl-N-ethylamino, N-Ethyl-N-(1-methyl-propyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methyl-propyl)amino, N-(1,1-Dimethylethyl)-N-(1-methylpropyl)amino und N-(1,1-Dimethylethyl)-N-(2-methylpropyl)amino, vorzugsweise Dimethylamino, Diethylamino, insbesondere Dimethylamino;
C₁-C₆-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl,2,2-Dimethylpropylcarbonyl, 1-Ethylpropylcarbonyl,Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise Methylcarbonyl, Ethylcarbonyl;
C₁-C₆-Alkoxycarbonyl wie Methoxycarbonyl, Ethoxycarbonyl, Propyloxycarbonyl, 1-Methyl-ethoxycarbonyl, Butyloxycarbonyl, 1-Methylpropyloxycarbonyl, 2-Methylpropyloxycarbonyl,1,1-Dimethylethoxycarbonyl, Pentyloxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethylpropoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyloxycarbonyl, 1-Ethyl-1-methylpropyloxycarbonyl und 1-Ethyl-2-methylpropyloxycarbonyl, vorzugsweise Methoxycarbonyl;
C₁-C₆-Alkylaminocarbonyl wie Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl, 1,1-Dimethylethylaminocarbonyl, Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl,3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl,3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl und 1-Ethyl-2-methylpropylaminocarbonyl, vorzugsweise Methylaminocarbonyl;
Di-C₁-C₆-alkylaminocarbonyl, besonders Di-C₁-C₄-alkylaminocarbonyl wie N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)aminocarbonyl, N-Ethyl-N-methylaminocarbonyl, N-Methyl-N-propyl-aminocarbonyl, N-Methyl-N-(1-methylethyl)aminocarbonyl, N-Butyl-N-methylaminocarbonyl, N-Methyl-N-(1-methylpropyl)amino-carbonyl, N-Methyl-N-(2-methylpropyl)aminocarbonyl, N-(1,1-Dimethylethyl)-N-methylaminocarbonyl, N-Ethyl-N-propyl-aminocarbonyl, N-Ethyl-N-(1-methylethyl)aminocarbonyl, N-ButylN-ethylaminocarbonyl, N-Ethyl-N-(1-methylpropyl)amino-carbonyl, N-Ethyl-N-(2-methylpropyl)aminocarbonyl, N-Ethyl-N-(1,1-dimethylethyl)aminocarbonyl, N-(1-Methylethyl)- -N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Di-methylethyl)-N-(1-methylethyl)aminocarbonyl, N-Butyl- N-(1-methylpropyl)aminocarbonyl, N-Butyl-N-(2-methylpropyl)- aminocarbonyl, N-Butyl-N-(1,1-dimethylethyl)aminocarbonyl, N-(1-Methylpropyl)-N-(2-methyl-propyl)aminocarbonyl, N-(1,1-Dimethylethyl)-N-(1-methylpropyl)aminocarbonyl und N- (1,1-Dimethylethyl)-N-(2-methylpropyl)aminocarbonyl, vorzugsweise Dimethylaminocarbonyl;
C₁-C₆-Alkylcarboxyl wie Methylcarboxyl, Ethylcarboxyl, Propylcarboxyl, 1-Methylethyl-carboxyl, Butylcarboxyl, 1-Methylpropylcarboxyl, 2-Methylpropylcarboxyl, 1,1-Dimethylethylcarboxyl, Pentylcarboxyl, 1-Methylbutylcarboxyl, 2-Methylbutylcarboxyl, 3-Methylbutylcarboxyl, 1,1-Dimethylpropylcarboxyl, 1,2-Dimethylpropylcarboxyl, 2,2-Dimethylpropylcarboxyl, 1-Ethylpropylcarboxyl, Hexylcarboxyl, 1-Methylpentylcarboxyl, 2-Methylpentylcarboxyl, 3-Methylpentylcarboxyl, 4-Methylpentylcarboxyl, 1,1-Dimethylbutylcarboxyl, 1,2-Dimethylbutylcarboxyl, 1,3-Dimethylbutylcarboxyl, 2,2-Dimethylbutylcarboxyl, 2,3-Dimethylbutylcarboxyl, 3,3-Dimethylbutylcarboxyl, 1-Ethylbutylcarboxyl, 2-Ethylbutylcarboxyl, 1,1,2-Trimethylpropylcarboxyl, 1,2,2-Trimethylpropylcarboxyl, 1-Ethyl-1-methylpropylcarboxyl und 1-Ethyl-2-methylpropylcarboxyl, vorzugsweise Methylcarboxyl;
Phenyl, Phenoxy, Phenylthio, Phenylamino, wobei die aromatischen Ringe ihrerseits ein bis fünf Halogenatome wie Fluor, Chlor, Brom und Jod, vorzugsweise Fluor, Chlor und Brom, insbesondere Fluor und Chlor;
und/oder ein bis drei der folgenden Reste tragen können:
Cyano, Nitro, C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Cyano, Methyl, insbesondere Methyl;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;
C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere Methoxy, Ethoxy;
C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, insbesondere Methylthio;
und C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio;
der Rest R³ steht außerdem für ein ggf. subst. über ein Kohlenstoffatom gebundenes aliphatisches Ringsystem, das neben Kohlenstoffatomen ein oder zwei Heteroatome aus der Gruppe Sauerstoff, Schwefel und Stickstoff enthalten kann; Beispiele für derartige Ringsysteme sind:
C₃-C₈-Cycloalkyl wie Cyclopropyl, Cyclobutyl, Cyclopentyl, Cyclohexyl, Cycloheptyl und Cyclooctyl, vorzugsweise Cyclopropyl, Cyclopentyl und Cyclohexyl, insbesondere Cyclopropyl;
C₅-C₈-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-1-enyl;
C₅-C₈-Cycloalkdienyl wie Cyclopenta-1,3-dien-1-yl, Cyclopenta-1,3-dien-2-yl, Cyclopenta-1,3-dien-5-yl, Cyclohexa-1,3-dien-1-yl, Cyclohexa-1,3-dien-2-yl, Cyclohexa-1,3-dien-5-yl, Cyclohexa-1,4-dien-1-yl, Cyclohexa-1,4-dien-3-yl, Cyclohepta-1,3-dien-1-yl, Cyclohepta-1,3-dien-2-yl, Cyclohepta-1,3-dien-5-yl, Cyclohepta-1,3-dien-6-yl, Cyclohepta-1,4-dien-1-yl, Cyclohepta-1,4-dien-2-yl, Cyclohepta-1,4-dien-3-yl, Cyclohepta-1,4-dien-6-yl, Cycloocta-1,3-dien-1-yl, Cycloocta-1,3-dien-2-yl, Cycloocta-1,3-dien-5-yl, Cycloocta-1,3-dien-6-yl, Cycloocta-1,4-dien-1-yl, Cycloocta-1,4-dien-2-yl, Cycloocta-1,4-dien-3-yl, Cycloocta-1,4-dien-6-yl, Cycloocta-1,4-dien-7-yl, Cycloocta-1,4-dien-1-yl und Cycloocta-1,4-dien-3-yl;
3- bis 6-gliedrige, gesättigte oder ungesättigte Heterocyclen, enthaltend ein bis drei Stickstoffatome und/oder ein Sauerstoff- oder Schwefelatom wie 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl,1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,5-Dihydrofur-2-yl, 2,5-Dihydrofur-3-yl, 2,3-Dihydrothien-2-yl, 2,3-Dihydrothien-3-yl, 2,5-Dihydrothien-2-yl, 2,5-Dihydrothien-3-yl, 2,3-Pyrrolin-2-yl, 2,3-Pyrrolin-3-yl, 2,5-Pyrrolin-2-yl, 2,5-Pyrrolin-3-yl, 2,3-Isoxazolin-3-yl, 2,4-Isoxazolin-3-yl, 4,5-Isoxazolin-3-yl, 2,3-Isoxazolin-4-yl, 2,5-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 2,5-Isothiazolin-3-yl, 4,5-Isothiazolin-3-yl, 2,3-Isothiazolin-4-yl, 2,5-Isothiazolin-4-yl, 4,5-Isothiazolin-4-yl, 2,3-Isothiazolin-5-yl, 2,5-Isothiazolin-5-yl, 4,5-Isothiazolin-5-yl, 2,3-Dihydropyrazol-3-yl, 2,3-Dihydropyrazol-4-yl, 2,3-Dihydropyrazol-5-yl,3,4-Dihydropyrazol-3-yl, 3,4-Dihydropyrazol-4-yl, 3,4-Dihydropyrazol-5-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-4-yl, 2,3-Dihydrooxazol-5-yl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl, 1,3-Dithianyl, 2-Tetrahydropyranyl, 3-Tetrahydropyranyl, 1,3-Dioxolauryl, Oxiranyl, vorzugsweise 2-Tetrahydrofuranyl, 3-Tetrahydrofuranyl, 2-Tetrahydropyranyl, insbesondere 2-Tetrahydrofuranyl, 2-Tetrahydropyranyl;
wobei diese vorstehend genannten Ringsysteme ein bis fünf Halogenatome wie Fluor, Chlor, Brom und Jod, vorzugsweise Fluor, Chlor und Brom, insbesondere Chlor;
und/oder ein bis vier der folgenden Reste tragen können: Cyano, Nitro, Amino, C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl,3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl und 1-Methylethyl;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trichlormethyl und Trifluormethyl, insbesondere Trifluormethyl;
C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy, insbesondere Methoxy, Ethoxy, 1-Methylethoxy und Propoxy;
C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
C₁-C₄-Alkoxy-C₁-C₄-alkyl wie Methoxymethyl, Ethoxymethyl, Propyloxymethyl, 1-Methylethoxymethyl, Butyloxymethyl, 1-Methyl-propyloxymethyl, 2-Methylpropyloxymethyl, 1,1-Dimethylethoxymethyl, 1-Methoxyethyl, 1-Ethoxyethyl, 1-Propyloxyethyl, 1-(1-Methylethoxy)ethyl, 1-Butyloxyethyl, 1-(1-Methyl-propyloxy)ethyl, 1-(2-Methylpropyloxy)ethyl, 1-(1,1-Dimethylethoxy)ethyl, 2-Methoxyethyl, 2-Ethoxyethyl, 2-Propyloxyethyl, 2-(1-Methylethoxy)ethyl, 2-Butyloxyethyl, 2-(1-Methyl-propyloxy)ethyl, 2-(2-Methylpropyloxy)ethyl, 2-(1,1-Dimethylethoxy)ethyl, 1-Methoxypropyl, 1-Ethoxypropyl, 1-Propyloxypropyl, 1-(1-Methylethoxy)propyl, 1-Butyloxypropyl, 1-(1-Methyl-propyloxy)propyl, 1-(2-Methylpropyloxy)propyl, 1-(1,1-Dimethylethoxy)propyl, 2-Methoxypropyl, 2-Ethoxypropyl, 2-Propyloxypropyl, 2-(1-Methylethoxy)propyl, 2-Butyloxypropyl, 2-(1-Methyl-propyloxy)propyl, 2-(2-Methylpropyloxy)propyl, 2-(1,1-Dimethylethoxy)propyl, 3-Methoxypropyl, 3-Ethoxypropyl, 3-Propyloxypropyl, 3-(1-Methylethoxy)propyl, 3-Butyloxypropyl, 3-(1-Methyl-propyloxy)propyl, 3-(2-Methylpropyloxy)propyl, 3-(1,1-Dimethylethoxy)propyl, 1-Methoxy-(1-methyl)-ethyl, 1-Ethoxy-(1-methyl)-ethyl, 1-Propyloxy-(1-methyl)-ethyl, 1-(1-Methylethoxy)-(1-methyl)-ethyl, 1-Butyloxy- (1-methyl)-ethyl, 1-(1-Methyl-propyloxy)-(1-methyl)-ethyl, 1-(2-Methylpropyloxy)-(1-methyl)-ethyl, 1-(1,1-Dimethylethoxy)-(1-methyl)-ethyl, 2-Methoxy-(1-methyl)-ethyl, 2-Ethoxy-(1-methyl)-ethyl, 2-Propyloxy-(1-methyl)-ethyl, 2-(1-Methylethoxy)-(1-methyl)-ethyl, 2-Butyloxy-(1-methyl)-ethyl, 2-(1-Methyl-propyloxy)-(1-methyl)-ethyl, 2-(2-Methylpropyloxy)-(1-methyl)-ethyl, 2-(1,1-Dimethylethoxy)-(1-methyl)-ethyl, 1-Methoxybutyl, 1-Ethoxybutyl, 1-Propyloxybutyl, 1-(1-Methylethoxy)butyl, 1-Butyloxybutyl, 1-(1-Methyl-propyloxy)butyl, 1-(2-Methylpropyloxy)butyl, 1-(1,1-Dimethylethoxy)butyl, 2-Methoxybutyl, 2-Ethoxybutyl, 2-Propyloxybutyl, 2-(1-Methylethoxy)butyl, 2-Butyloxybutyl, 2-(1-Methyl-propyloxy)butyl, 2-(2-Methylpropyloxy)butyl, 2-(1,1-Dimethylethoxy)butyl, 3-Methoxybutyl, 3-Ethoxybutyl, 3-Propyloxybutyl, 3-(1-Methylethoxy)butyl, 3-Butyloxybutyl, 3-(1-Methyl-propyloxy)butyl, 3-(2-Methylpropyloxy)butyl, 3-(1,1-Dimethylethoxy)butyl, 4-Methoxybutyl, 4-Ethoxybutyl, 4-Propyloxybutyl, 4-(1-Methylethoxy)butyl, 4-Butyloxybutyl, 4-(1-Methyl-propyloxy)butyl, 4-(2-Methylpropyloxy)butyl, 4-(1,1-Dimethylethoxy)butyl, 1-Methoxy-(1-methyl)-propyl, 1-Ethoxy-(1-methyl)-propyl, 1-Propyloxy-(1-methyl)-propyl, 1-(1-Methylethoxy)-(1-methyl)-propyl, 1-Butyloxy-(1-methyl)-propyl, 1-(1-Methyl-propyloxy)-(1-methyl)-propyl, 1-(2-Methylpropyloxy)-(1-methyl)-propyl, 1-(1,1-Dimethylethoxy)-(1-methyl)-propyl, 2-Methoxy-(1-methyl)-propyl, 2-Ethoxy-(1-methyl)-propyl, 2-Propyloxy-(1-methyl)-propyl, 2-(1-Methylethoxy)-(1-methyl)-propyl, 2-Butyloxy-(1-methyl)-propyl, 2-(1-Methyl-propyloxy)-(1-methyl)-propyl, 2-(2-Methylpropyloxy)-(1-methyl)-propyl, 2-(1,1-Dimethylethoxy)-(1-methyl)-propyl, 3-Methoxy-(1-methyl)-propyl, 3-Ethoxy- (1-methyl)-propyl, 3-Propyloxy- (1-methyl) -propyl, 3-(1-Methylethoxy)-(1-methyl)-propyl, 3-Butyloxy-(1-methyl)-propyl, 3-(1-Methyl-propyloxy)-(1-methyl)-propyl, 3-(2-Methylpropyloxy)-(1-methyl)-propyl, 3-(1,1-Dimethylethoxy)-(1-methyl)-propyl, 1-Methoxy-(2-methyl)-propyl, 1-Ethoxy-(2-methyl)-propyl, 1-Propyloxy-(2-methyl)-propyl, 1-(1-Methylethoxy)-(2-methyl)-propyl, 1-Butyloxy-(2-methyl)-propyl, 1-(1-Methyl-propyloxy)-(2-methyl)-propyl, 1-(2-Methylpropyloxy)-(2-methyl)-propyl, 1-(1,1-Dimethylethoxy)-(2-methyl)-propyl, 2-Methoxy-(2-methyl)-propyl, 2-Ethoxy-(2-methyl)-propyl, 2-Propyloxy-(2-methyl)-propyl, 2-(1-Methylethoxy)-(2-methyl)-propyl, 2-Butyloxy- (2-methyl)-propyl, 2- (1-Methyl-propyloxy )-(2-methyl)-propyl, 2-(2-Methylpropyloxy)-(2-methyl)-propyl, 2-(1,1-Dimethylethoxy)-(2-methyl)-propyl, 3-Methoxy-(2-methyl)-propyl, 3-Ethoxy-(2-methyl)-propyl, 3-Propyloxy-(2-methyl)-propyl, 3-(1-Methylethoxy)-(2-methyl)-propyl, 3-Butyloxy-(2-methyl)-propyl, 3-(1-Methyl-propyloxy)-(2-methyl)-propyl, 3-(2-Methylpropyloxy)-(2-methyl)-propyl, 3-(1,1-Dimethylethoxy)-(2-methyl)-propyl, 2-Methoxy-(1,1-dimethyl)-ethyl, 2-Ethoxy-(1,1-dimethyl)-ethyl, 2-Propyloxy-(1,1-dimethyl)-ethyl, 2-(1-Methylethoxy)-(1,1-dimethyl)-ethyl, 2-Butyloxy-(1,1-dimethyl)-ethyl, 2-(1-Methyl-propyloxy)-(1,1-dimethyl)-ethyl, 2-(2-Methylpropyloxy)-(1,1-dimethyl)-ethyl, 2-(1,1-Dimethylethoxy)-(1,1-dimethyl)-ethyl, insbesondere Methoxymethyl;
C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethyl-butylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio, vorzugsweise C₁-C₃-Alkylthio, insbesondere Methylthio;
C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio;
C₁-C₄-Alkylthio-C₁-C₄-alkyl, wie Methylthiomethyl, Ethylthiomethyl, Propylthiomethyl, 1-Methylethylthiomethyl, Butylthiomethyl, 1-Methyl-propylthiomethyl, 2-Methylpropylthiomethyl, 1,1-Dimethylethylthiomethyl, 1-Methylthioethyl, 1-Ethylthioethyl, 1-Propylthioethyl, 1-(1-Methylethylthio)ethyl, 1-Butylthioethyl, 1-(1-Methyl-propylthio)ethyl, 1-(2-Methylpropylthio)ethyl, 1-(1,1-Dimethylethylthio)ethyl, 2-Methylthioethyl, 2-Ethylthioethyl, 2-Propylthioethyl, 2-(1-Methylethylthio)ethyl, 2-Butylthioethyl, 2-(1-Methylpropylthio)ethyl, 2-(2-Methylpropylthio)ethyl, 2-(1,1-Dimethylethylthio)ethyl, 1-Methylthiopropyl, 1-Ethylthiopropyl, 1-Propylthiopropyl, 1-(1-Methylethylthio)propyl, 1-Butylthiopropyl, 1-(1-Methyl-propylthio)propyl, 1-(2-Methylpropylthio)propyl, 1-(1,1-Dimethylethylthio)propyl, 2-Methylthiopropyl, 2-Ethylthiopropyl, 2-Propylthiopropyl, 2-(1-Methylethylthio)propyl, 2-Butylthiopropyl, 2-(1-Methyl-propylthio)propyl, 2-(2-Methylpropylthio)propyl, 2-(1,1-Dimethylethylthio)propyl, 3-Methylthiopropyl, 3-Ethylthiopropyl, 3-Propylthiopropyl, 3-(1-Methylethylthio)propyl, 3-Butylthiopropyl, 3-(1-Methylpropylthio)propyl, 3-(2-Methylpropylthio)propyl, 3-(1,1-Dimethylethylthio)propyl, 1-Methylthio-(1-methyl)-ethyl, 1-Ethylthio-(1-methyl)-ethyl, 1-Propylthio-(1-methyl)-ethyl, 1-(1-Methylethylthio)-(1-methyl)-ethyl, 1-Butylthio-(1-methyl)-ethyl, 1-(1-Methyl-propylthio)-(1-methyl)-ethyl, 1-(2-Methylpropylthio)-(1-methyl)-ethyl, 1-(1,1-Dimethylethylthio)-(1-methyl)-ethyl, 2-Methylthio-(1-methyl)-ethyl, 2-Ethylthio-(1-methyl)-ethyl, 2-Propylthio-(1-methyl)-ethyl, 2-(1-Methylethylthio)-(1-methyl)-ethyl, 2-Butylthio-(1-methyl)-ethyl, 2-(1-Methyl-propylthio)-(1-methyl)-ethyl, 2-(2-Methylpropylthio)-(1-methyl)-ethyl, 2-(1,1-Dimethylethylthio)-(1-methyl)-ethyl, 1-Methylthiobutyl, 1-Ethylthiobutyl, 1-Propylthiobutyl, 1-(1-Methylethylthio)butyl, 1-Butylthiobutyl, 1-(1-Methylpropylthio)butyl, 1-(2-Methylpropylthio)butyl, 1-(1,1-Dimethylethylthio)butyl, 2-Methylthiobutyl, 2-Ethylthiobutyl, 2-Propylthiobutyl, 2-(1-Methylethylthio)butyl, 2-Butylthiobutyl, 2-(1-Methyl-propylthio)butyl, 2-(2-Methylpropylthio)butyl, 2-(1,1-Dimethylethylthio)butyl, 3-Methylthiobutyl, 3-Ethylthiobutyl, 3-Propylthiobutyl, 3-(1-Methylethylthio)butyl, 3-Butylthiobutyl, 3-(1-Methyl-propylthio)butyl, 3-(2-Methylpropylthio)butyl, 3-(1,1-Dimethylethylthio)butyl, 4-Methylthiobutyl, 4-Ethylthiobutyl, 4-Propylthiobutyl, 4-(1-Methylethylthio)butyl, 4-Butylthiobutyl, 4-(1-Methyl-propylthio)butyl, 4-(2-Methylpropylthio)butyl, 4-(1,1-Dimethylethylthio)butyl, 1-Methylthio-(1-methyl)-propyl, 1-Ethylthio-(1-methyl)-propyl, 1-Propylthio-(1-methyl)-propyl, 1-(1-Methylethylthio)-(1-methyl)-propyl, 1-Butylthio-(1-methyl)-propyl, 1-(1-Methyl-propylthio)-(1-methyl)-propyl, 1-(2-Methylpropylthio)-(1-methyl)-propyl, 1-(1,1-Dimethylethylthio)-(1-methyl)-propyl, 2-Methylthio-(1-methyl)-propyl, 2-Ethylthio-(1-methyl)-propyl, 2-Propylthio-(1-methyl)-propyl, 2-(1-Methylethylthio)-(1-methyl)-propyl, 2-Butylthio-(1-methyl)-propyl, 2-(1-Methylpropylthio)-(1-methyl)-propyl, 2-(2-Methylpropylthio)-(1-methyl)-propyl, 2-(1,1-Dimethylethylthio)-(1-methyl)-propyl, 3-Methylthio-(1-methyl)-propyl, 3-Ethylthio-(1-methyl)-propyl, 3-Propylthio-(1-methyl)-propyl, 3-(1-Methylethylthio)-(1-methyl)-propyl, 3-Butylthio-(1-methyl)-propyl, 3-(1-Methyl-propylthio)-(1-methyl)-propyl, 3-(2-Methylpropylthio)-(1-methyl)-propyl, 3-(1,1-Dimethylethylthio)-(1-methyl)-propyl, 1-Methylthio-(2-methyl)-propyl, 1-Ethylthio-(2-methyl)-propyl, 1-Propylthio-(2-methyl)-propyl, 1-(1-Methylethylthio)-(2-methyl)-propyl, 1-Butylthio-(2-methyl)-propyl, 1-(1-Methyl-propylthio)-(2-methyl)-propyl, 1-(2-Methylpropylthio)-(2-methyl)-propyl, 1-(1,1-Dimethylethylthio)-(2-methyl)-propyl, 2-Methylthio-(2-methyl)-propyl, 2-Ethylthio-(2-methyl)-propyl, 2-Propylthio-(2-methyl)-propyl, 2-(1-Methylethylthio)-(2-methyl)-propyl, 2-Butylthio-(2-methyl)-propyl, 2-(1-Methyl-propylthio)-(2-methyl)-propyl, 2-(2-Methylpropylthio)-(2-methyl)-propyl, 2-(1,1-Dimethylethylthio)-(2-methyl)-propyl, 3-Methylthio- (2-methyl)-propyl, 3-Ethylthio- (2-methyl)-propyl, 3-Propylthio-(2-methyl)-propyl, 3-(1-Methylethylthio)-(2-methyl)-propyl, 3-Butylthio-(2-methyl)-propyl, 3-(1-Methylpropylthio)-(2-methyl)-propyl, 3-(2-Methylpropylthio)-(2-methyl)-propyl, 3-(1,1-Dimethylethylthio)-(2-methyl)-propyl, 2-Methylthio-(1,1-dimethyl)-ethyl, 2-Ethylthio-(1,1-dimethyl)-ethyl, 2-Propylthio-(1,1-dimethyl)-ethyl, 2-(1-Methylethylthio)-(1,1-dimethyl)-ethyl, 2-Butylthio-(1,1-dimethyl)-ethyl, 2-(1-Methyl-propylthio)-(1,1-dimethyl)-ethyl, 2-(2-Methylpropylthio)-(1,1-dimethyl)-ethyl, 2-(1,1-Dimethylethylthio)-(1,1-dimethyl)-ethyl, insbesondere Methylthiomethyl;
C₃-C₈-Cycloalkyl wie Cyclopropyl, Cyclobutyl, Cyclopentyl, Cyclohexyl, Cycloheptyl und Cyclooctyl, insbesondere Cyclopropyl und Cyclohexyl;
Di-C₁-C₆-alkylamino, besonders Di-C₁-C₄-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methyl-ethyl)amino, N-Butyl-N-ethylamino, N-Ethyl-N-(1-methyl-propyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methyl-propyl)amino, N-Butyl-N-(2-methylpropyl)amino, N-Butyl-N-(1,1-dimethylethyl)amino, N-(1-Methylpropyl)-N-(2-methyl-propyl)amino, N-(1,1-Dimethylethyl)-N-(1-methylpropyl)amino und N-(1,1-Dimethylethyl)-N-(2-methylpropyl)amino, vorzugsweise Dimethylamino und Diethylamino, insbesondere Dimethylamino;
C₁-C₆-Alkylcarboxyl wie Methylcarboxyl, Ethylcarboxyl, Propylcarboxyl, 1-Methylethyl-carboxyl, Butylcarboxyl, 1-Methylpropylcarboxyl, 2-Methylpropylcarboxyl, 1,1-Dimethylethylcarboxyl, Pentylcarboxyl, 1-Methylbutylcarboxyl, 2-Methylbutylcarboxyl, 3-Methylbutylcarboxyl, 1,1-Dimethylpropylcarboxyl, 1,2-Dimethylpropylcarboxyl, 2,2-Dimethylpropylcarboxyl, 1-Ethylpropylcarboxyl, Hexylcarboxyl, 1-Methylpentylcarboxyl, 2-Methylpentylcarboxyl, 3-Methylpentylcarboxyl, 4-Methylpentylcarboxyl, 1,1-Dimethylbutylcarboxyl, 1,2-Dimethylbutylcarboxyl, 1,3-Dimethylbutylcarboxyl, 2,2-Dimethylbutylcarboxyl, 2,3-Dimethylbutylcarboxyl, 3,3-Dimethylbutylcarboxyl, 1-Ethylbutylcarboxyl, 2-Ethylbutylcarboxyl, 1,1,2-Trimethylpropylcarboxyl, 1,2,2-Trimethylpropylcarboxyl, 1-Ethyl-1-methylpropylcarboxyl und 1-Ethyl-2-methylpropylcarboxyl, insbesondere Methylcarboxyl;
Phenyl, Phenoxy, Phenylthio;
Phenyl-C₁-C₄-alkyl wie Benzyl, 1-Phenylethyl, 2-Phenylethyl, 1-Phenylpropyl, 2-Phenylpropyl, 3-Phenylpropyl, 1-Methyl-1-phenylethyl, 1-Methyl-2-phenylethyl, 1-Phenylbutyl, 2-Phenylbutyl, 3-Phenylbutyl, 4-Phenylbutyl, 1-Methyl-1-phenylpropyl, 1-Methyl-2-phenylpropyl, 1-Methyl-3-phenylpropyl, 2-Methyl-1-phenylpropyl, 2-Methyl-2-phenylpropyl, 2-Methyl-3-phenylpropyl und 1,1-Dimethyl-2-phenylethyl, vorzugsweise Benzyl;
Phenoxy-C₁-C₄-alkyl wie Phenoxymethyl, 1-Phenoxyethyl, 2-Phenoxyethyl, 1-Phenoxypropyl, 2-Phenoxypropyl, 3-Phenoxypropyl, 1-Methyl-1-phenoxyethyl, 1-Methyl-2-phenoxyethyl, 1-Phenoxybutyl, 2-Phenoxybutyl, 3-Phenoxybutyl, 4-Phenoxybutyl, 1-Methyl-1-phenoxypropyl, 1-Methyl-2-phenoxypropyl, 1-Methyl-3-phenoxypropyl, 2-Methyl-1-phenoxypropyl, 2-Methyl-2-phenoxypropyl, 2-Methyl-3-phenoxypropyl und 1,1-Dimethyl-2-phenoxyethyl;
Phenylthio-C₁-C₄-alkyl wie Phenylthiomethyl, 1-Phenylthioethyl, 2-Phenylthioethyl, 1-Phenylthiopropyl, 2-Phenylthiopropyl, 3-Phenylthiopropyl, 1-Methyl-1-phenylthioethyl, 1-Methyl-2-phenylthioethyl, 1-Phenylthiobutyl, 2-Phenylthiobutyl, 3-Phenylthiobutyl, 4-Phenylthiobutyl, 1-Methyl-1-phenylthiopropyl, 1-Methyl-2-phenylthiopropyl, 1-Methyl-3-phenylthiopropyl, 2-Methyl-1-phenylthiopropyl, 2-Methyl-2-phenylthiopropyl, 2-Methyl-3-phenylthiopropyl und 1,1-Dimethyl-2-phenylthioethyl;
wobei die aromatischen Ringe ihrerseits ein bis fünf Halogenatome wie Fluor, Chlor, Brom und Jod, vorzugsweise Fluor, Chlor und Brom, insbesondere Fluor und Chlor;
und/oder ein bis drei der folgenden Reste tragen können:
Cyano, Nitro;
C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl und 1-Methylethyl, insbesondere Methyl;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;
C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy und Ethoxy, insbesondere Methoxy;
C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, insbesondere Methylthio;
und C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio.

Bevorzugte Cyanooximether der Formel I sind solche, in denen R³ die folgende Bedeutung hat:
C₁-C₈-Alkyl wie vorstehend im allgemeinen und im besonderen genannt, insbesondere C₁-C₃-Alkyl, welches ein bis neun Halogenatome wie vorstehend im allgemeinen und im besonderen genannt, insbesondere Chlor und Brom und/oder einen der folgenden Reste tragen kann:
Cyano, Nitro, Amino;
C₁-C₆-Alkoxy wie vorstehend im allgemeinen und im besonderen genannt, insbesondere C₁-C₃-Alkoxy;
C₁-C₄-Halogenalkoxy wie vorstehend im allgemeinen und im besonderen genannt;
C₁-C₆-Alkylthio wie vorstehend im allgemeinen und im besonderen genannt, insbesondere C₁-C₂-Alkylthio;
C₁-C₄-Halogenalkylthio wie vorstehend im allgemeinen und im besonderen genannt;
C₃-C₈-Cycloalkyl wie vorstehend im allgemeinen und im besonderen genannt, insbesondere Cyclopropyl;
C₃-C₈-Cycloalkoxy wie vorstehend im allgemeinen und im besonderen genannt, insbesondere Cyclopropyloxy;
Di-C₁-C₆-alkylamino wie vorstehend im allgemeinen und im besonderen genannt, insbesondere Dimethylamino;
C₁-C₆-Alkylcarbonyl wie vorstehend im allgemeinen und im besonderen genannt, insbesondere Methylcarbonyl;
C₁-C₆-Alkoxycarbonyl wie vorstehend im allgemeinen und im besonderen genannt, insbesondere Methoxycarbonyl;
C₁-C₆-Alkylaminocarbonyl wie vorstehend im allgemeinen und im besonderen genannt, insbesondere Methylaminocarbonyl;
Di-C₁-C₆-alkylaminocarbonyl wie vorstehend im allgemeinen und im besonderen genannt, insbesondere Dimethylaminocarbonyl;
C₁-C₆-Alkylcarboxyl wie vorstehend im allgemeinen und im besonderen genannt, insbesondere Methylcarboxyl;
Phenyl, Phenoxy, Phenylthio, Phenyl-C₁-C₄-alkyl wie vorstehend im allgemeinen und im besonderen genannt, insbesondere Phenyl, Phenoxy;
Phenoxy-C₁-C₄-alkyl wie vorstehend im allgemeinen und im besonderen genannt, insbesondere Phenoxymethyl;
Phenylthio-C₁-C₄-alkyl wie vorstehend im allgemeinen und im besonderen genannt, insbesondere Phenylthiomethyl;
Phenylamino-C₁-C₄-alkyl wie vorstehend im allgemeinen und im besonderen genannt, insbesondere Phenylaminomethyl;
wobei die aromatischen Ringe ihrerseits ein bis fünf Halogenatome wie vorstehend im allgemeinen und im besonderen genannt, insbesondere Fluor und Chlor;
und/oder ein bis drei der folgenden Reste tragen können:
Cyano, Nitro;
C₁-C₄-Alkyl wie vorstehend im allgemeinen und im besonderen genannt, insbesondere Methyl;
C₁-C₄-Halogenalkyl wie vorstehend im allgemeinen und im besonderen genannt, insbesondere Trifluormethyl;
C₁-C₄-Alkoxy wie vorstehend im allgemeinen und im besonderen genannt, insbesondere Methoxy, Ethoxy;
C₁-C₄-Halogenalkoxy wie vorstehend im allgemeinen und im besonderen genannt;
C₁-C₄-Alkylthio wie vorstehend im allgemeinen und im besonderen genannt, insbesondere Methylthio;
oder in denen R³ ein über ein Kohlenstoffatom gebundenes aliphatisches Ringsystem, das neben Kohlenstoffatomen ein bis zwei Heteroatome aus der Gruppe Sauerstoff, Schwefel und Stickstoff enthalten kann wie vorstehend im allgemeinen und im besonderen genannt, insbesondere Cyclopropyl, Cyclohexyl, 2-Tetrahydrofuranyl, 2-Tetrahydropyranyl, bedeutet, wobei dieses Ringsystem ein bis fünf Halogenatome wie vorstehend im allgemeinen und im besonderen genannt, insbesondere Fluor, Chlor;
und/oder ein bis vier der folgenden Reste tragen kann:
Cyano, Nitro, Amino, C₁-C₆-Alkyl wie vorstehend im allgemeinen und im besonderen genannt, insbesondere Methyl;
C₁-C₄-Halogenalkyl wie vorstehend im allgemeinen und im besonderen genannt, insbesondere Trifluormethyl;
C₁-C₆-Alkoxy wie vorstehend im allgemeinen und im besonderen genannt, insbesondere Methoxy, Ethoxy;
C₁-C₄-Halogenalkoxy wie vorstehend im allgemeinen und im besonderen genannt;
C₁-C₄-Alkoxy-C₁-C₄-alkyl wie vorstehend im allgemeinen und im besonderen genannt, insbesondere Methoxymethyl;
C₁-C₆-Alkylthio wie vorstehend im allgemeinen und im besonderen genannt, insbesondere Methylthio;
C₁-C₄-Alkylthio-C₁-C₄-alkyl wie vorstehend im allgemeinen und im besonderen genannt;
C₃-C₈-Cycloalkyl wie vorstehend im allgemeinen und im besonderen genannt;
Di-C₁-C₆-alkylamino wie vorstehend im allgemeinen und im besonderen genannt, insbesondere Dimethylamino;
C₁-C₆-Alkylcarboxyl wie vorstehend im allgemeinen und im besonderen genannt, insbesondere Methylcarboxyl;
Phenyl, Phenoxy, Phenylthio;
Phenyl-C₁-C₄-alkyl wie vorstehend im allgemeinen und im besonderen genannt, insbesondere Benzyl;
Phenoxy-C₁-C₄-alkyl wie vorstehend im allgemeinen und im besonderen genannt;
Phenylthio-C₁-C₄-alkyl wie vorstehend im allgemeinen und im besonderen genannt;
wobei die aromatischen Ringe ihrerseits ein bis fünf Halogenatome wie vorstehend im allgemeinen und im besonderen genannt, insbesondere Fluor und Chlor;
und/oder ein bis drei der folgenden Reste tragen können:
Cyano, Nitro;
C₁-C₄-Alkyl wie vorstehend im allgemeinen und im besonderen genannt, insbesondere Methyl;
C₁-C₄-Halogenalkyl wie vorstehend im allgemeinen und im besonderen genannt, insbesondere Trifluormethyl;
C₁-C₄-Alkoxy wie vorstehend im allgemeinen und im besonderen genannt, insbesondere Methoxy;
C₁-C₄-Halogenalkoxy wie vorstehend im allgemeinen und im besonderen genannt;
C₁-C₄-Alkylthio wie vorstehend im allgemeinen und im besonderen genannt, insbesondere Methylthio.

Außerdem werden Cyanooximether der Formel I bevorzugt, in denen R² Phenyl, 4-Oxazolyl oder 5-Isoxazolyl bedeutet, wobei diese Ringe ein bis fünf Halogenatome wie vorstehend im allgemeinen und im besonderen genannt, insbesondere Fluor und Chlor;
und/oder ein bis drei der folgenden Reste tragen kann:
Cyano, Cyanato, Thiocyanato, Nitro, Amino, Hydroxy, Carboxyl, C₁-C₆-Alkyl wie vorstehend im allgemeinen und im besonderen genannt, insbesondere Methyl, 1-Methylethyl, 1,1-Dimethylethyl, 1-Methylpropyl;
C₁-C₄-Halogenalkyl wie vorstehend im allgemeinen und im besonderen genannt, insbesondere Trifluormethyl;
C₁-C₆-Alkoxy wie vorstehend im allgemeinen und im besonderen genannt, insbesondere Methoxy, Ethoxy;
C₁-C₄-Halogenalkoxy wie vorstehend im allgemeinen und im besonderen genannt;
C₁-C₄-Alkoxy-C₁-C₄-alkyl wie vorstehend im allgemeinen und im besonderen genannt, insbesondere Methoxymethyl;
C₁-C₆-Alkylthio wie vorstehend im allgemeinen und im besonderen genannt, insbesondere Methylthio;
C₁-C₄-Halogenalkylthio wie vorstehend im allgemeinen und im besonderen genannt;
C₁-C₄-Alkylthio-C₁-C₄-alkyl wie vorstehend im allgemeinen und im besonderen genannt;
C₃-C₁₅-Alkenyl wie vorstehend im allgemeinen und im besonderen genannt;
C₃-C₁₅-Alkenyloxy wie vorstehend im allgemeinen und im besonderen genannt;
C₃-C₈-Cycloalkyl wie vorstehend im allgemeinen und im besonderen genannt, insbesondere Cyclopropyl, Cyclohexyl;
C₅-C₈-Cycloalkenyl wie vorstehend im allgemeinen und im besonderen genannt;
C₃-C₈-Cycloalkoxy wie vorstehend im allgemeinen und im besonderen genannt;
C₅-C₈-Cycloalkenyloxy wie vorstehend im allgemeinen und im besonderen genannt;
C₁-C₆-Alkylamino wie vorstehend im allgemeinen und im besonderen genannt;
Di-C₁-C₆-alkylamino wie vorstehend im allgemeinen und im besonderen genannt;
C₁-C₆-Alkylcarbonyl wie vorstehend im allgemeinen und im besonderen genannt;
C₁-C₆-Alkoxycarbonyl wie vorstehend im allgemeinen und im besonderen genannt;
C₁-C₆-Alkylaminocarbonyl wie vorstehend im allgemeinen und im besonderen genannt;
Di-C₁-C₆-alkylaminocarbonyl wie vorstehend im allgemeinen und im besonderen genannt;
C₁-C₆-Alkylcarboxyl wie vorstehend im allgemeinen und im besonderen genannt;
Phenyl, Phenoxy, Phenylthio, Phenylamino, Phenyl-C₁-C₄-alkyl wie vorstehend im allgemeinen und im besonderen genannt, insbesondere Phenyl;
Phenoxy-C₁-C₄-alkyl wie vorstehend im allgemeinen und im besonderen genannt;
Phenylthio-C₁-C₄-alkyl wie vorstehend im allgemeinen und im besonderen genannt;
Phenylamino-C₁-C₄-alkyl wie vorstehend im allgemeinen und im besonderen genannt;
wobei die aromatischen Ringe ihrerseits ein bis fünf Halogenatome wie vorstehend im allgemeinen und im besonderen genannt, insbesondere Fluor, Chlor und Brom;
und/oder ein bis drei der folgenden Reste tragen können: Cyano, Nitro, C₁-C₄-Alkyl wie vorstehend im allgemeinen und im besonderen genannt, insbesondere Methyl;
C₁-C₄-Halogenalkyl wie vorstehend im allgemeinen und im besonderen genannt, insbesondere Trifluormethyl;
C₁-C₄-Alkoxy wie vorstehend im allgemeinen und im besonderen genannt, insbesondere Methoxy;
C₁-C₄-Halogenalkoxy wie vorstehend im allgemeinen und im besonderen genannt;
C₁-C₄-Alkylthio wie vorstehend im allgemeinen und im besonderen genannt, insbesondere Methylthio;
und C₁-C₄-Halogenalkylthio wie vorstehend im allgemeinen und im besonderen genannt;
ein über ein Kohlenstoffatom gebundenes fünf- oder sechsgliedriges aromatisches Ringsystem, das neben Kohlenstoffatomen ein bis drei Stickstoffatome oder ein oder zwei Stickstoffatome und ein Sauerstoff- oder ein Schwefelatom oder ein Sauerstoff- oder ein Schwefelatom enthalten kann wie vorstehend genannt, insbesondere
funfring Heteroaromaten wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4- Triazol-2-yl,
oder sechsring Heteroaromaten wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl,
wobei diese Ringsysteme ein bis vier Halogenatome wie vorstehend im allgemeinen und im besonderen genannt, insbesondere Fluor, Chlor und Brom und/oder ein bis drei der folgenden Reste tragen können:
C₁-C₄-Alkyl wie vorstehend im allgemeinen und im besonderen genannt, insbesondere Methyl;
C₁-C₄-Halogenalkyl wie vorstehend im allgemeinen und im besonderen genannt, insbesondere Trifluormethyl;
C₁-C₄-Alkoxy wie vorstehend im allgemeinen und im besonderen genannt, insbesondere Methoxy;
C₁-C₄-Halogenalkoxy wie vorstehend im allgemeinen und im besonderen genannt, insbesondere Trifluormethoxy;
C₁-C₄-Alkylthio wie vorstehend im allgemeinen und im besonderen genannt, insbesondere Methylthio;
und C₁-C₄-Halogenalkylthio wie vorstehend im allgemeinen und im besonderen genannt, insbesondere Trifluormethylthio;

Desweiteren werden Cyanooximether der Formel I bevorzugt, in denen R³ die folgende Bedeutung hat:
C₁-C₄-Alkyl wie vorstehend im allgemeinen und im besonderen genannt, insbesondere Methyl, Ethyl, 1,1-Dimethylethyl;
welches ein bis neun Halogenatome wie vorstehend im allgemeinen und im besonderen genannt, insbesondere Chlor und Brom;
und/oder einen der folgenden Reste tragen kann:
C₁-C₄-Alkoxy wie vorstehend im allgemeinen und im besonderen genannt, insbesondere Methoxy, Ethoxy, Propoxy, 1-Methylethoxy;
C₁-C₄-Halogenalkoxy wie vorstehend im allgemeinen und im besonderen genannt;
C₁-C₄-Alkylthio wie vorstehend im allgemeinen und im besonderen genannt, insbesondere Methylthio, Ethylthio;
C₃-C₈-Cycloalkyl wie vorstehend im allgemeinen und im besonderen genannt;
C₃-C₈-Cycloalkoxy wie vorstehend im allgemeinen und im besonderen genannt;
Phenyl, Phenoxy, Phenylthio, Phenylamino, Phenyl-C₁-C₄-alkyl wie vorstehend im allgemeinen und im besonderen genannt;
Phenoxy-C₁-C₄-alkyl wie vorstehend im allgemeinen und im besonderen genannt;
Phenylthio-C₁-C₄-alkyl wie vorstehend im allgemeinen und im besonderen genannt;
wobei die aromatischen Ringe ihrerseits ein bis fünf Halogenatome wie vorstehend im allgemeinen und im besonderen genannt, insbesondere Chlor;
und/oder ein bis drei der folgenden Reste tragen können:
C₁-C₄-Alkyl wie vorstehend im allgemeinen und im besonderen genannt, insbesondere Methyl;
C₁-C₄-Halogenalkyl wie vorstehend im allgemeinen und im besonderen genannt, insbesondere Trifluormethyl;
C₁-C₄-Alkoxy wie vorstehend im allgemeinen und im besonderen genannt, insbesondere Methoxy;
C₁-C₄-Halogenalkoxy wie vorstehend im allgemeinen und im besonderen genannt;
C₁-C₄-Alkylthio wie vorstehend im allgemeinen und im besonderen genannt;
oder in denen R³ ein über ein Kohlenstoffatom gebundenes gesättigtes oder einfach ungesättigtes 3- bis 7-gliedriges Ringsystem, das neben Kohlenstoffatomen ein oder zwei Heteroatome aus der Gruppe Sauerstoff, Schwefel und Stickstoff enthalten kann, bedeutet, wie vorstehend im allgemeinen und im besonderen genannt, insbesondere Cyclopropyl, 2-Tetrahydropyranyl;
wobei dieses Ringsystem ein bis drei der folgenden Reste tragen kann:
C₁-C₄-Alkyl wie vorstehend im allgemeinen und im besonderen genannt, insbesondere Methyl;
C₁-C₄-Alkoxy wie vorstehend im allgemeinen und im besonderen genannt;
C₁-C₄-Alkoxy-C₁-C₄-alkyl wie vorstehend im allgemeinen und im besonderen genannt;
C₁-C₄-Alkylthio wie vorstehend im allgemeinen und im besonderen genannt;
C₁-C₄-Alkylthio-C₁-C₄-alkyl wie vorstehend im allgemeinen und im besonderen genannt;
Phenyl, Phenoxy, Phenylthio;
Phenyl-C₁-C₄-alkyl wie vorstehend im allgemeinen und im besonderen genannt;
Phenoxy-C₁-C₄-alkyl wie vorstehend im allgemeinen und im besonderen genannt;
Phenylthio-C₁-C₄-alkyl wie vorstehend im allgemeinen und im besonderen genannt;
wobei die aromatischen Ringe ihrerseits ein bis fünf Halogenatome wie vorstehend im allgemeinen und im besonderen genannt;
und/oder ein bis drei der folgenden Reste tragen können:
C₁-C₄-Alkyl wie vorstehend im allgemeinen und im besonderen genannt;
C₁-C₄-Halogenalkyl wie vorstehend im allgemeinen und im besonderen genannt;
C₁-C₄-Alkoxy wie vorstehend im allgemeinen und im besonderen genannt;
C₁-C₄-Halogenalkoxy wie vorstehend im allgemeinen und im besonderen genannt;
C₁-C₄-Alkylthio wie vorstehend im allgemeinen und im besonderen genannt;

Besonders bevorzugte Verbindungen der Formel I sind in den nachstehenden Tabellen A, B und C zusammengestellt:

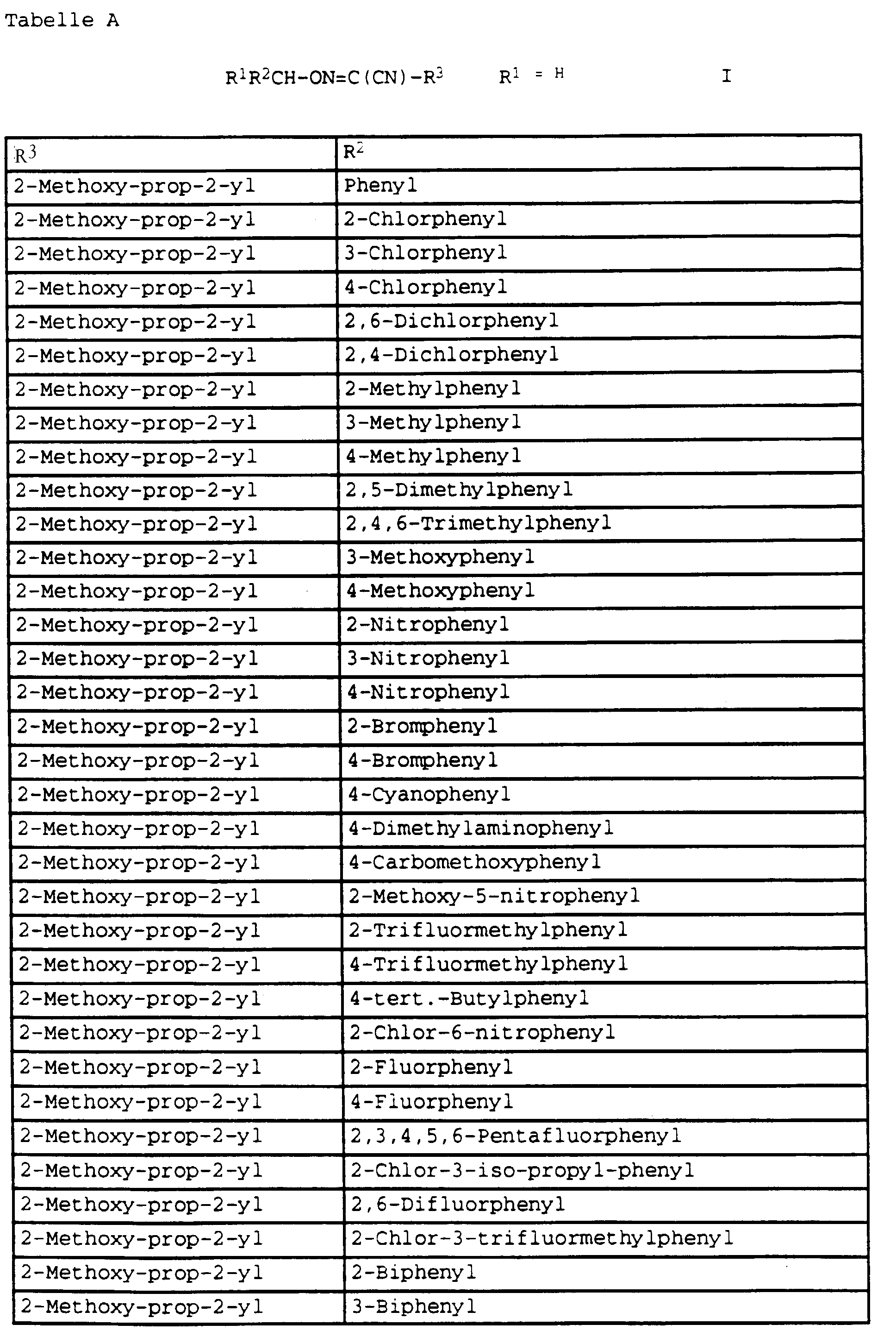

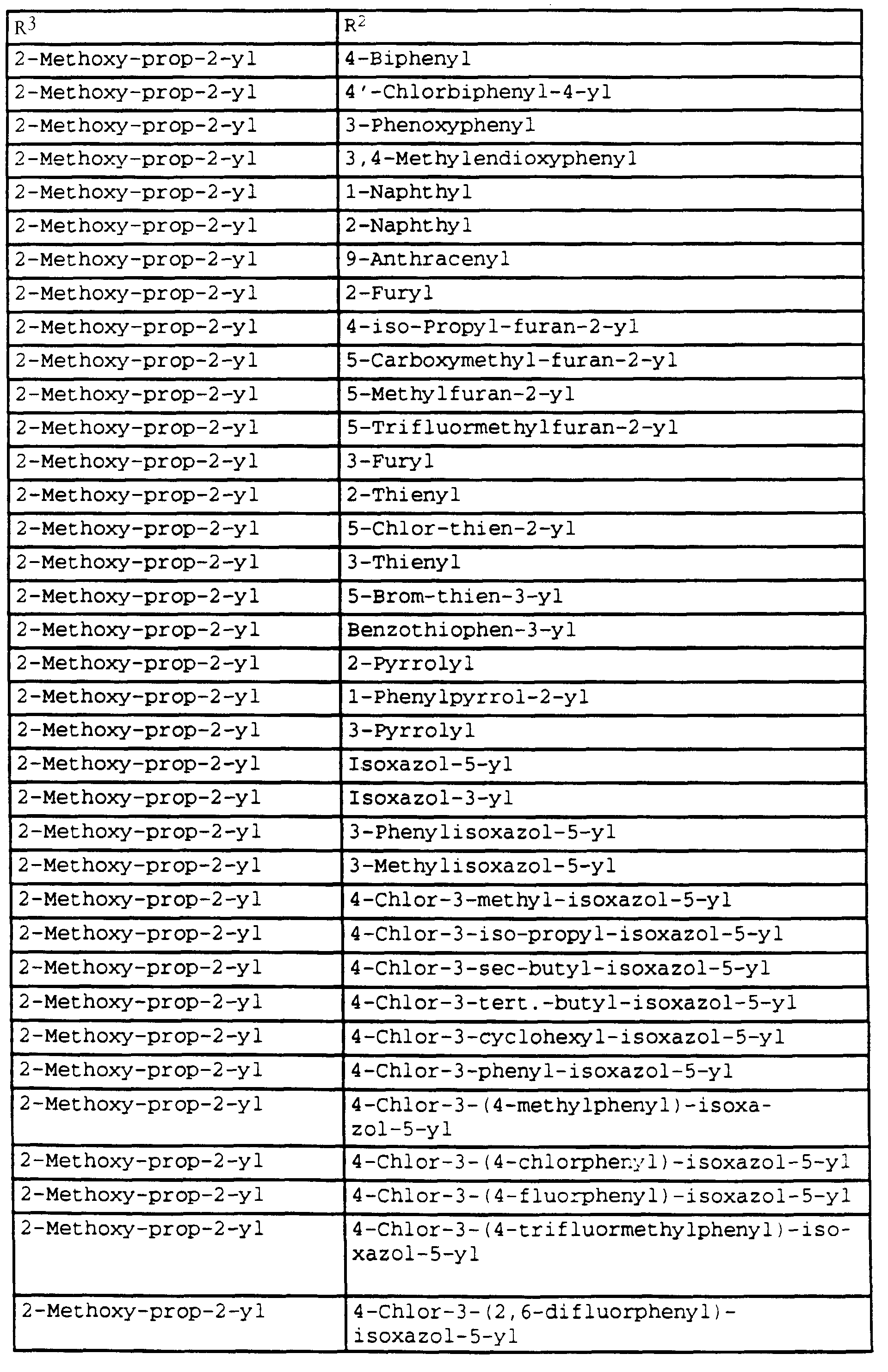

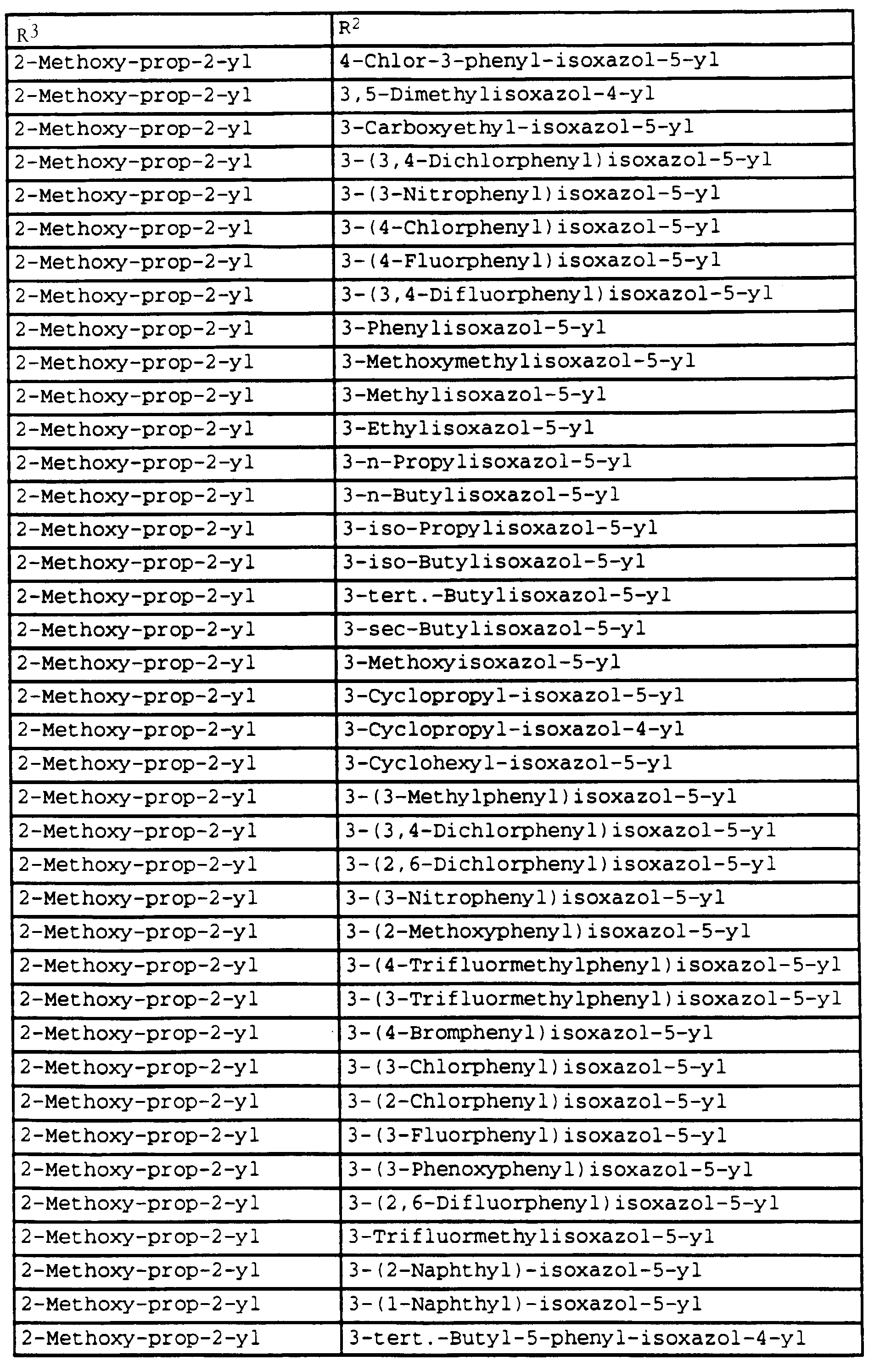

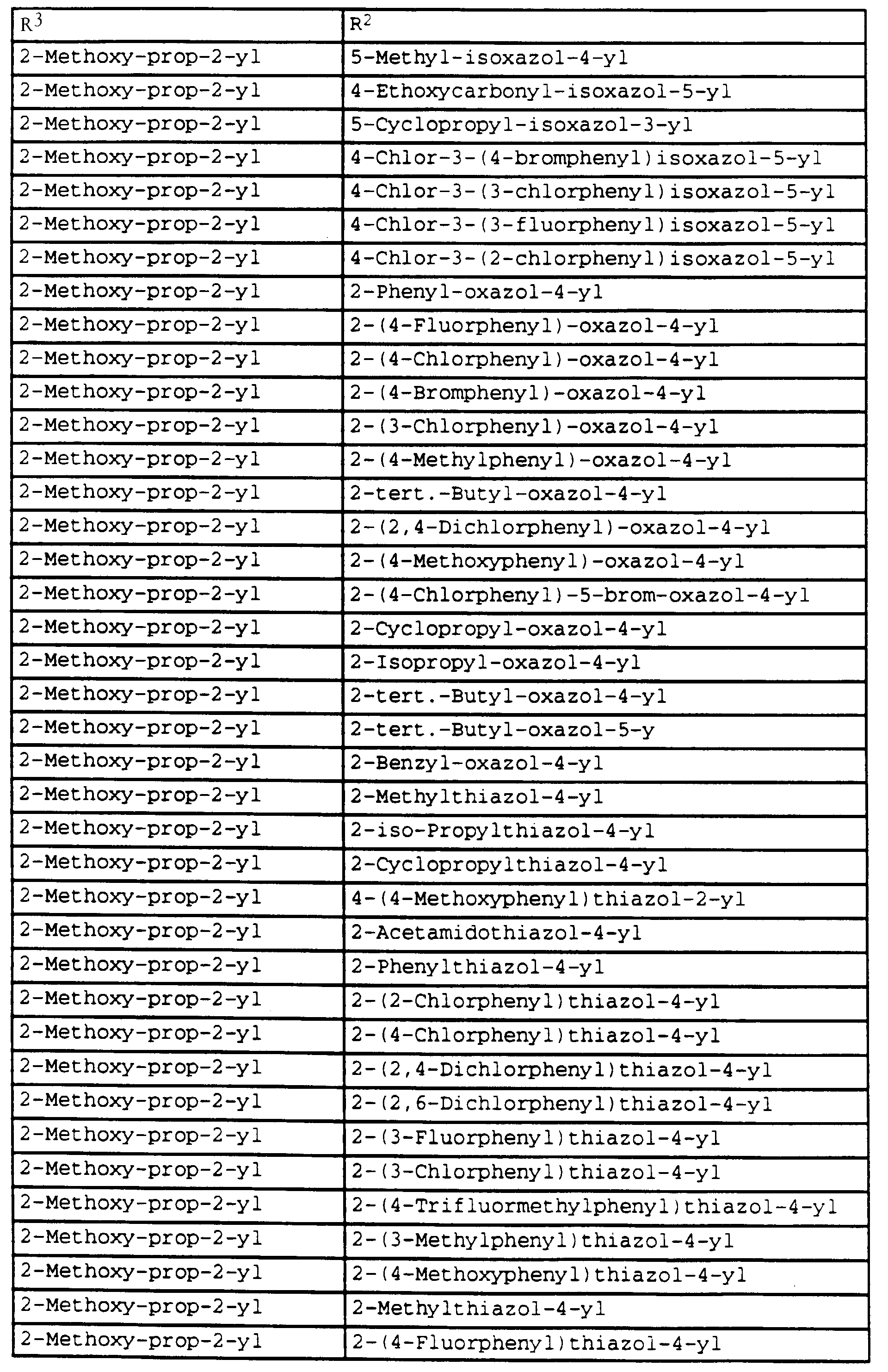

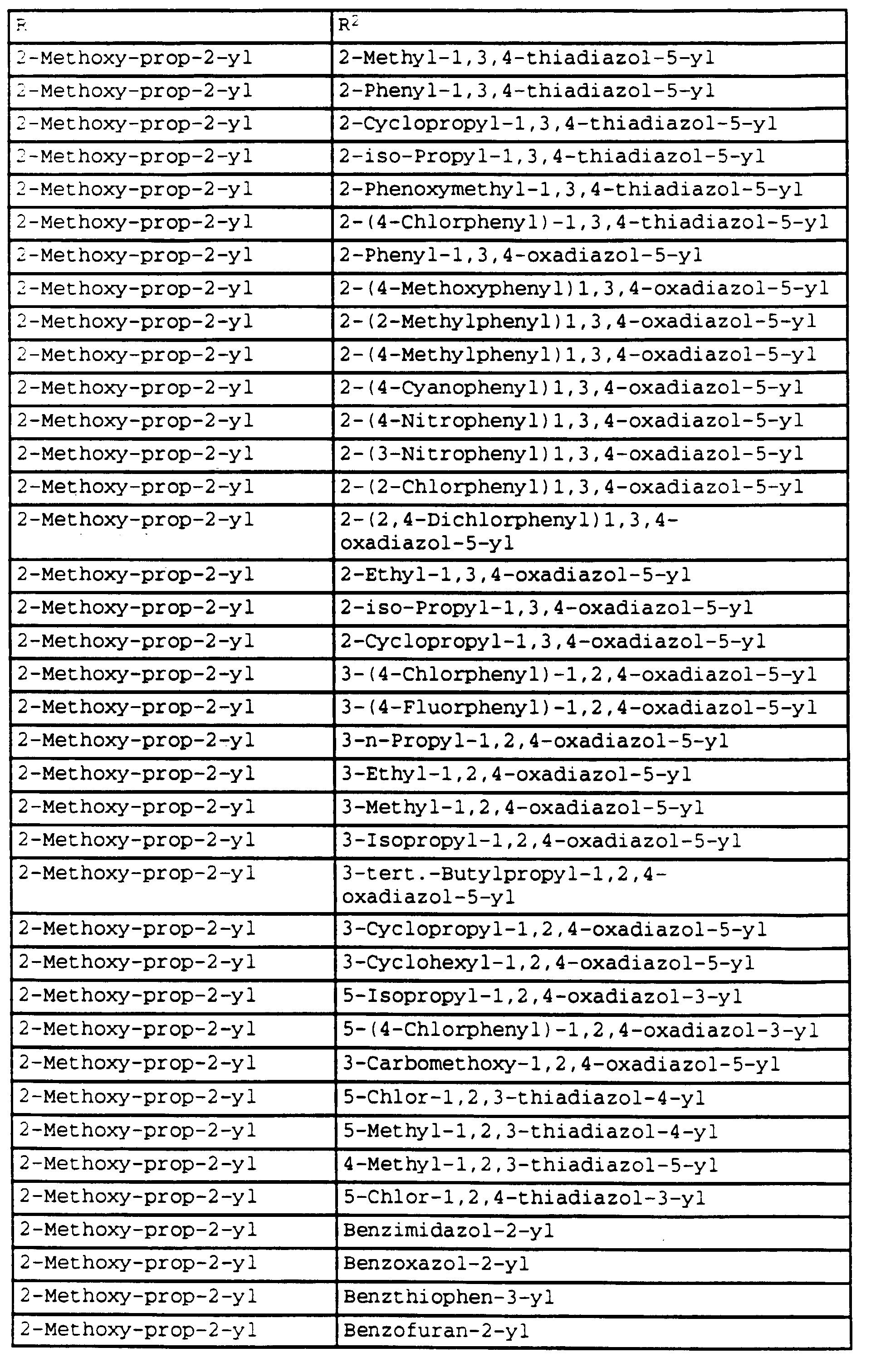

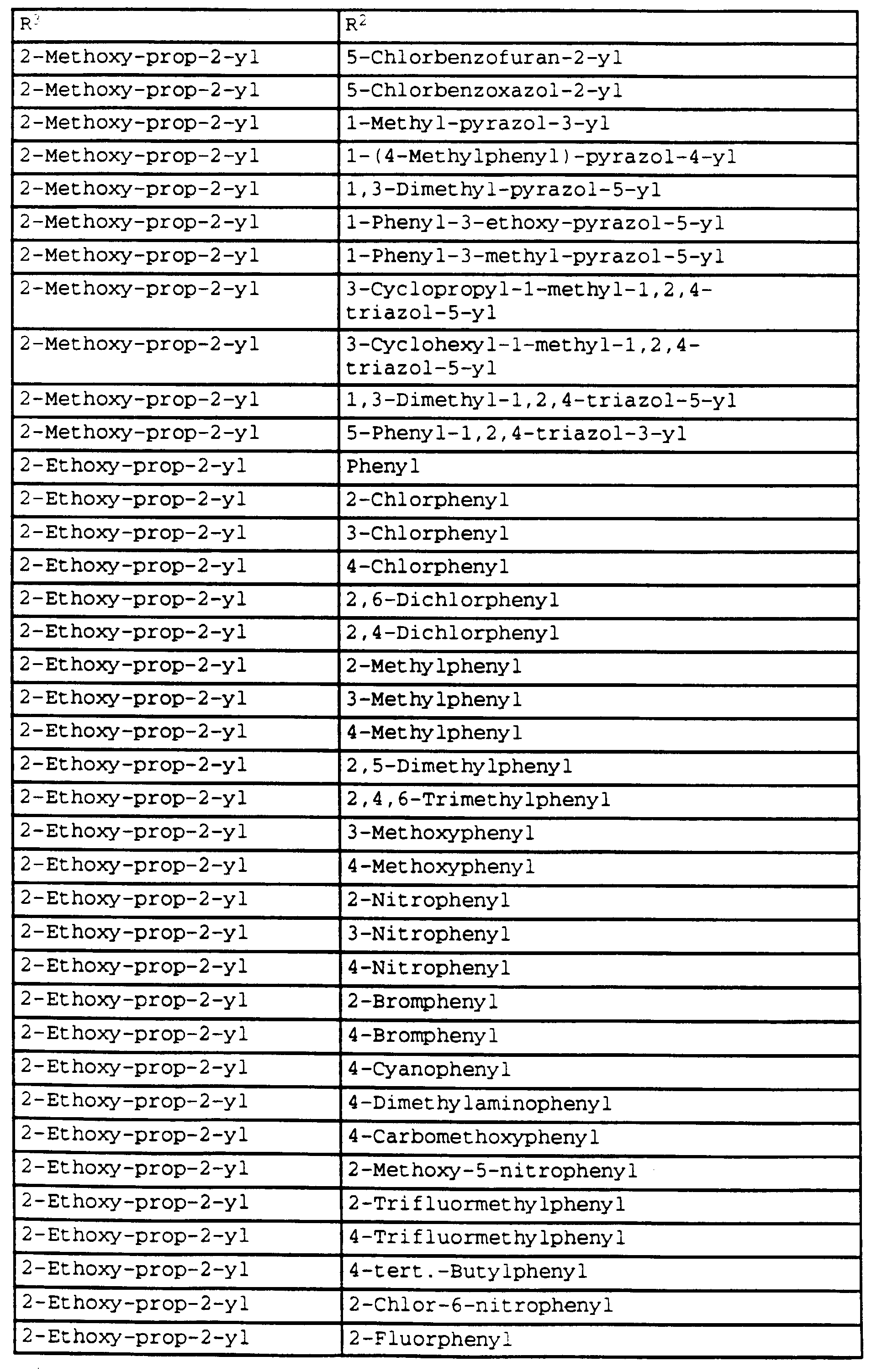

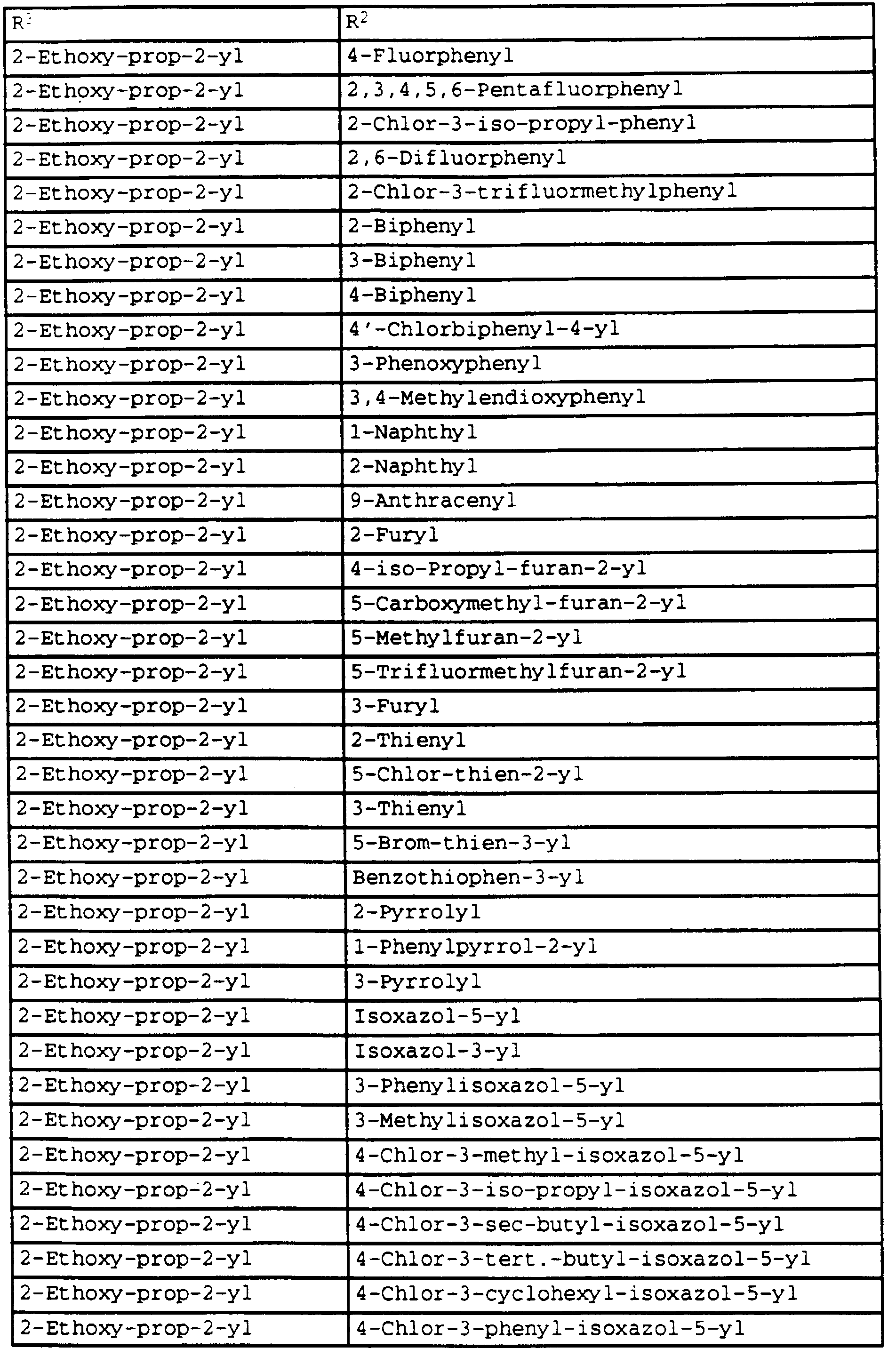

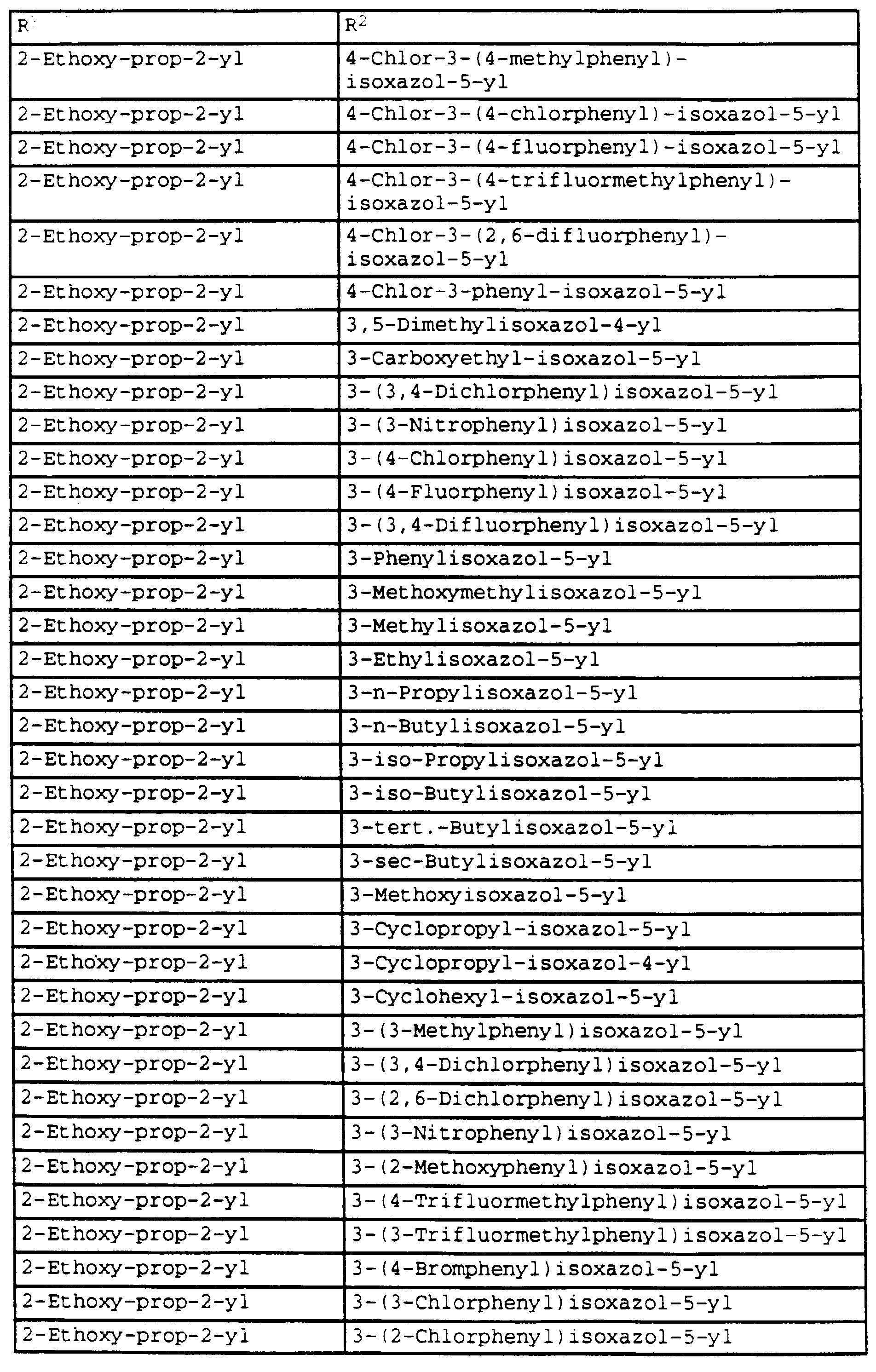

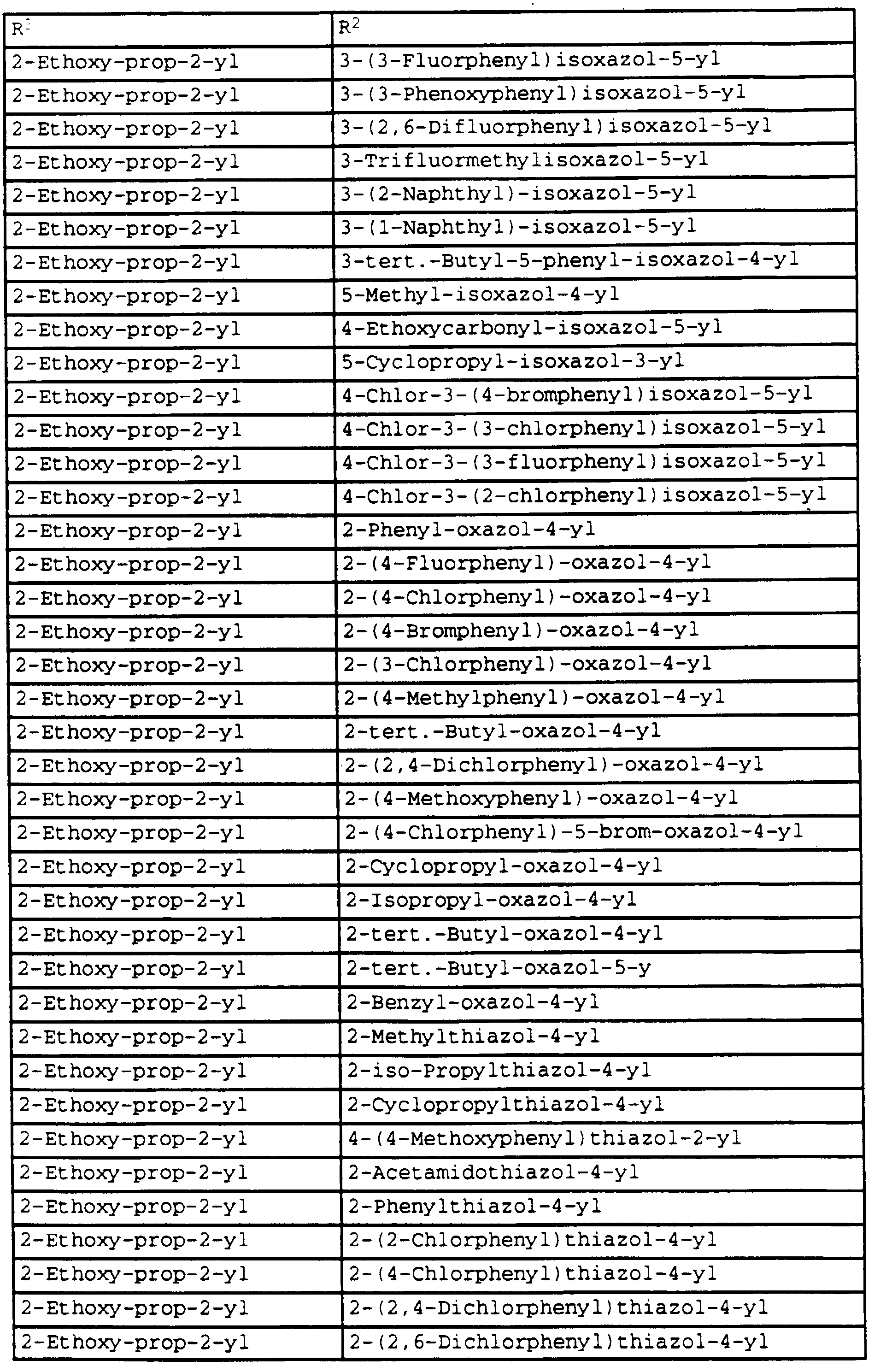

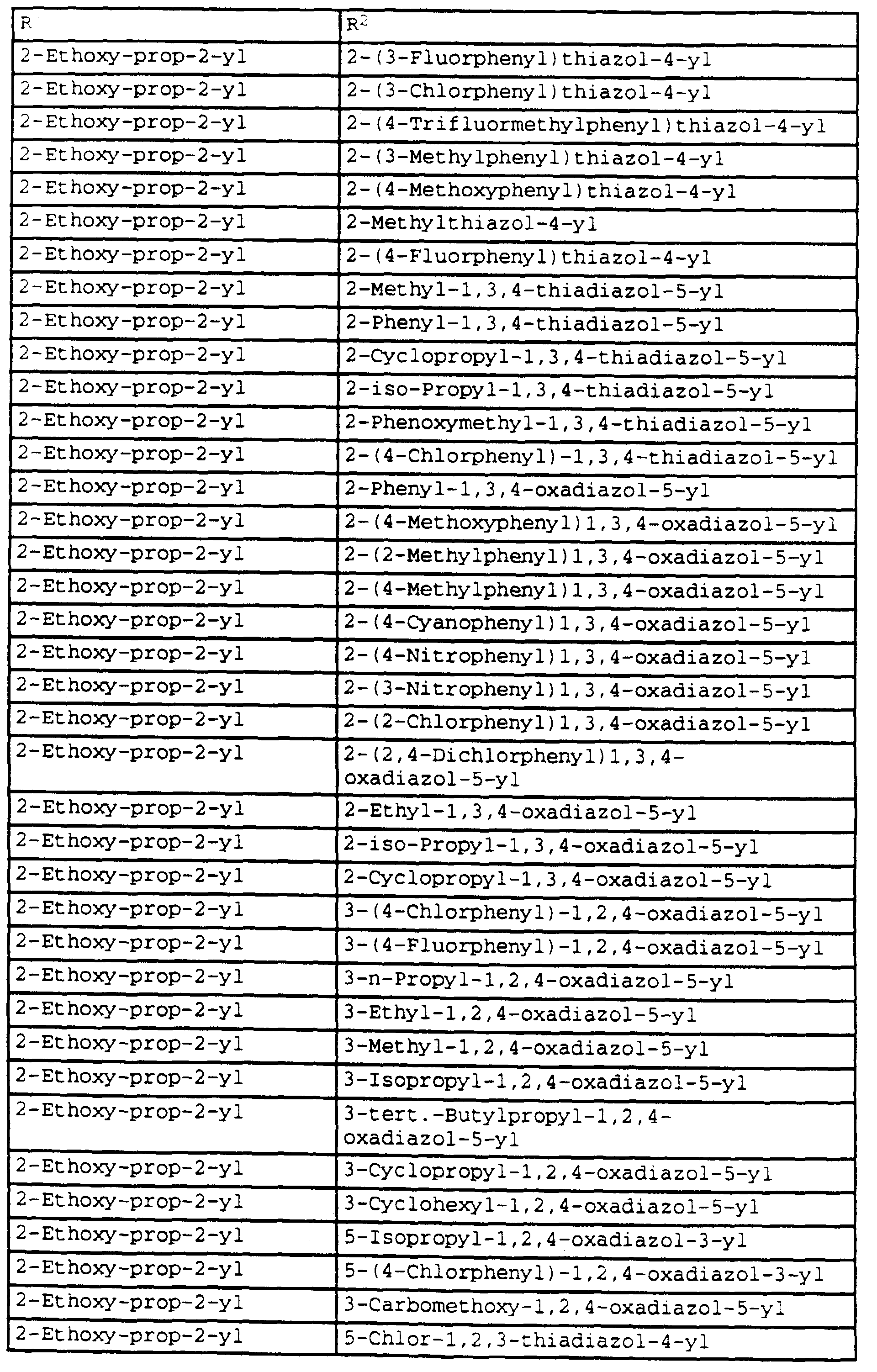

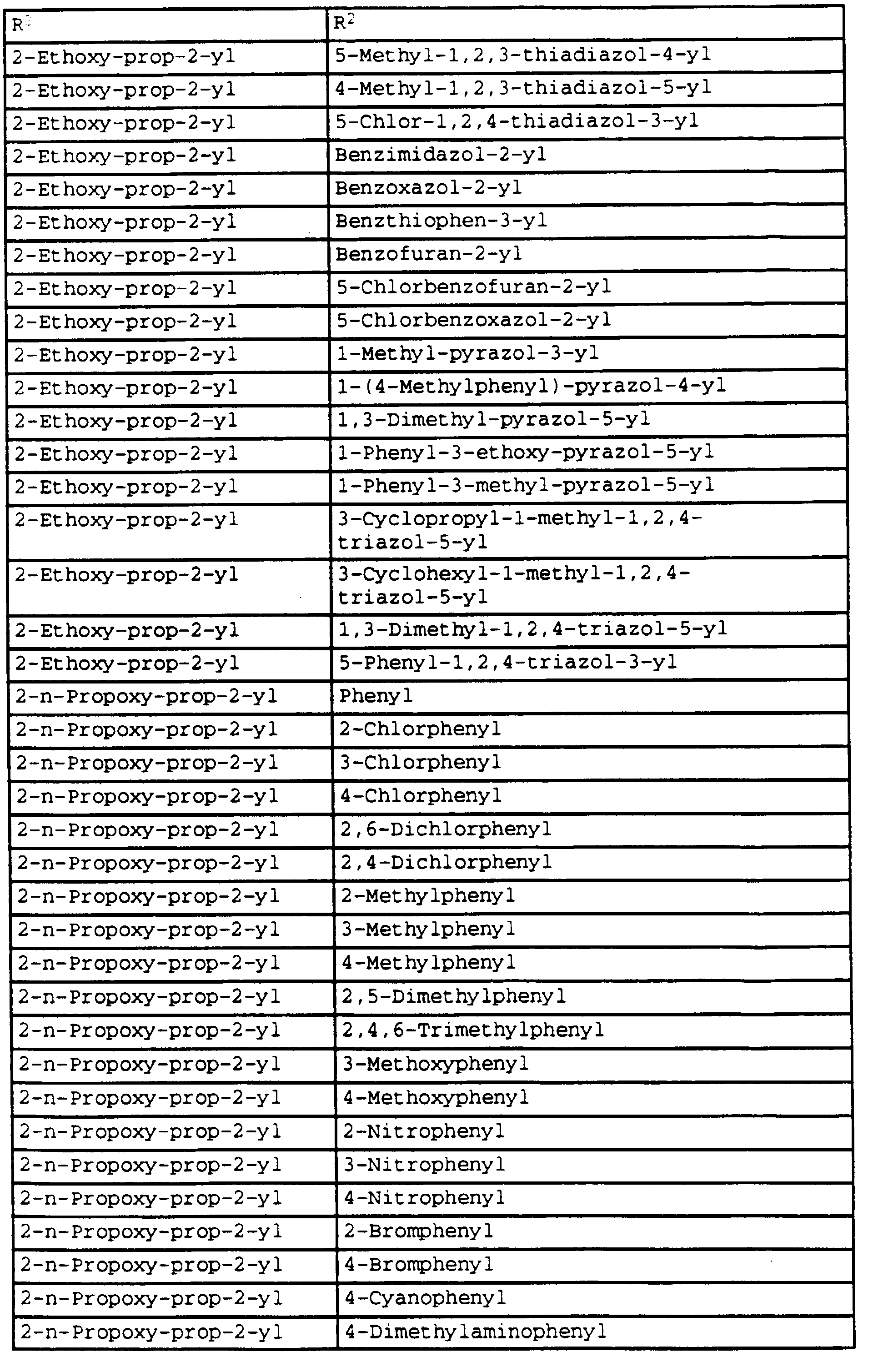

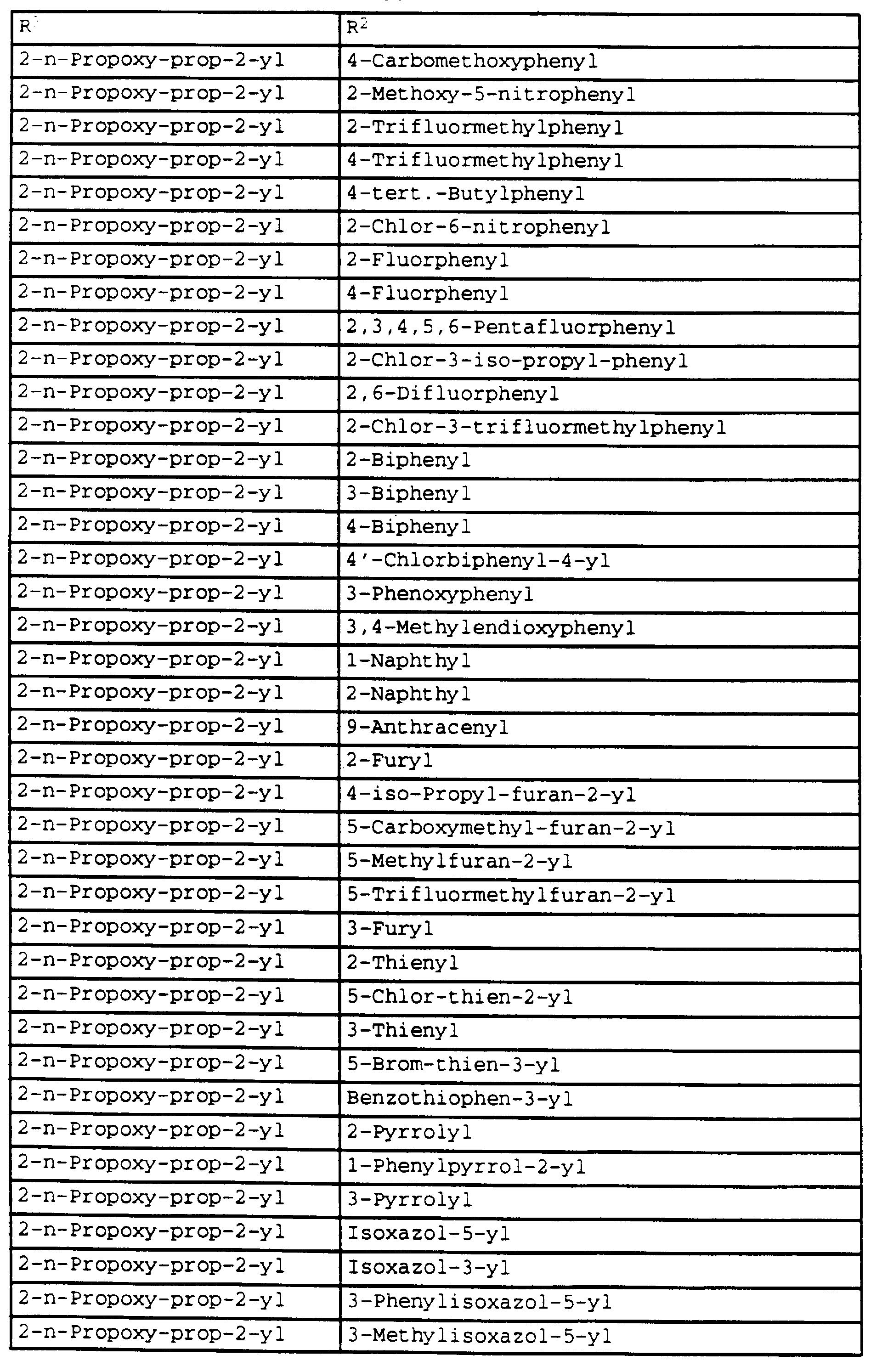

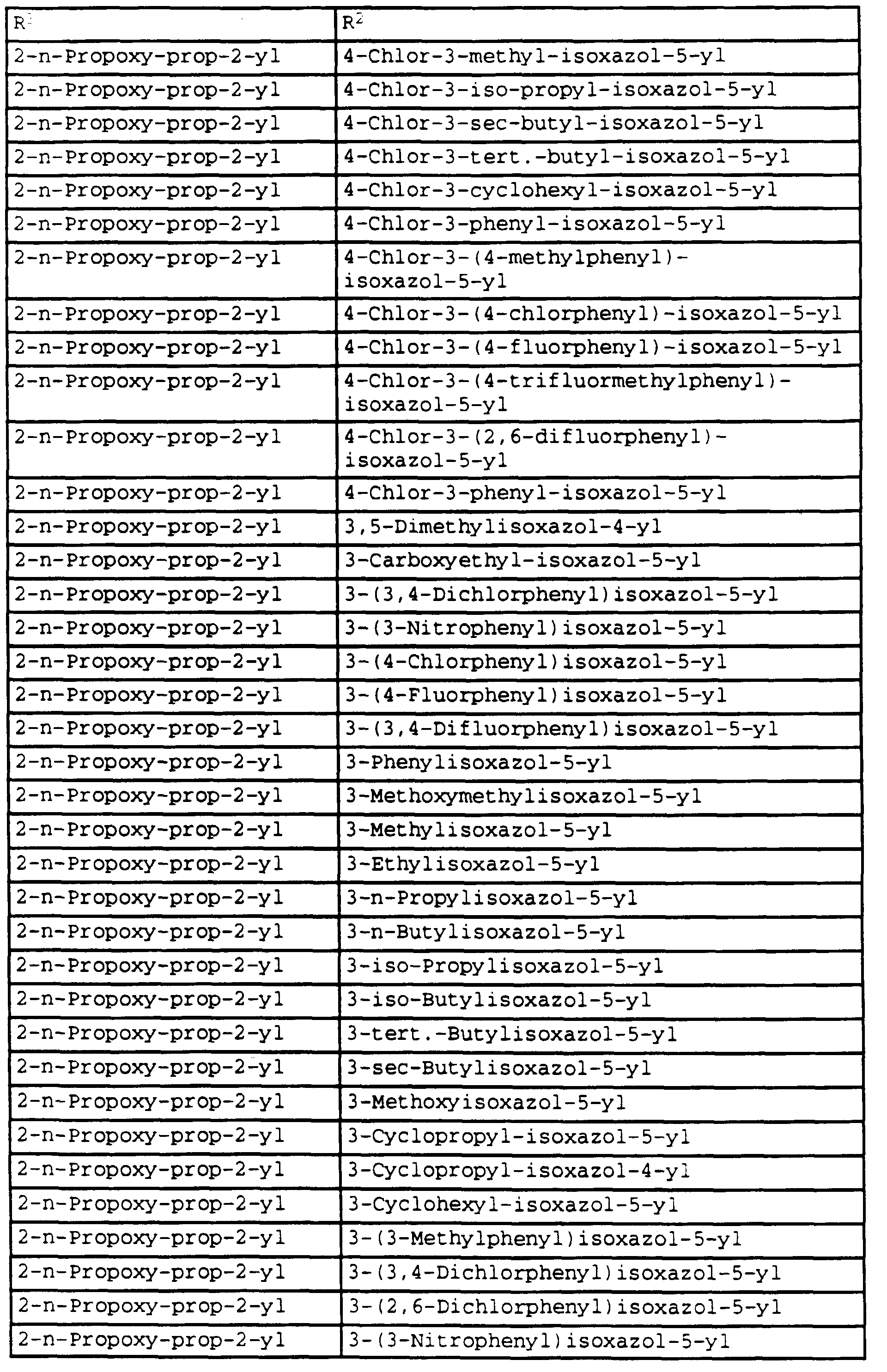

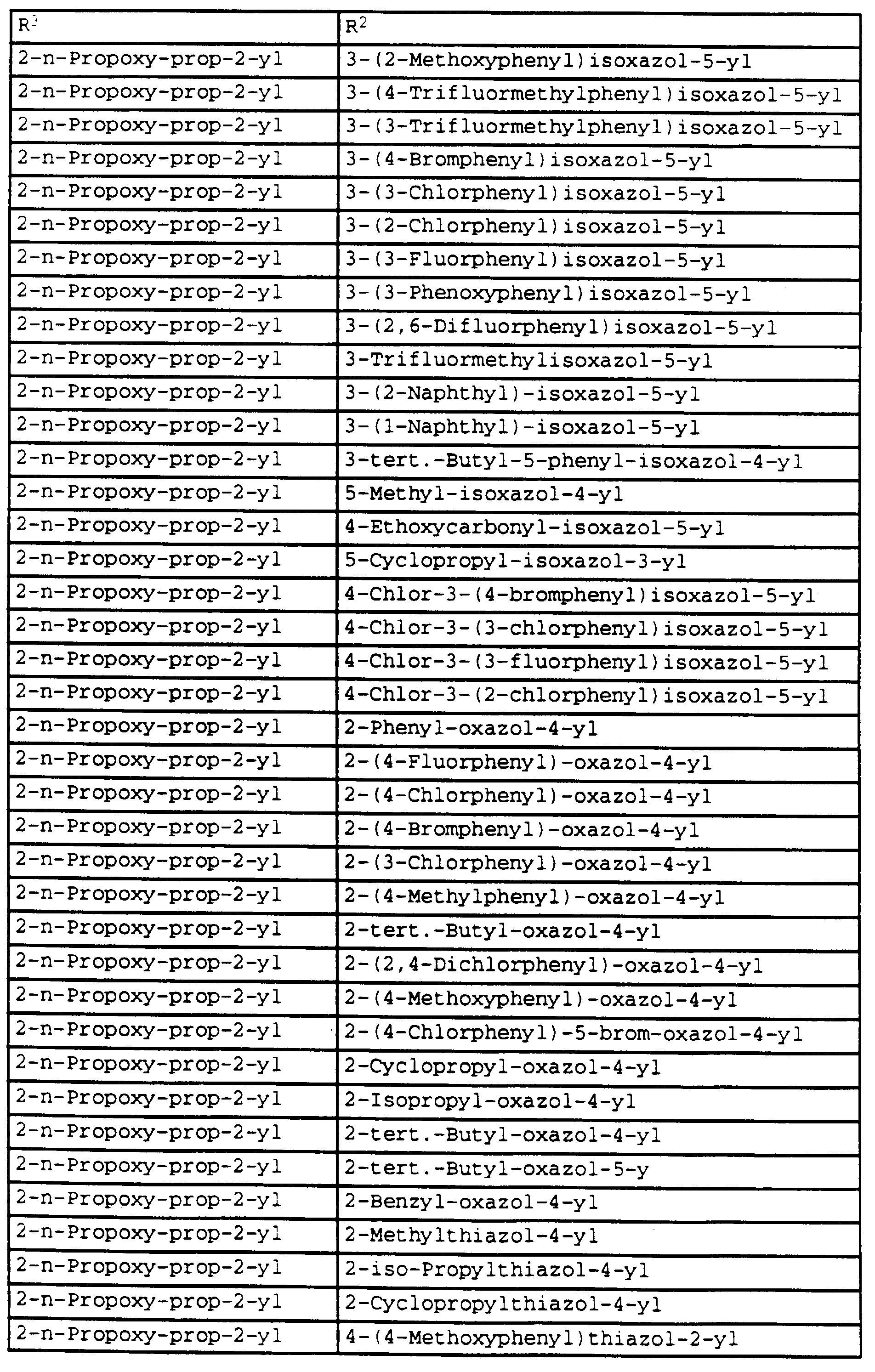

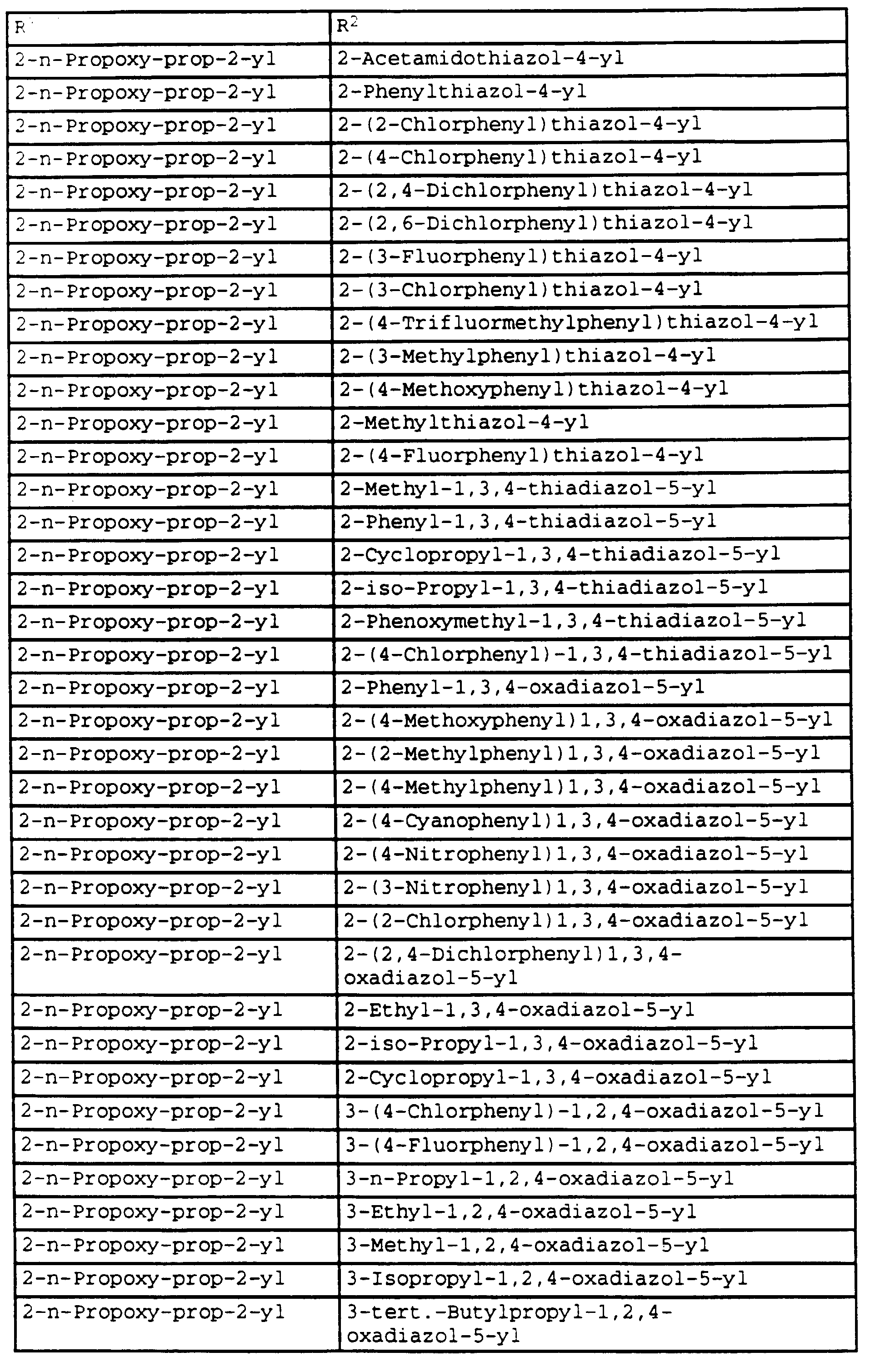

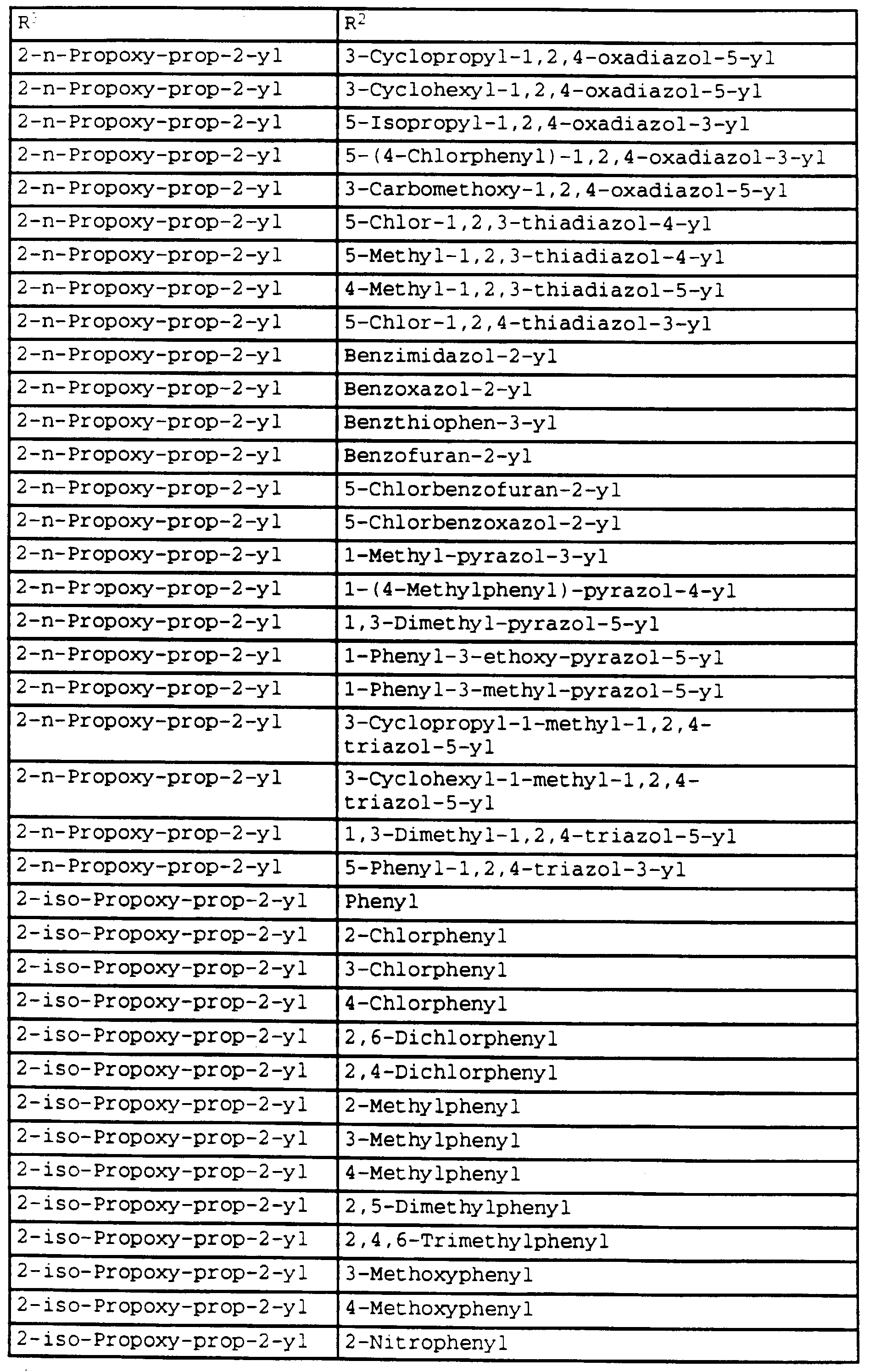

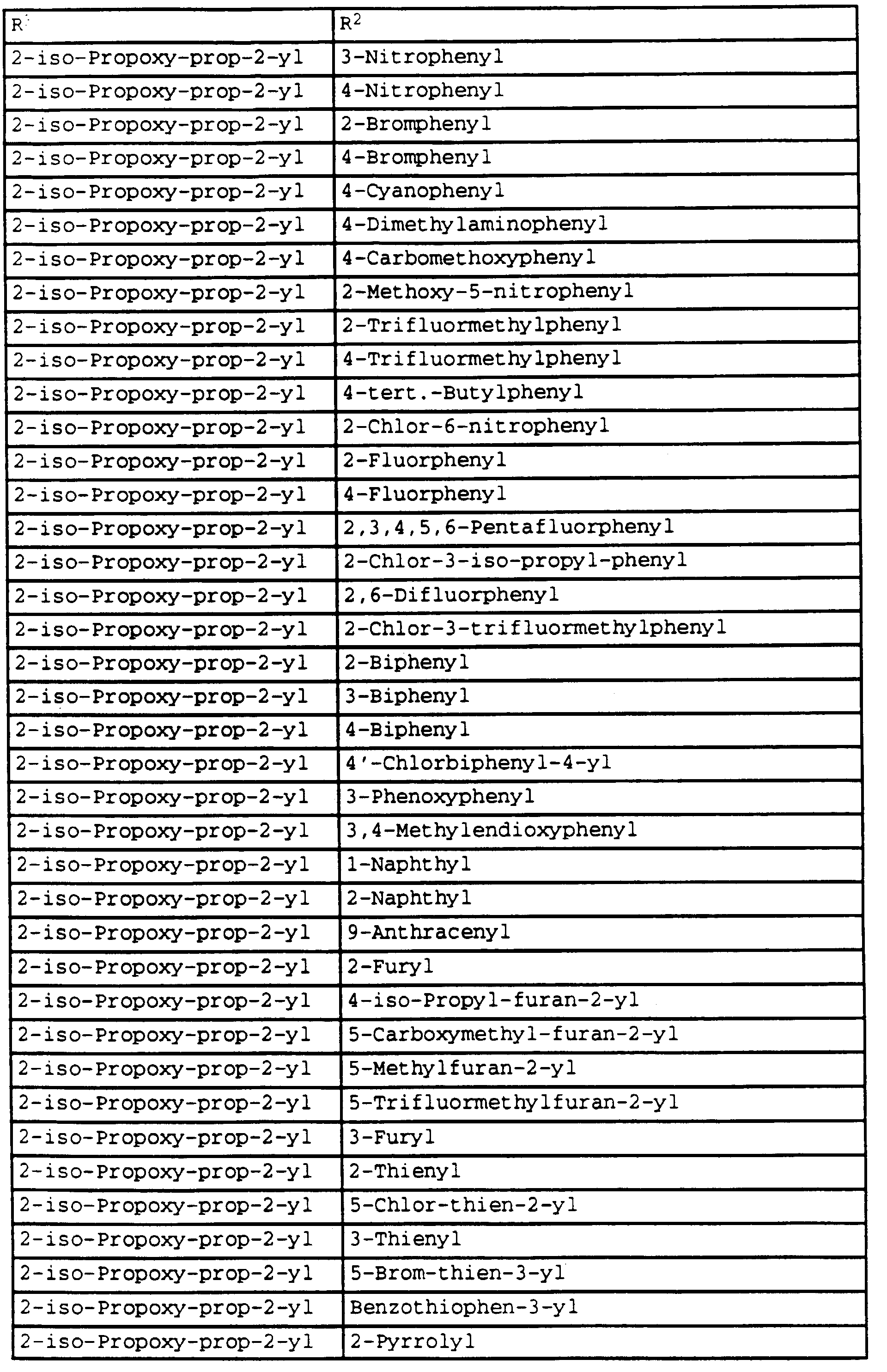

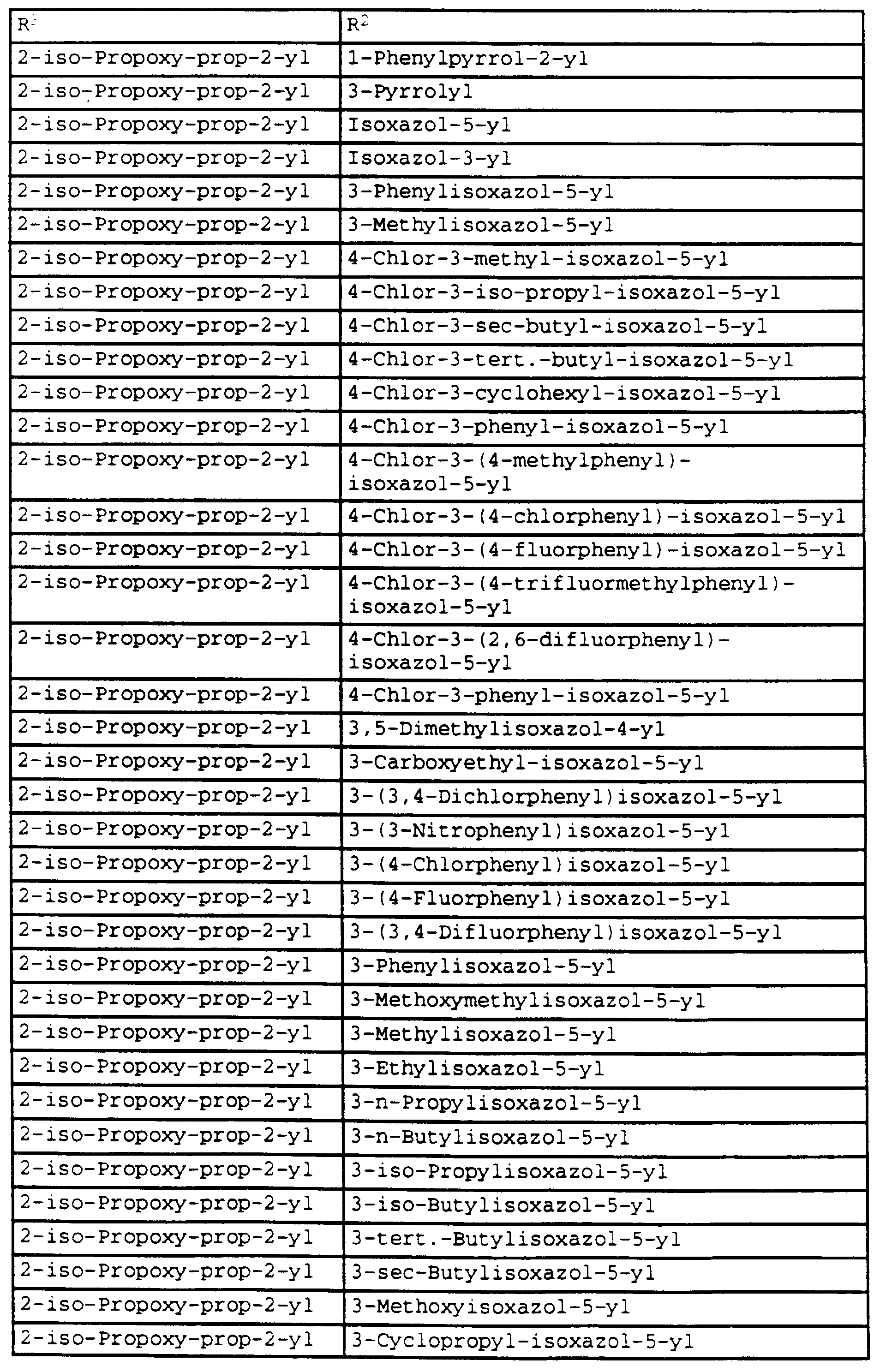

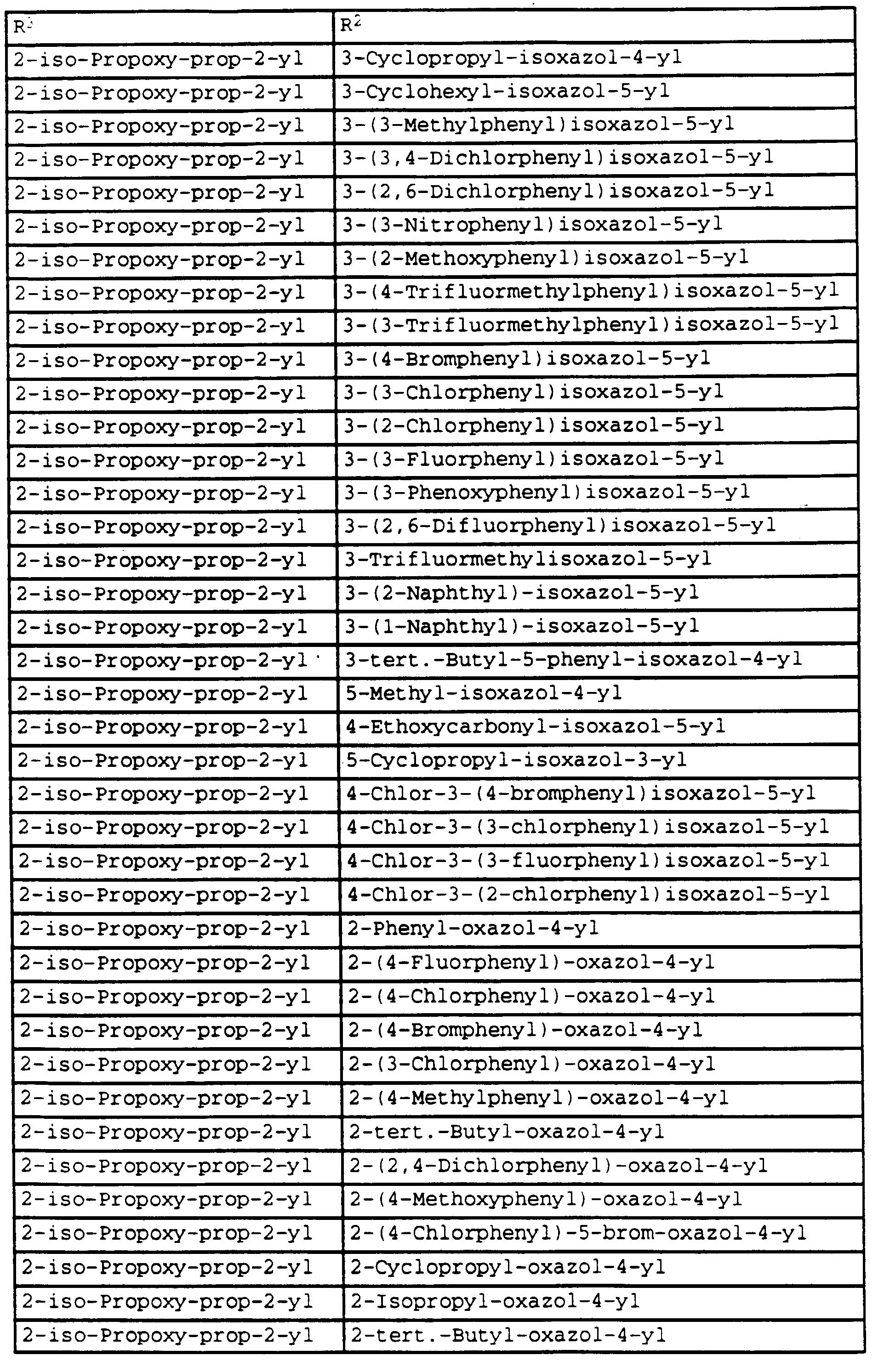

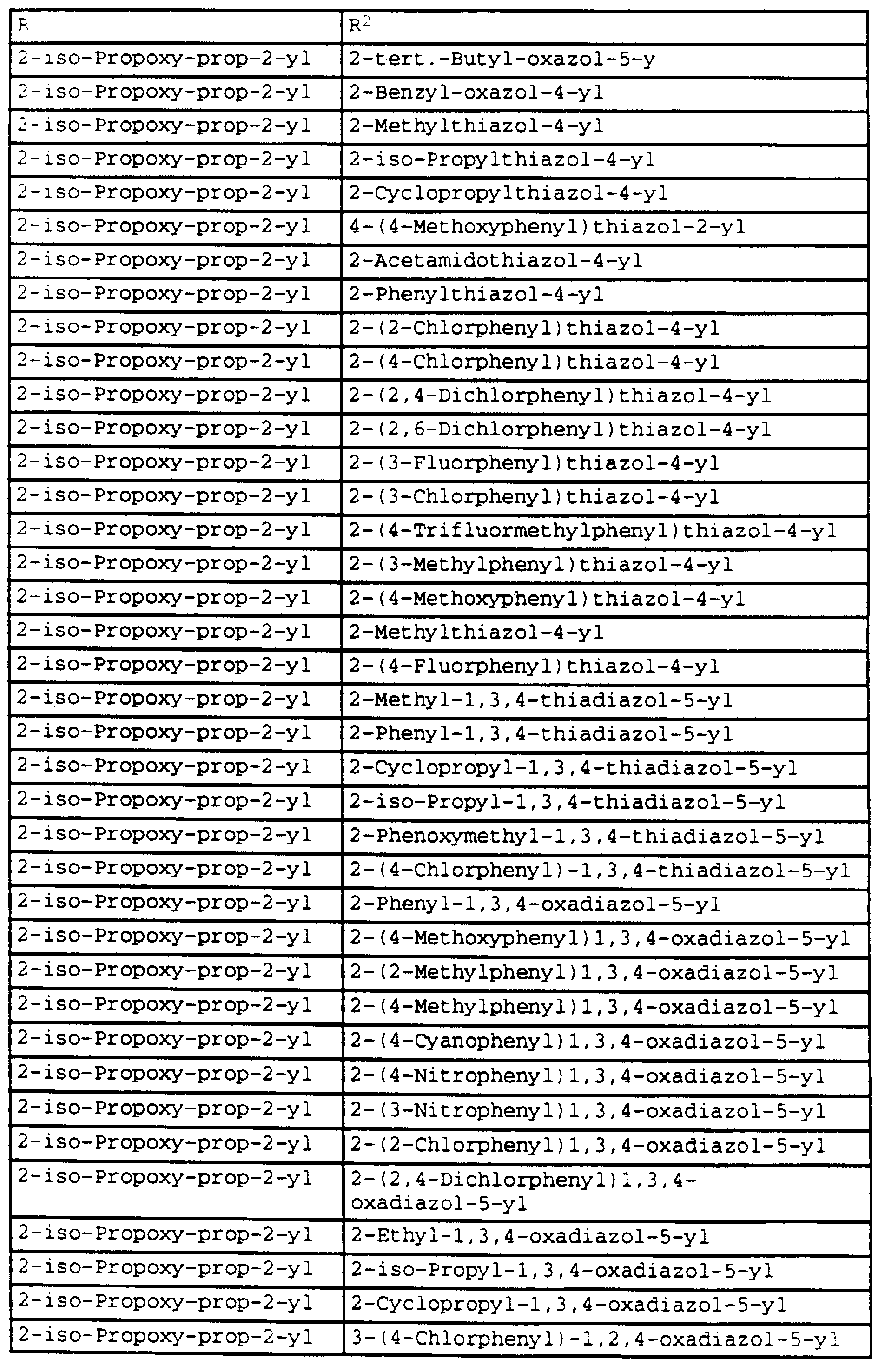

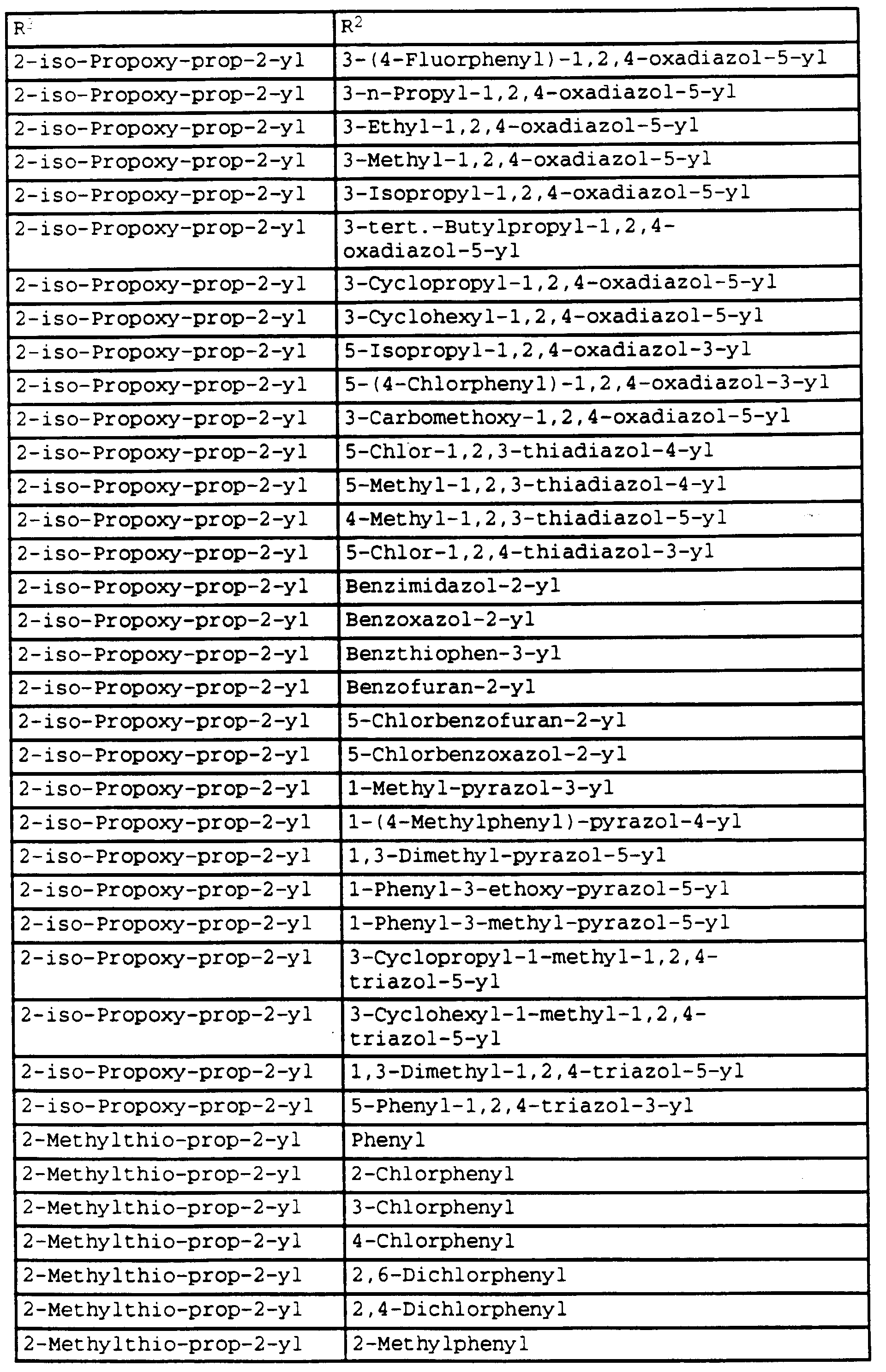

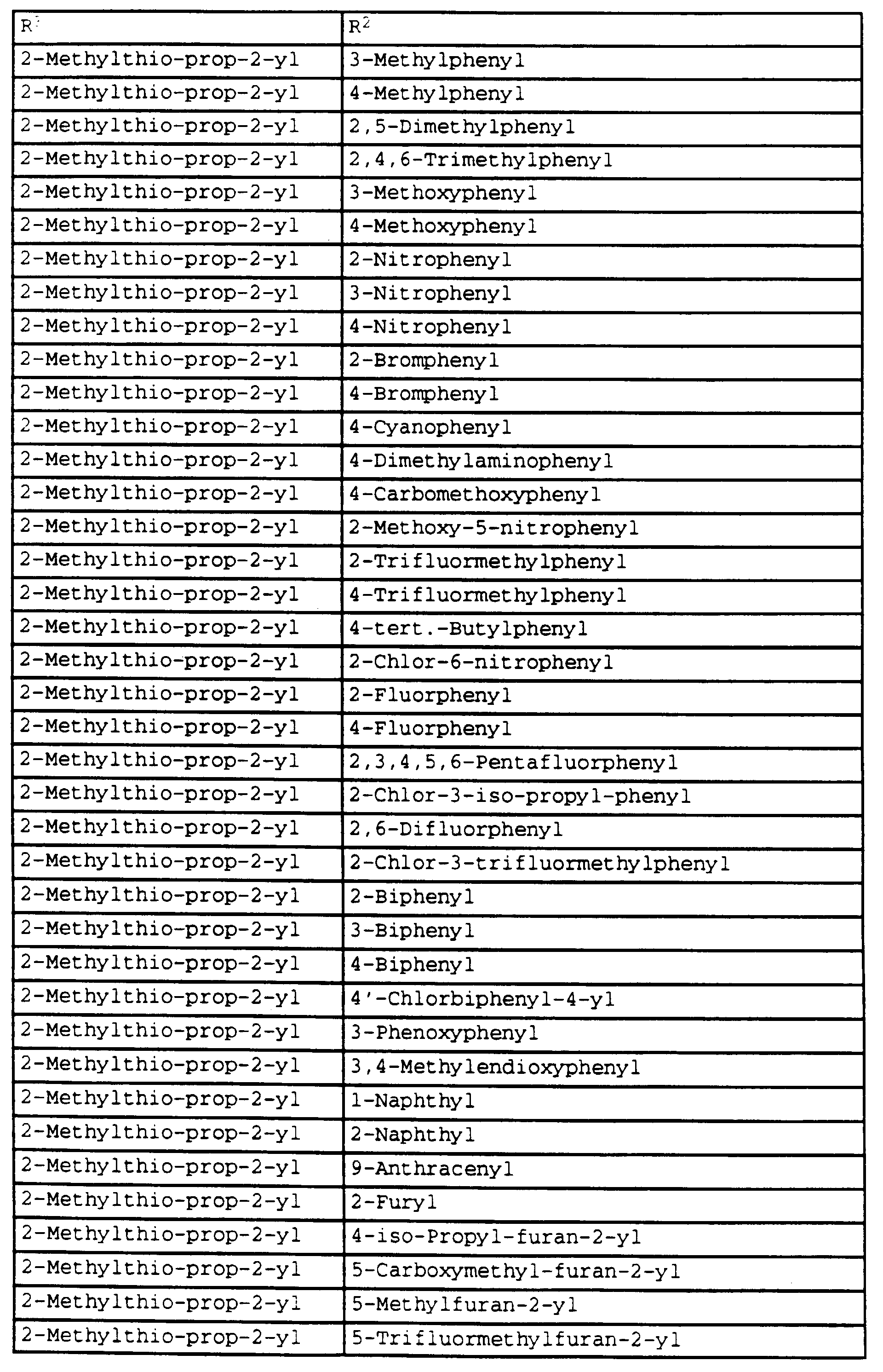

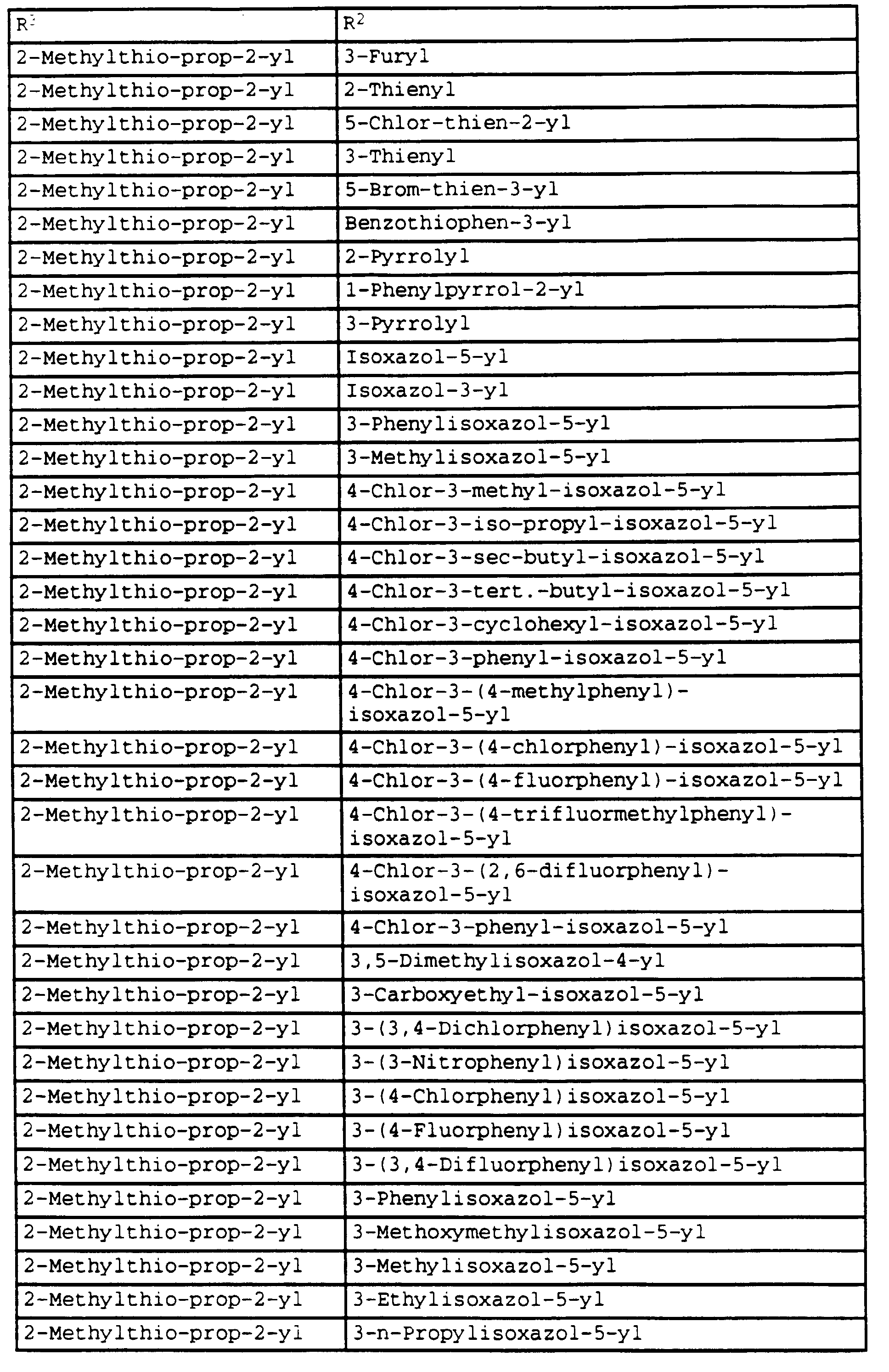

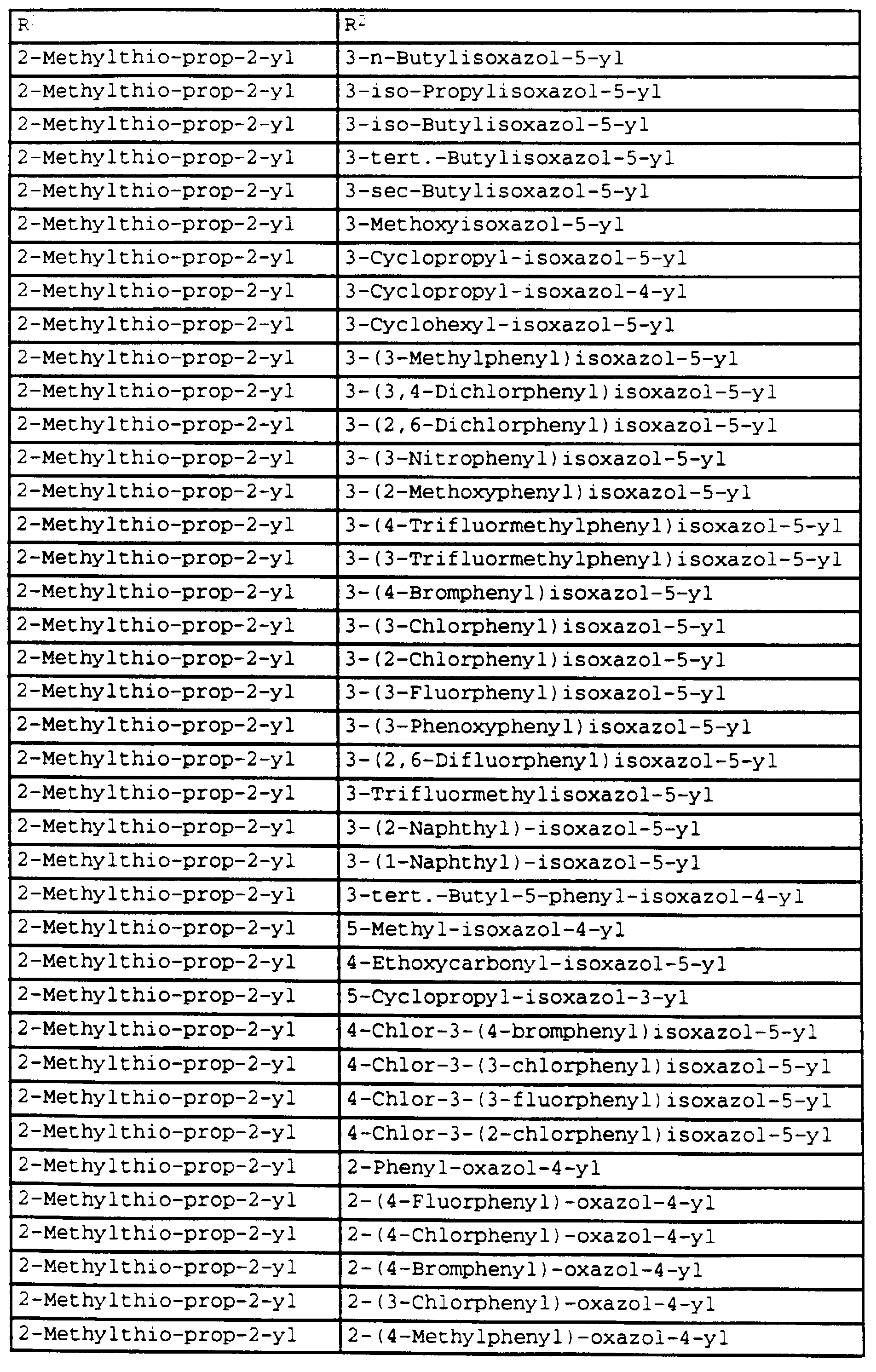

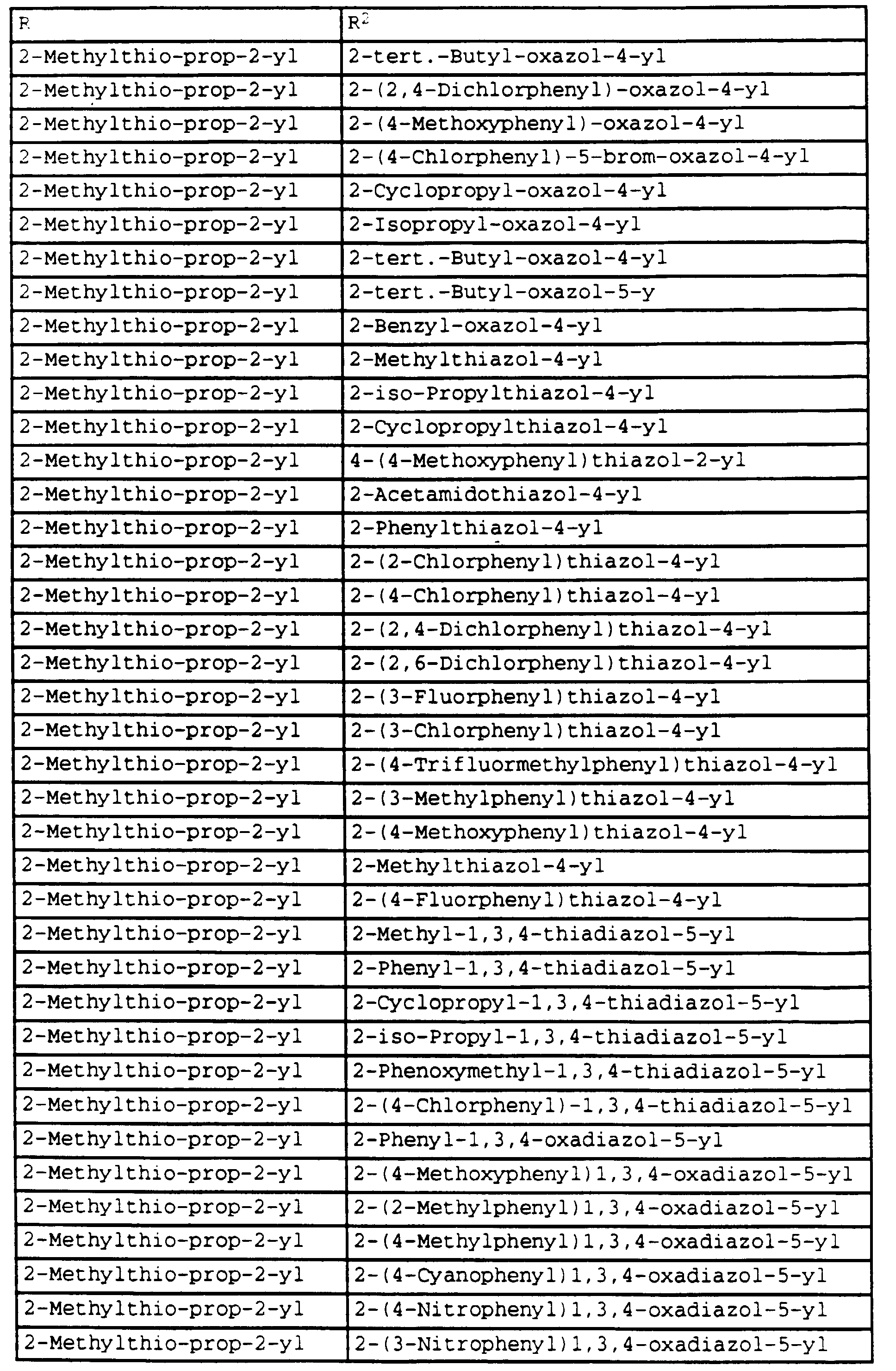

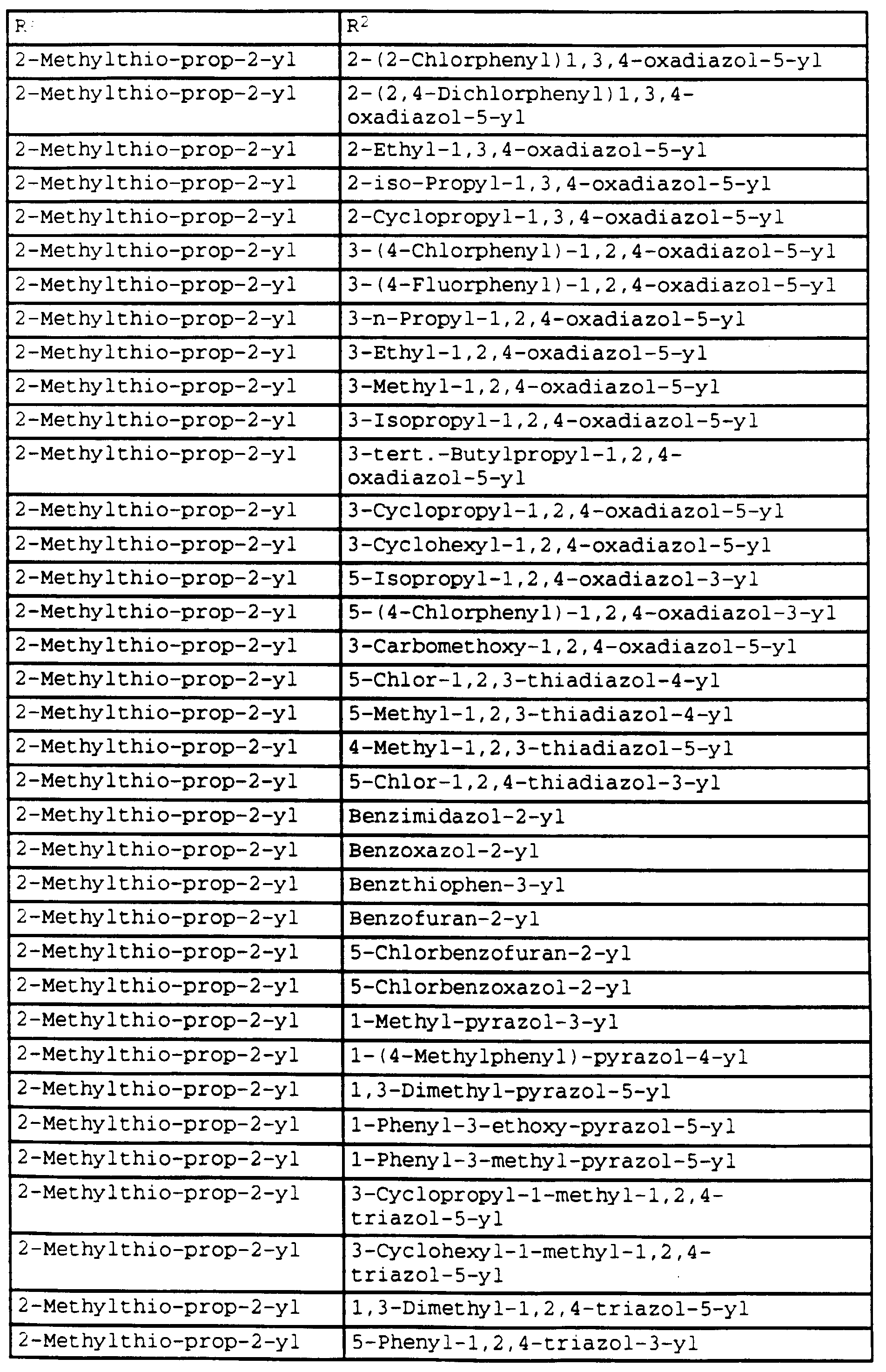

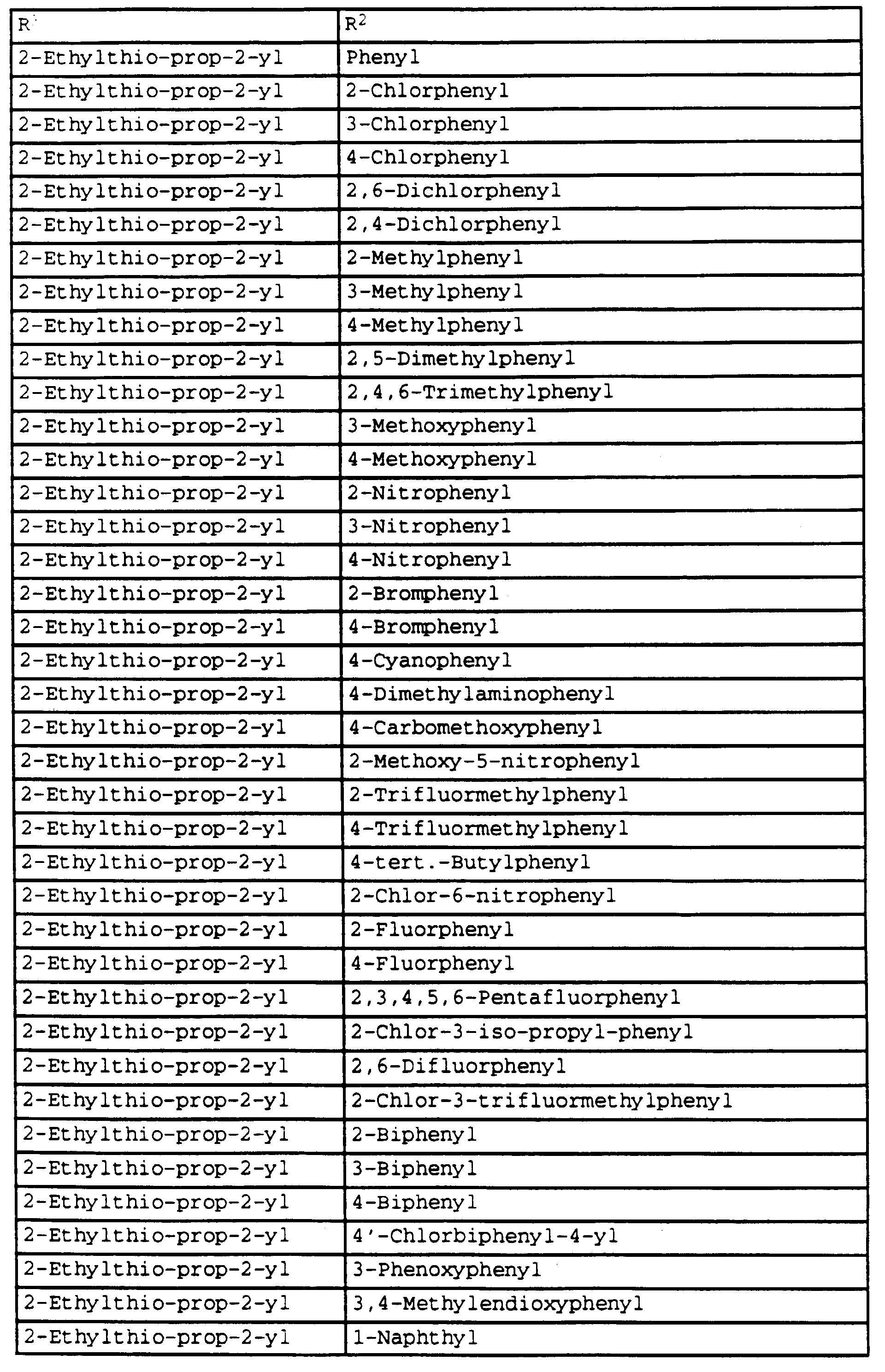

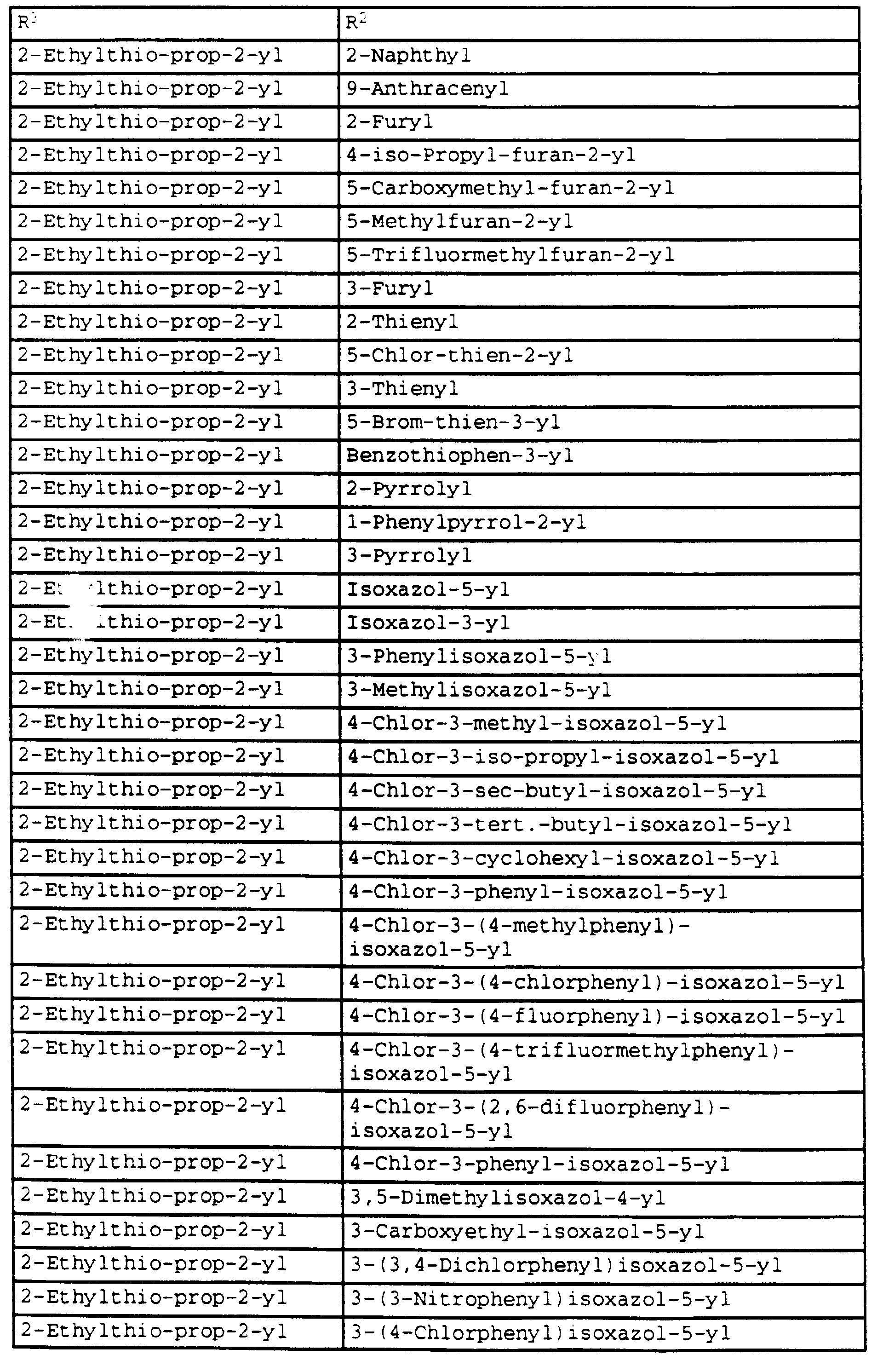

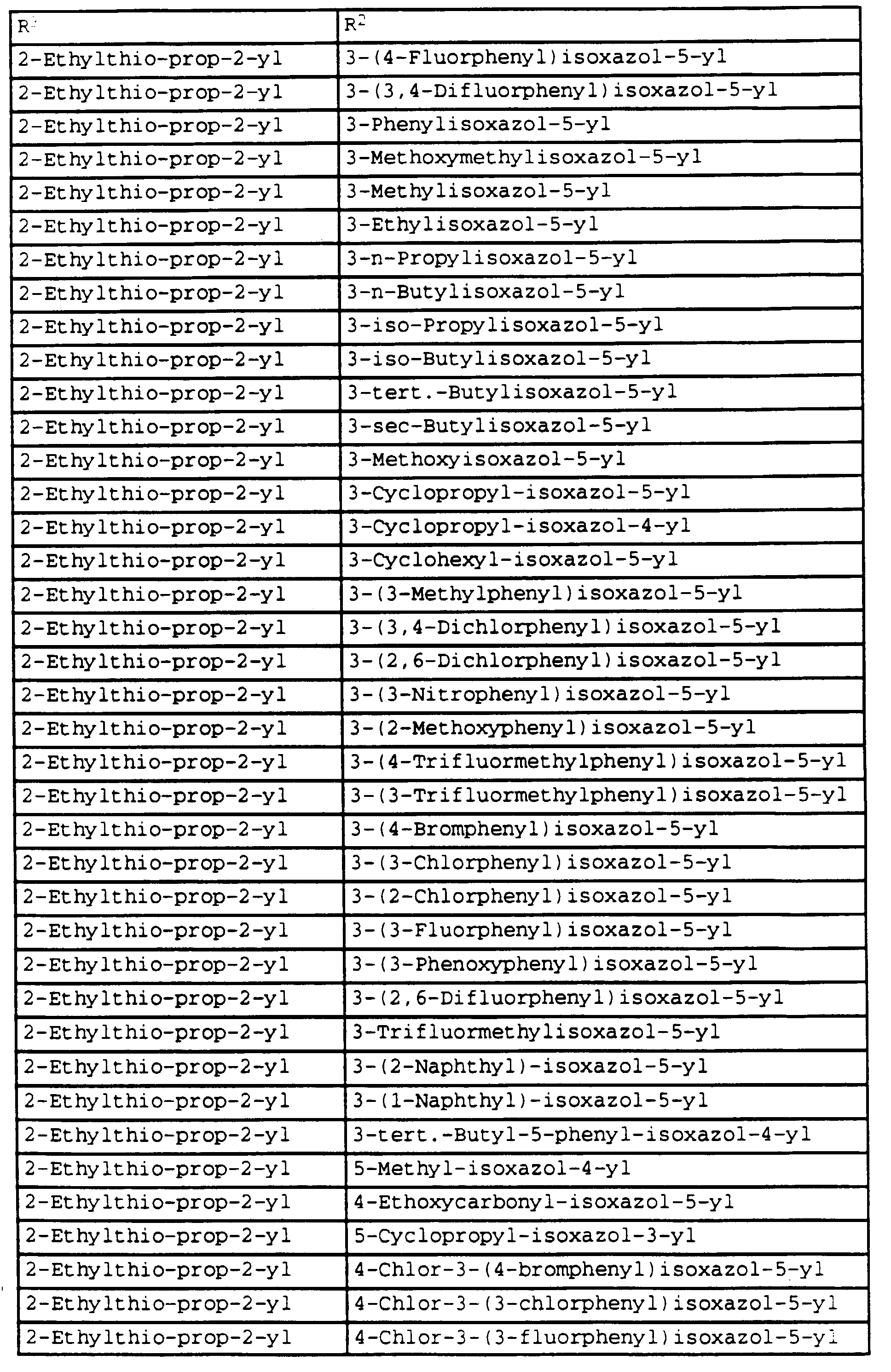

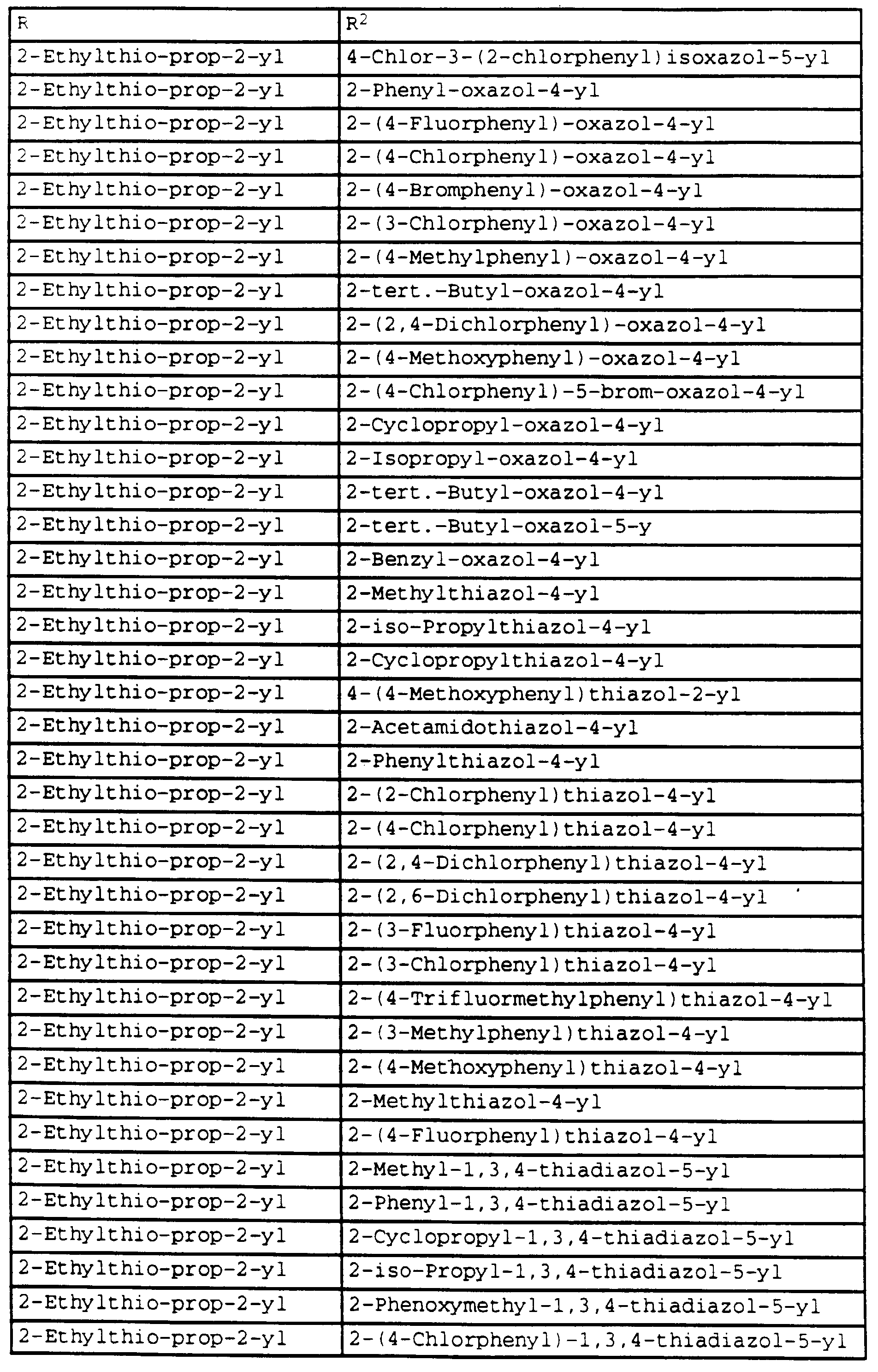

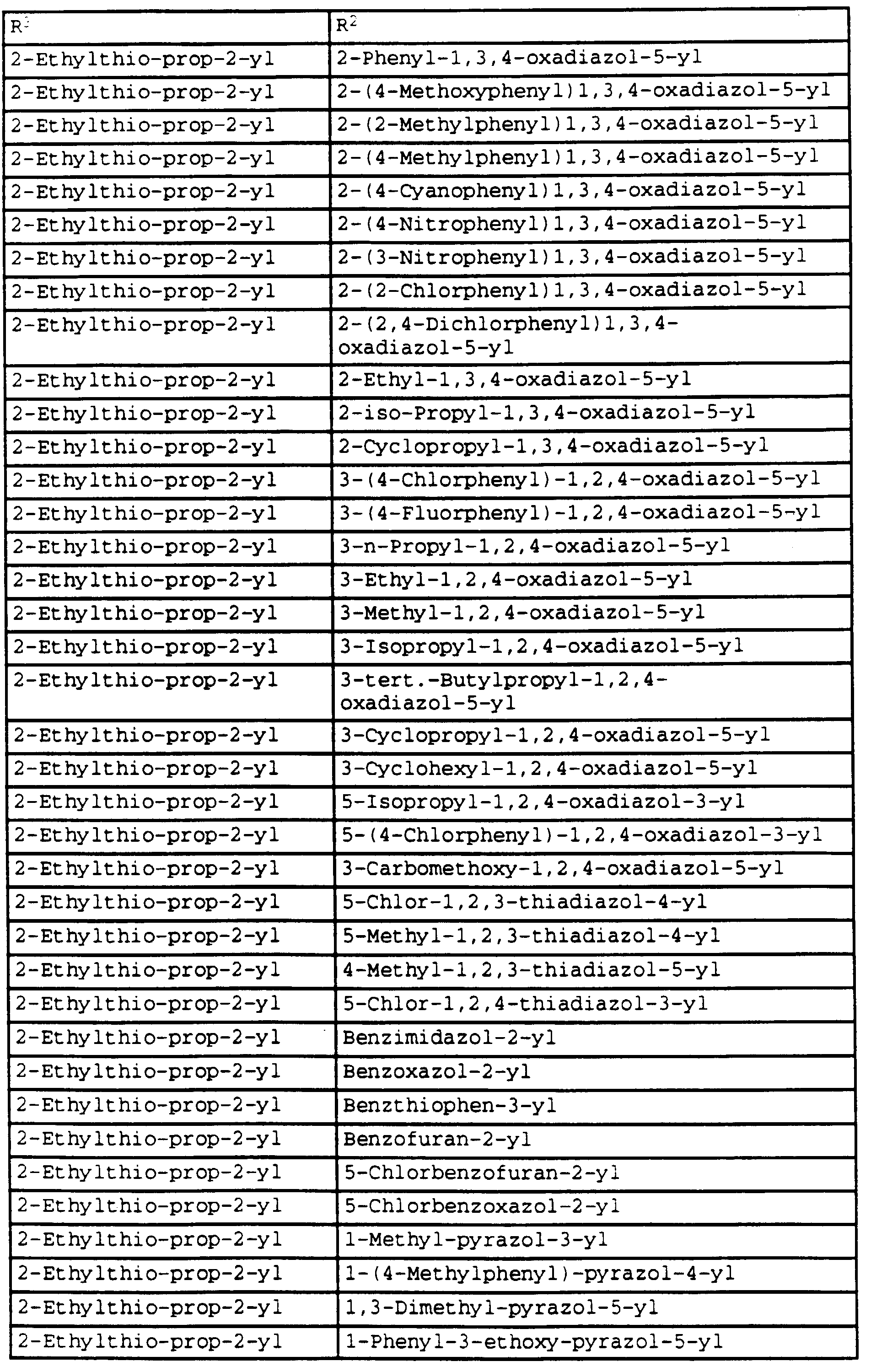

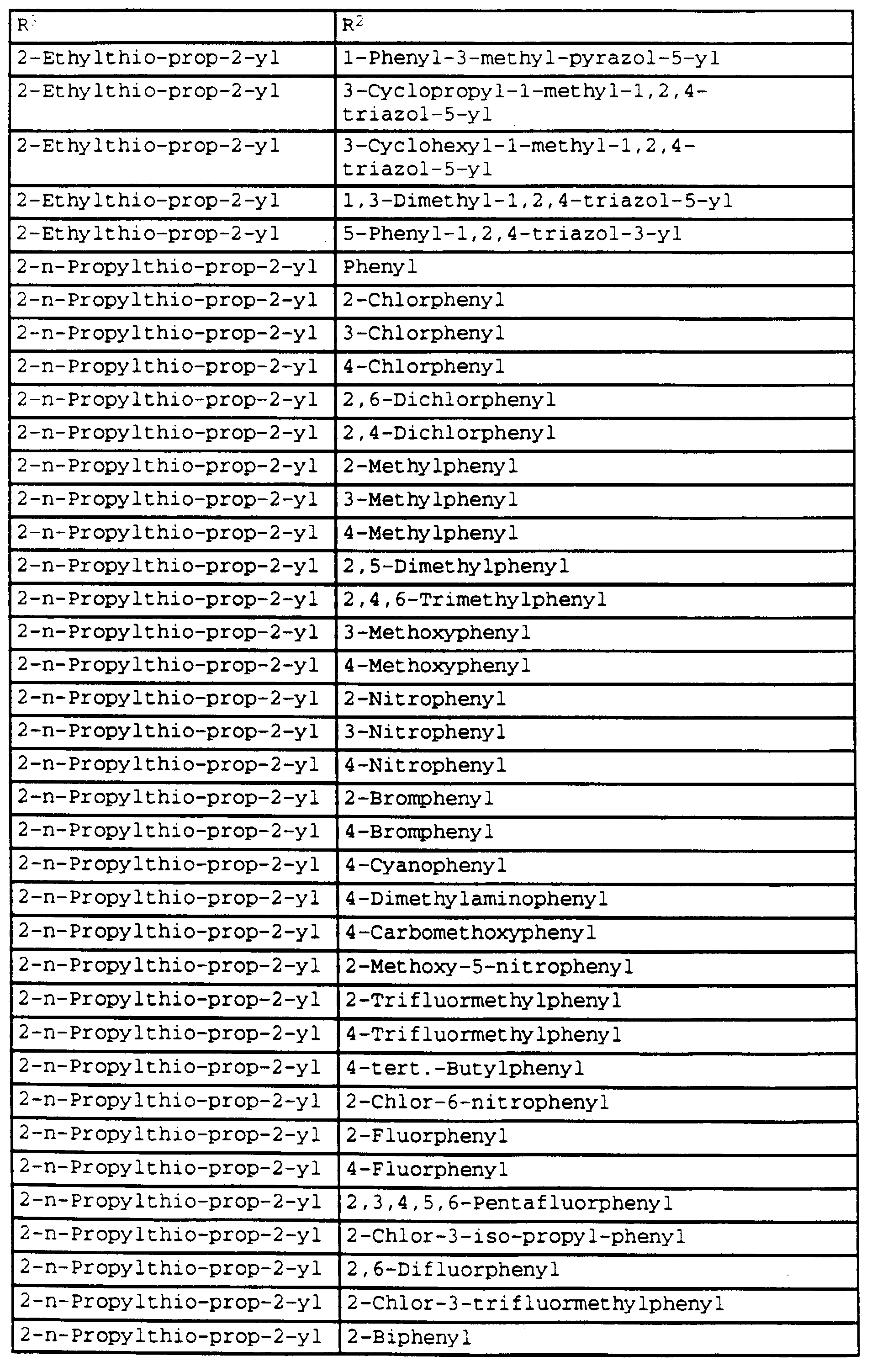

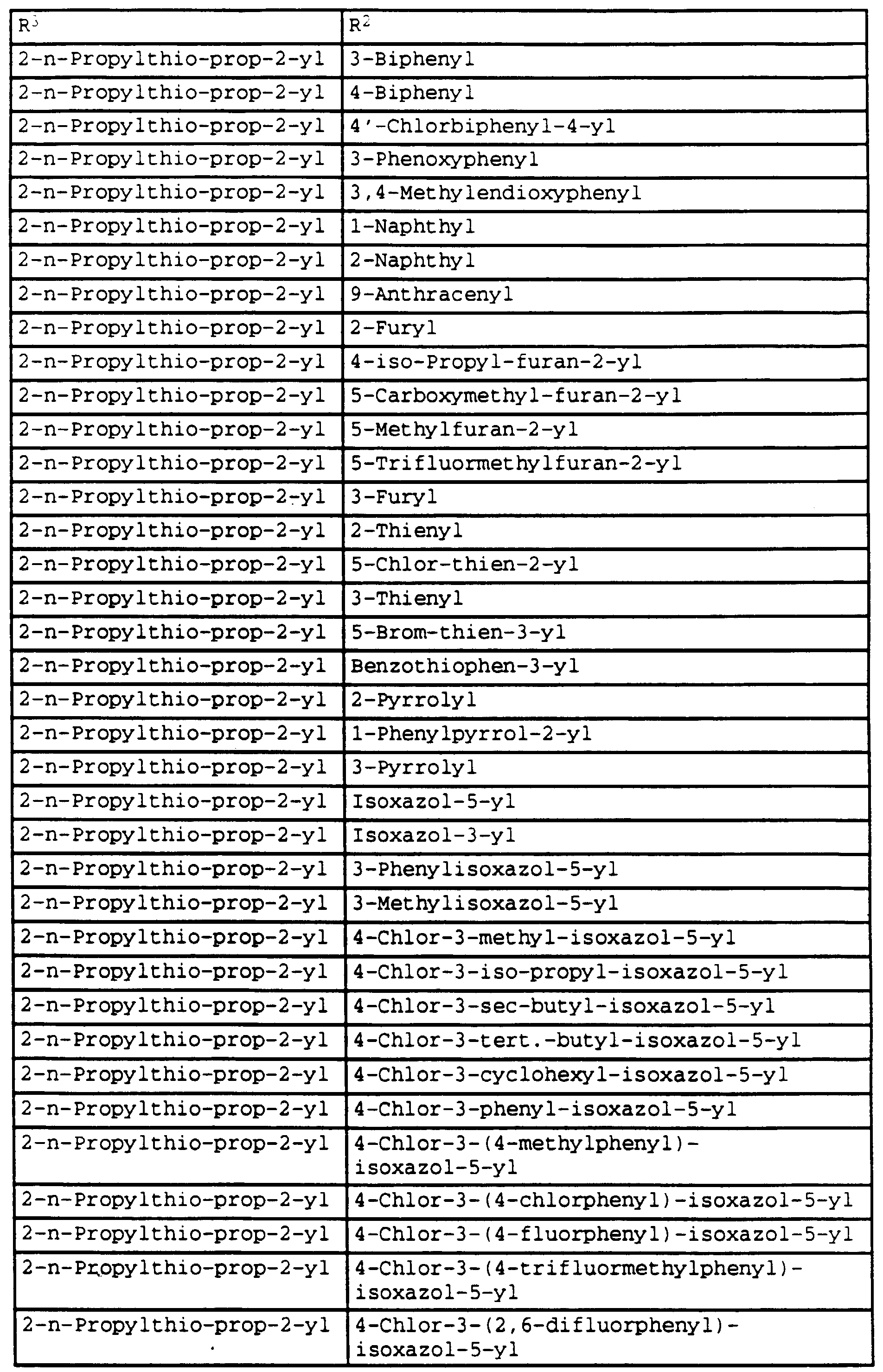

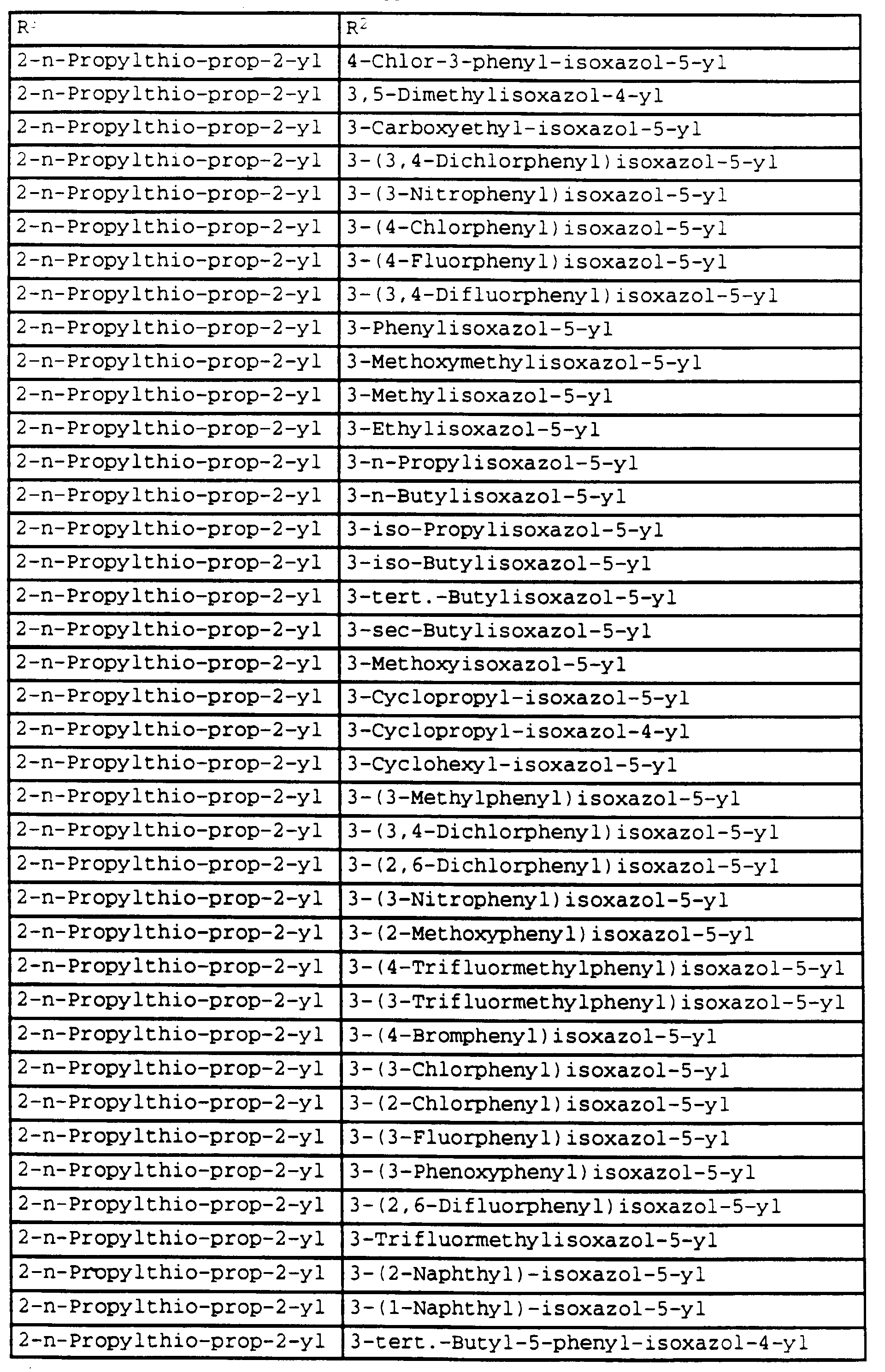

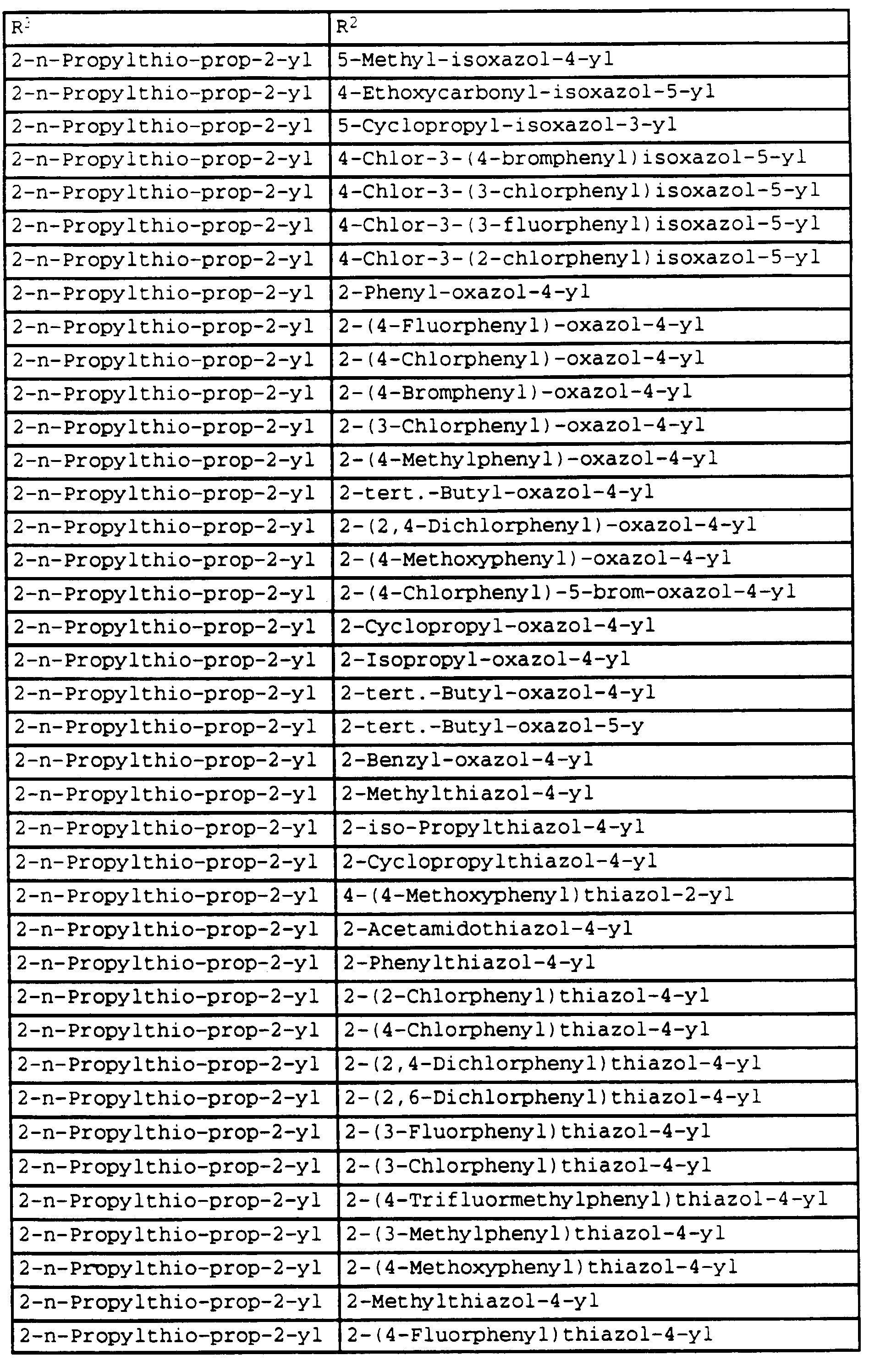

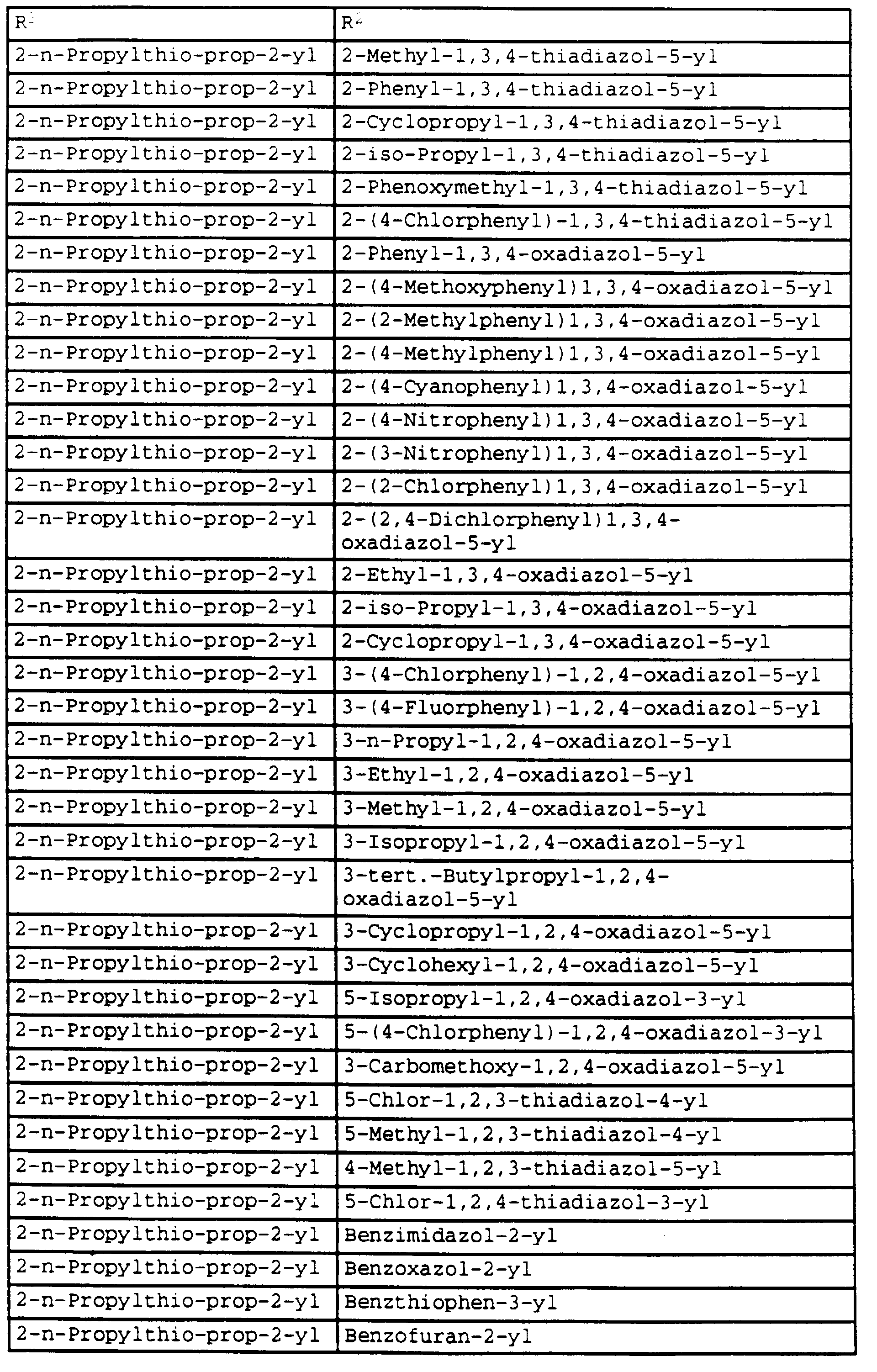

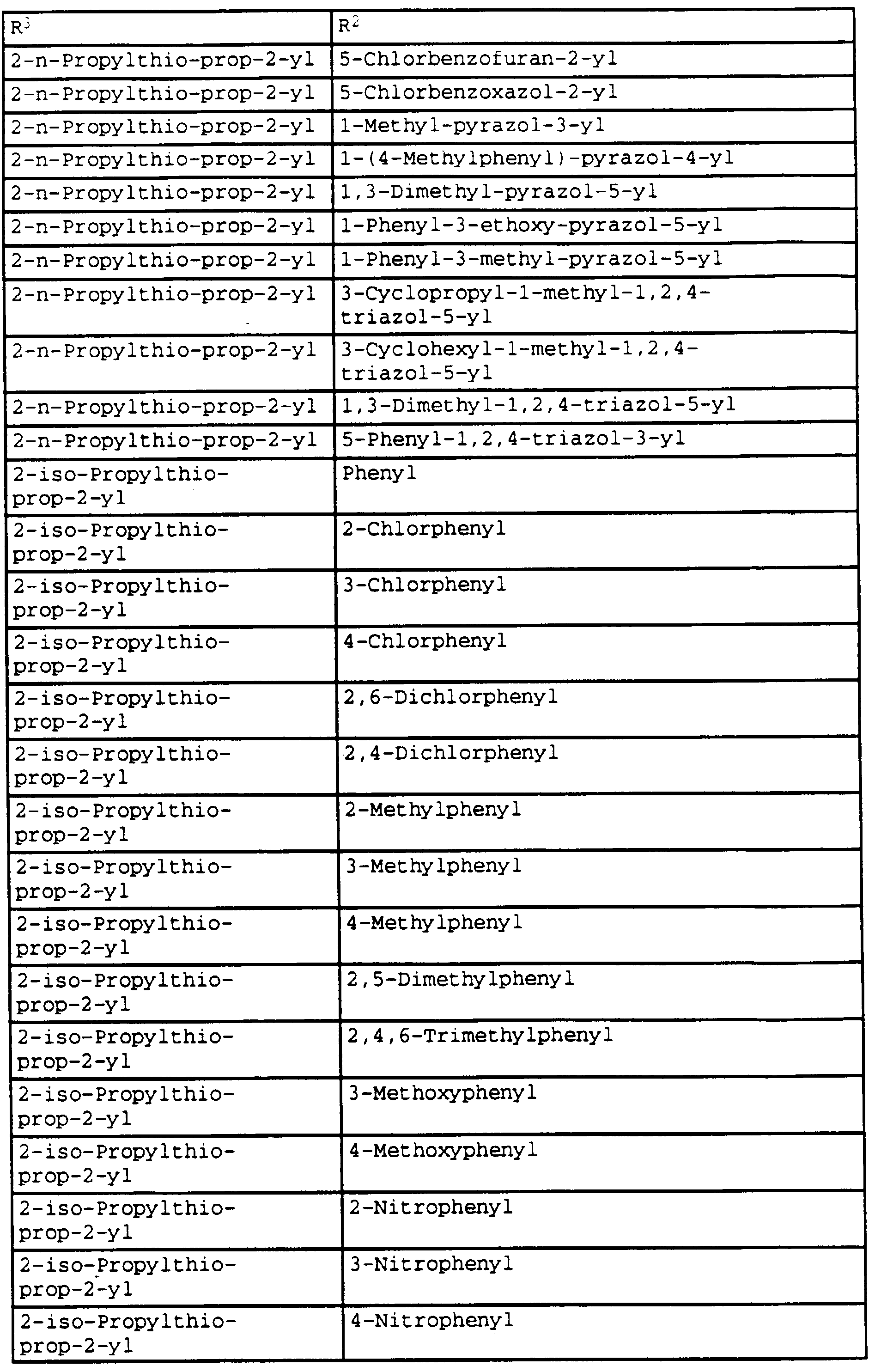

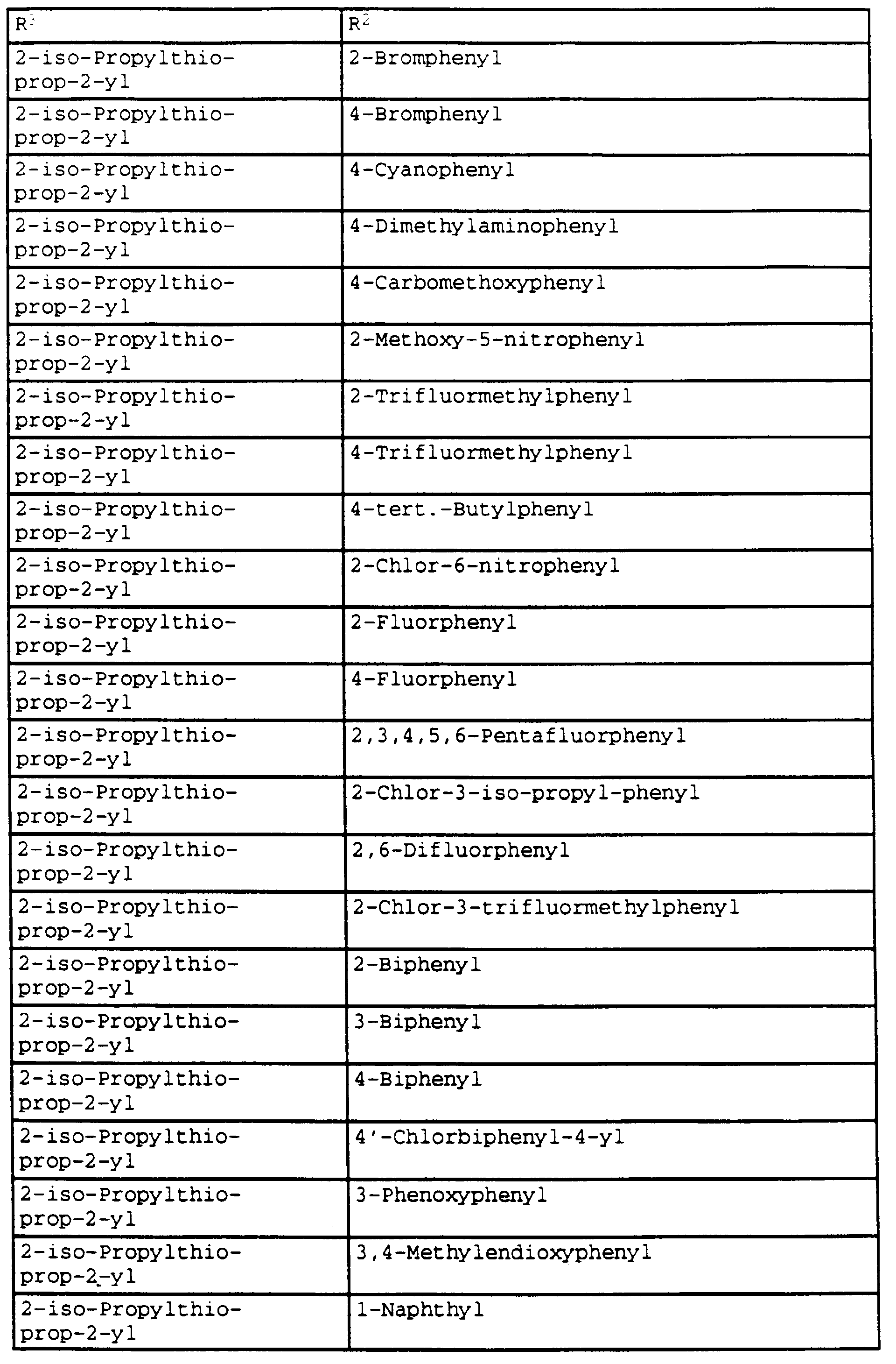

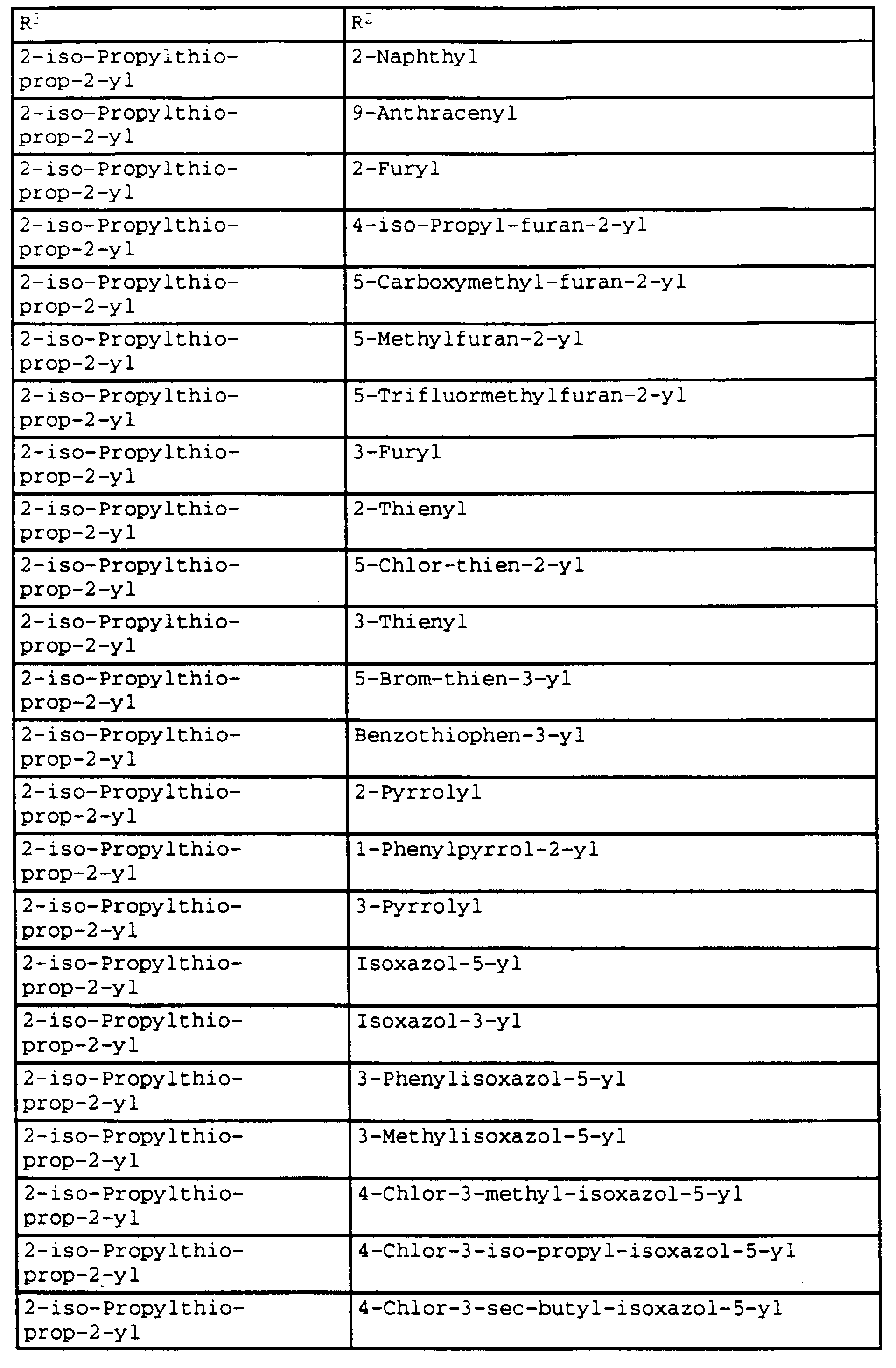

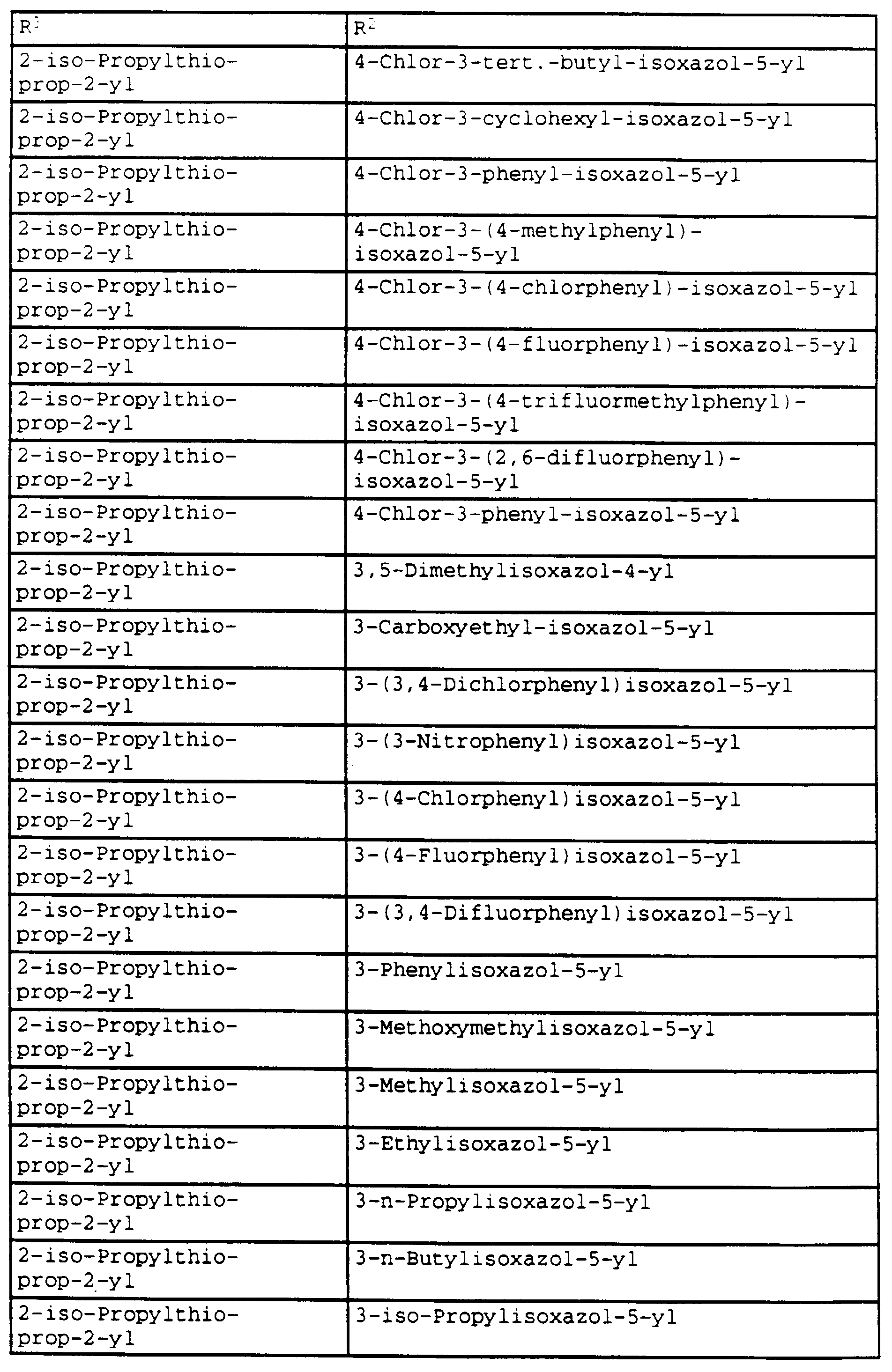

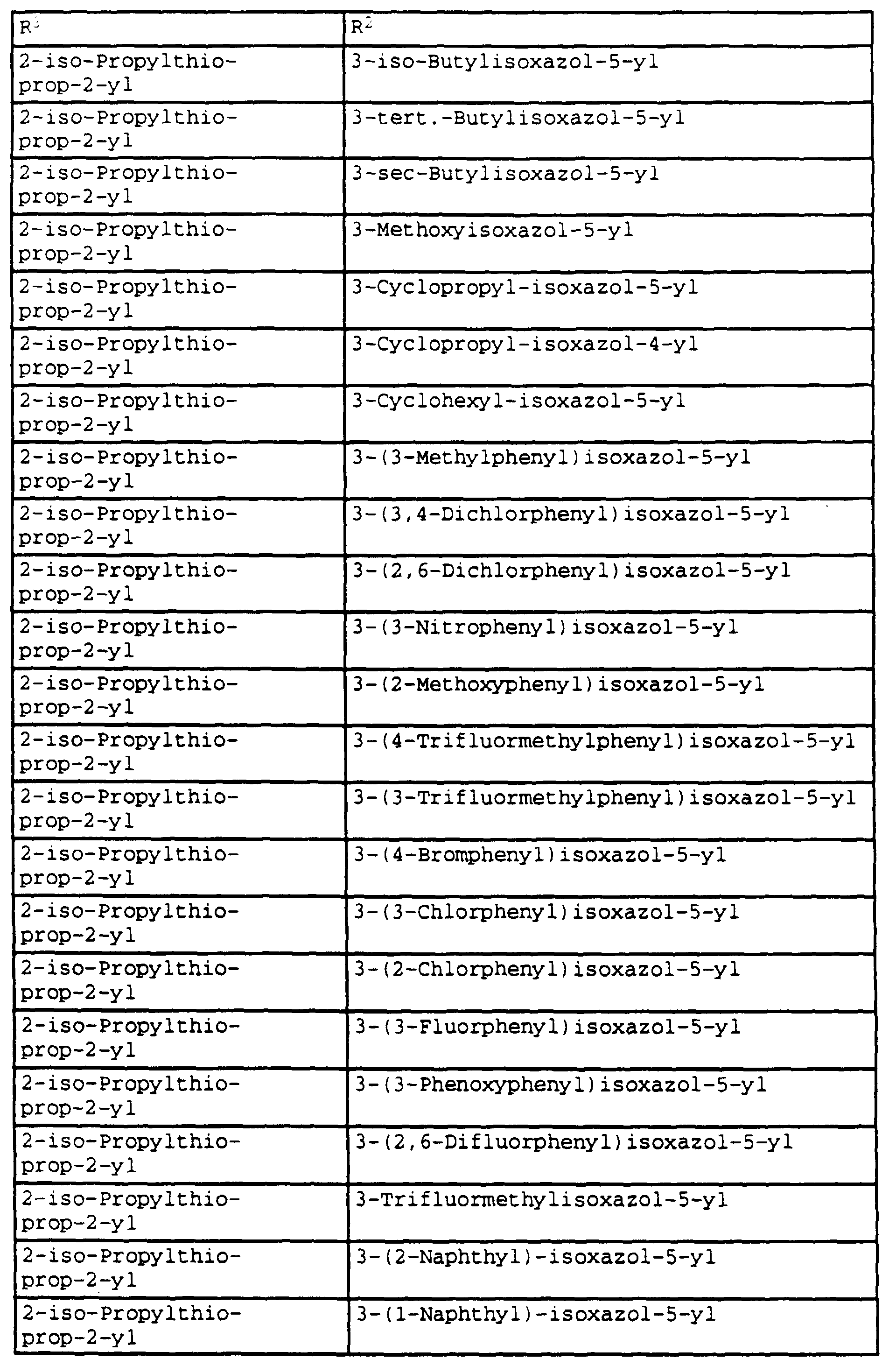

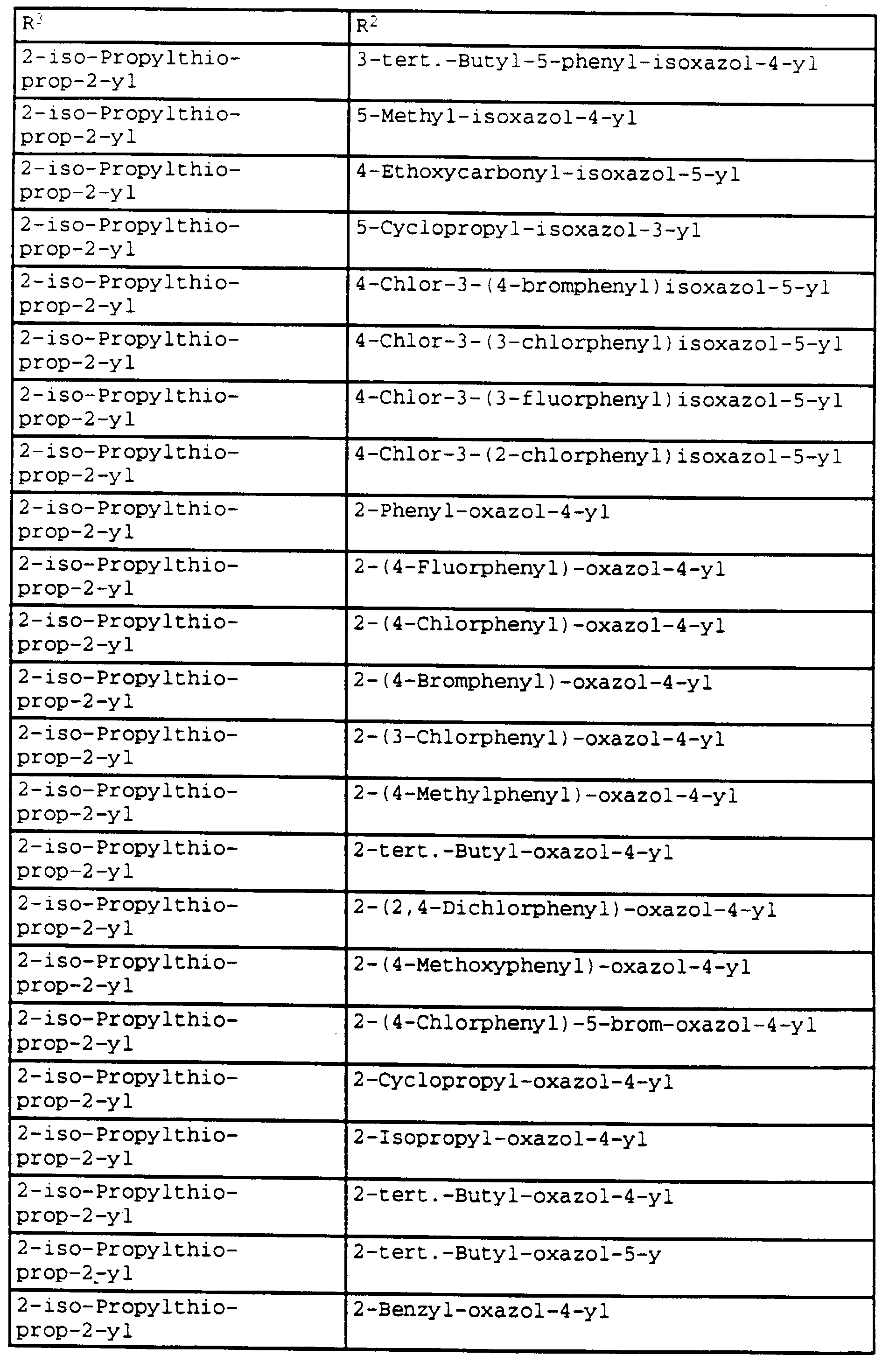

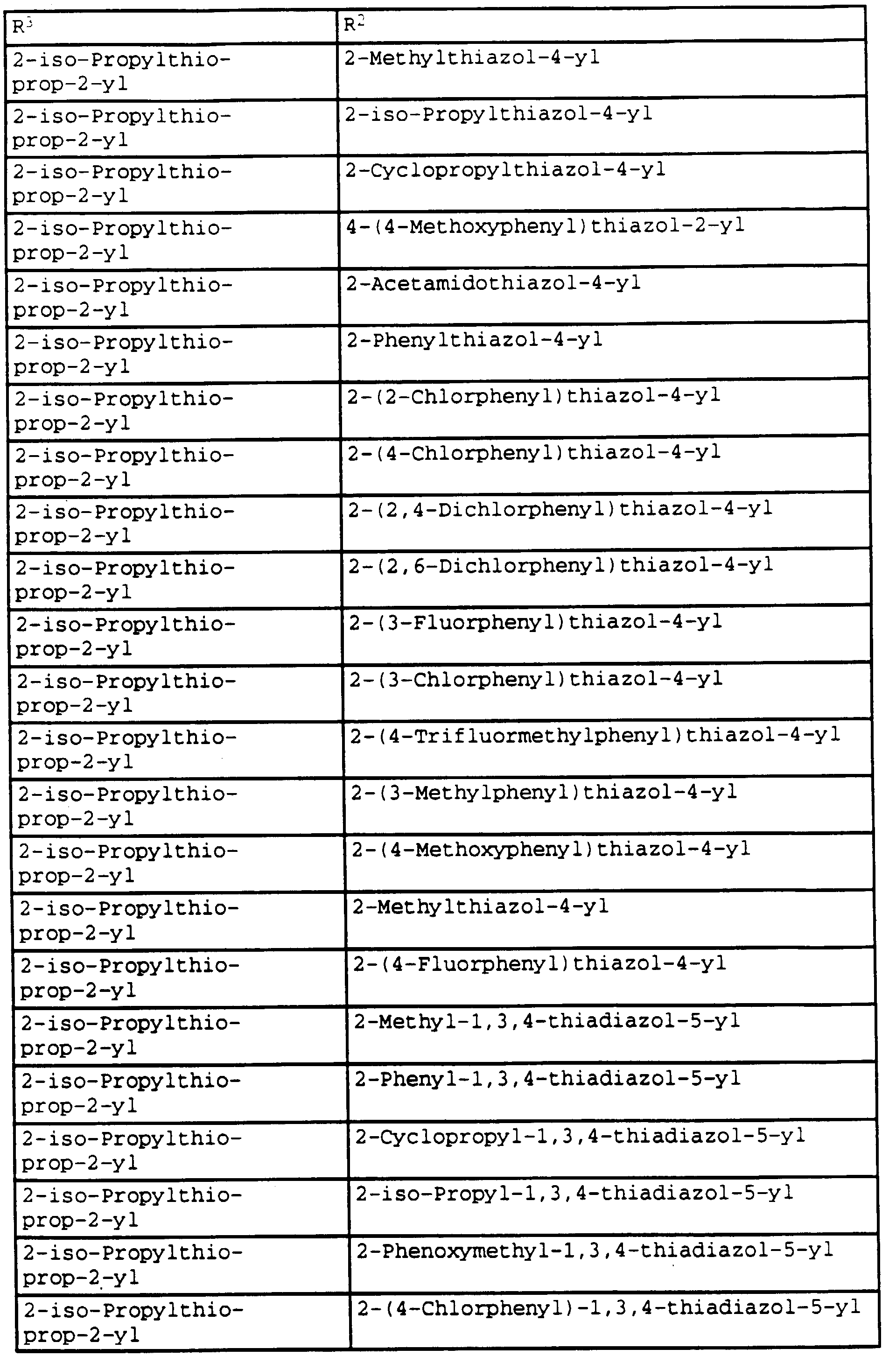

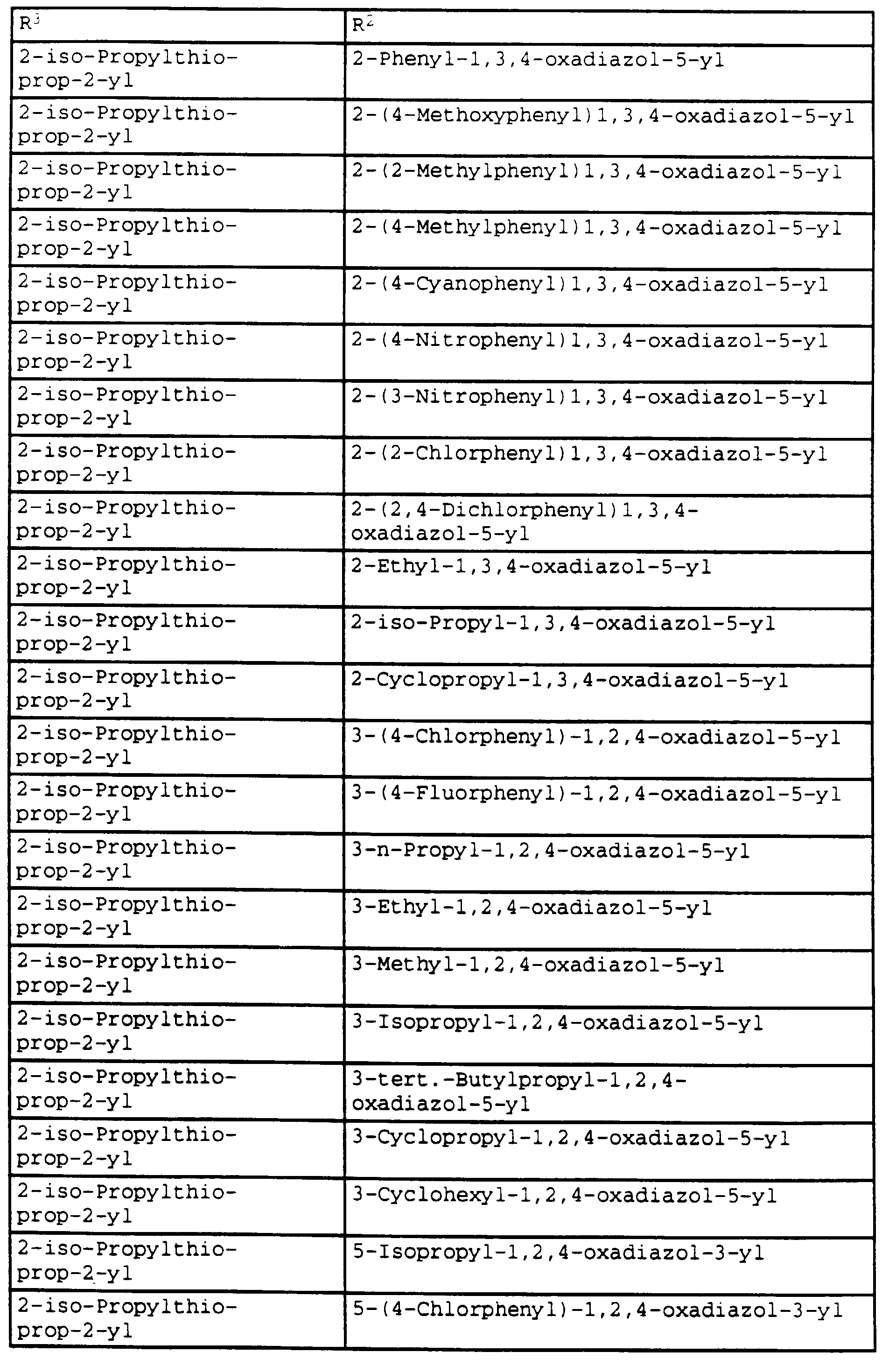

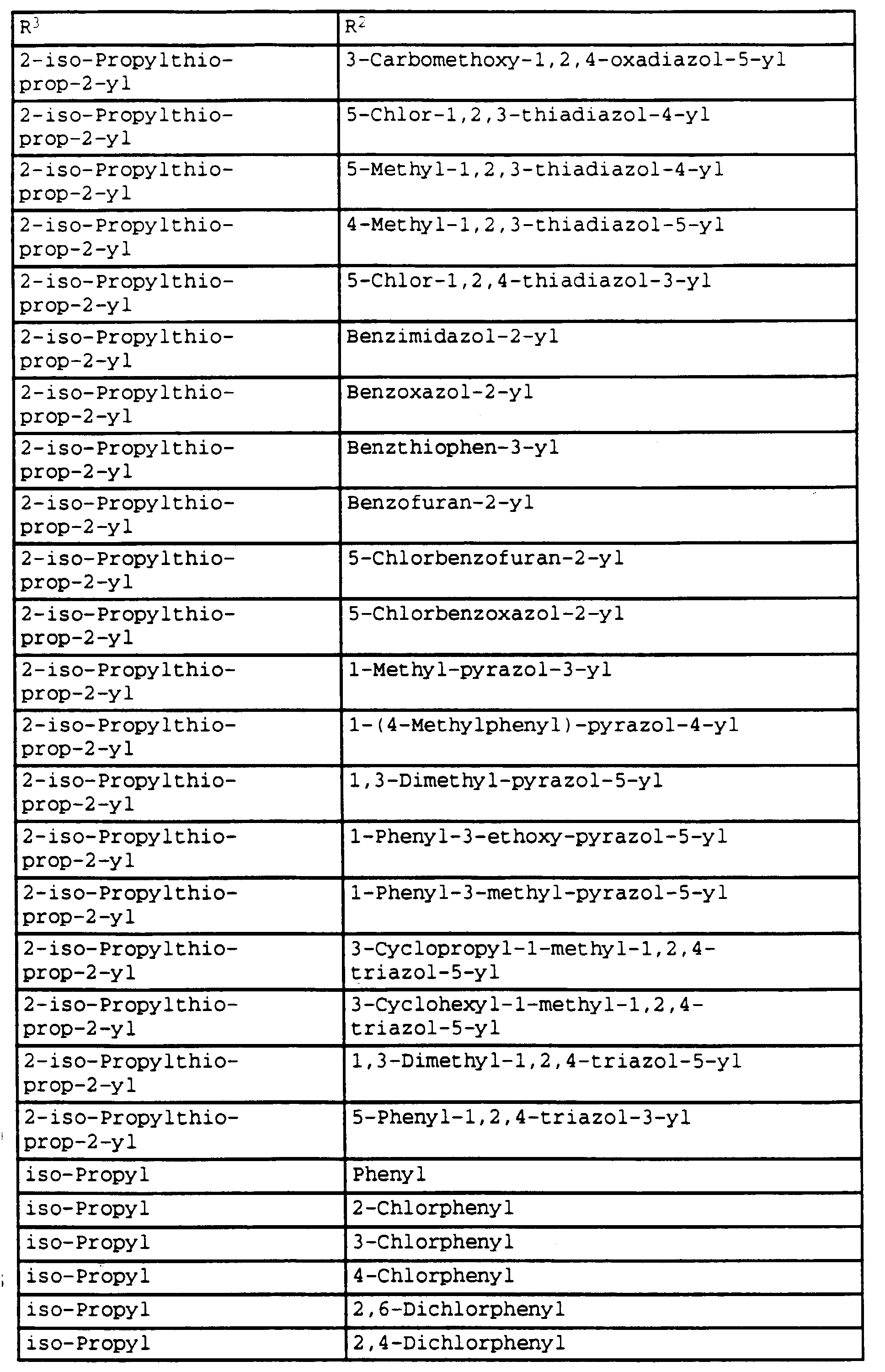

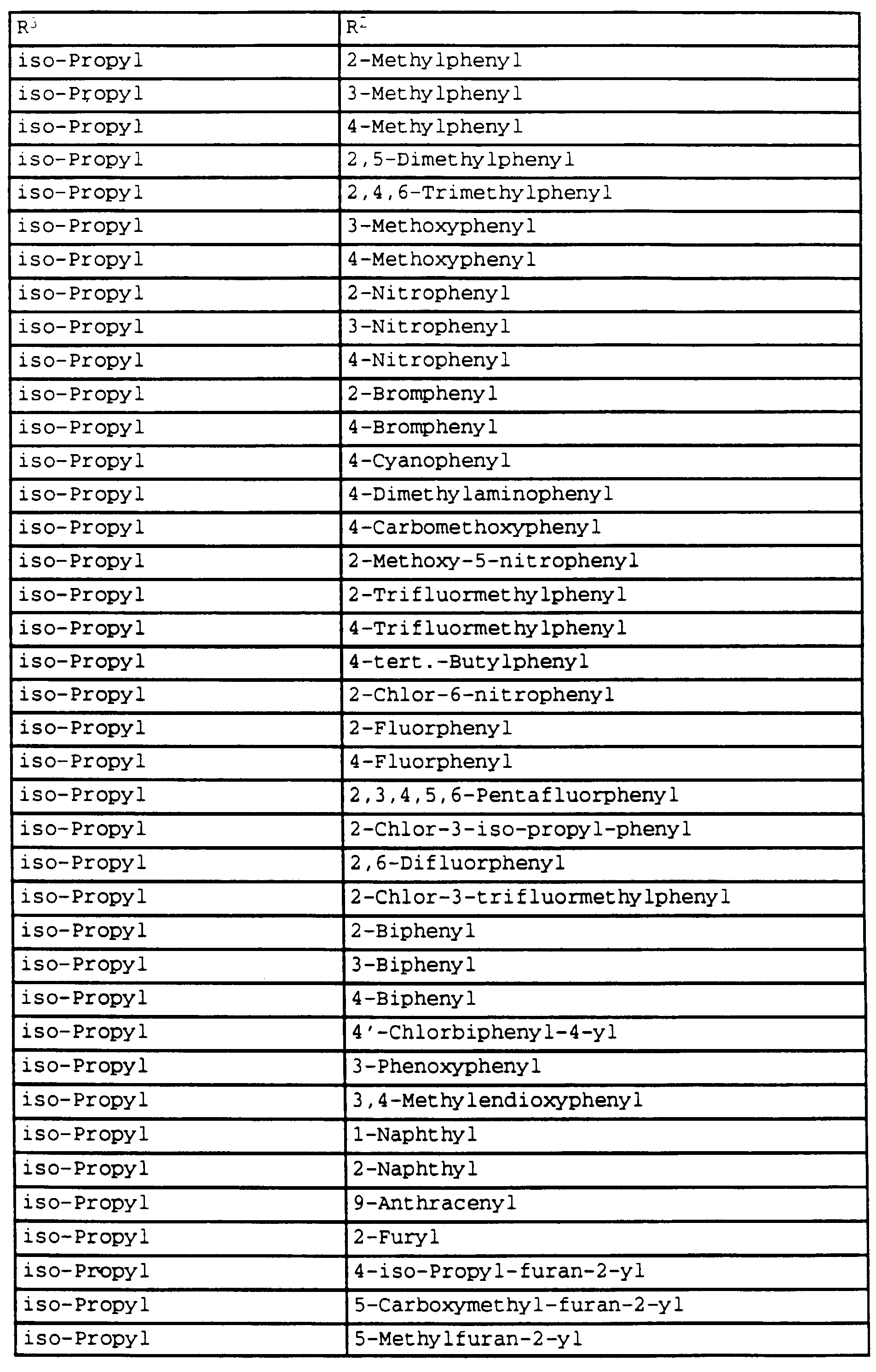

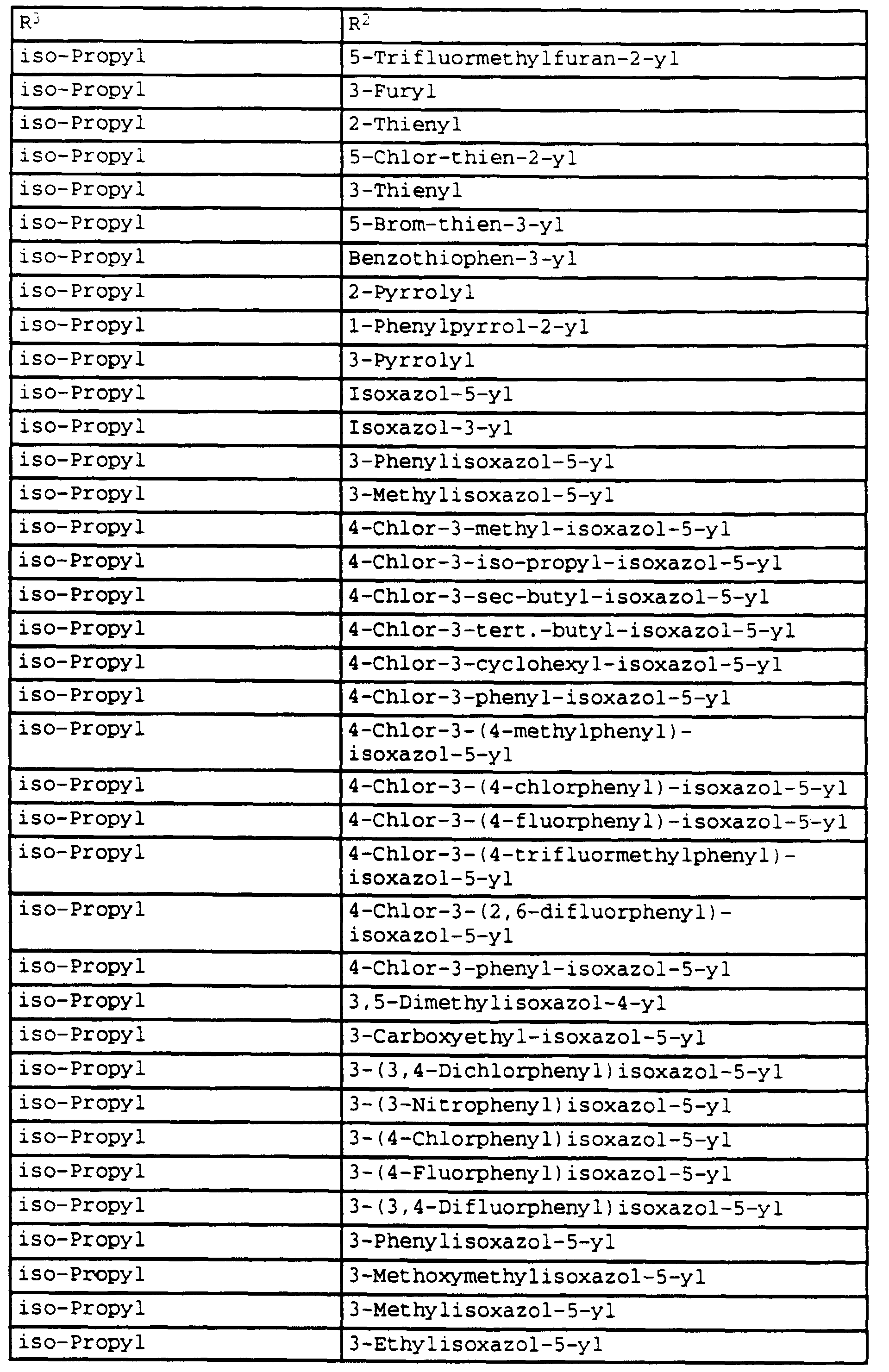

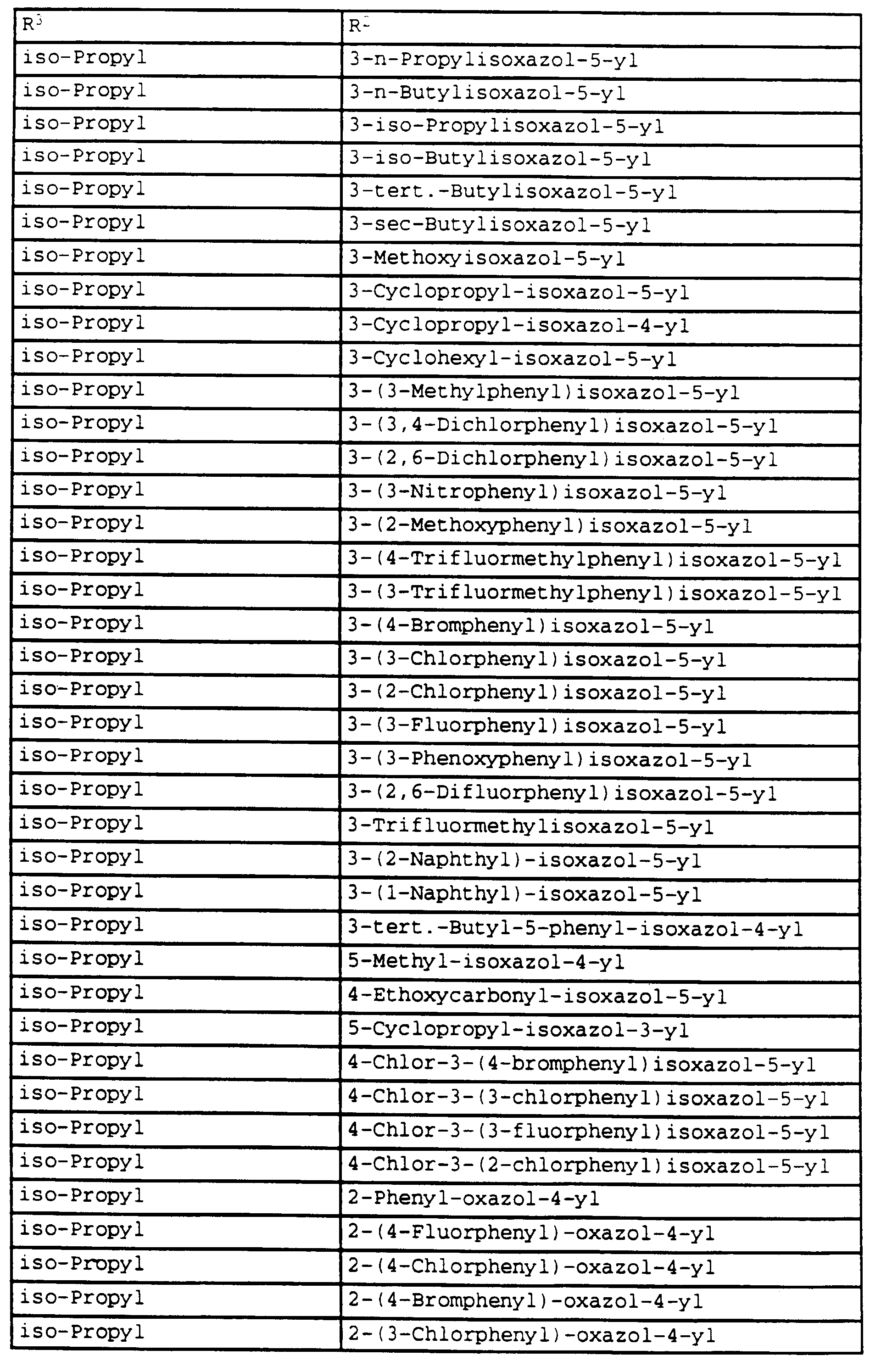

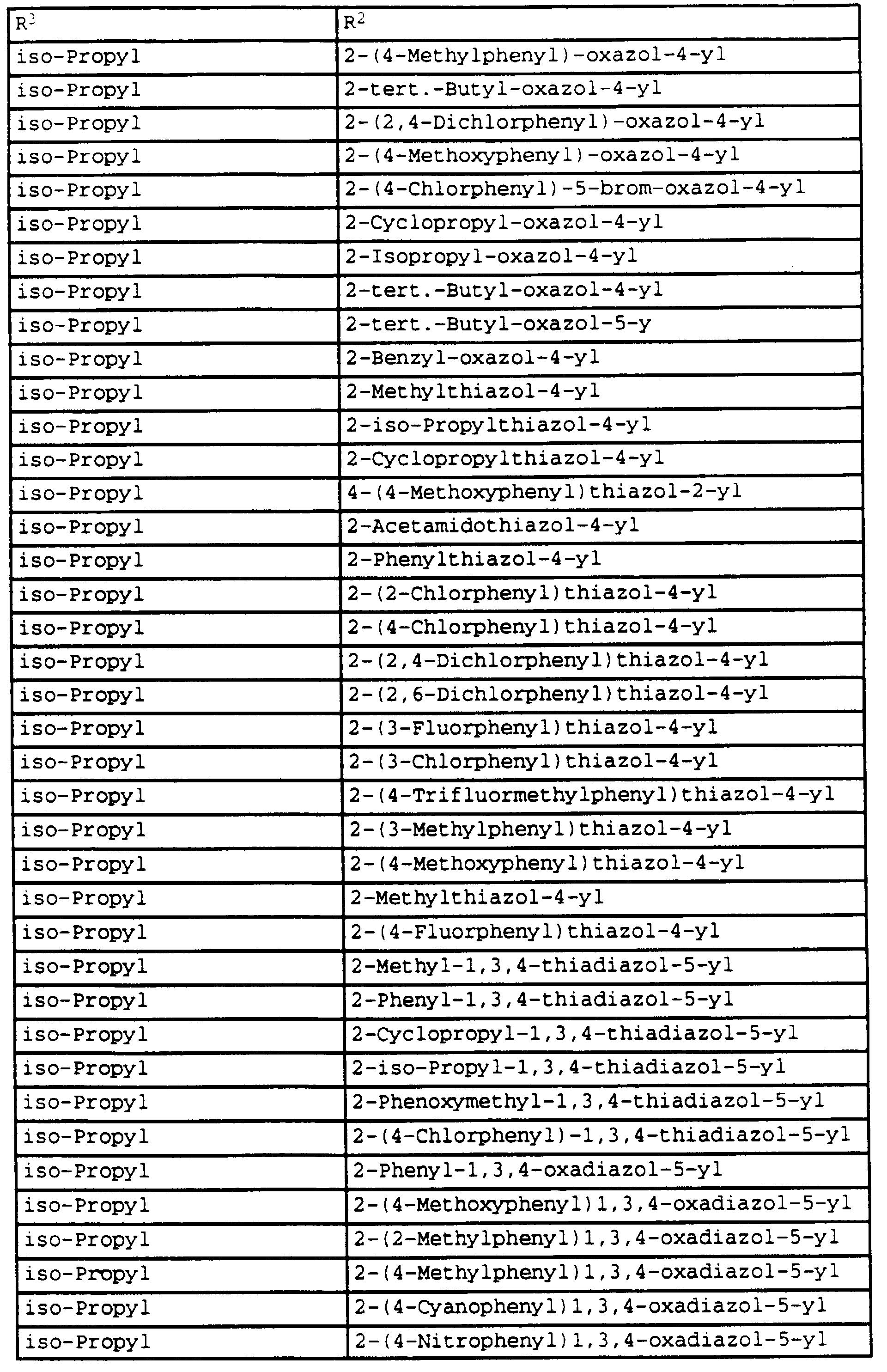

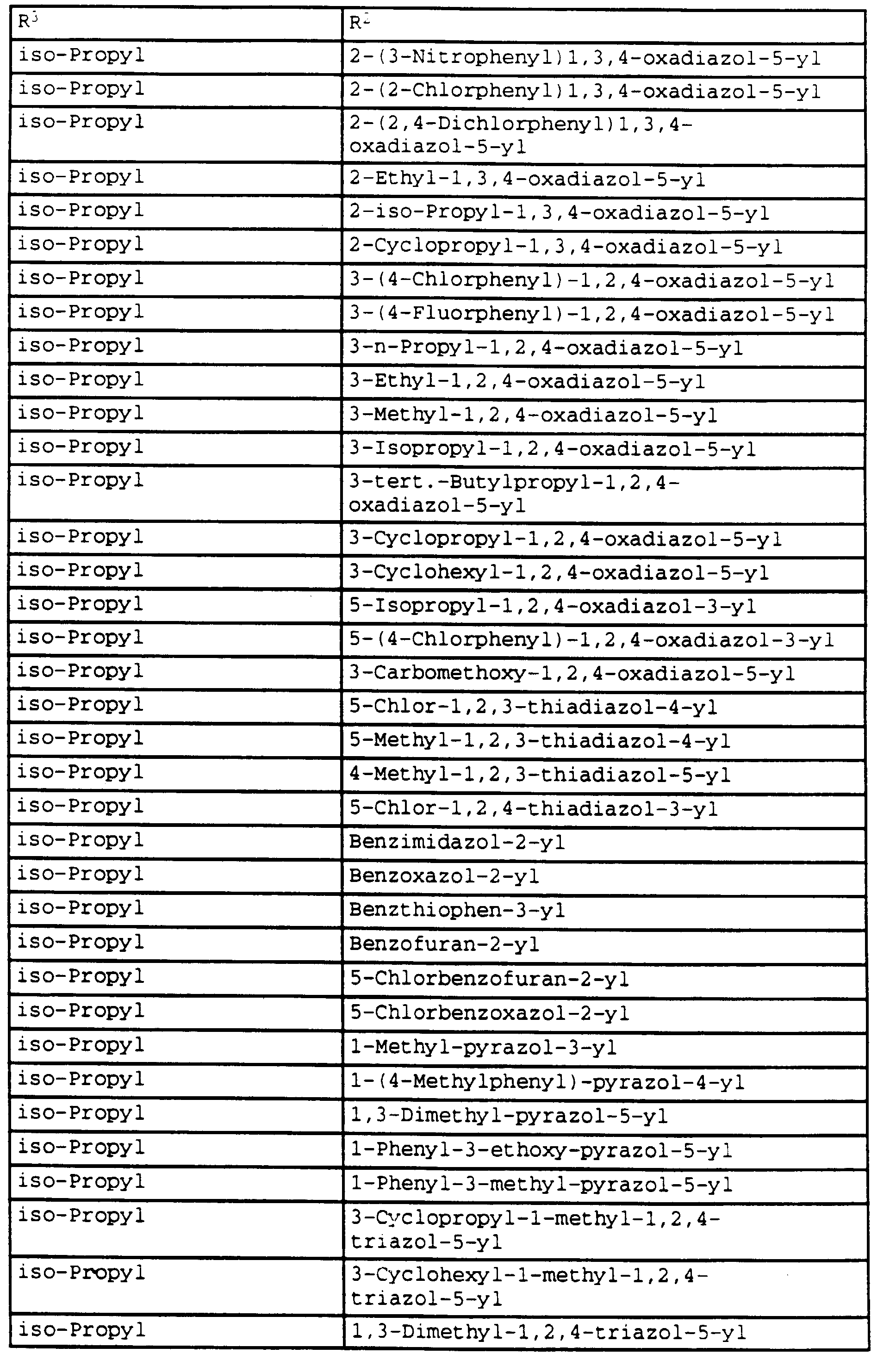

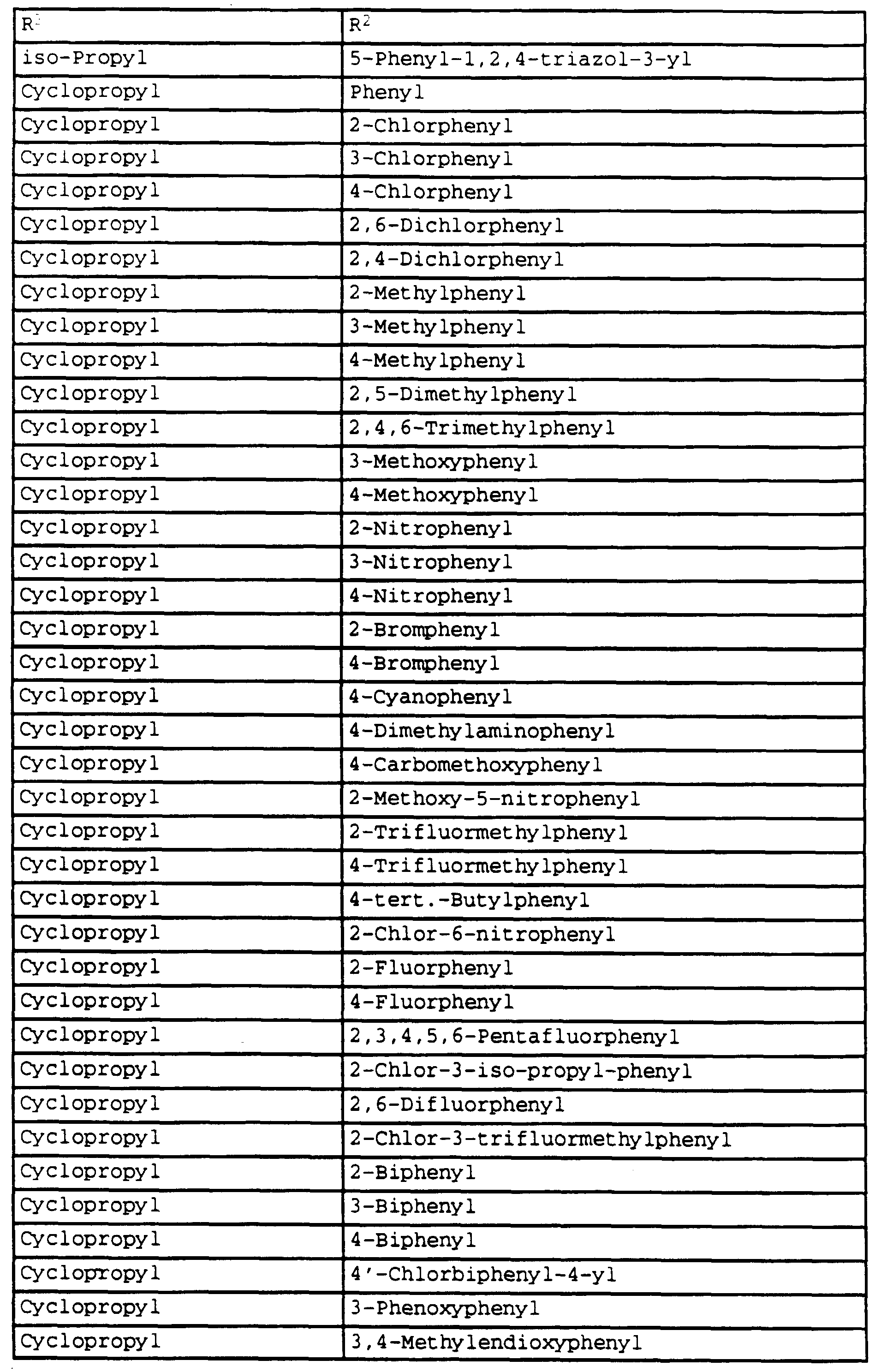

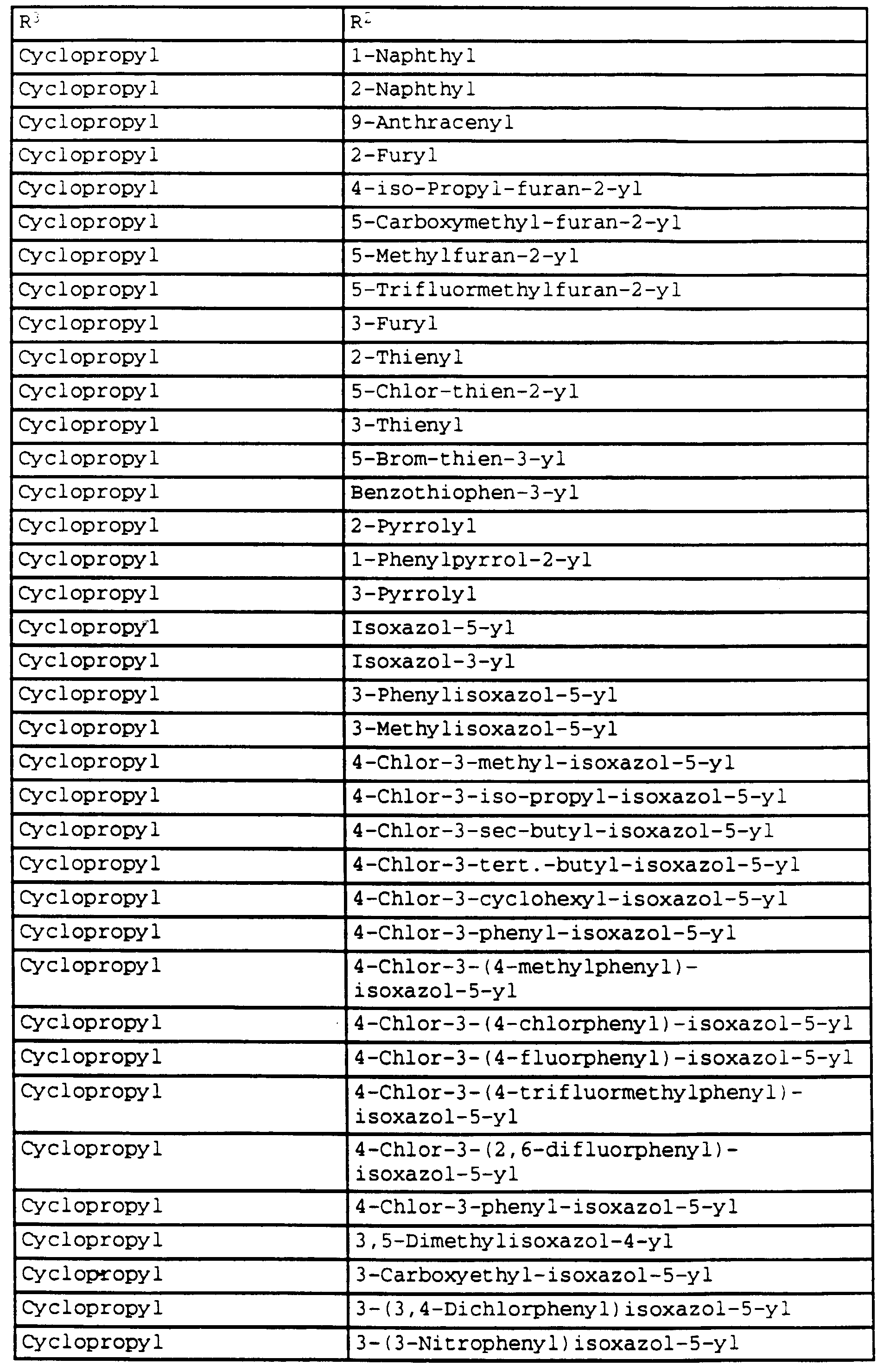

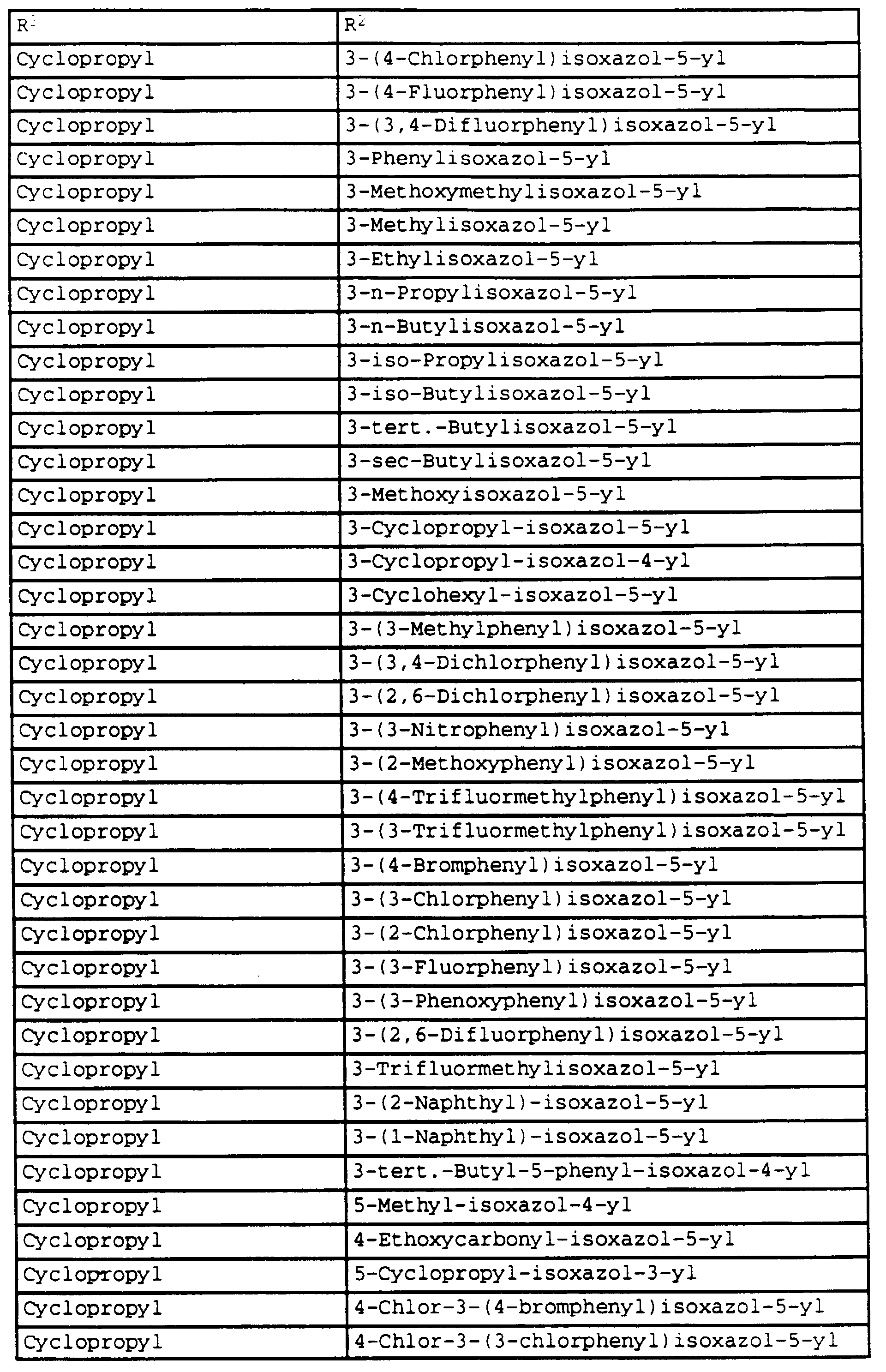

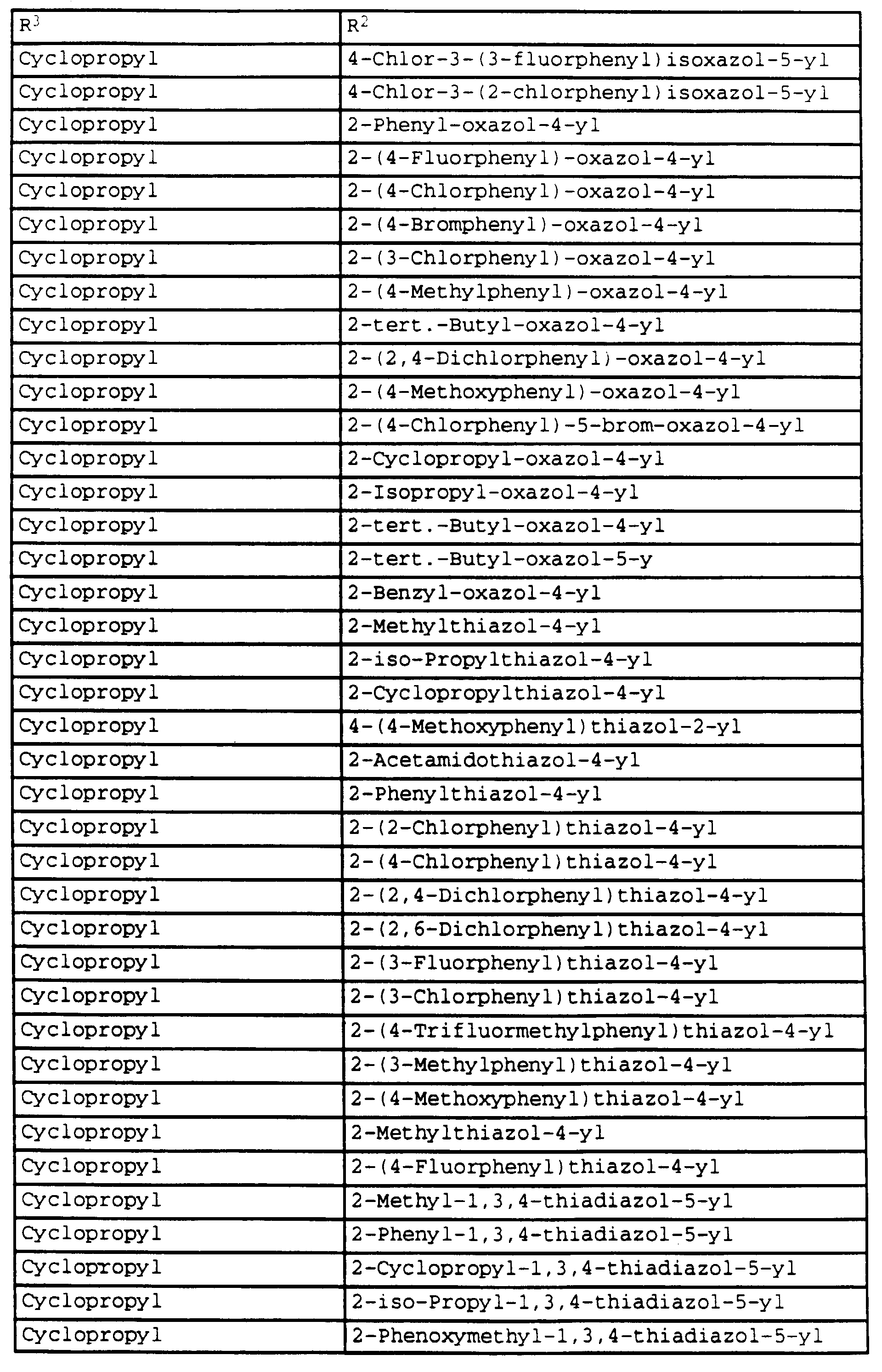

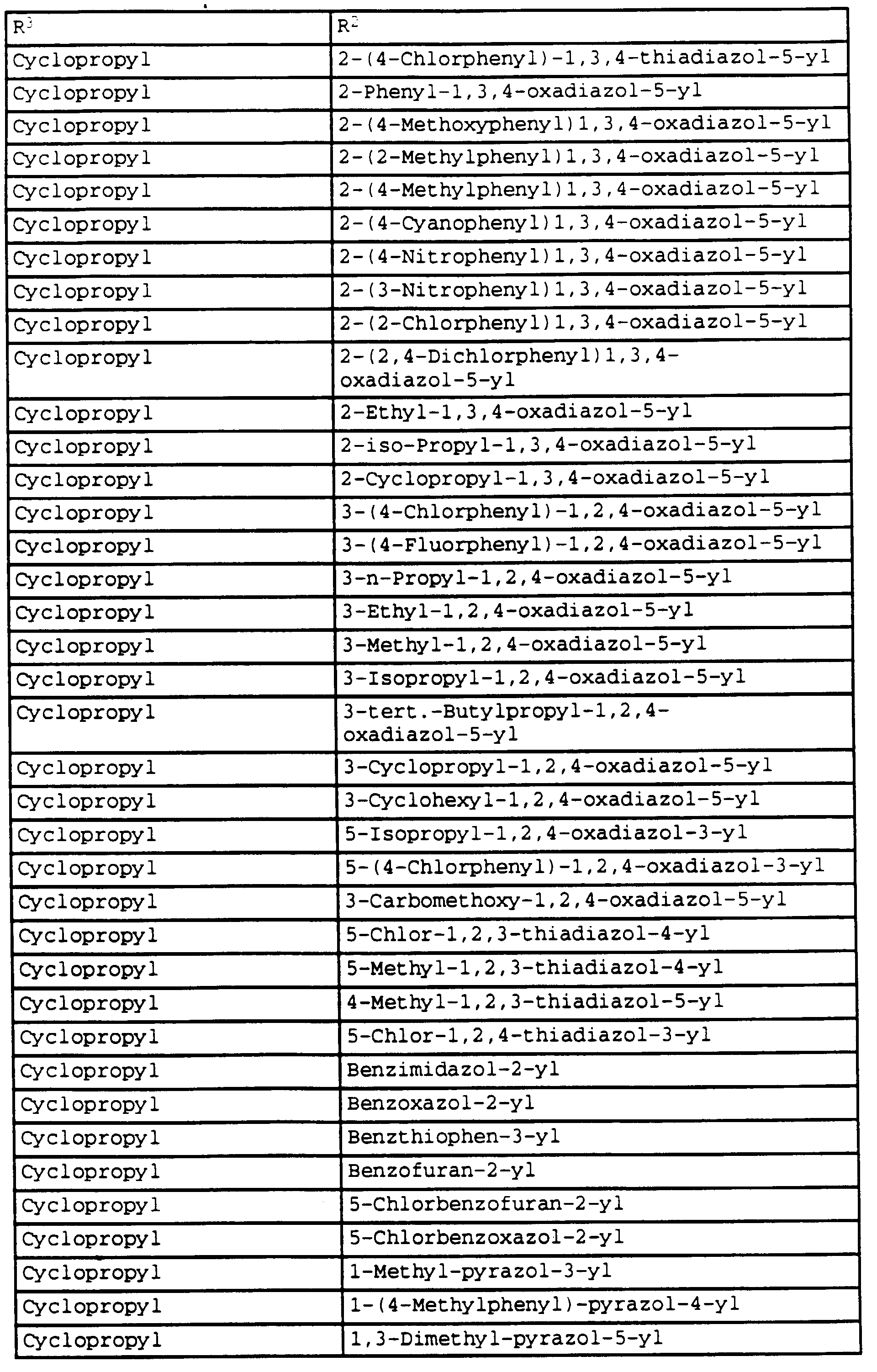

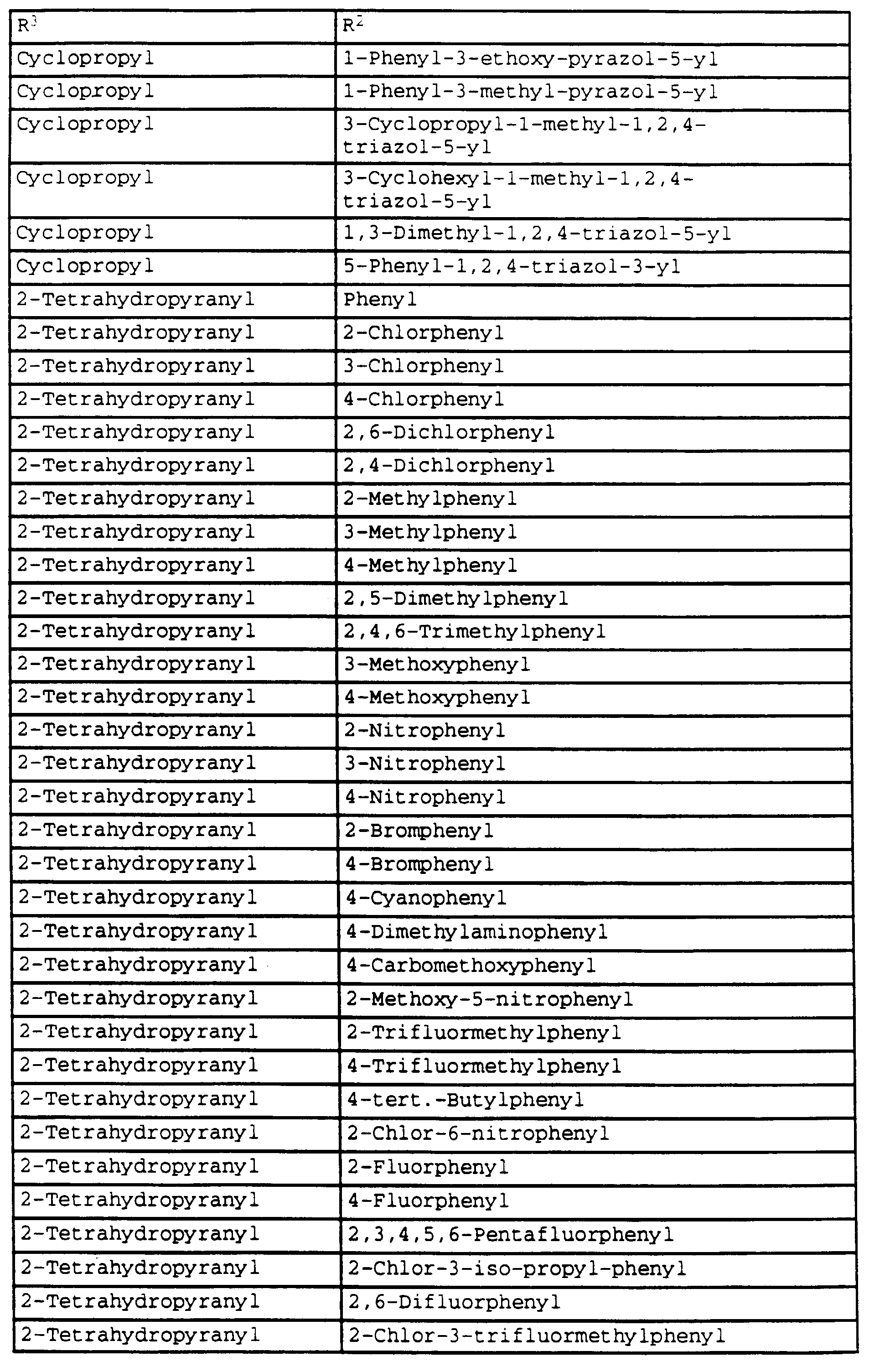

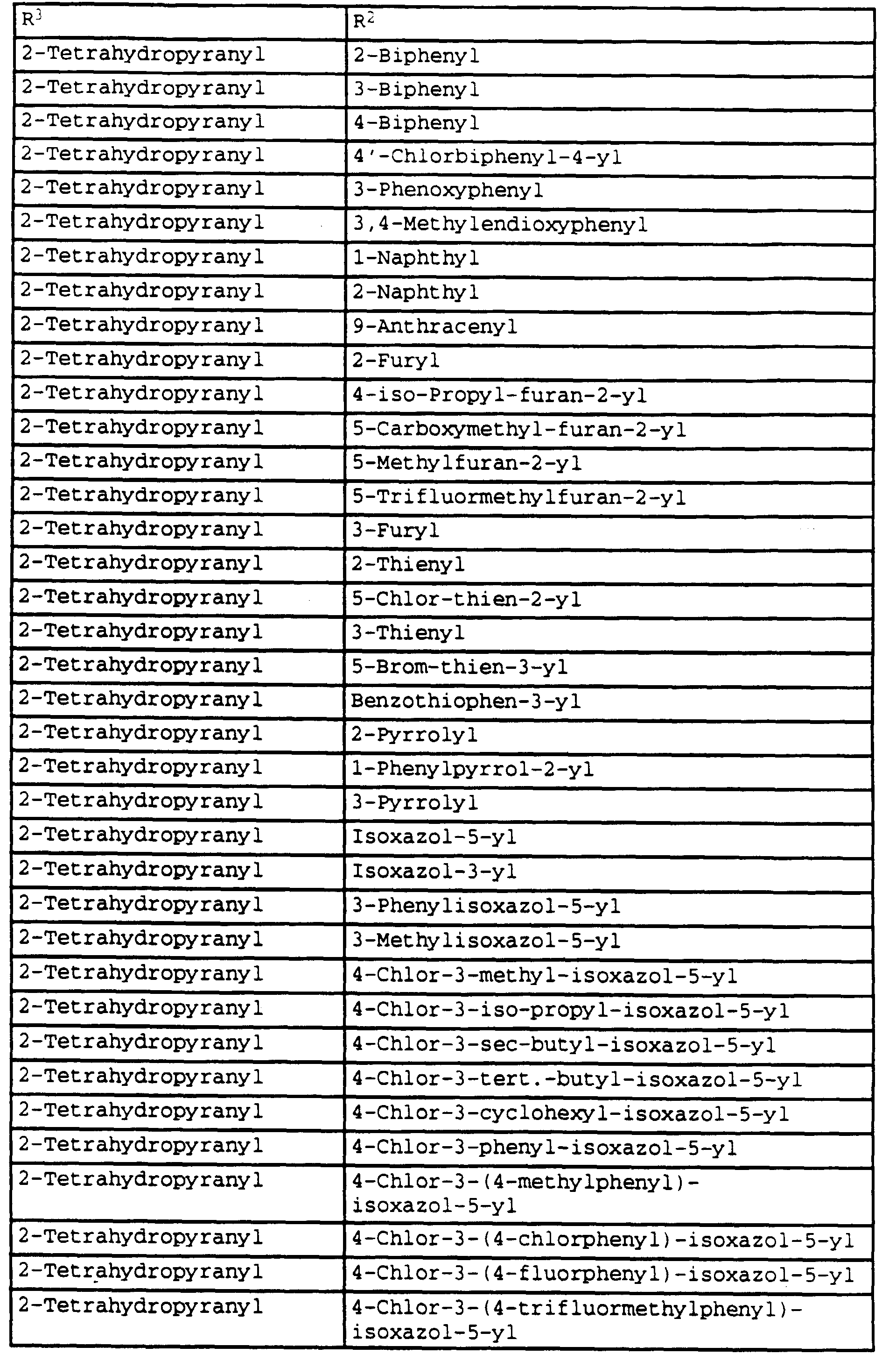

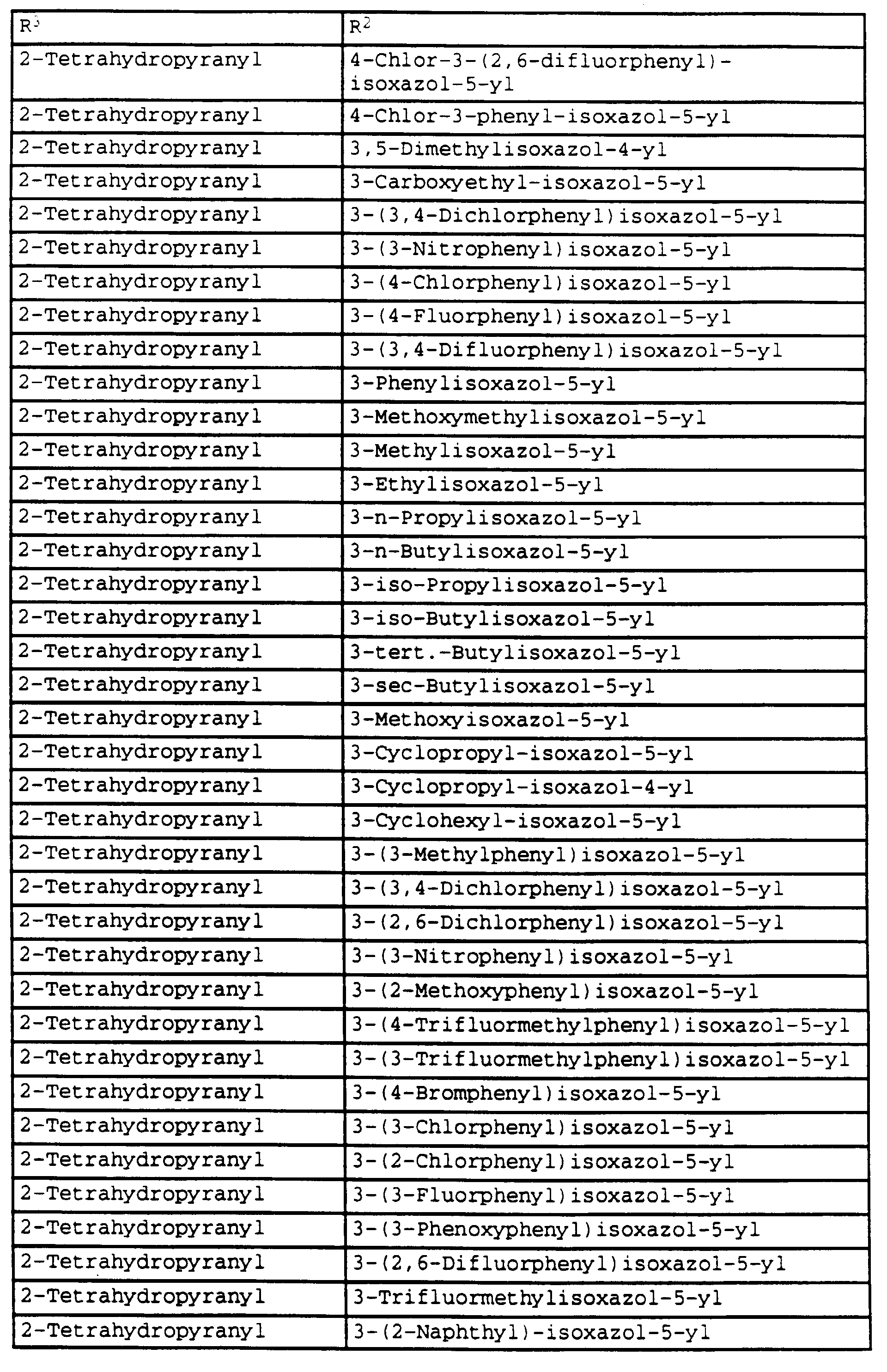

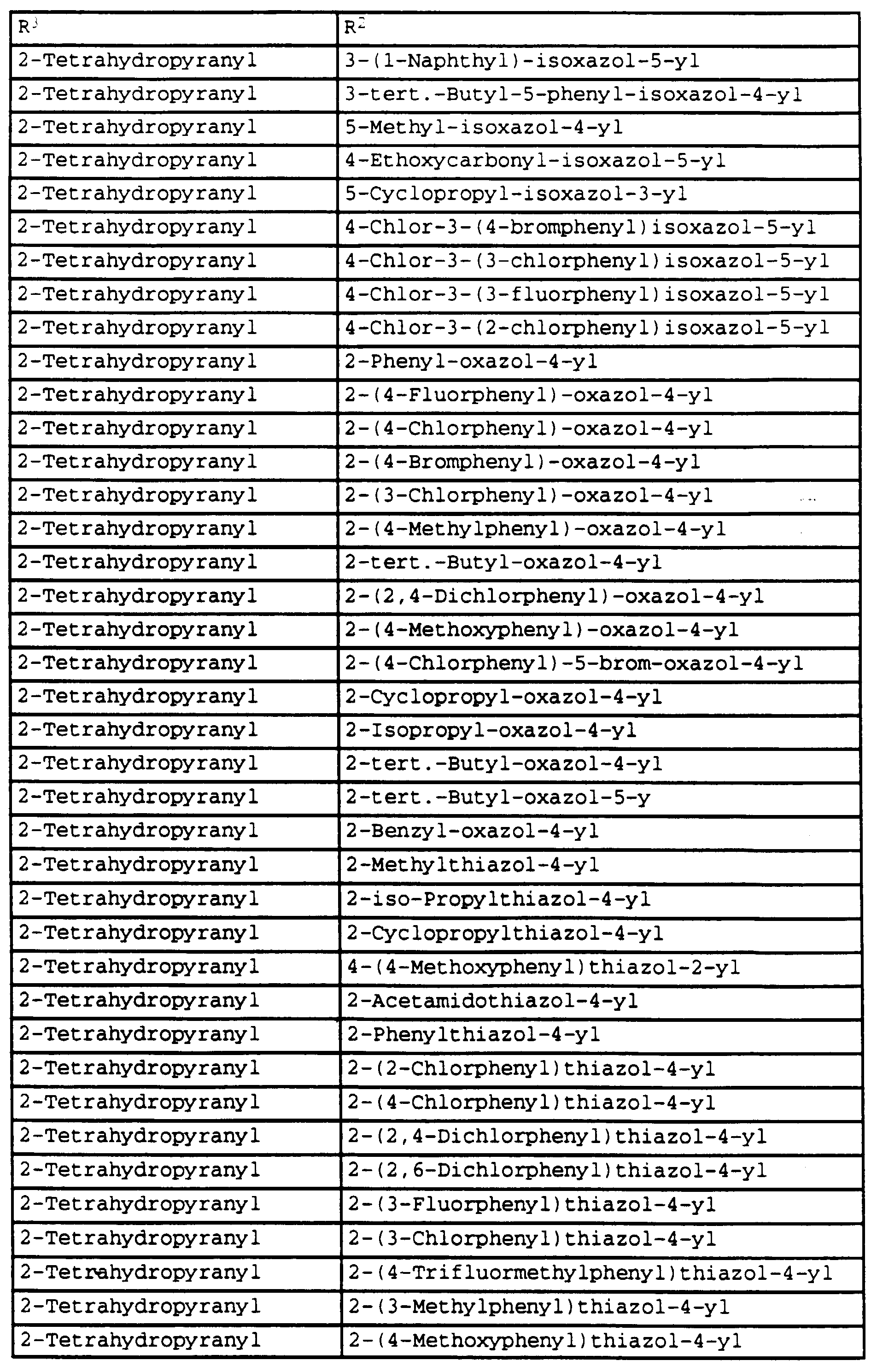

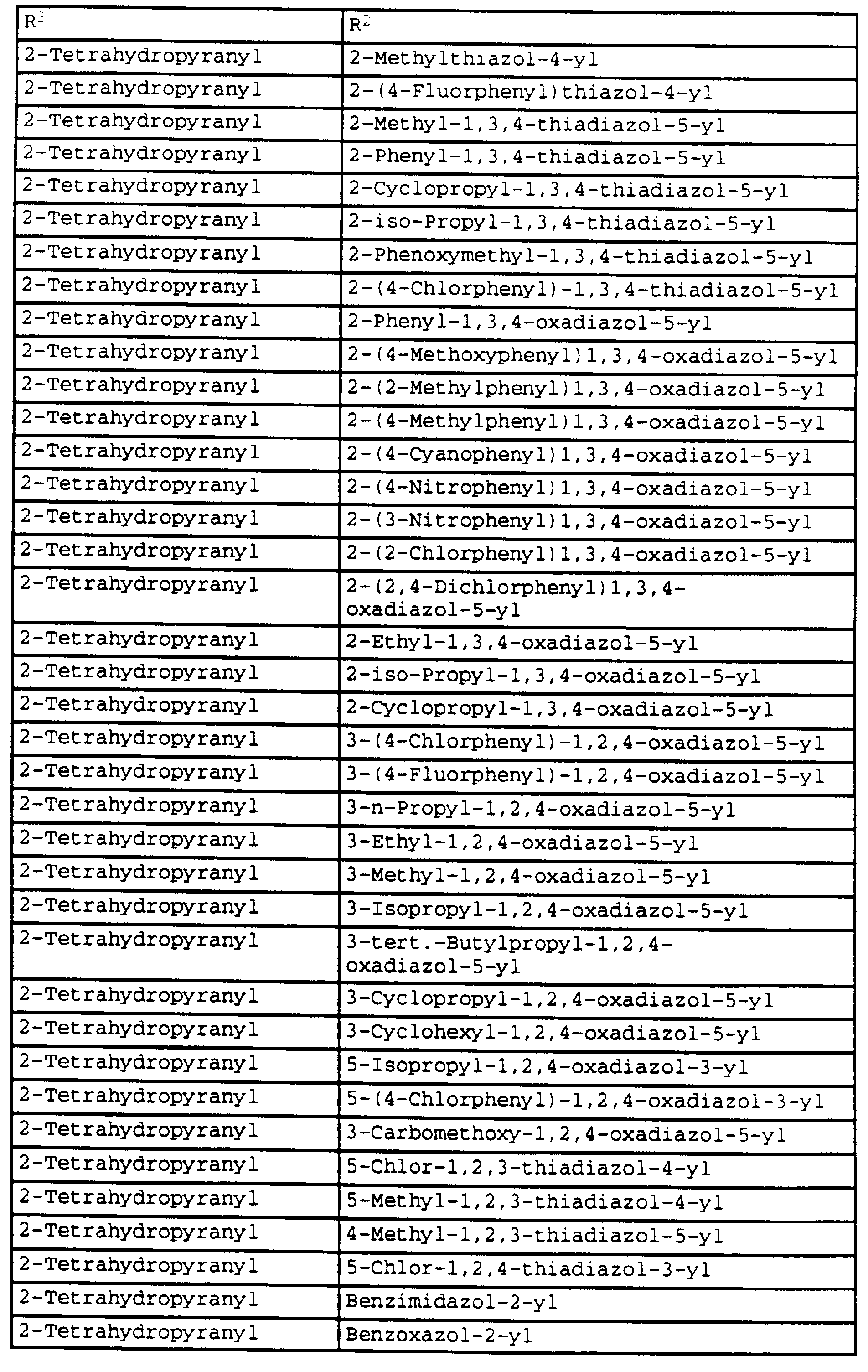

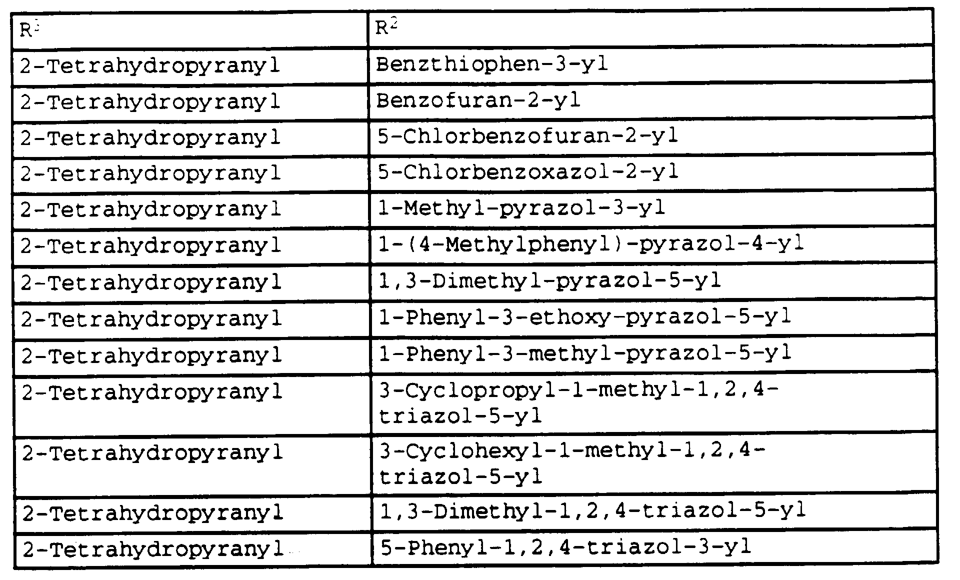

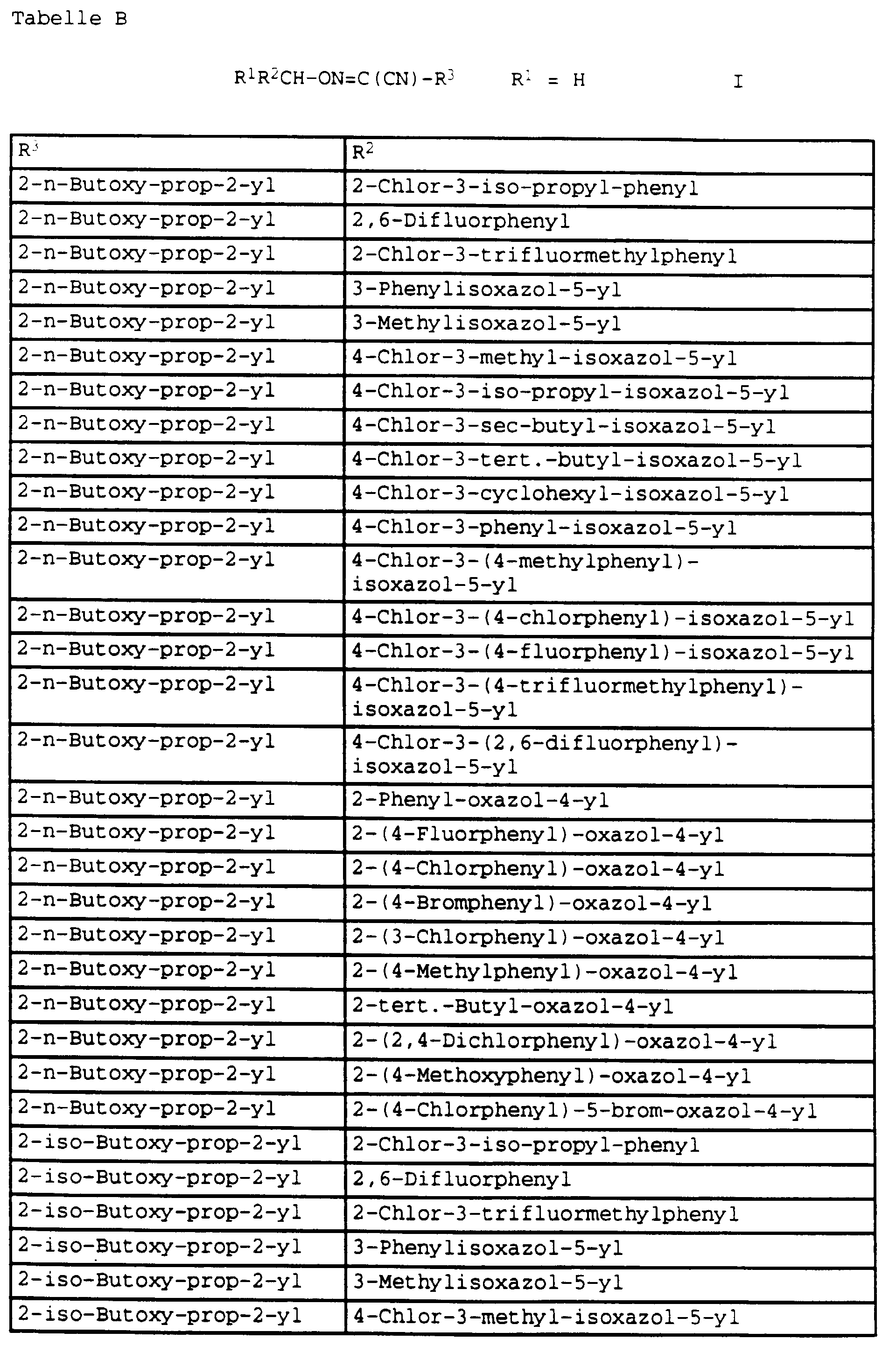

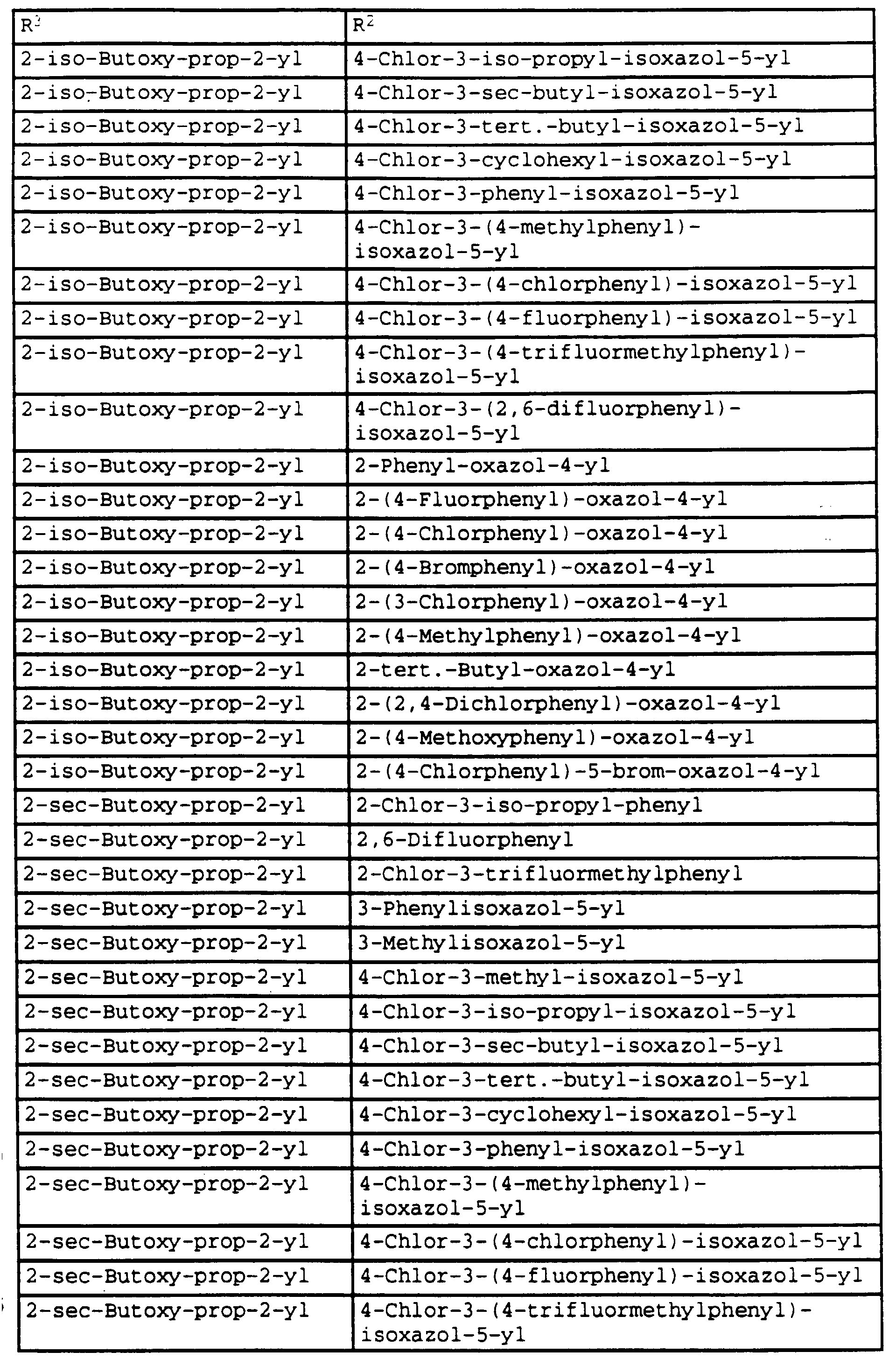

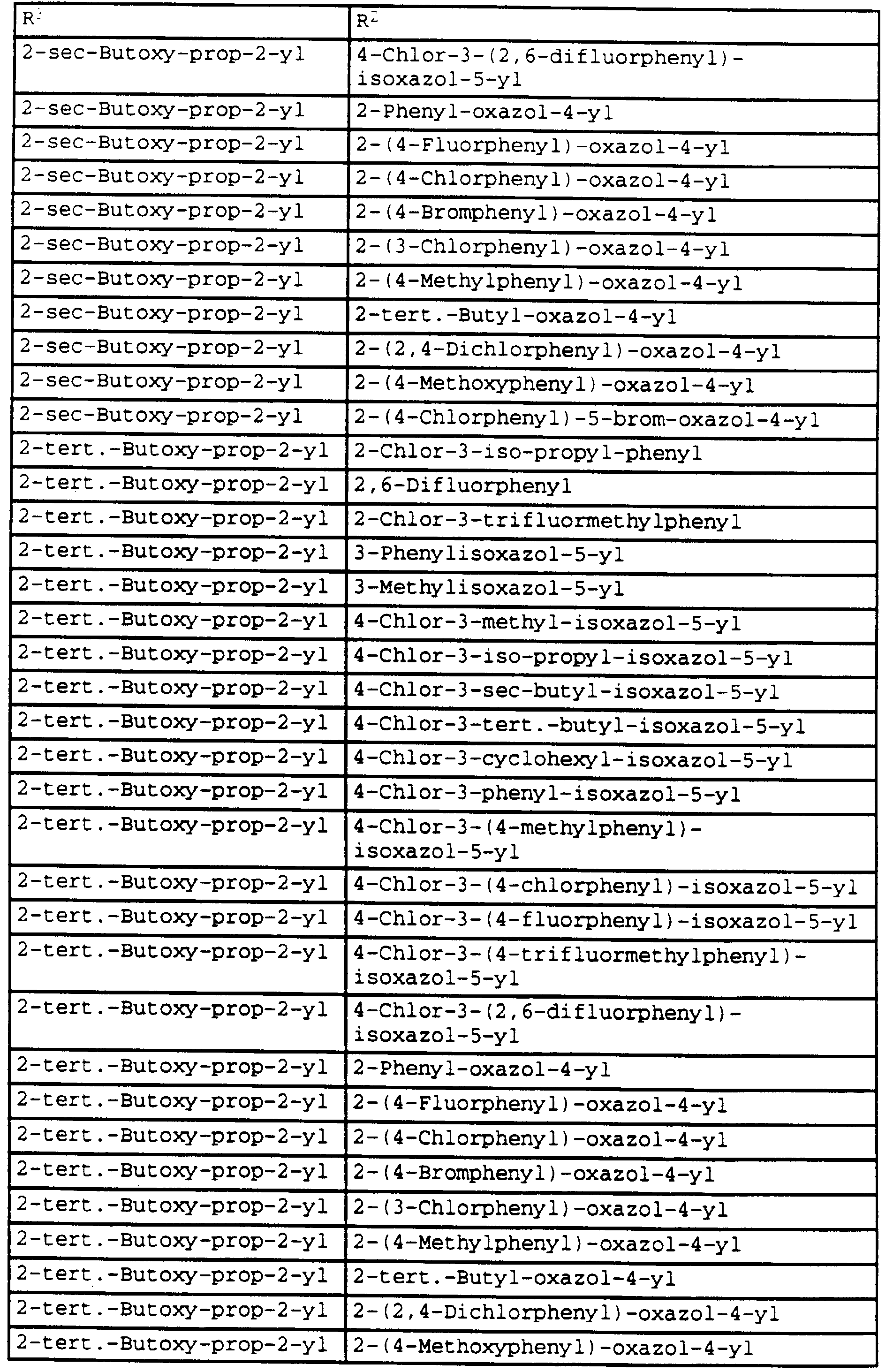

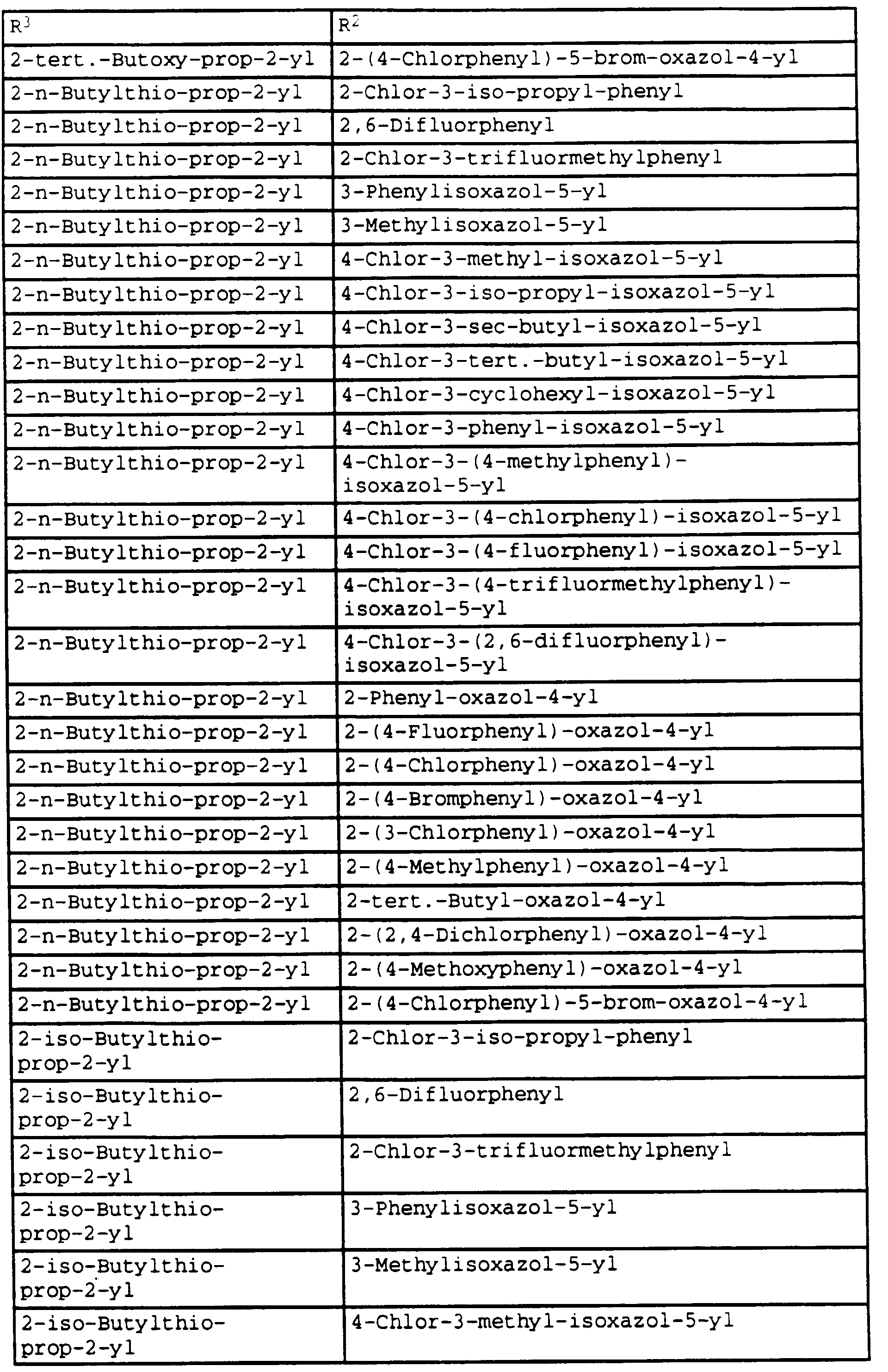

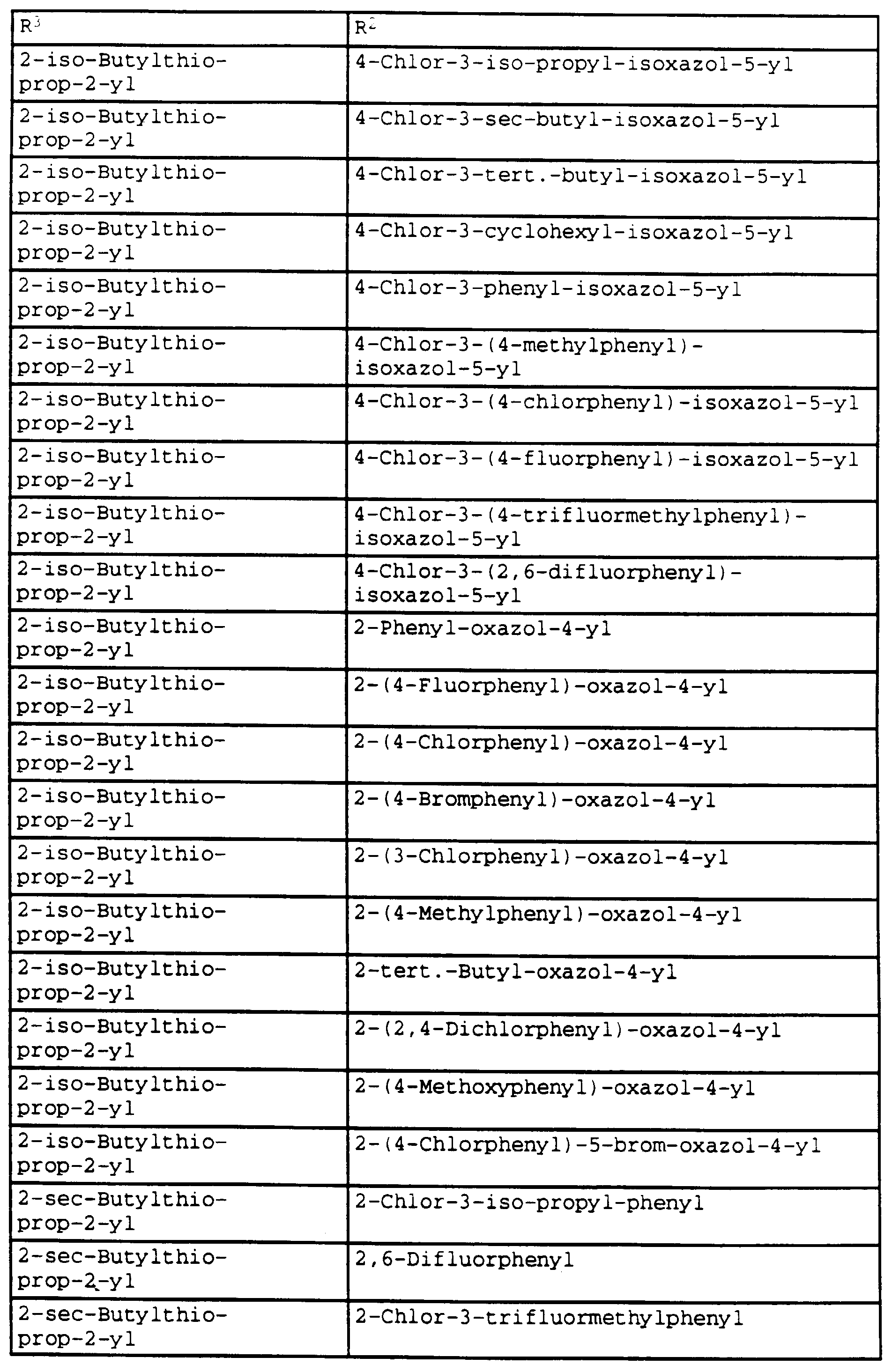

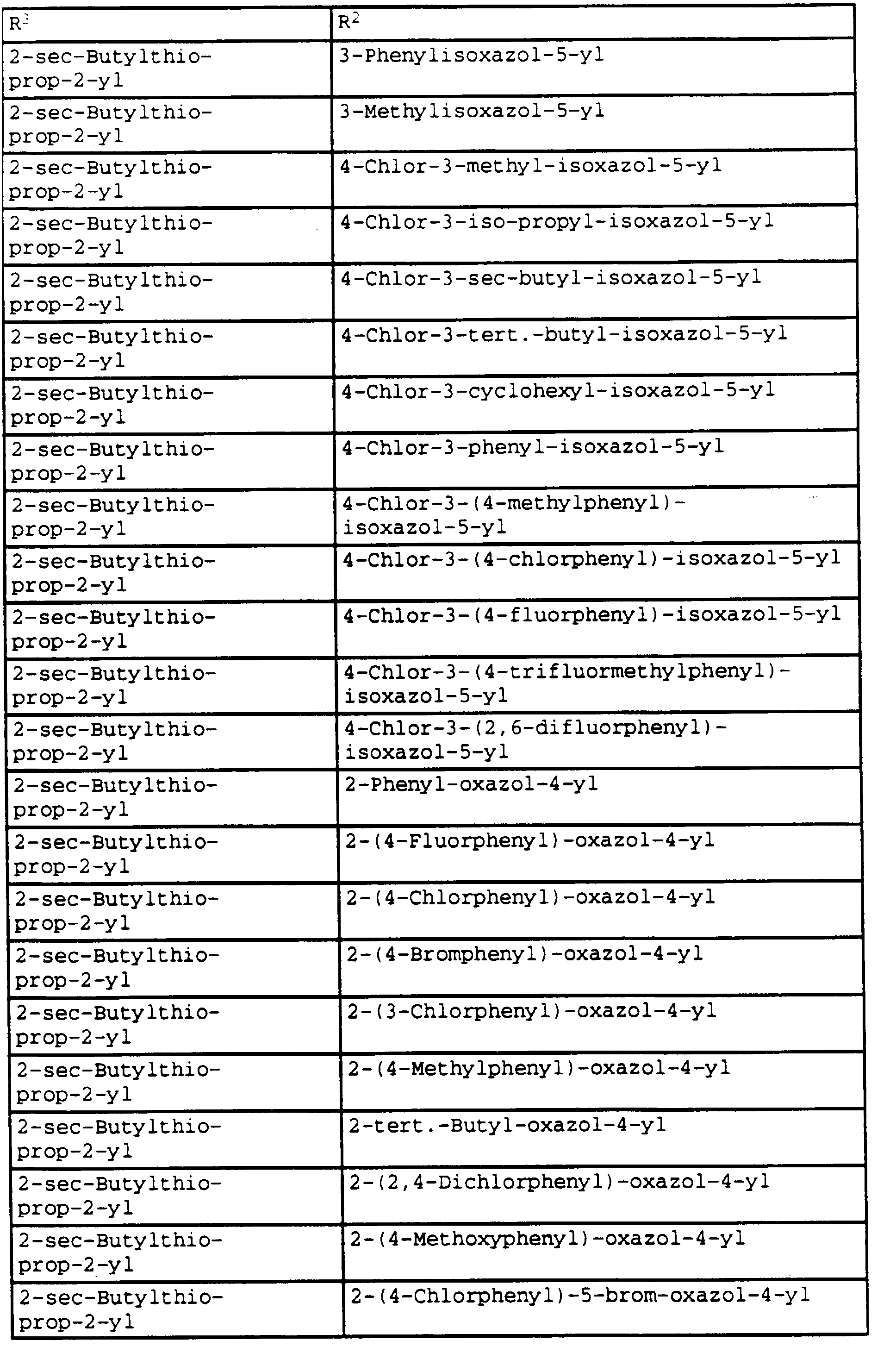

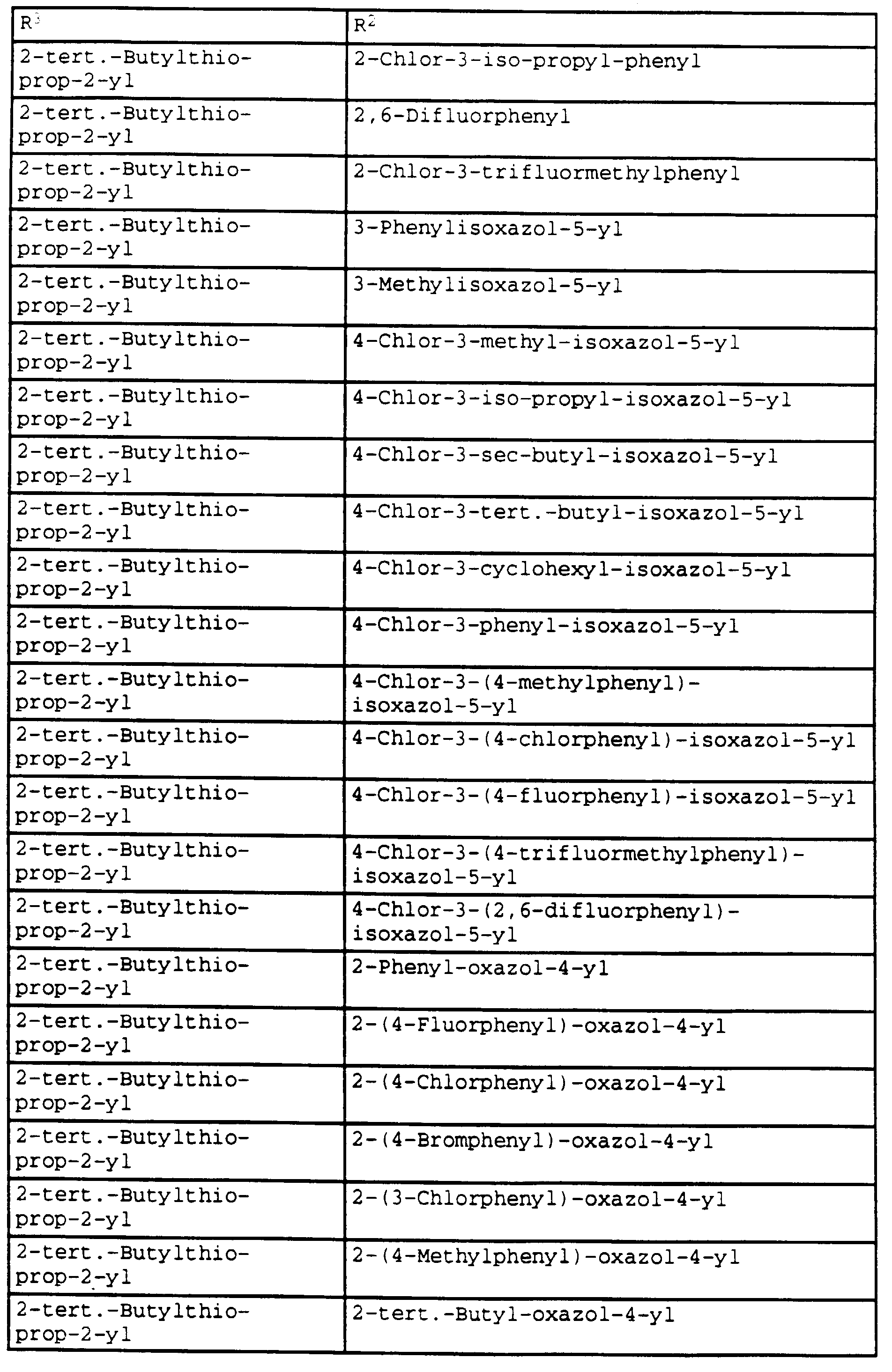

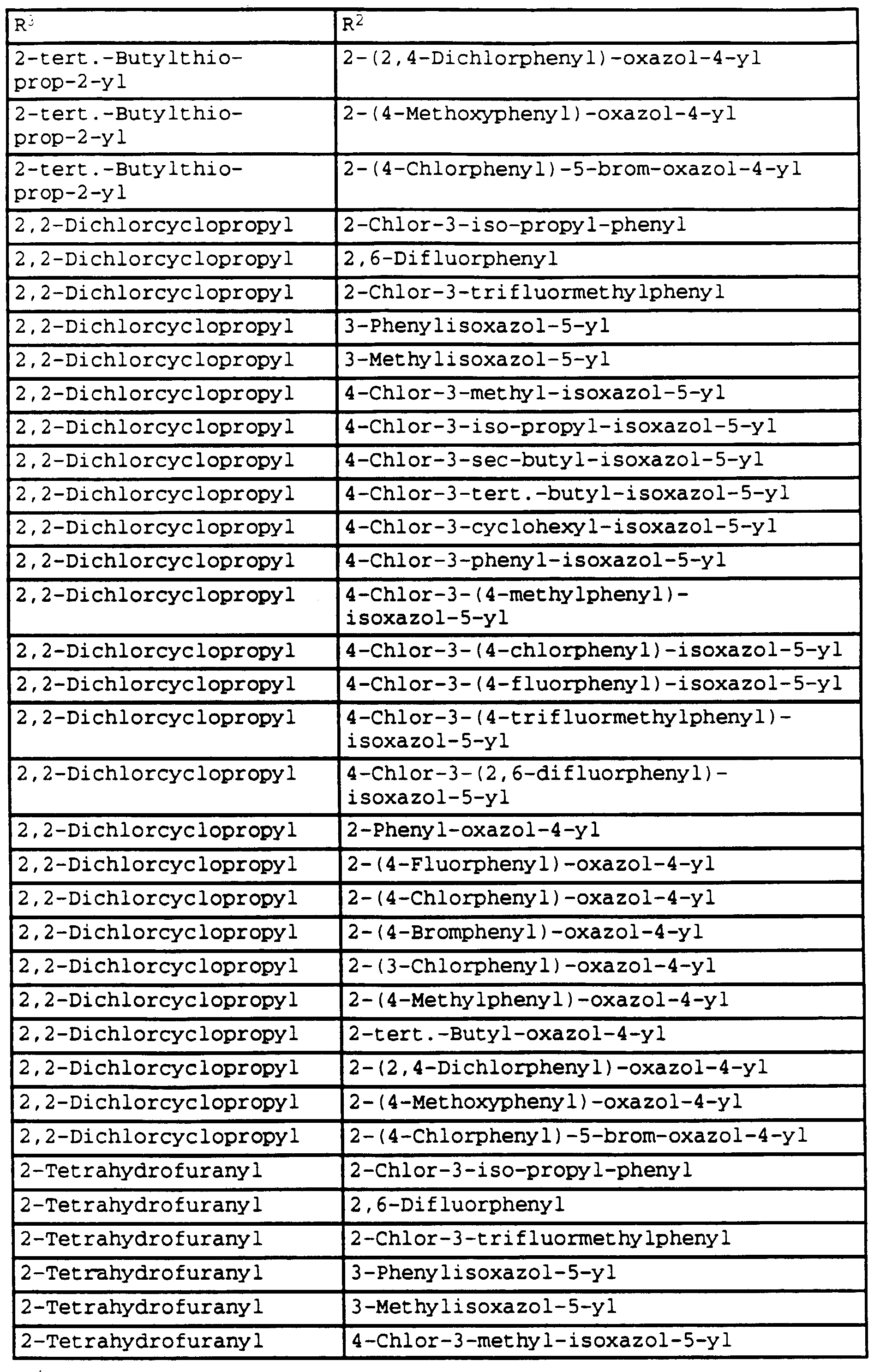

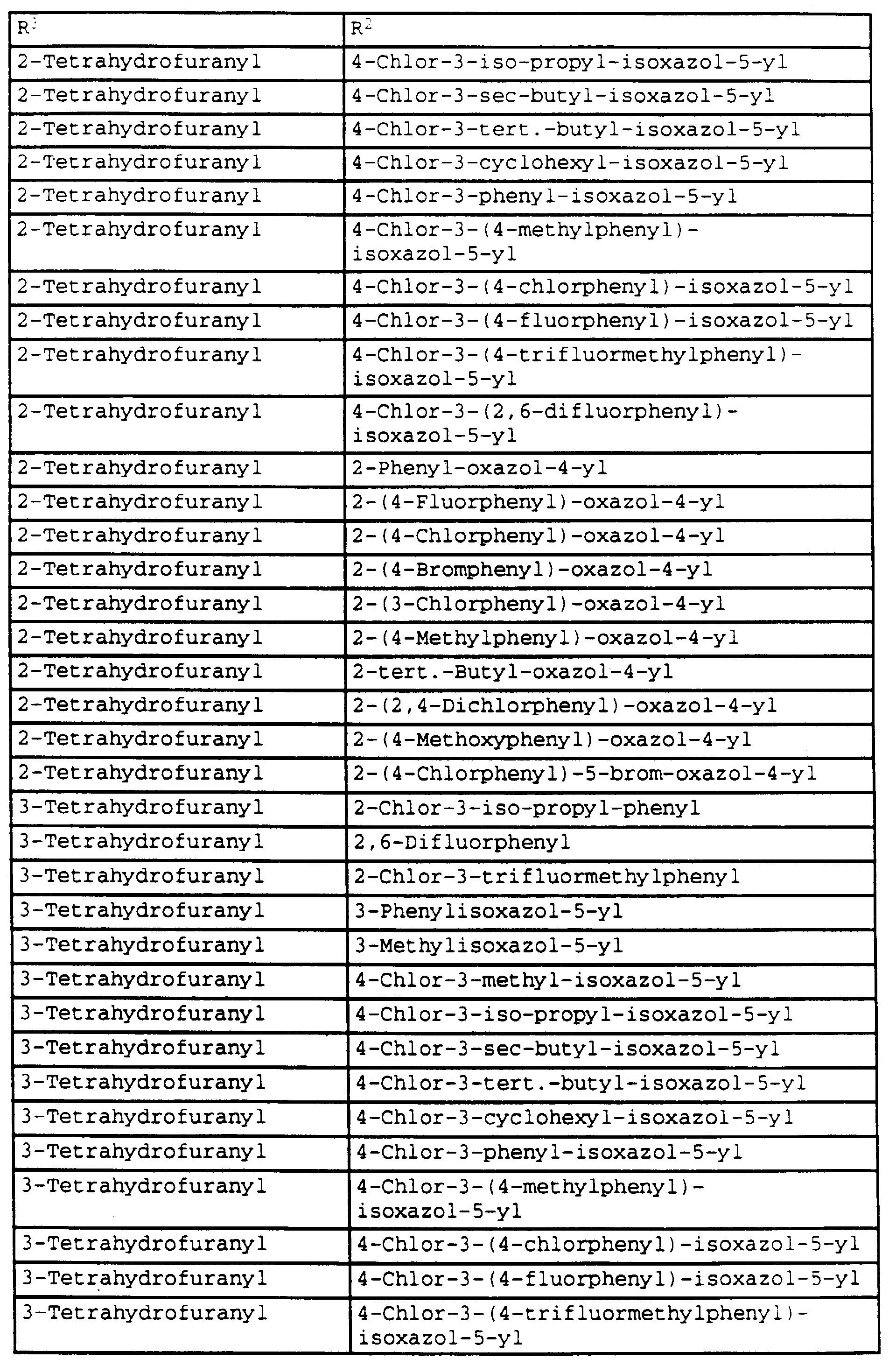

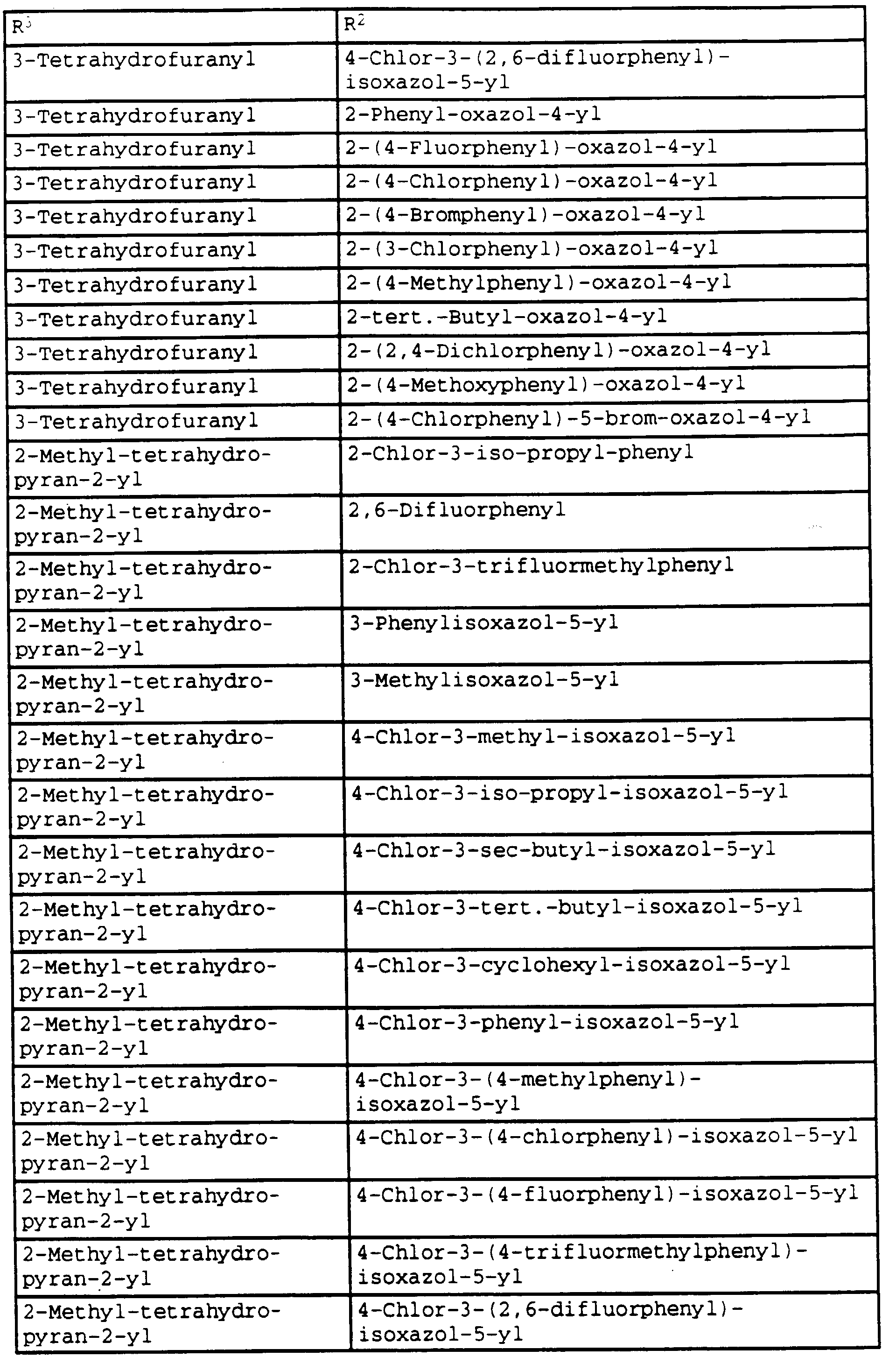

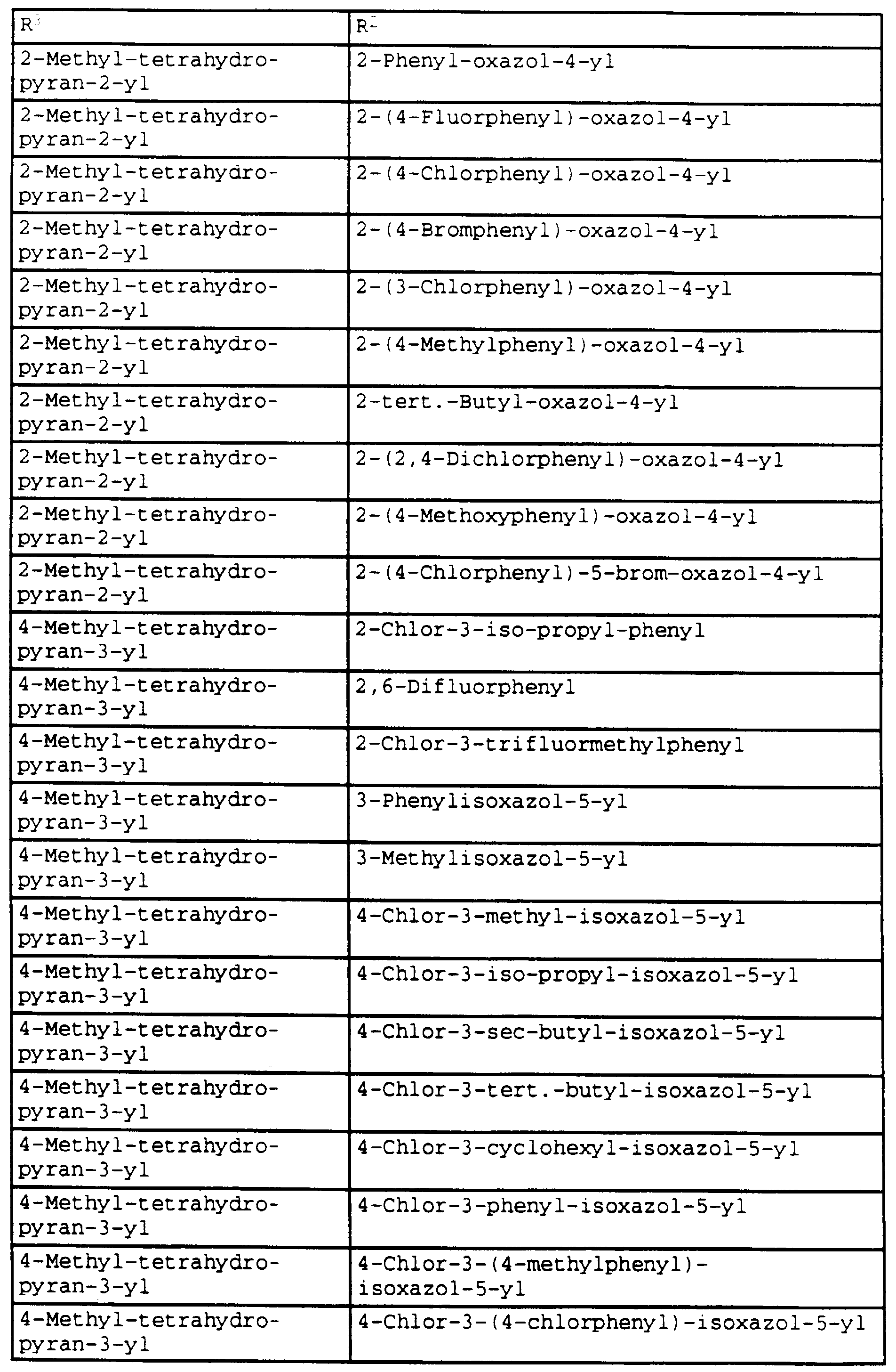

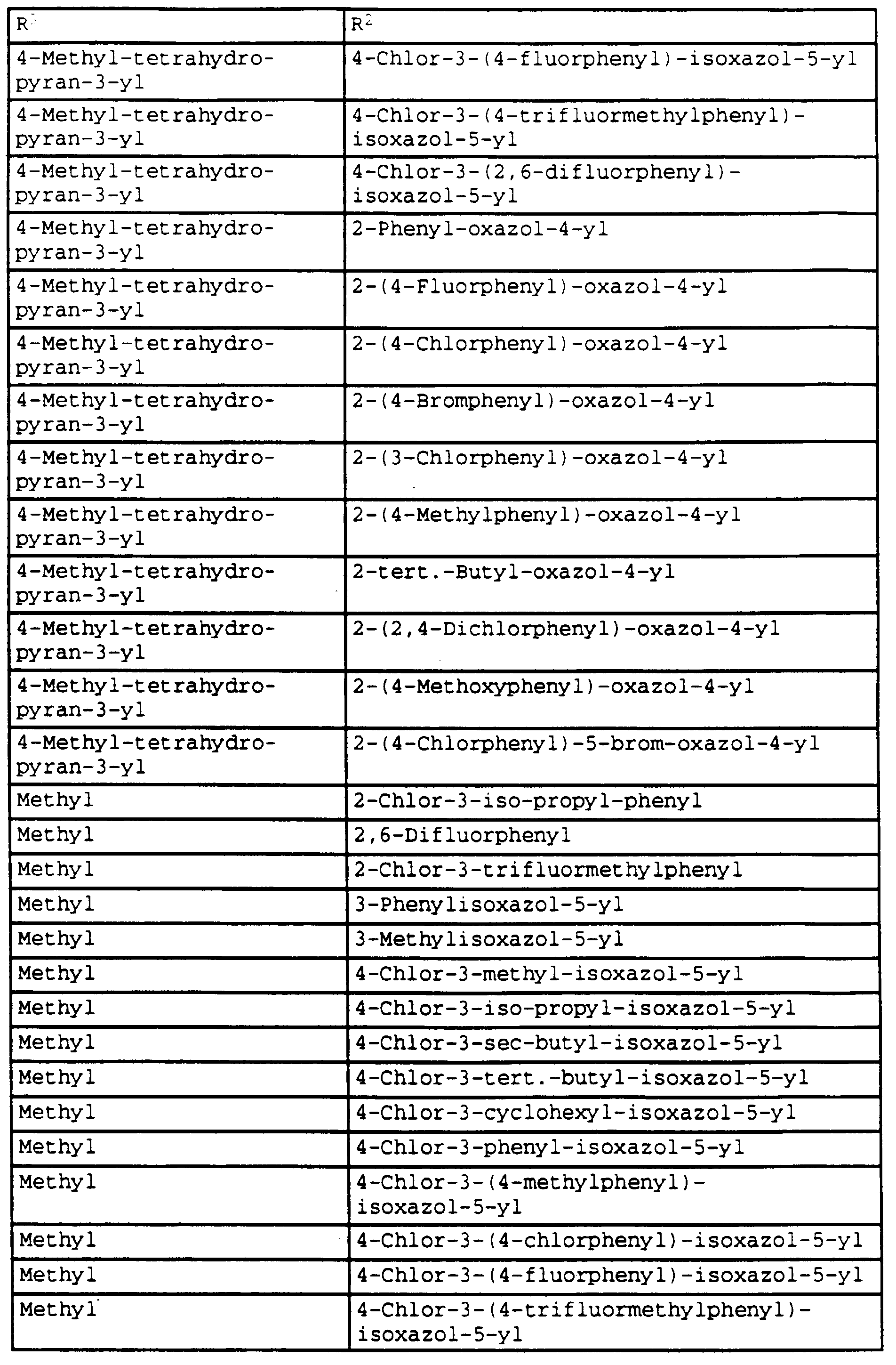

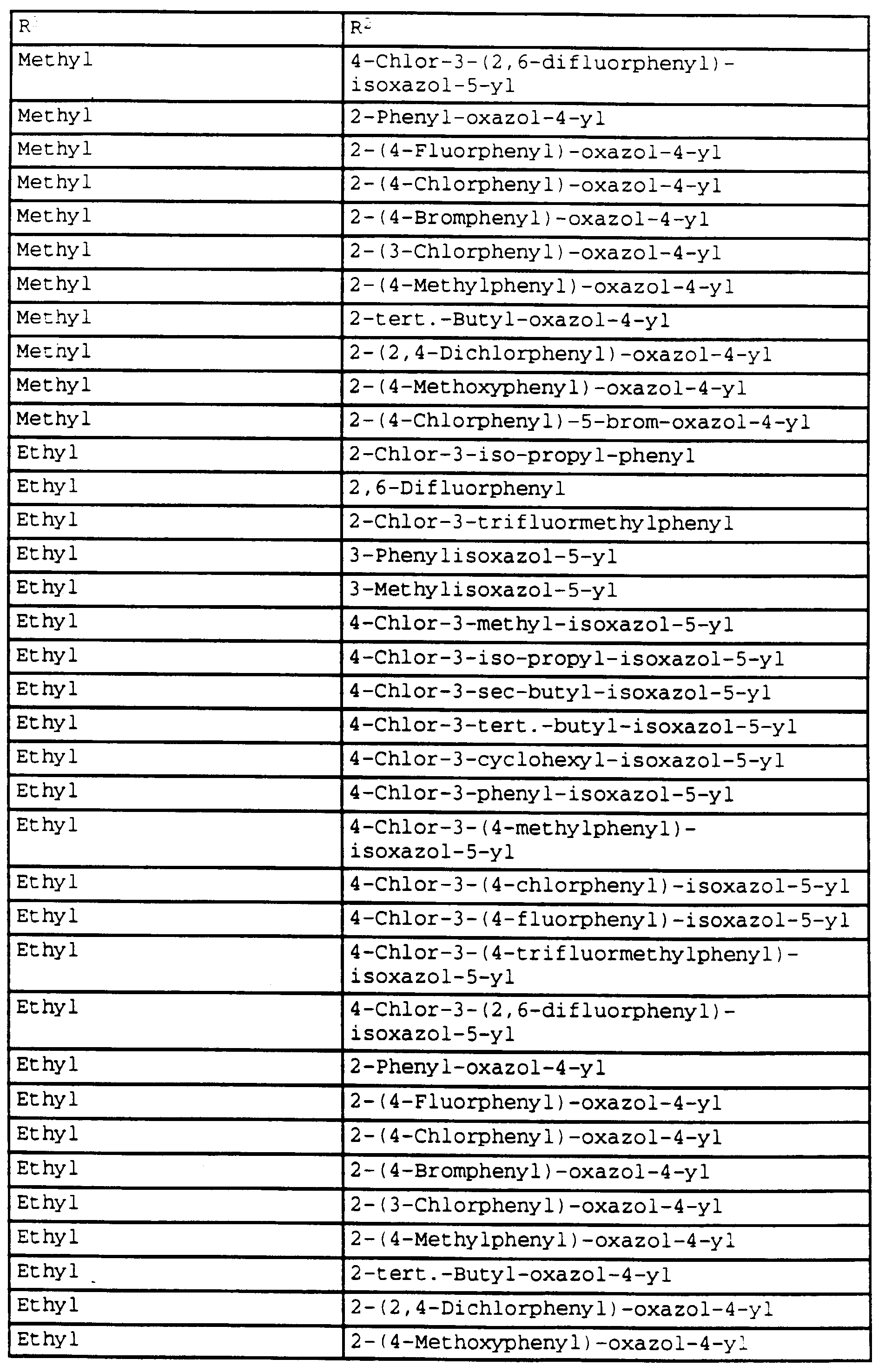

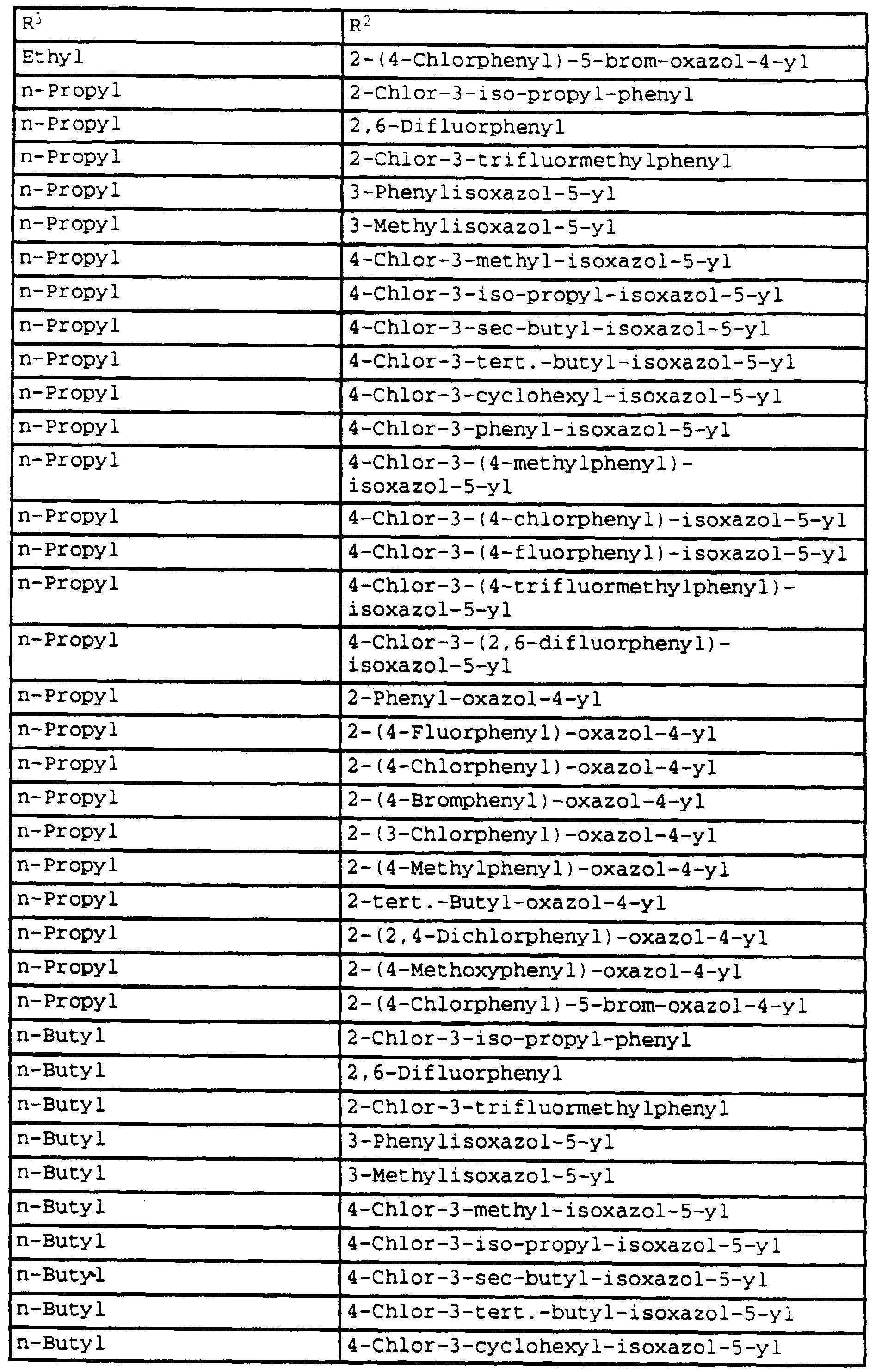

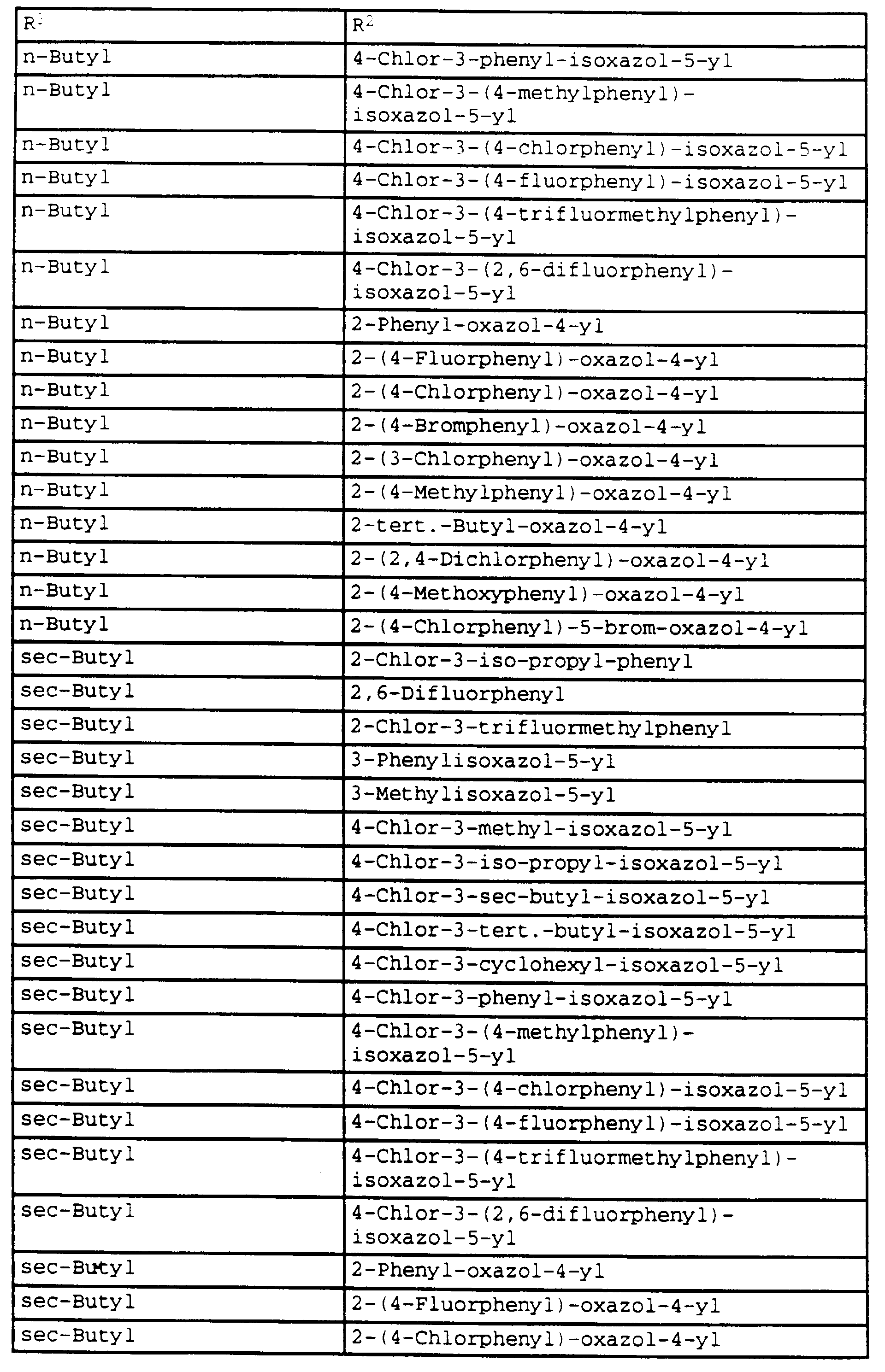

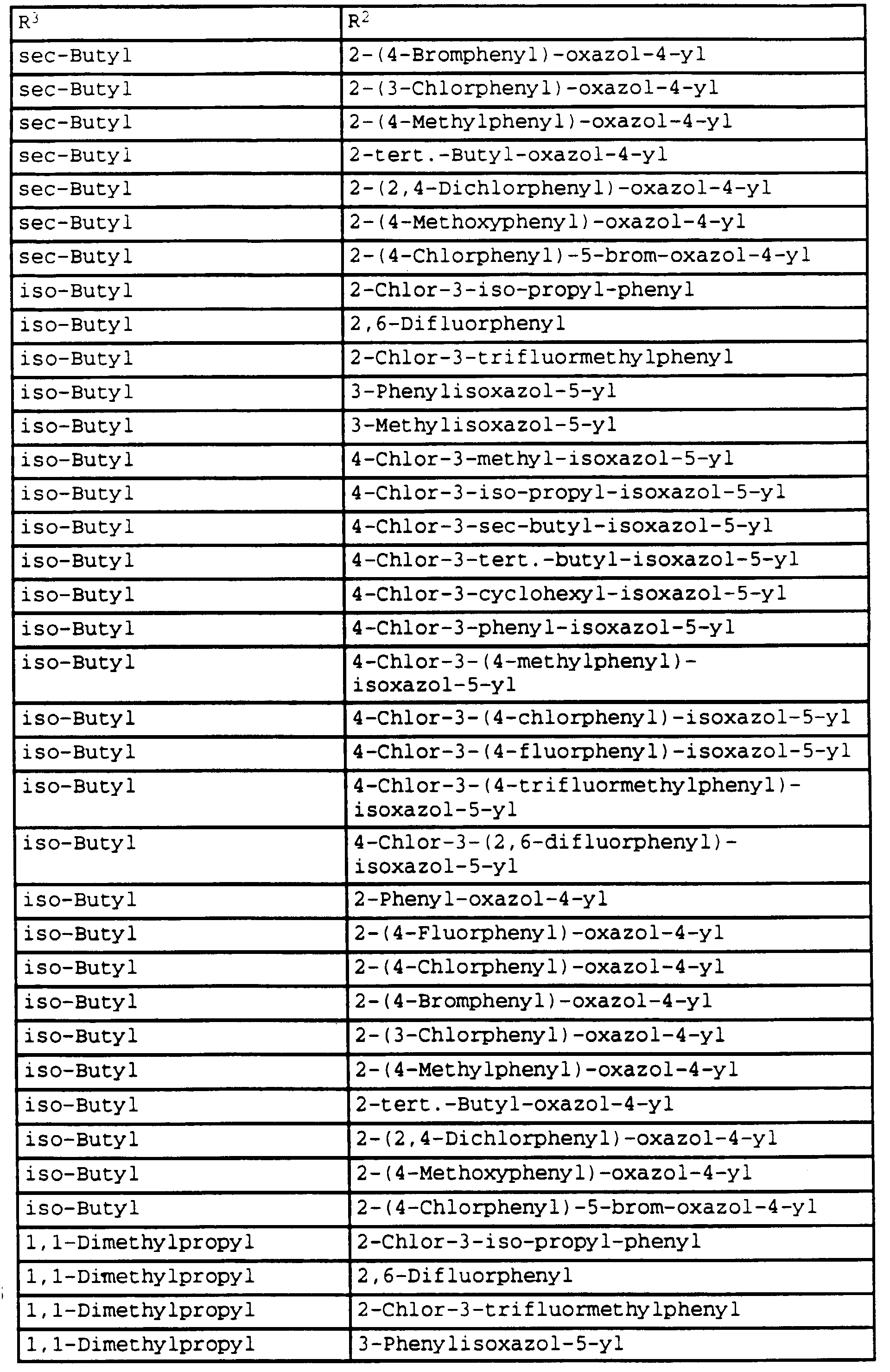

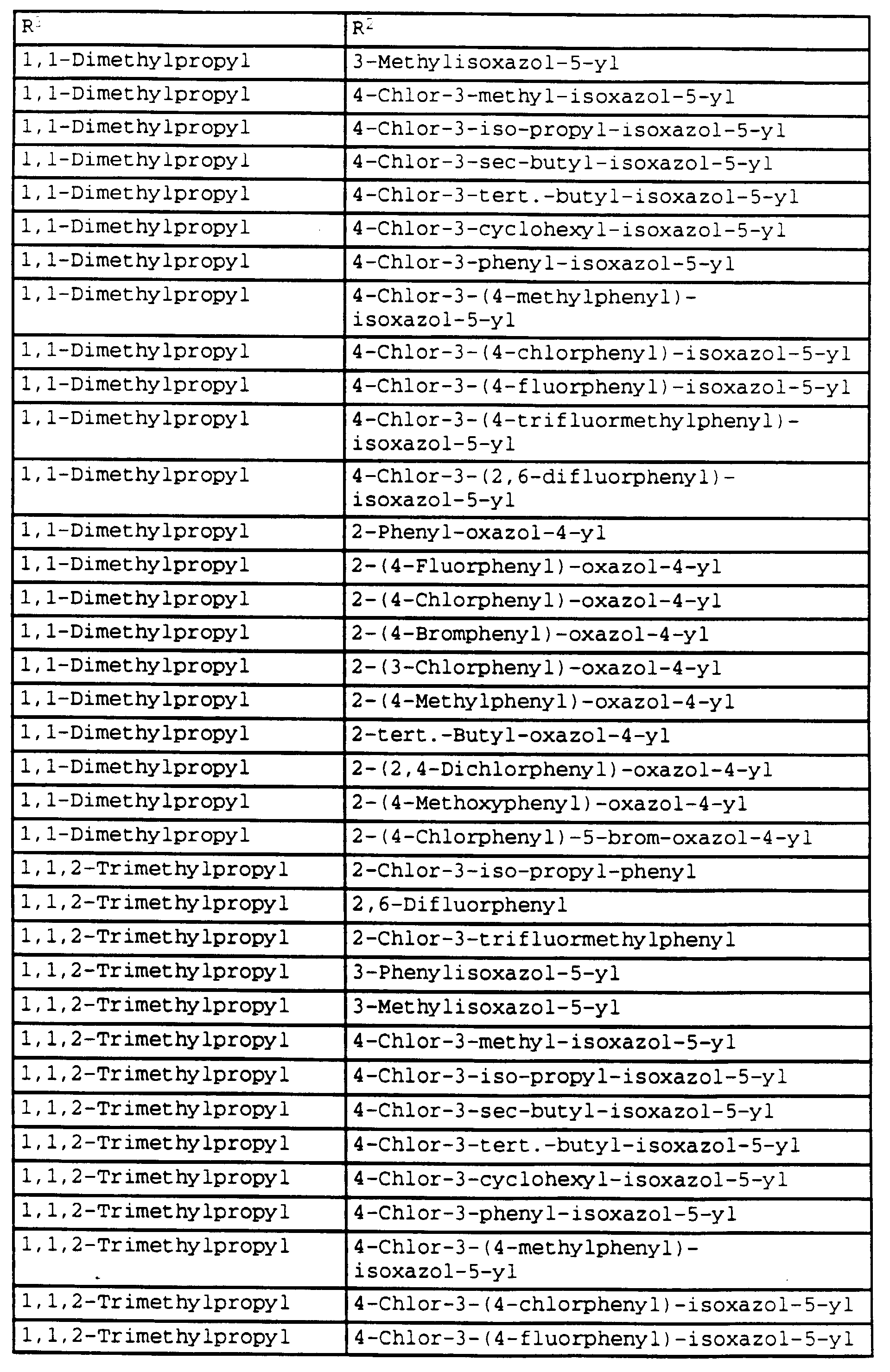

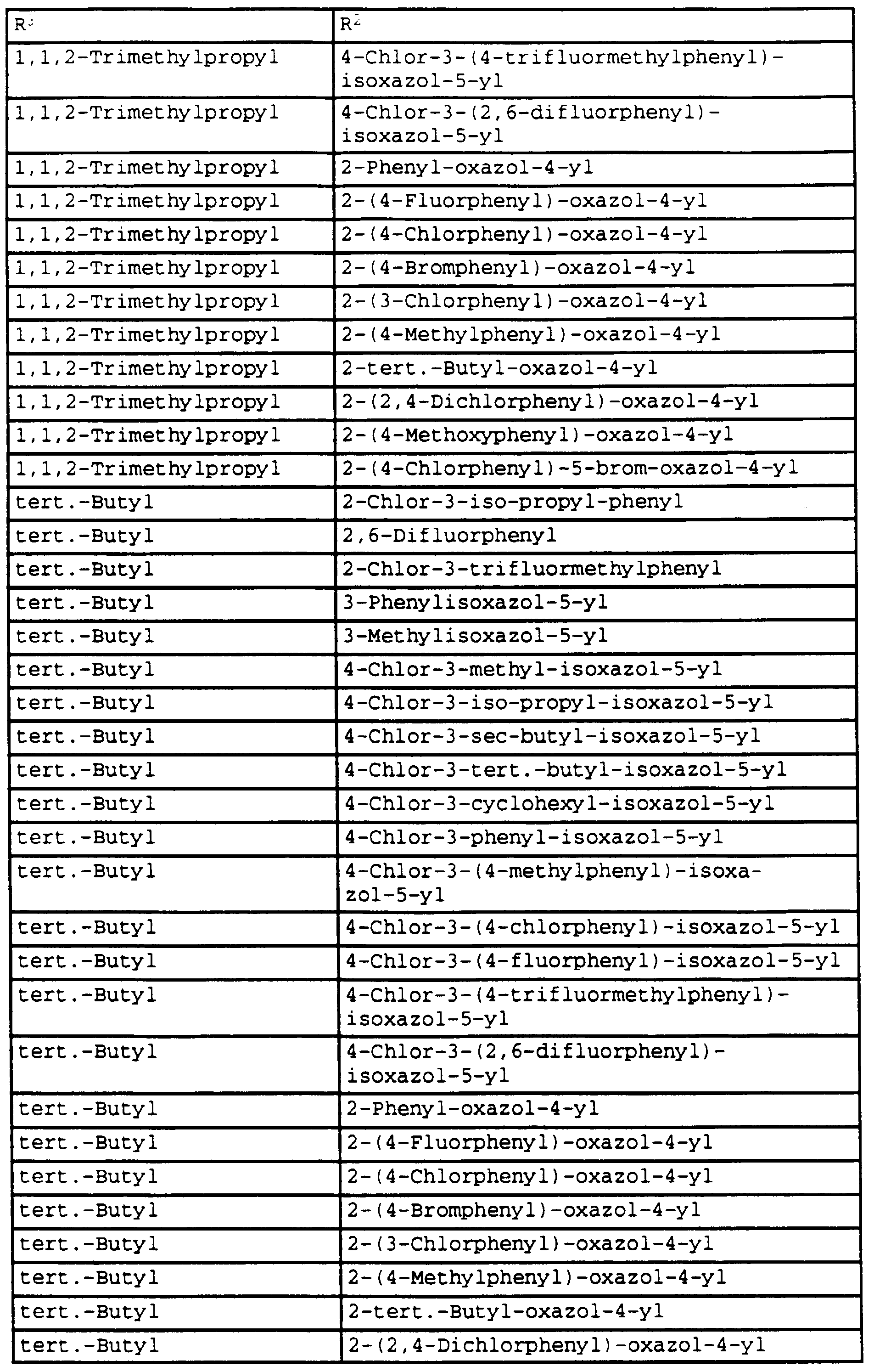

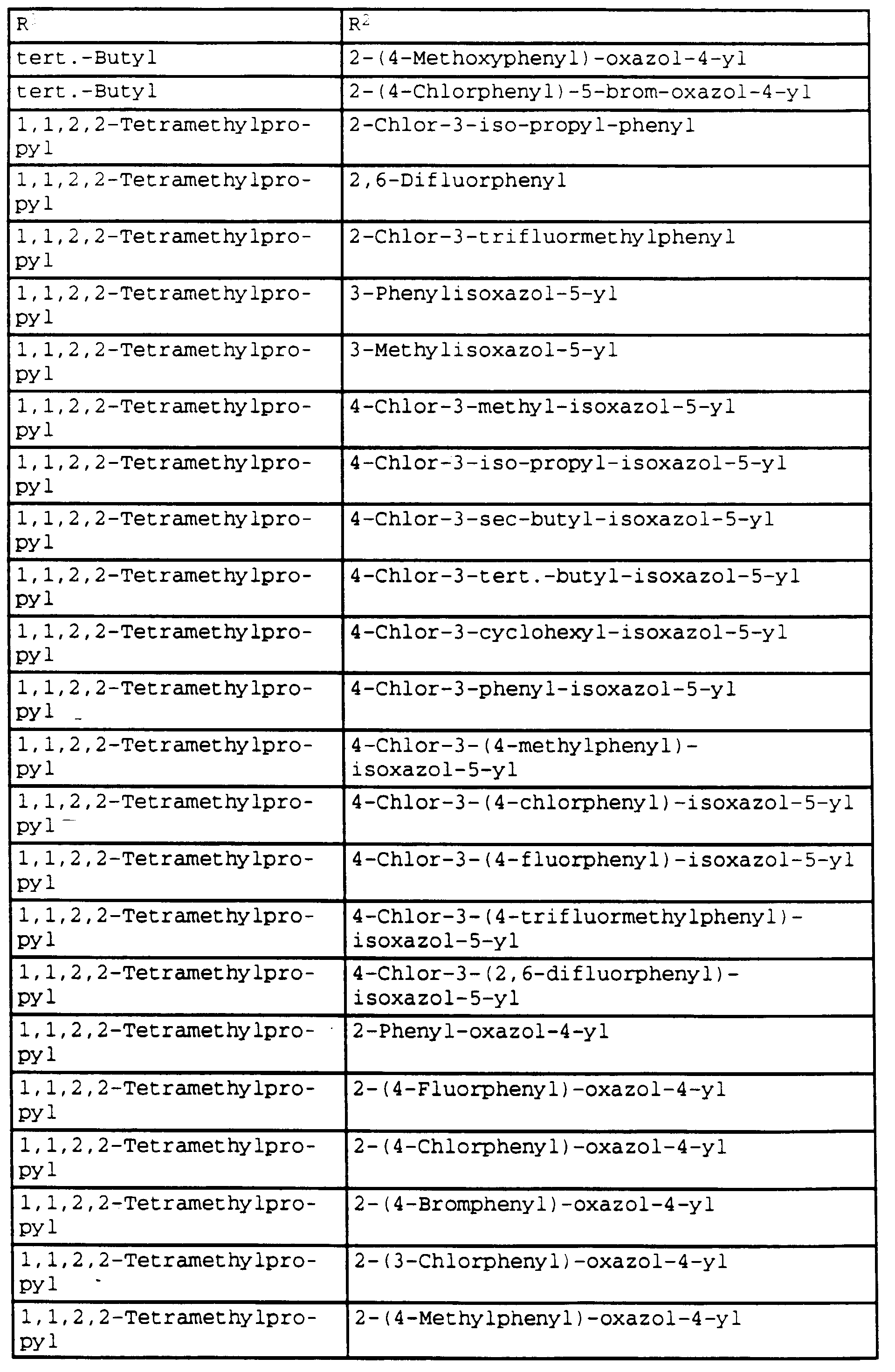

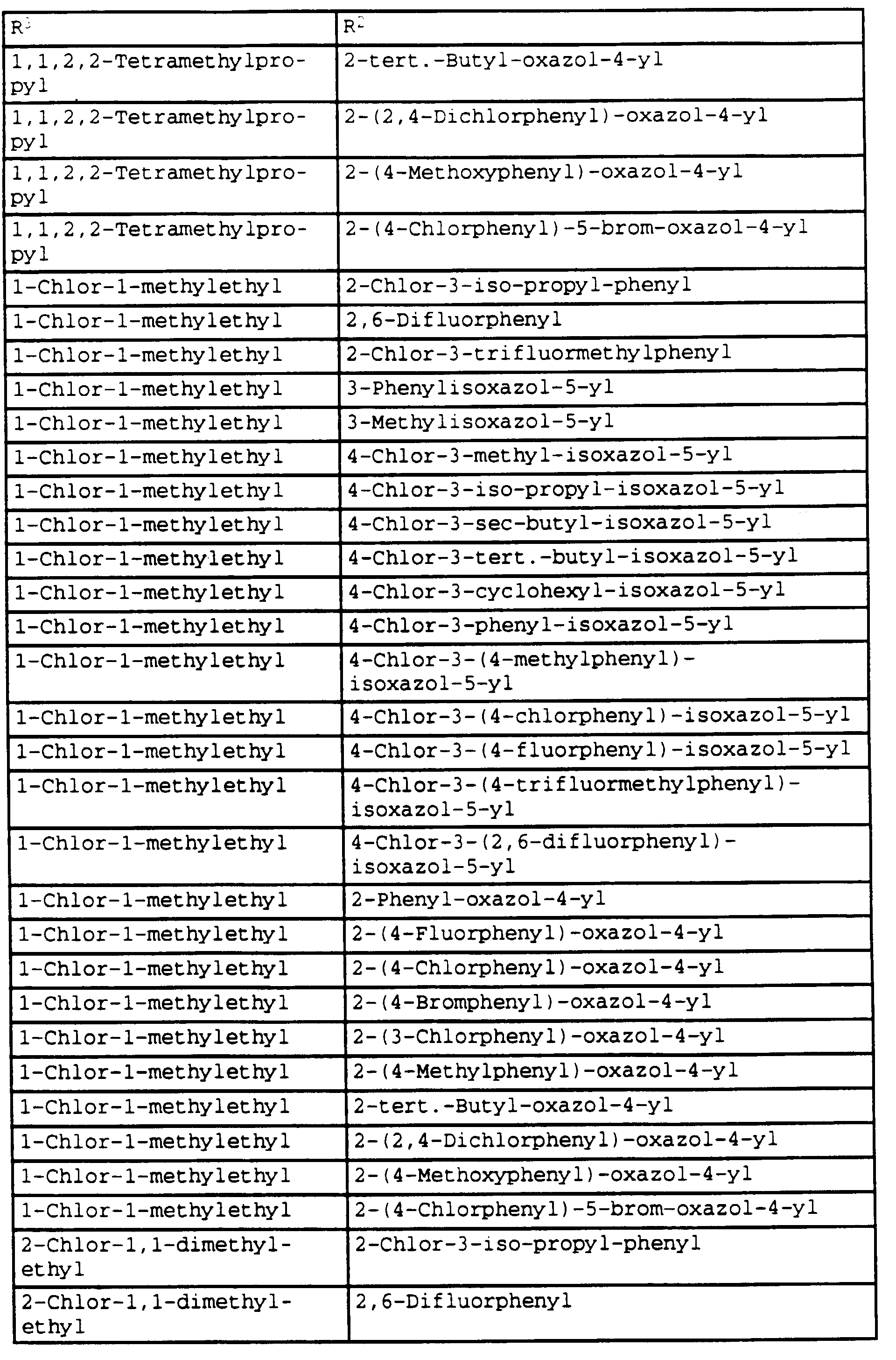

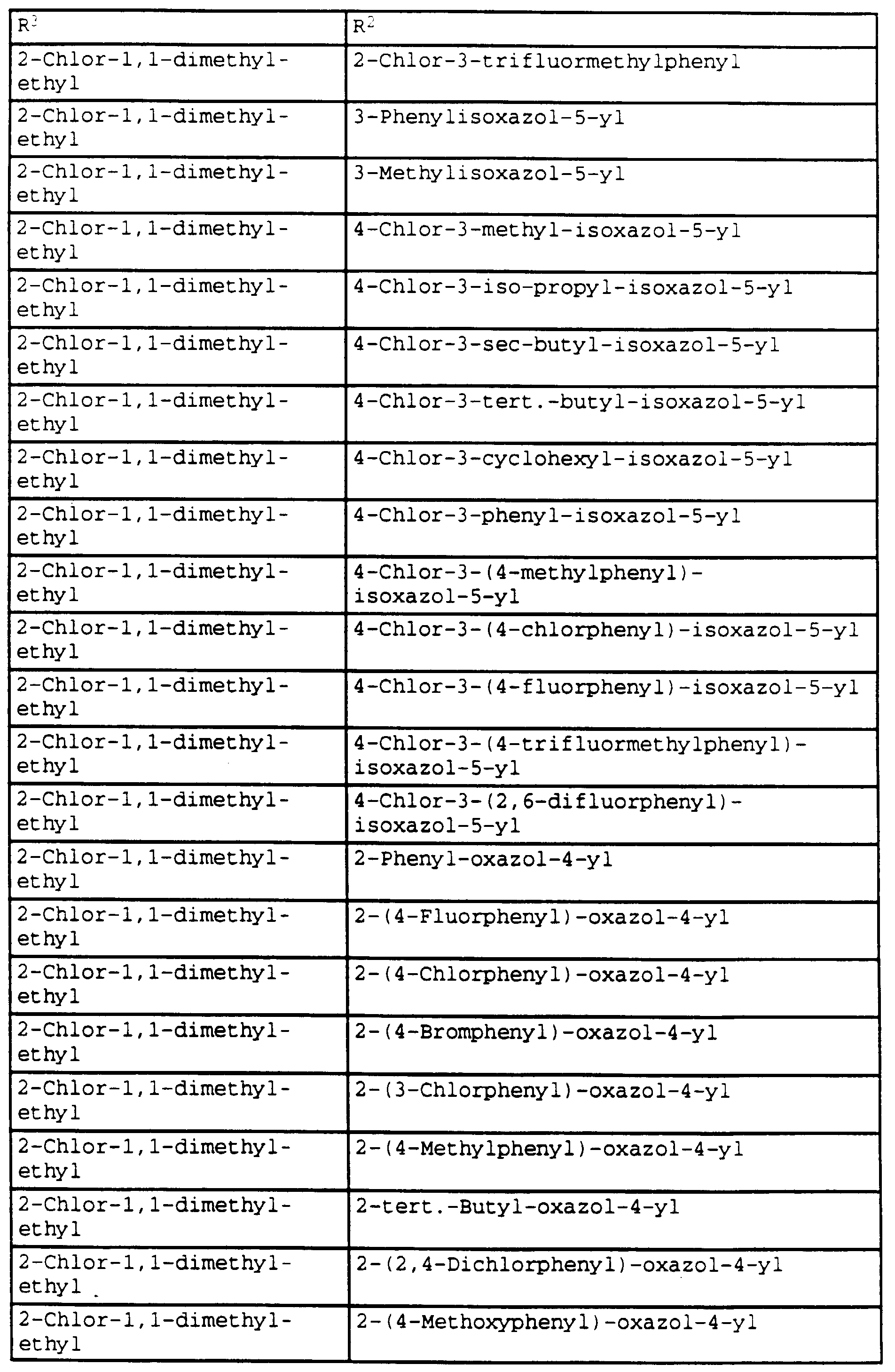

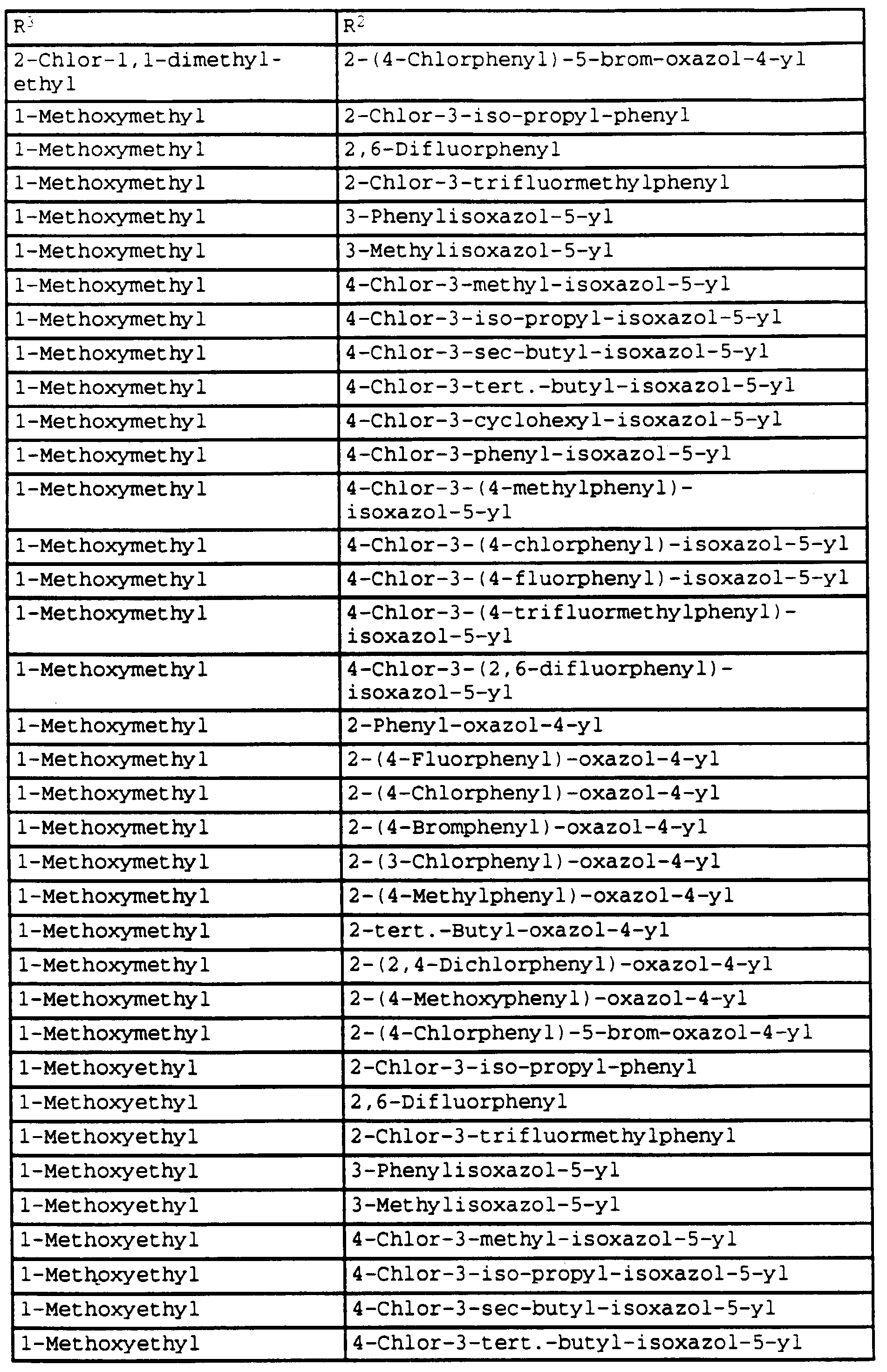

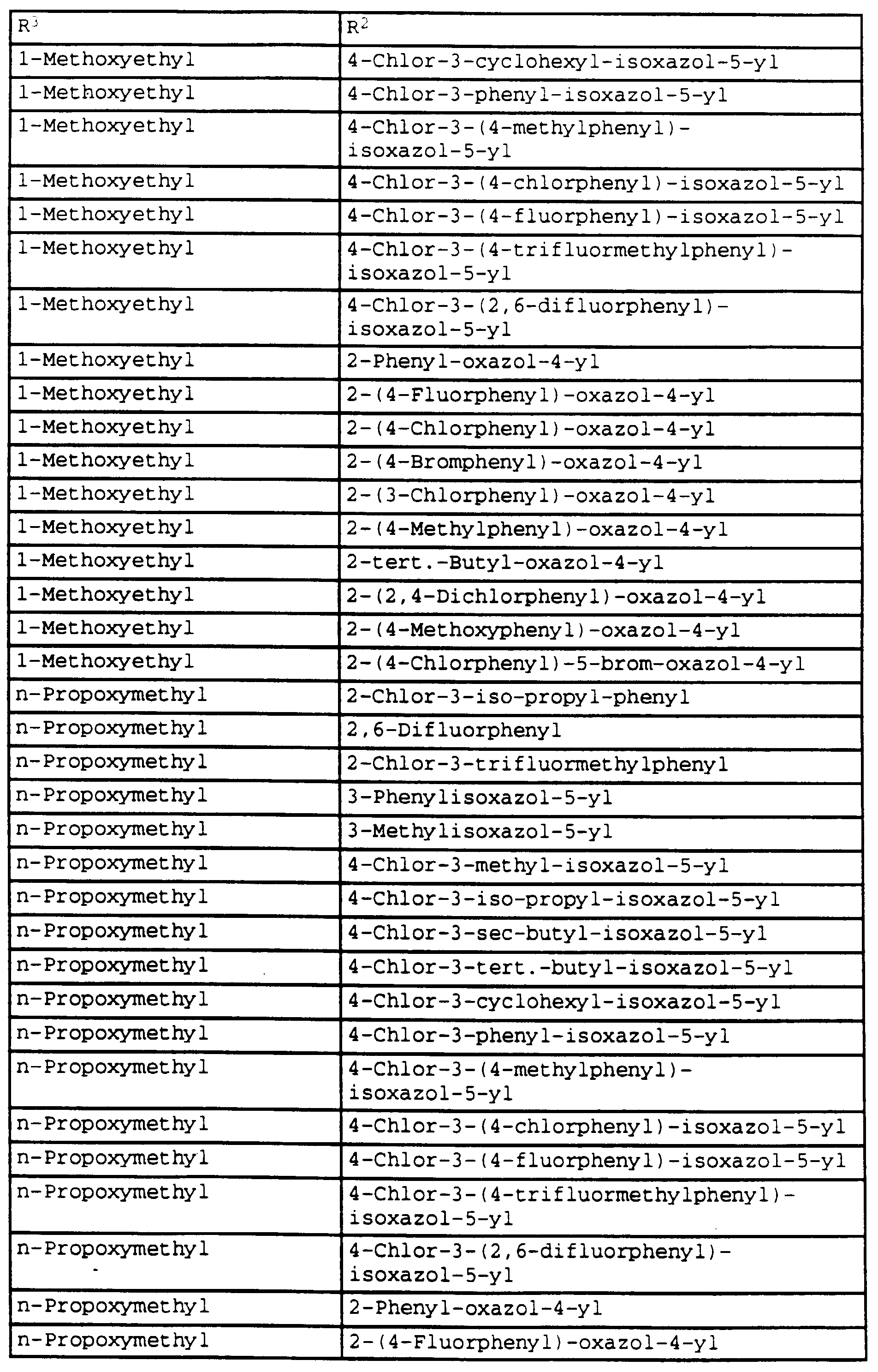

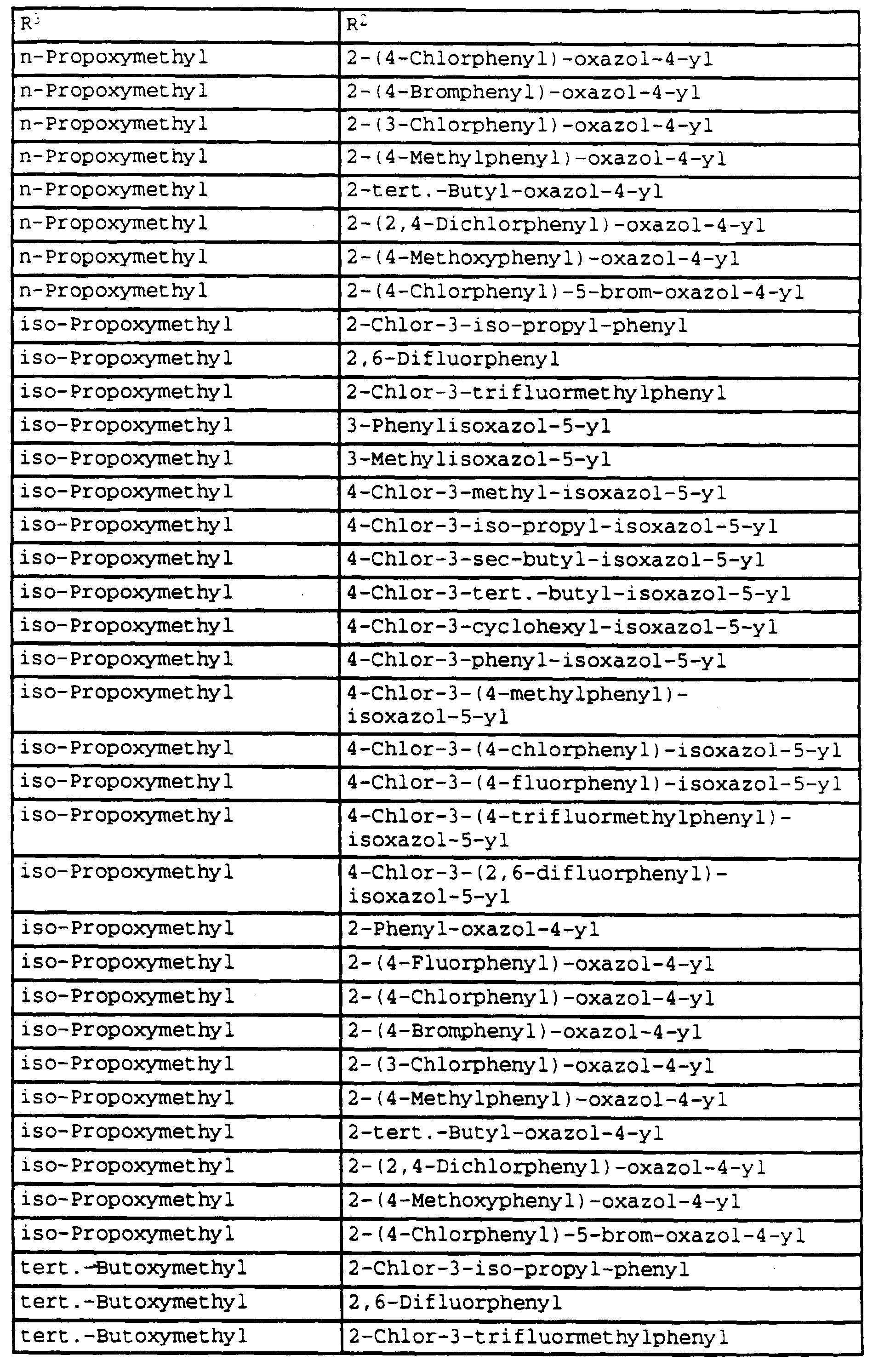

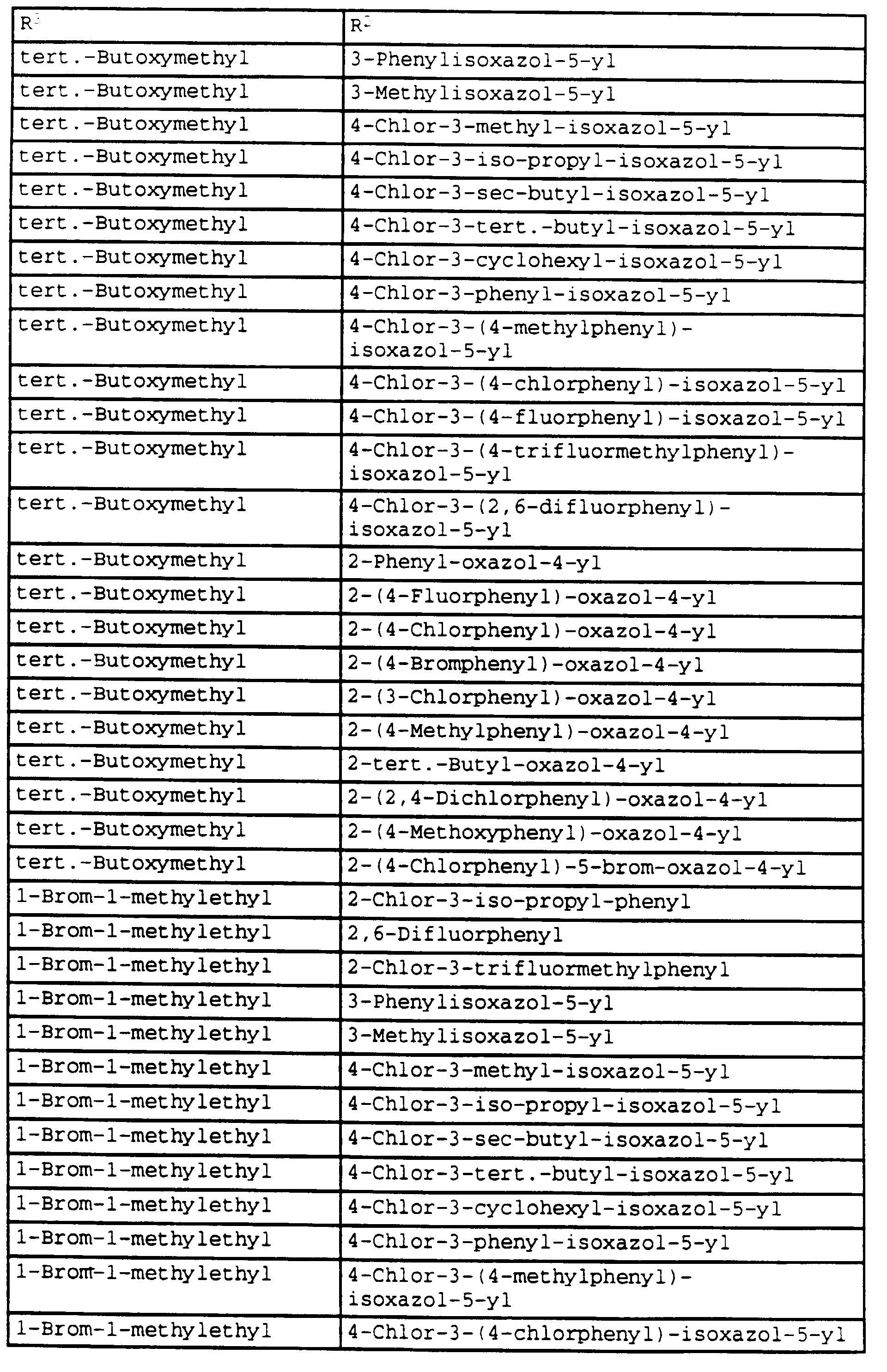

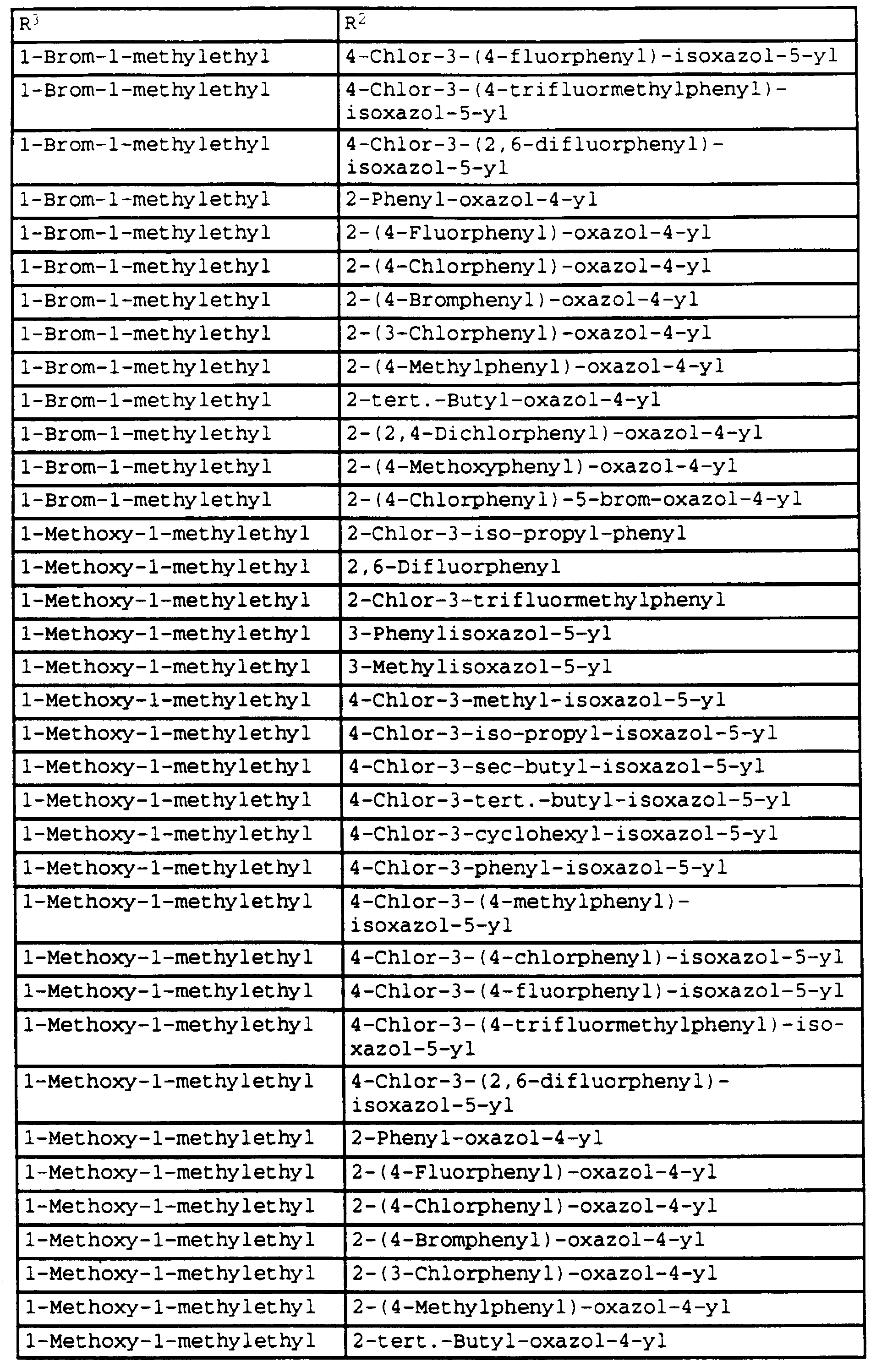

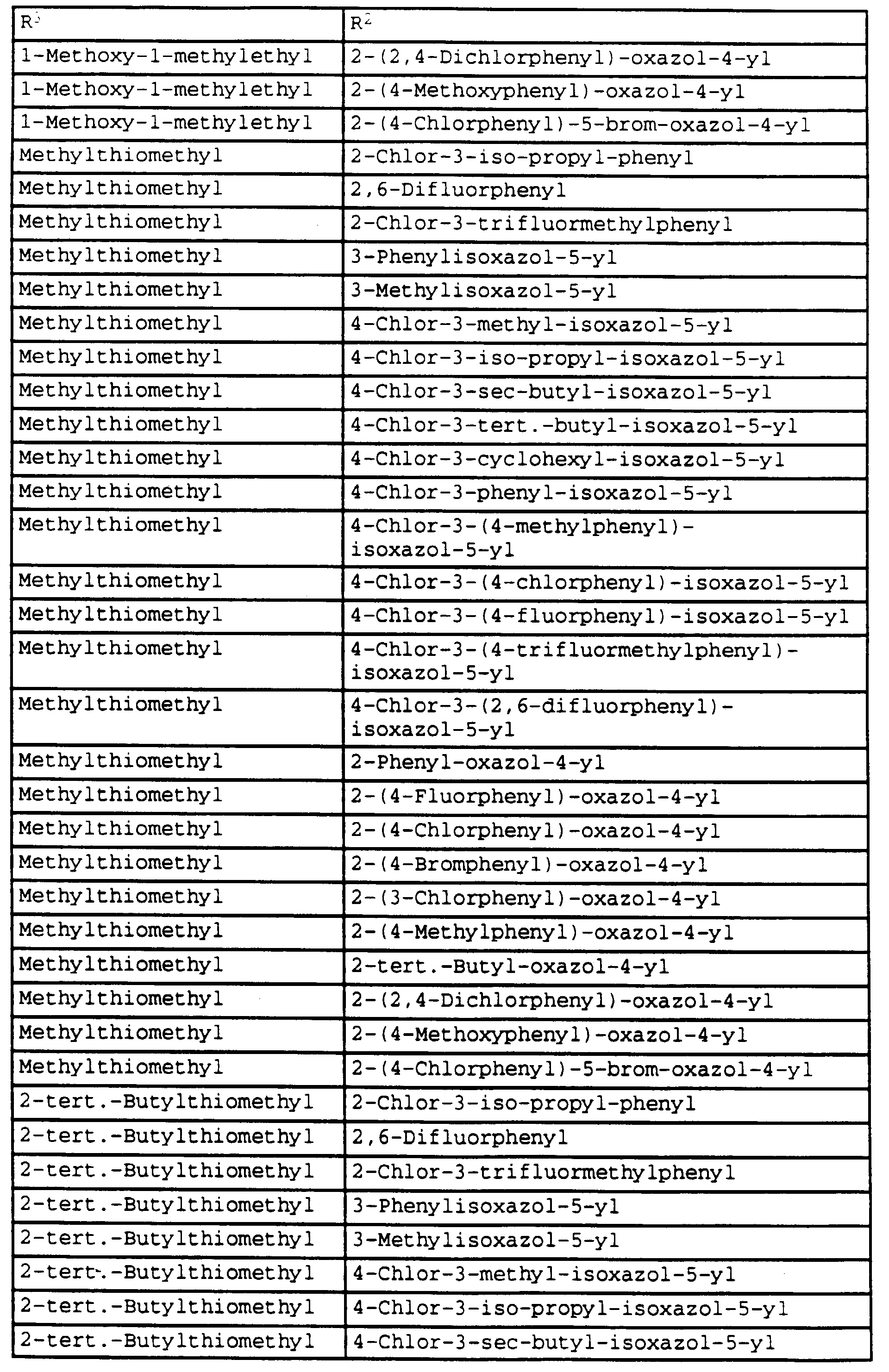

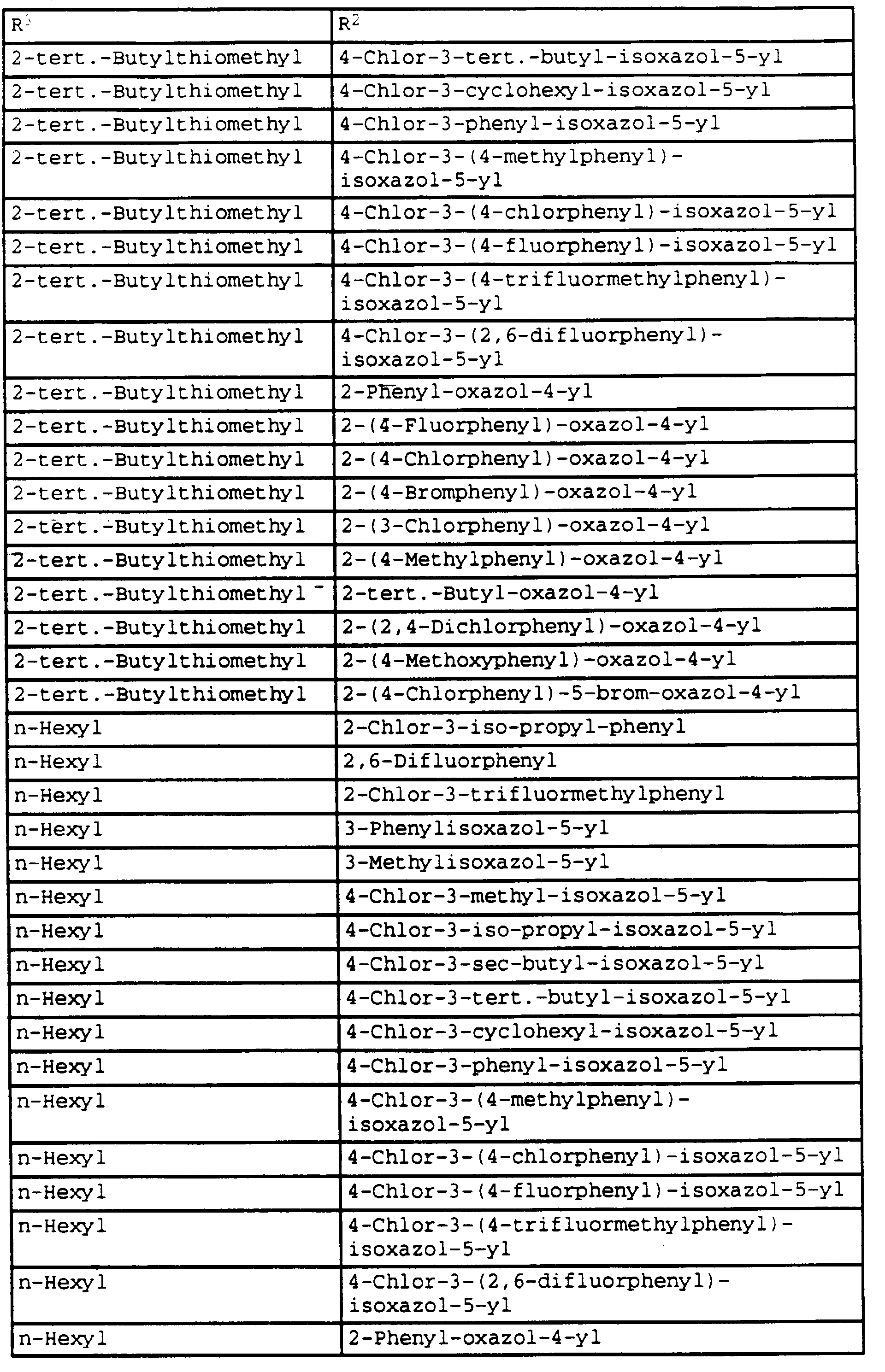

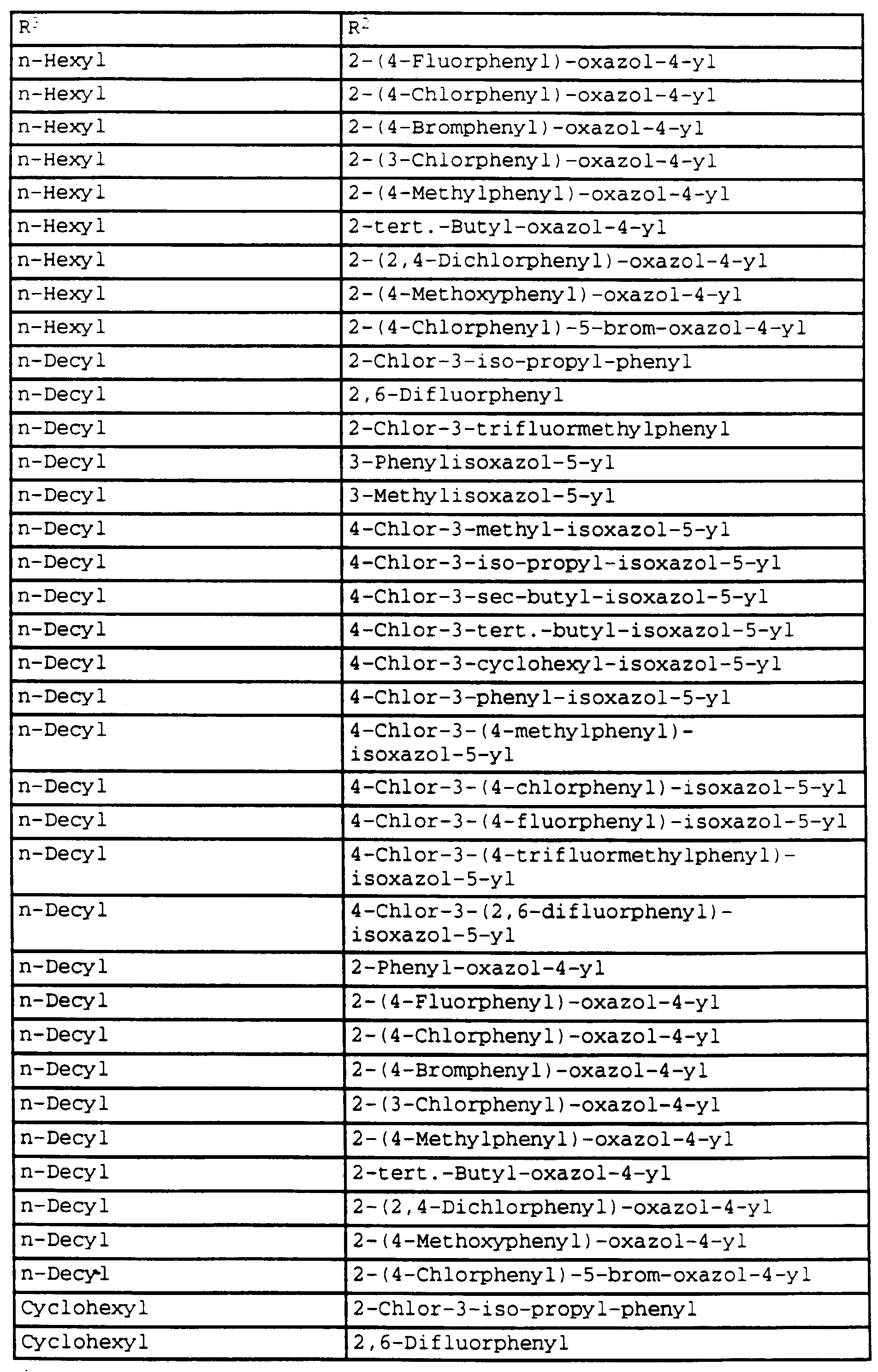

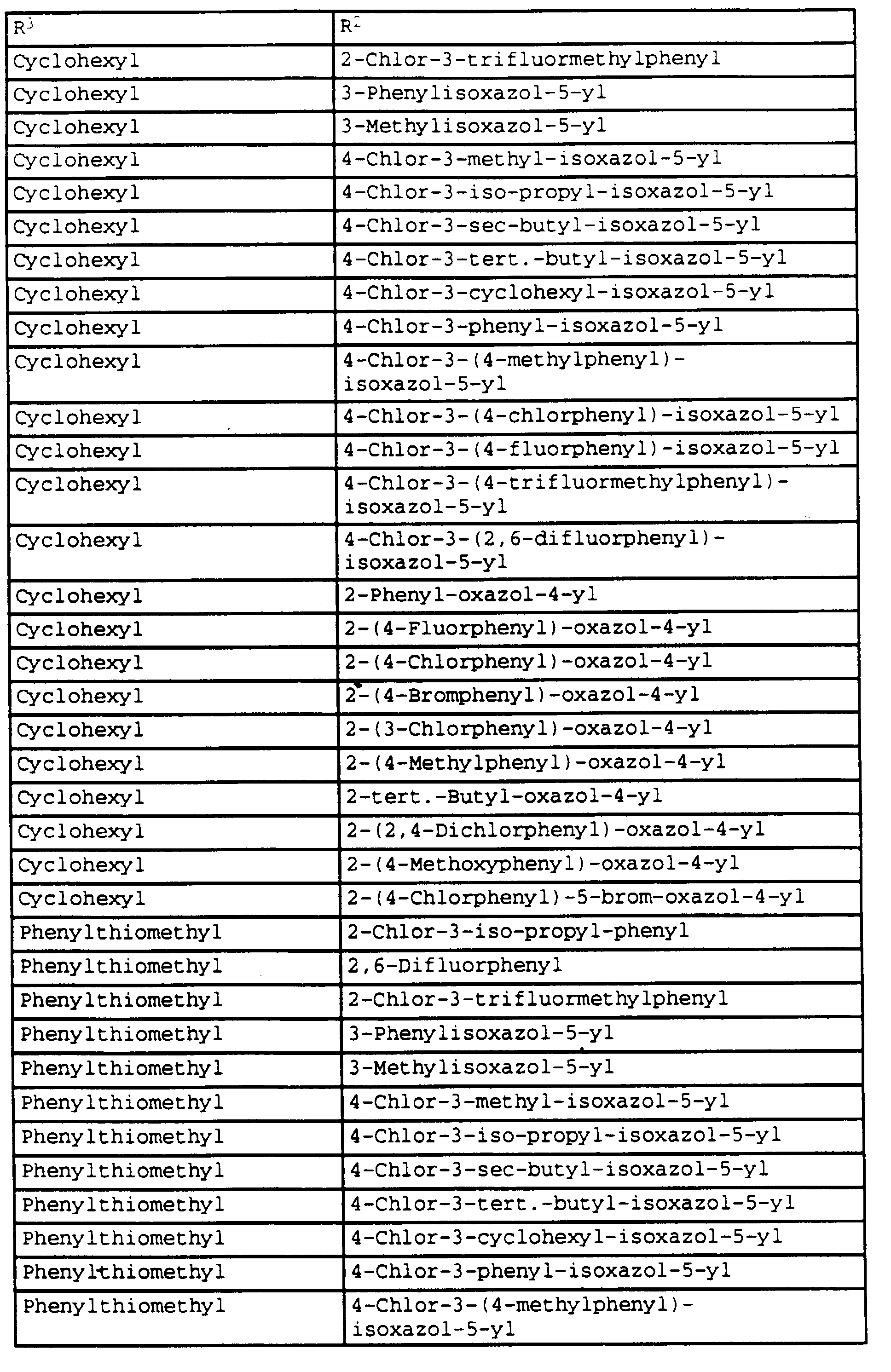

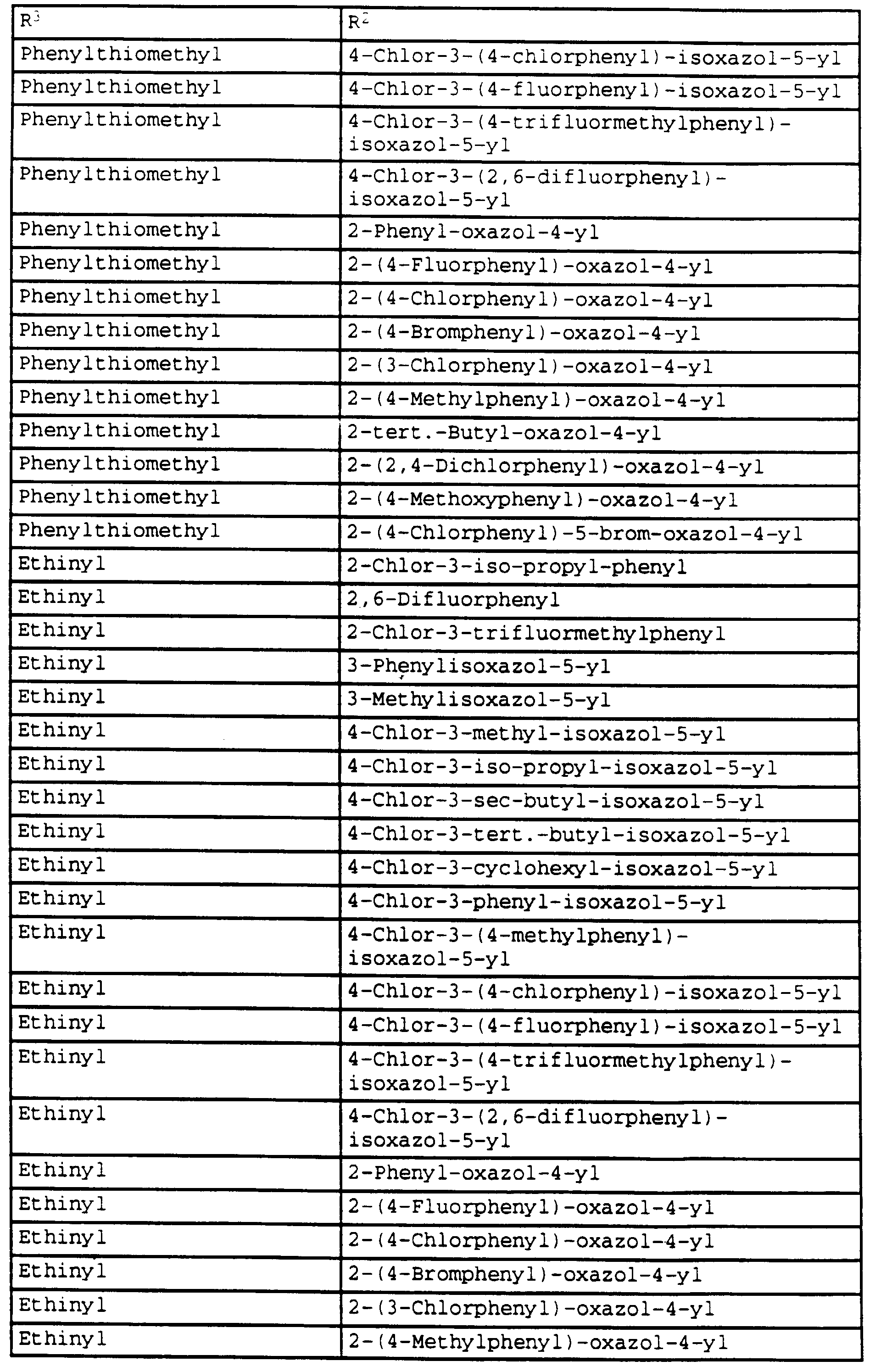

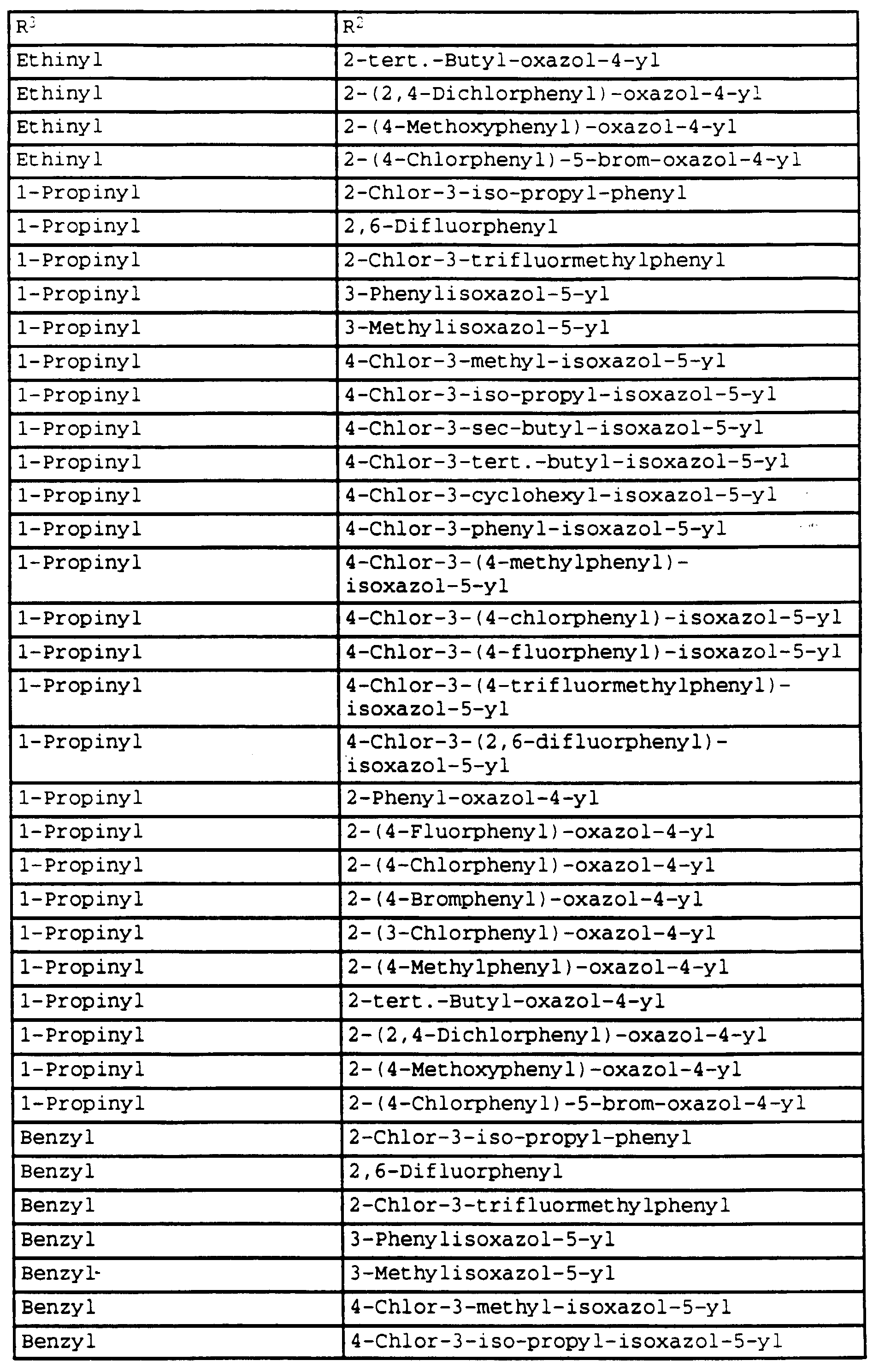

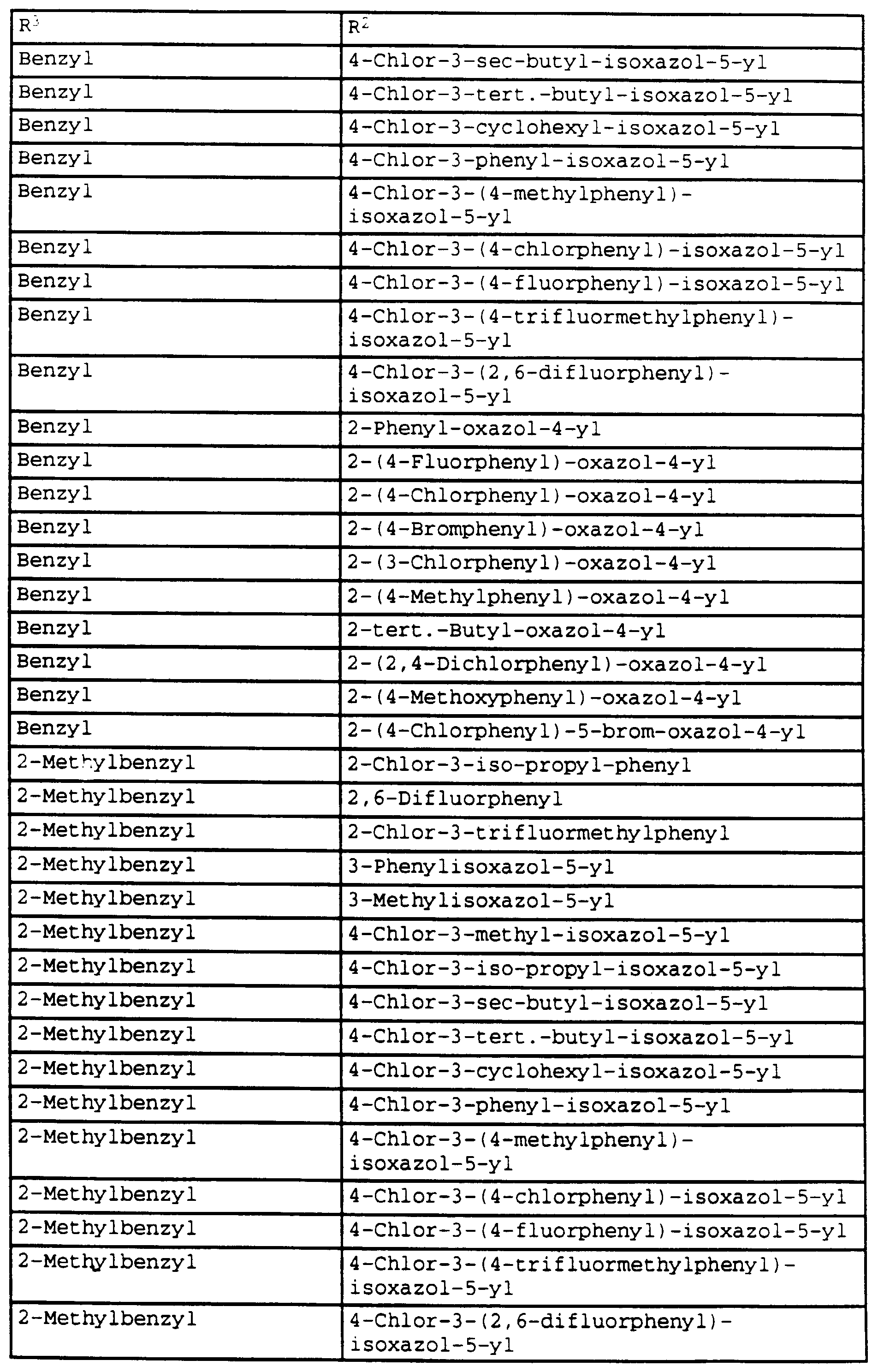

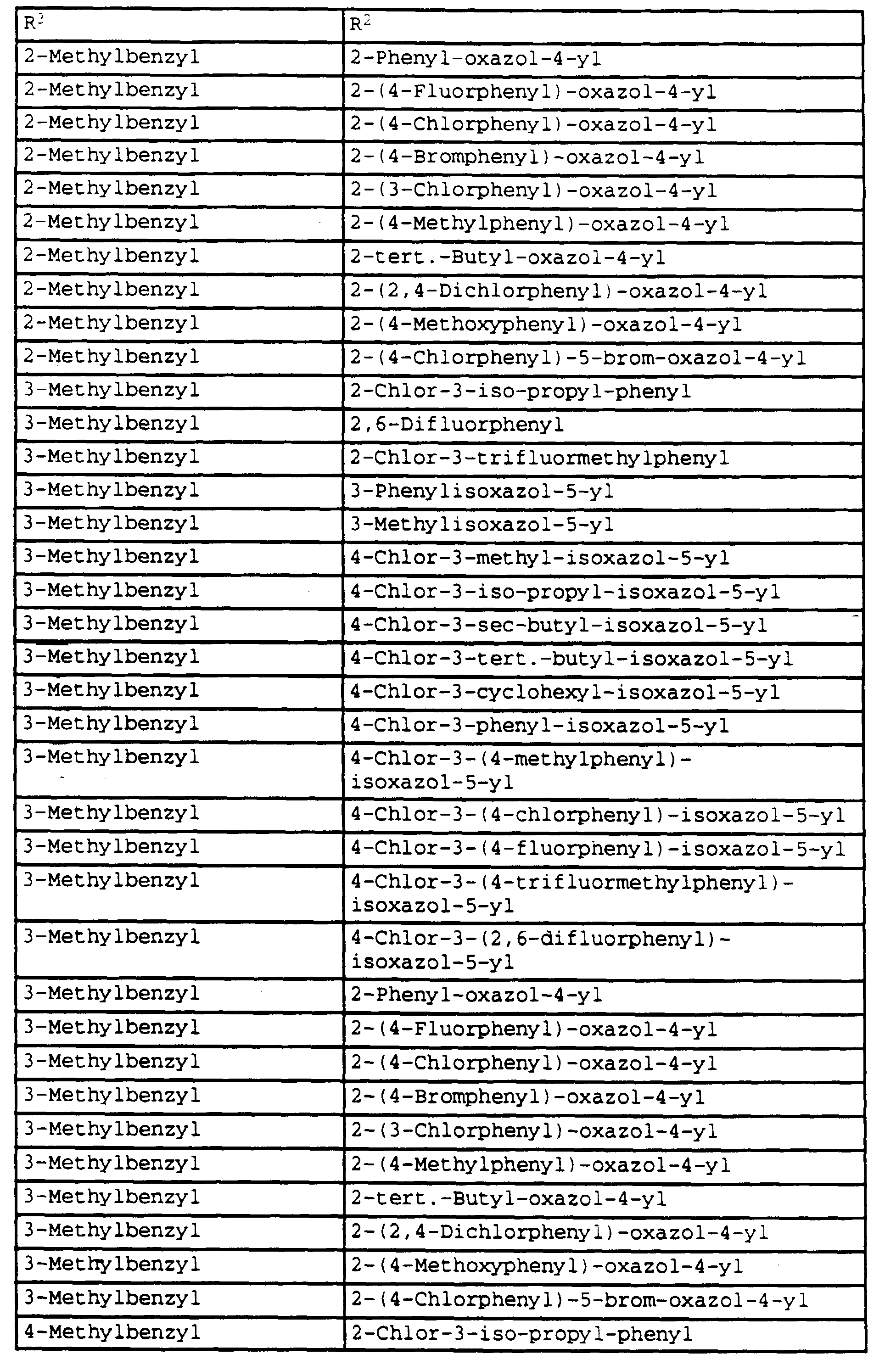

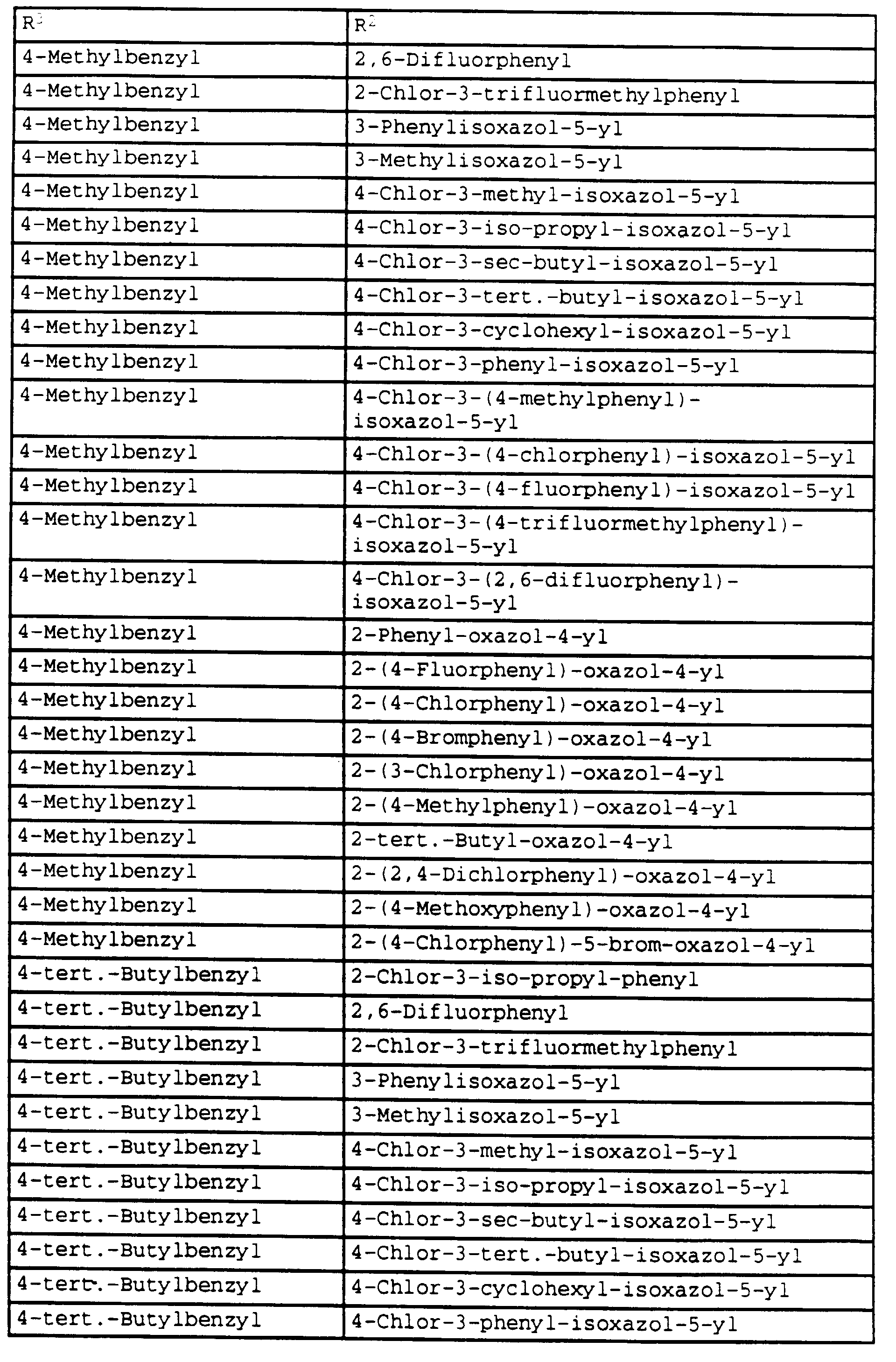

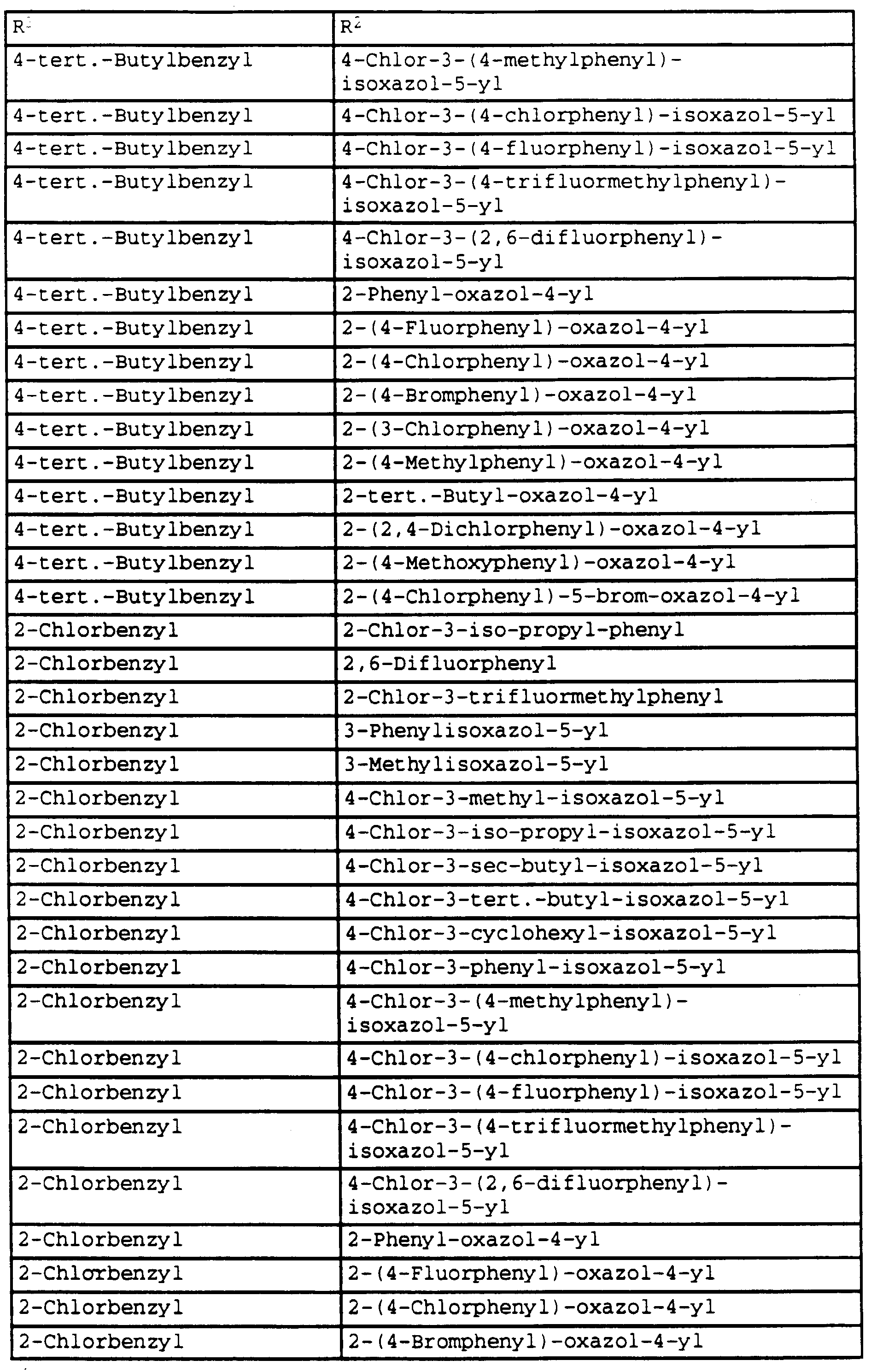

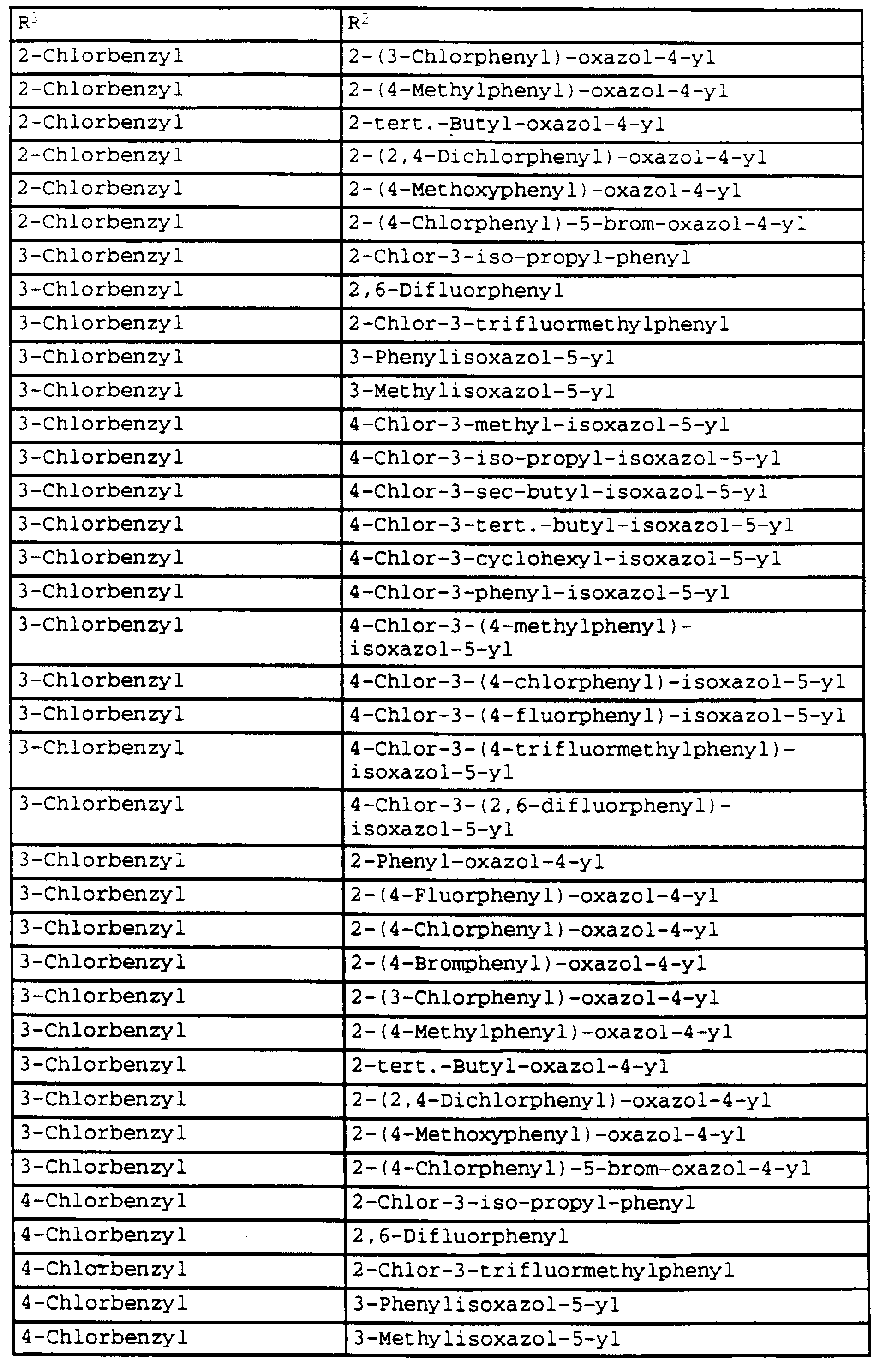

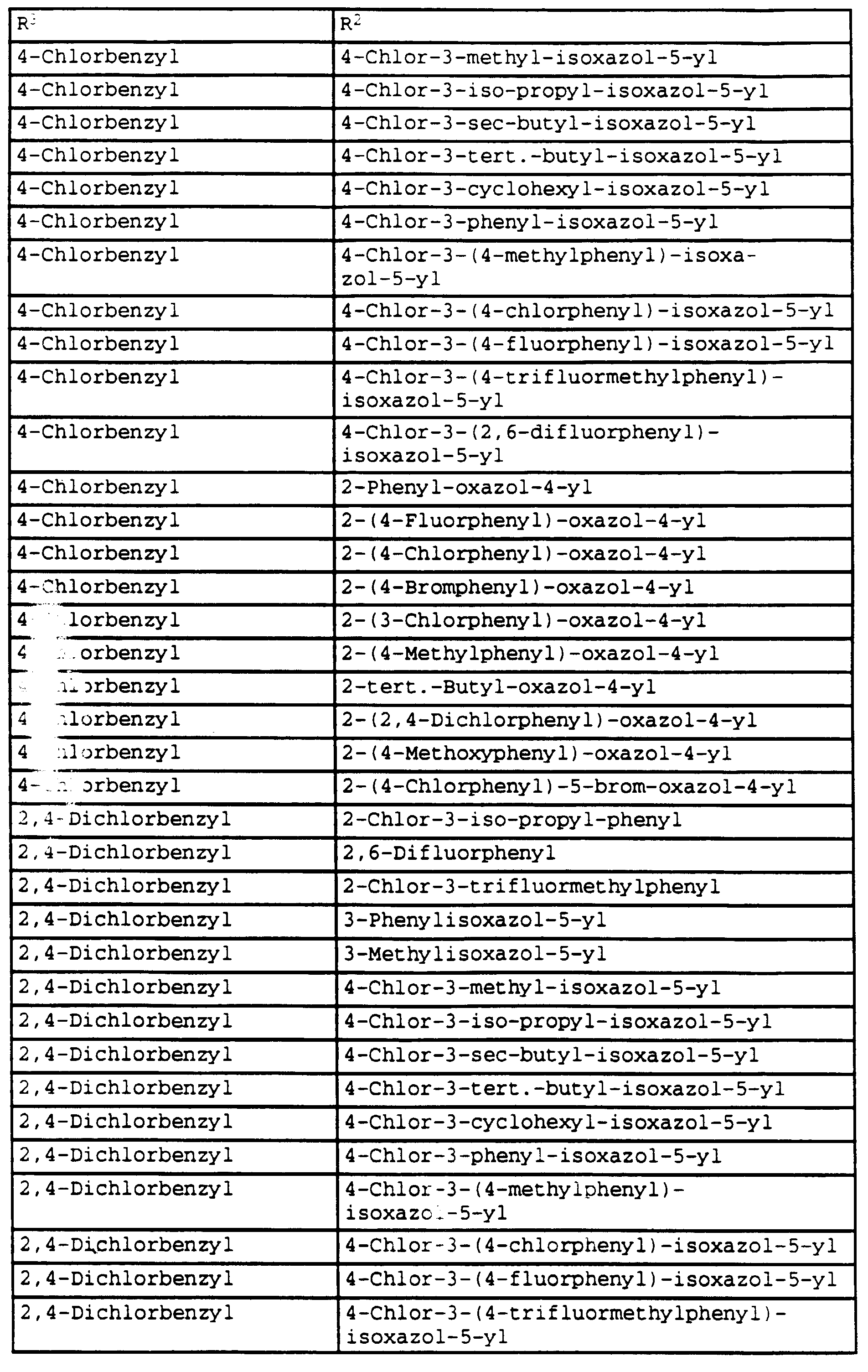

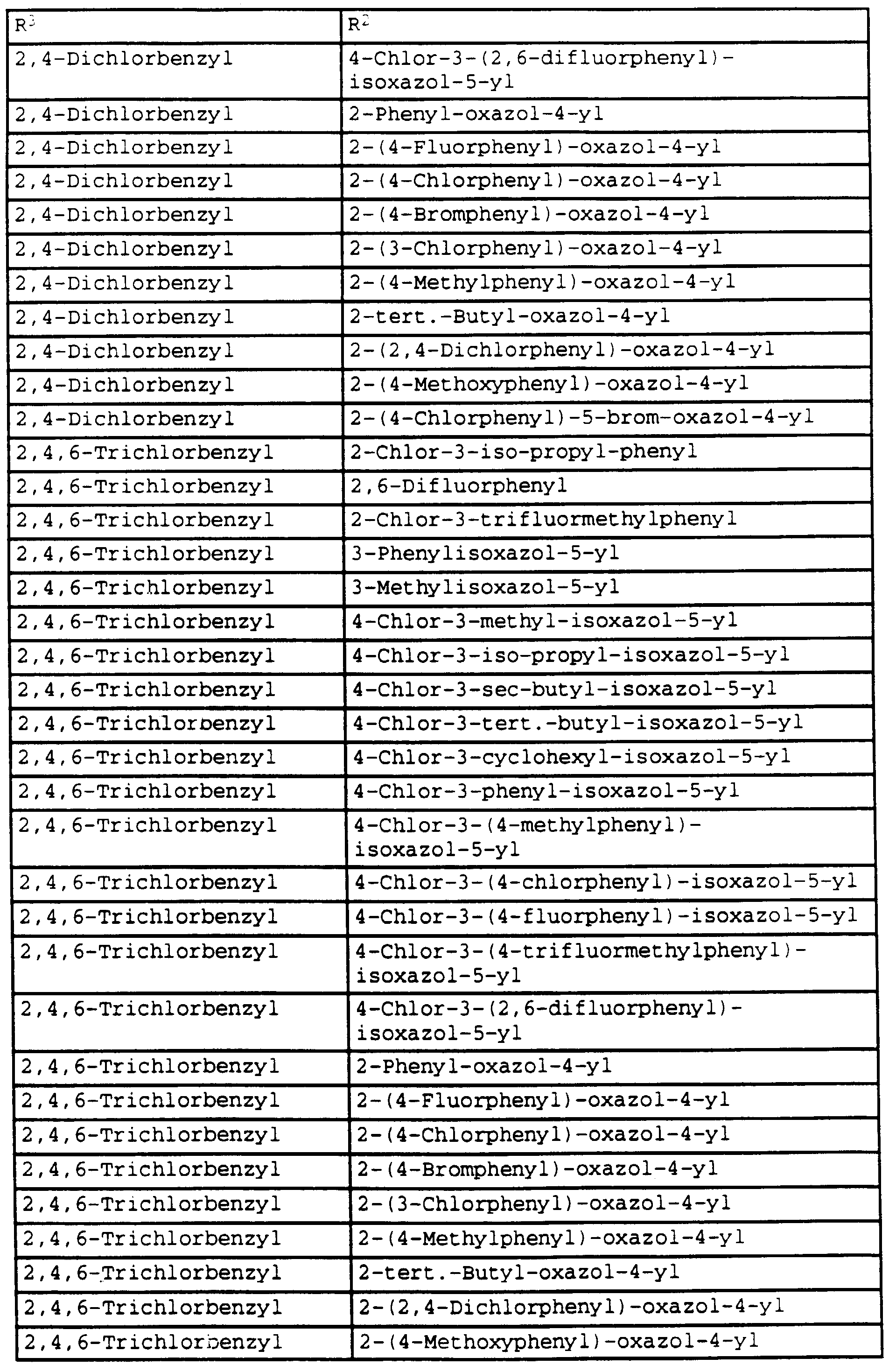

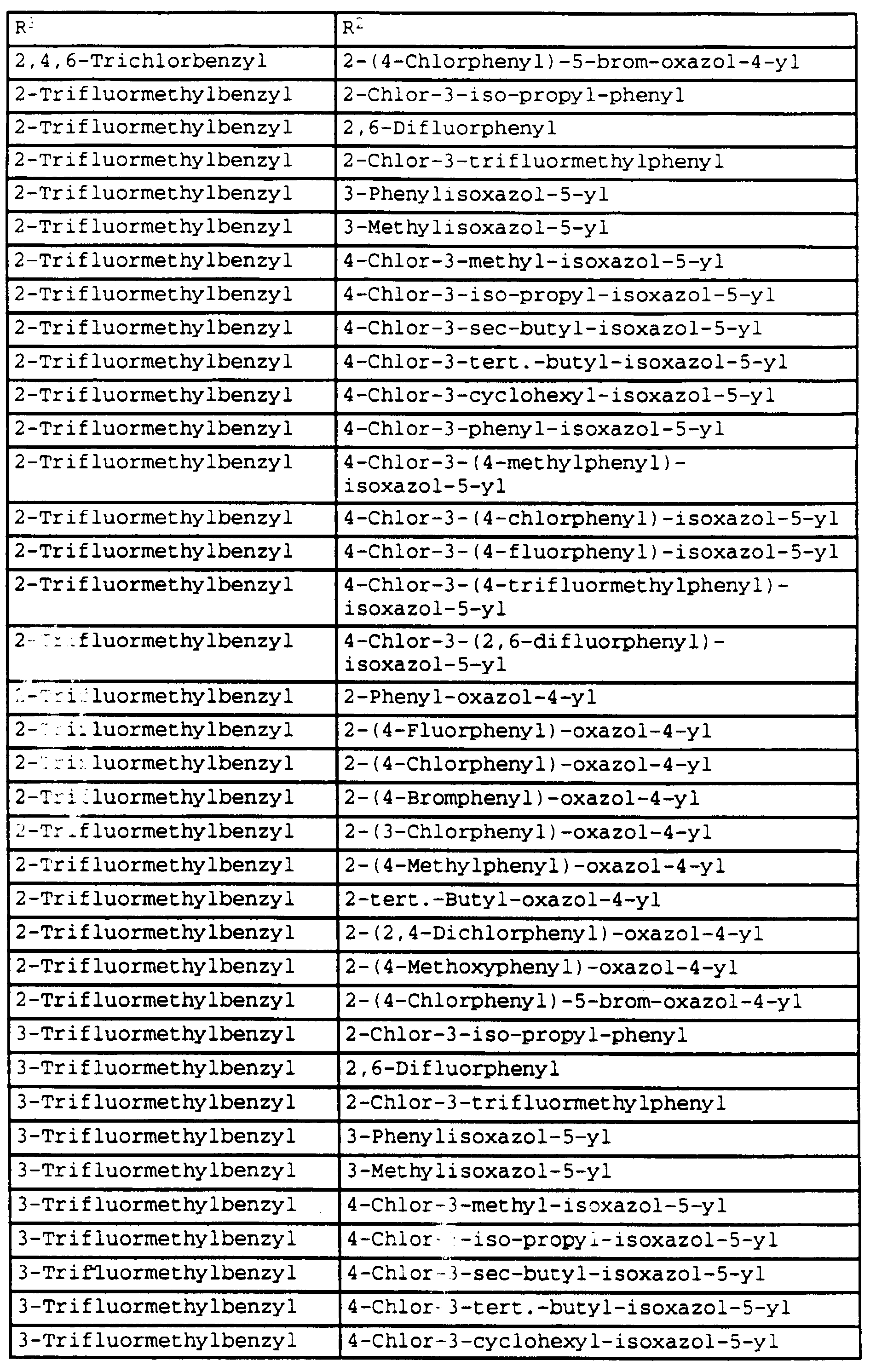

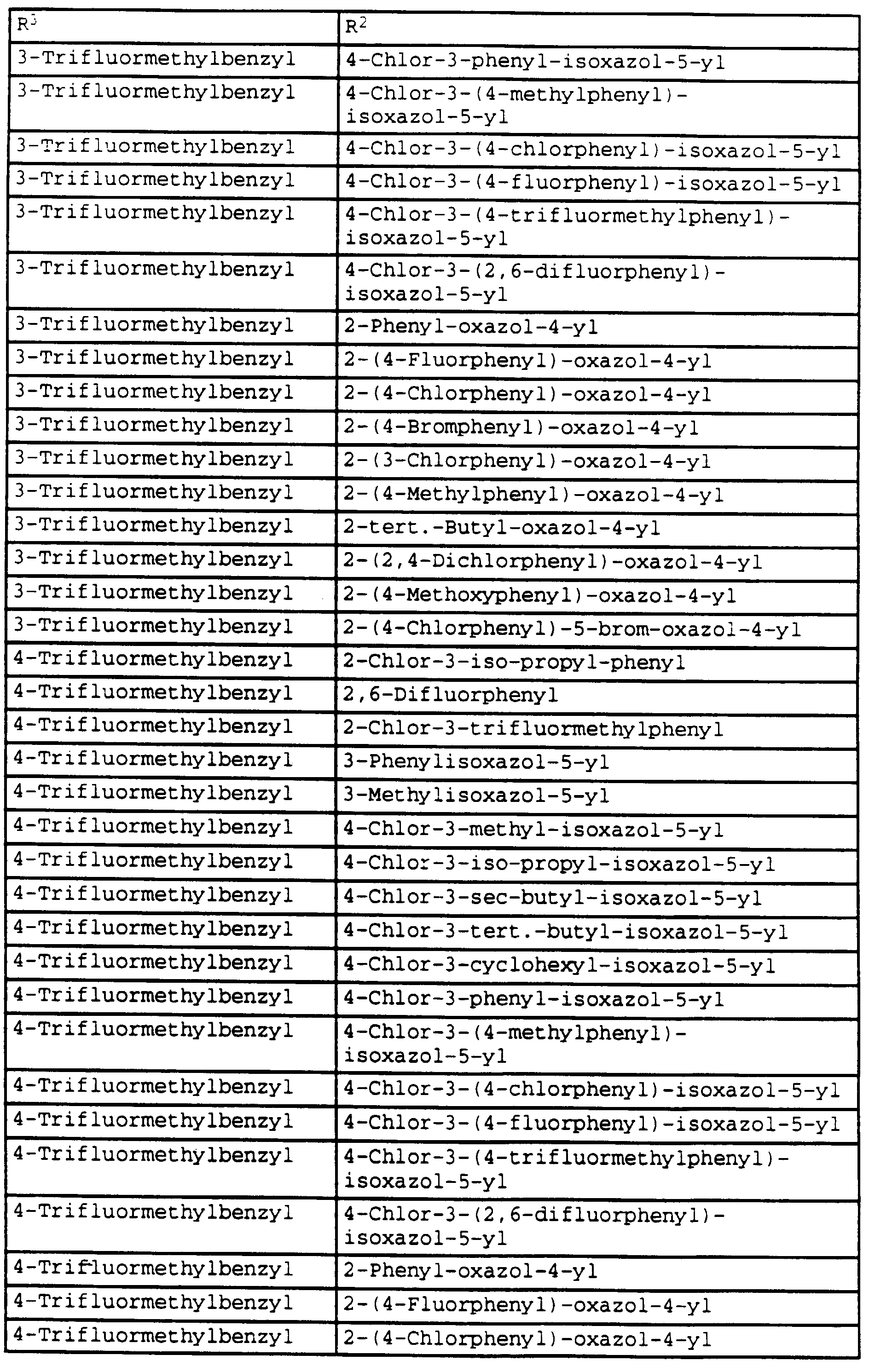

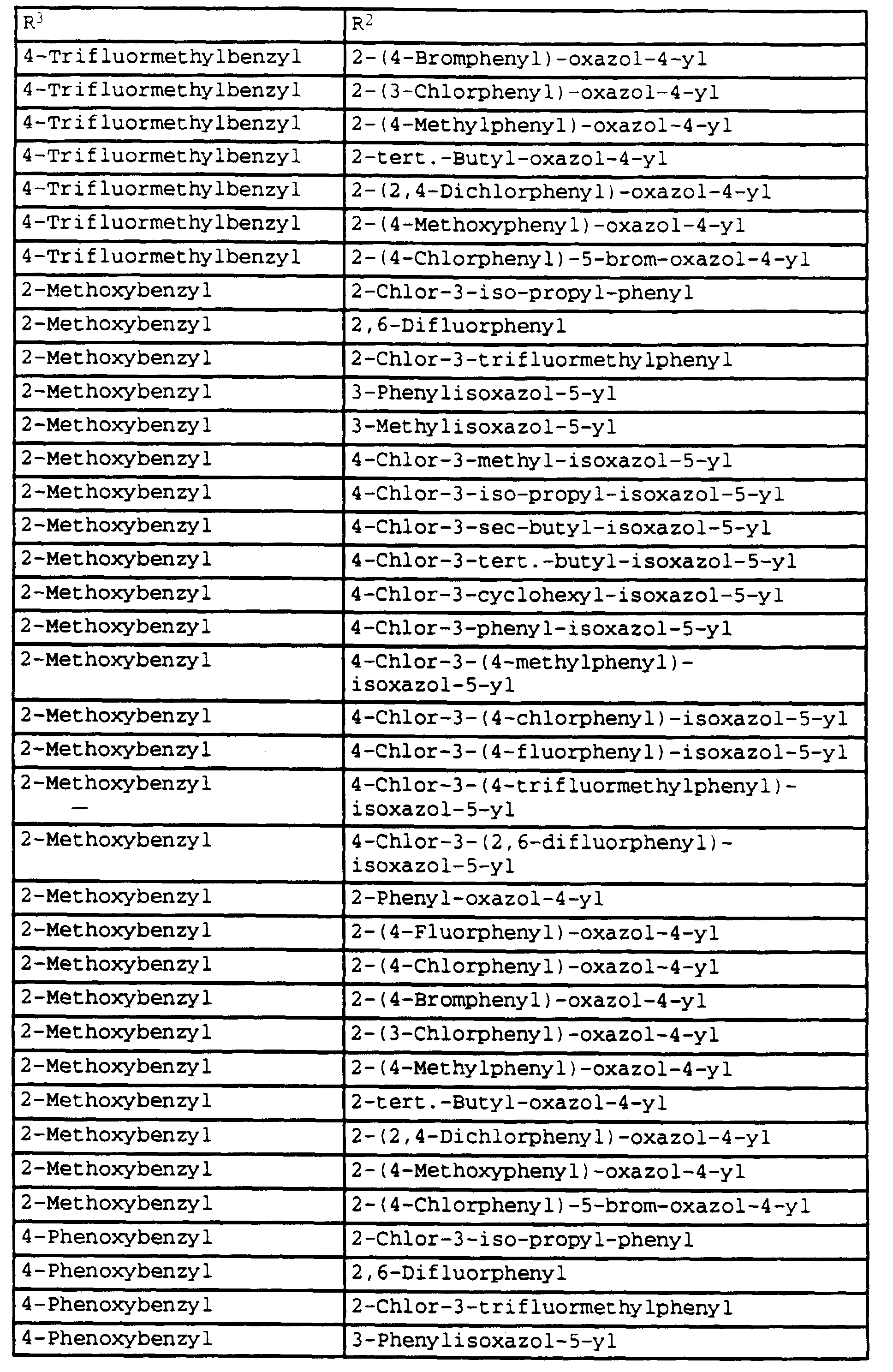

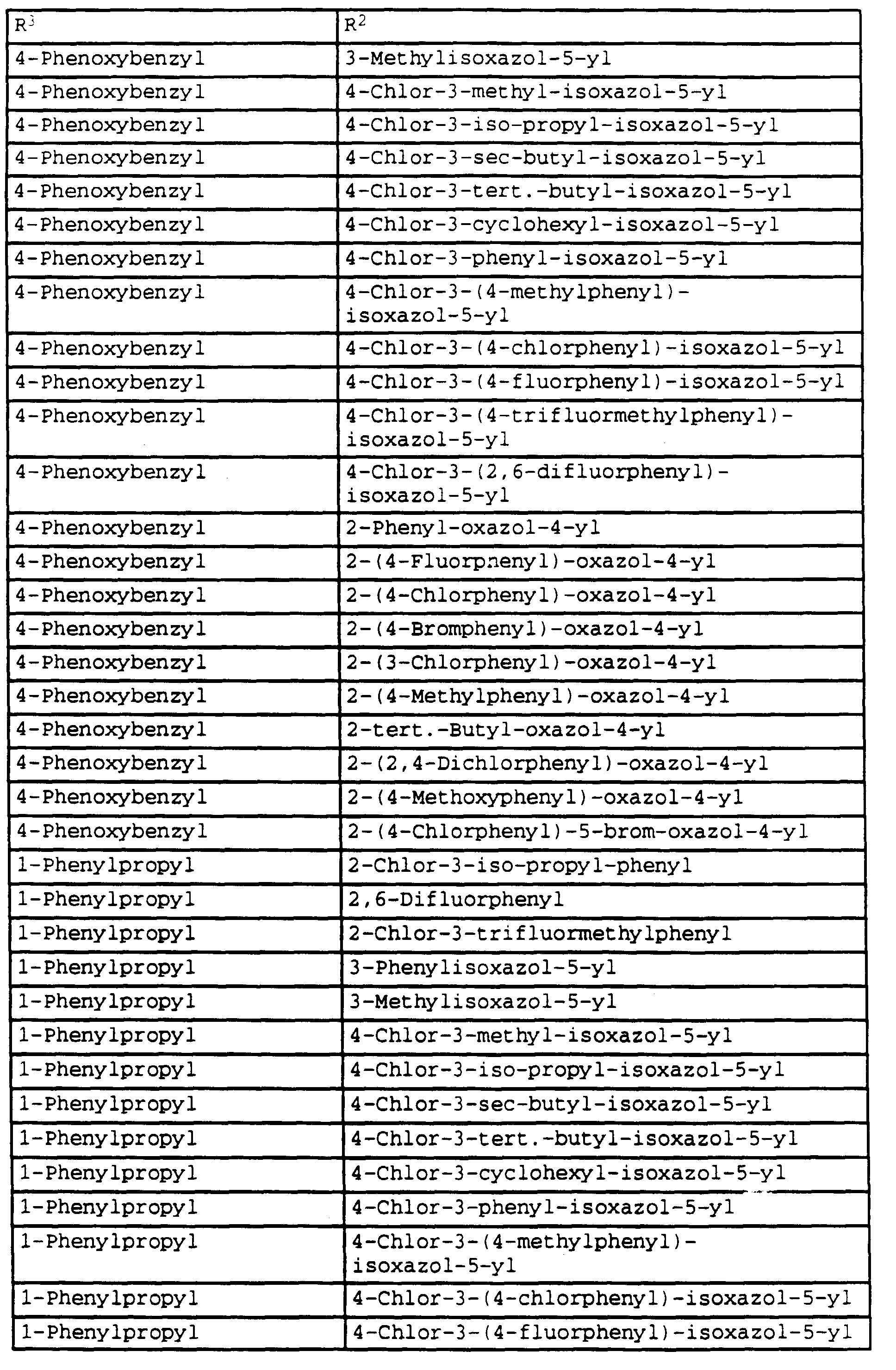

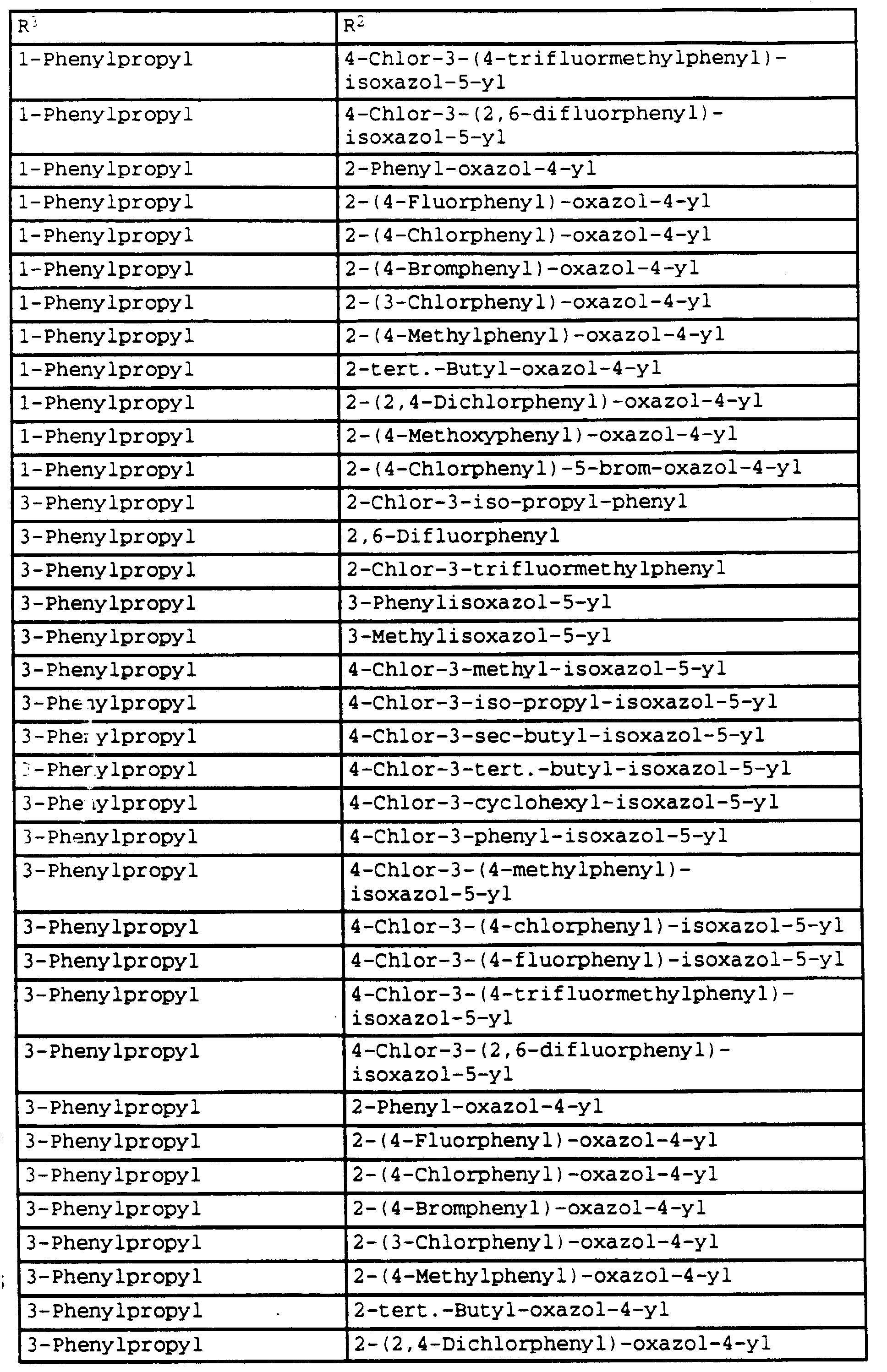

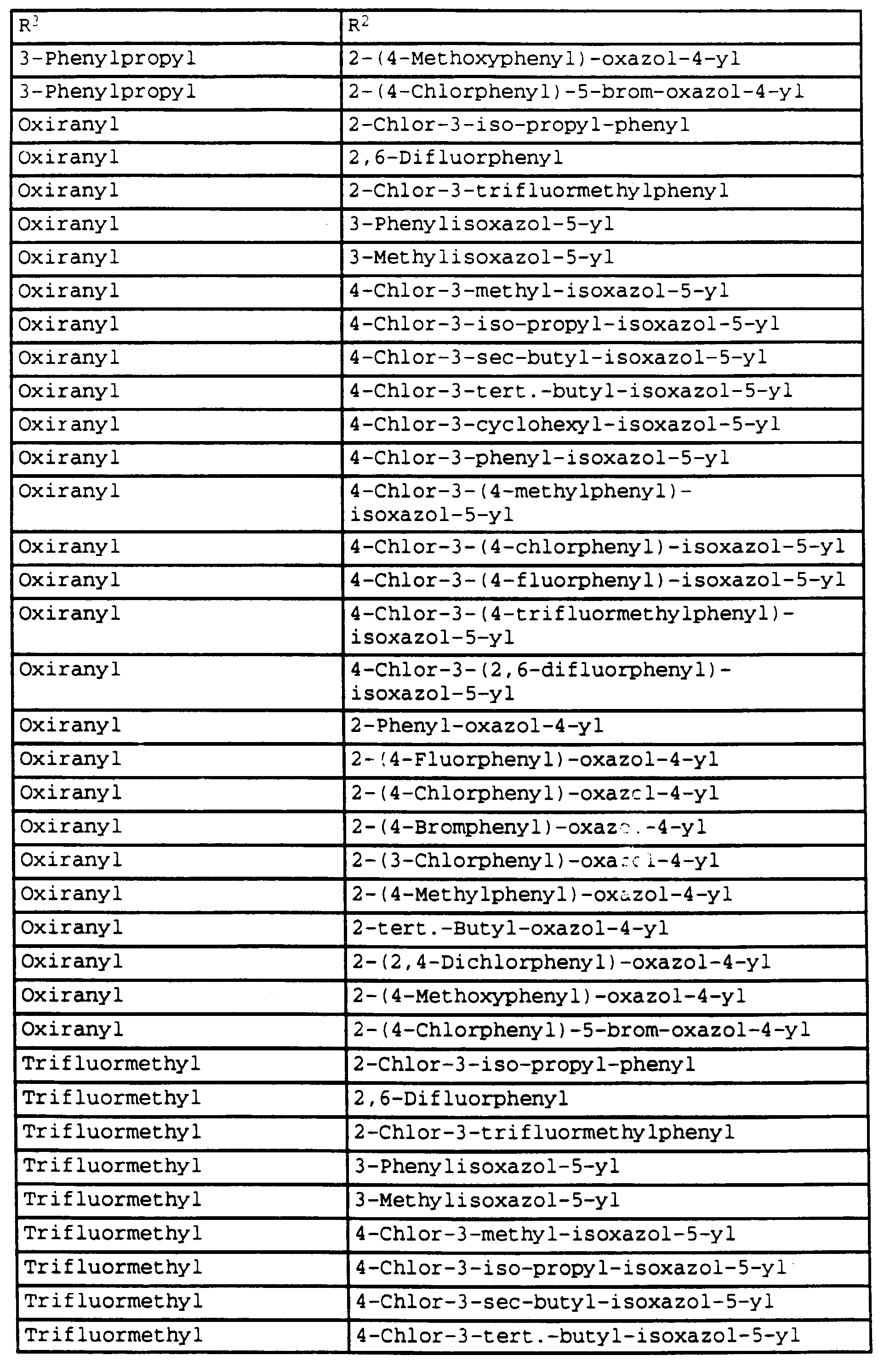

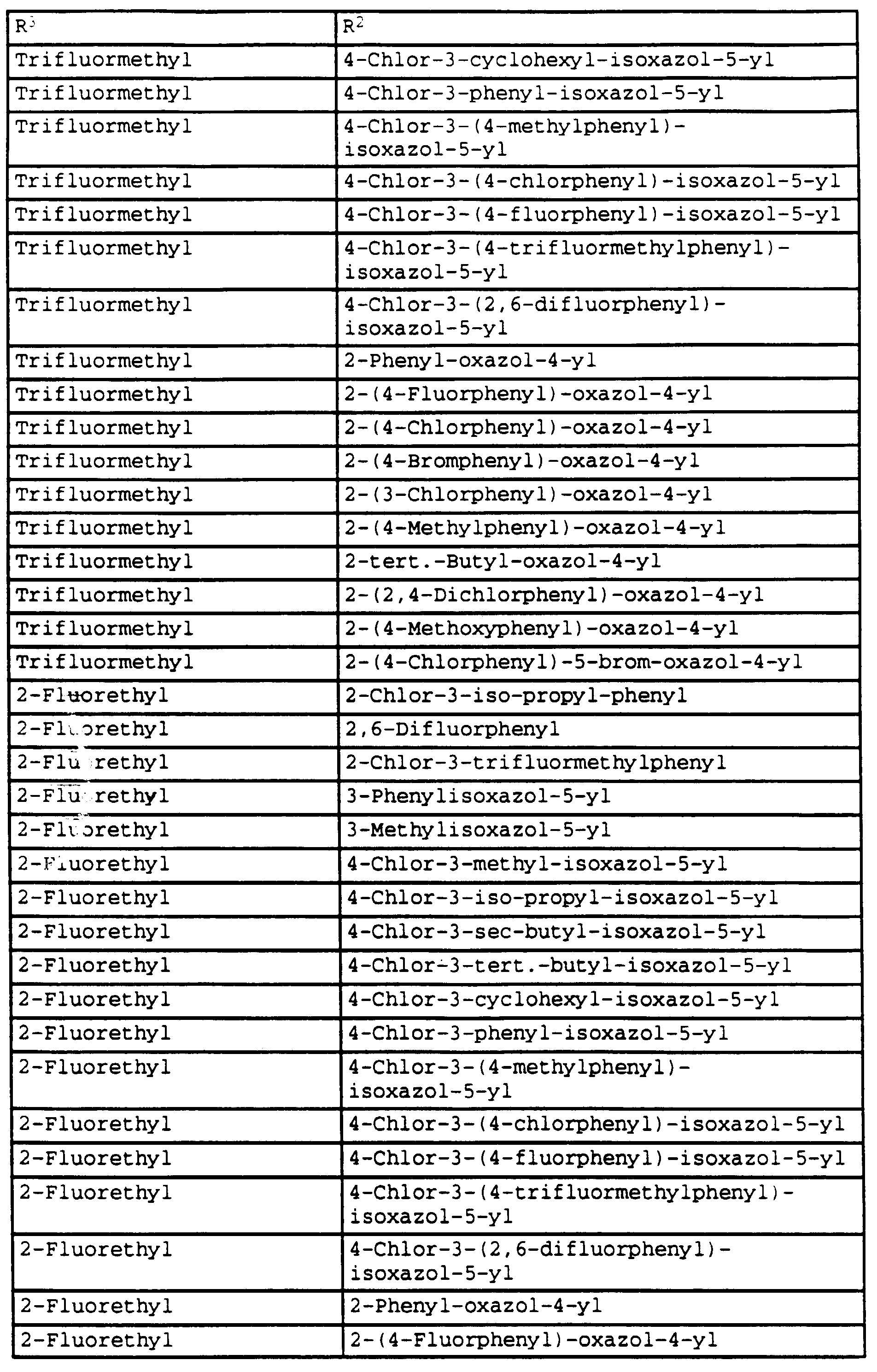

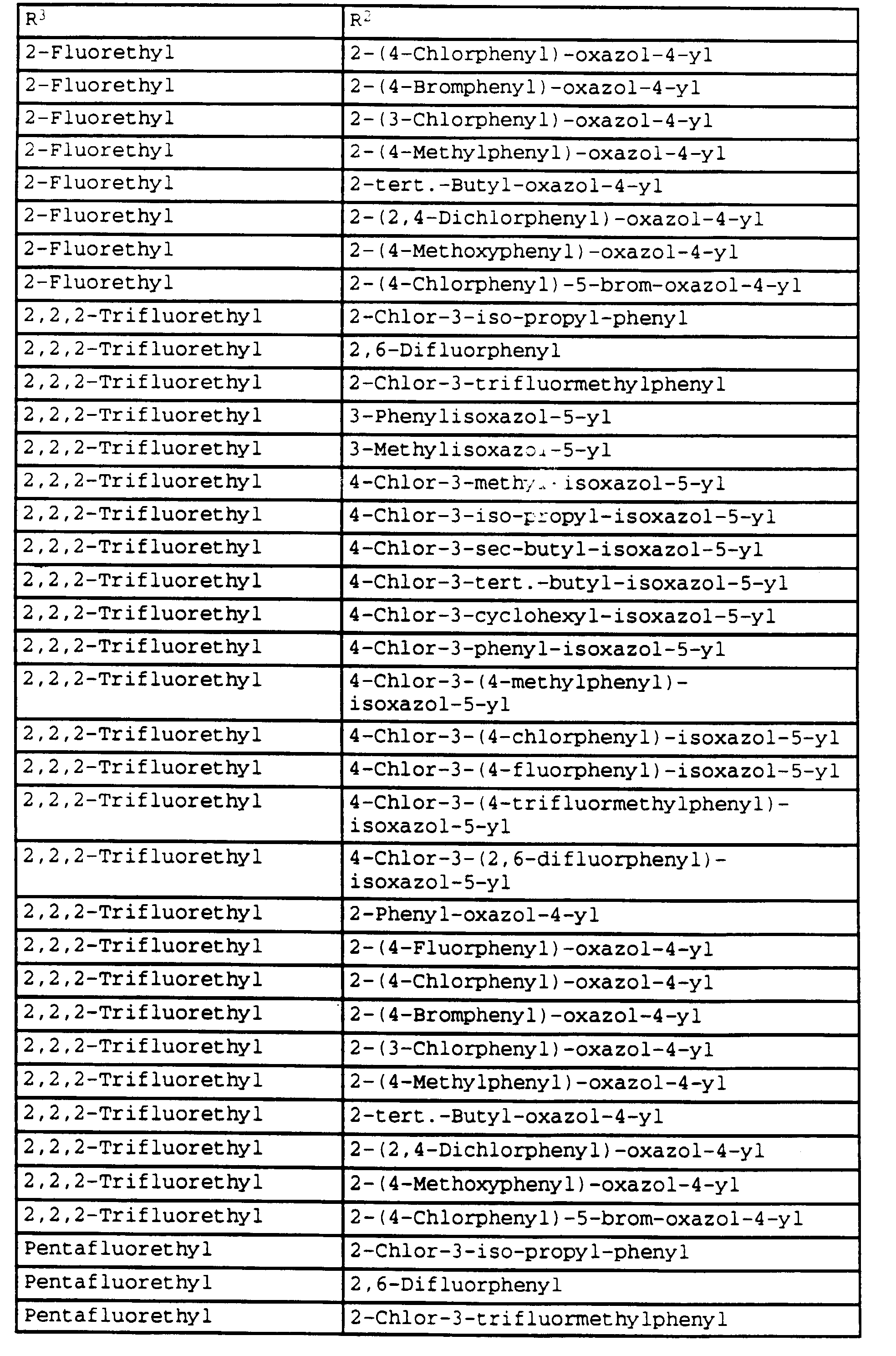

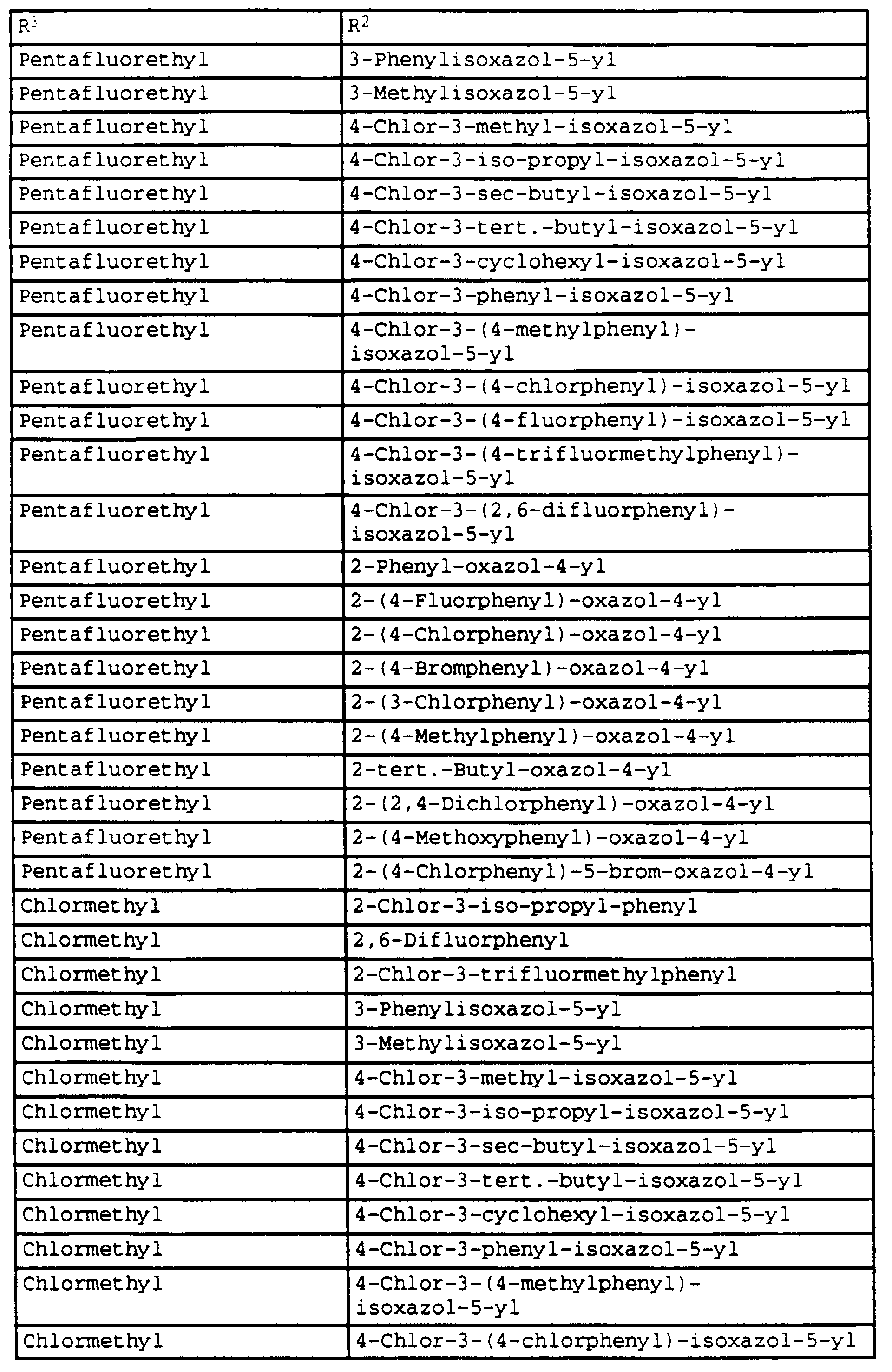

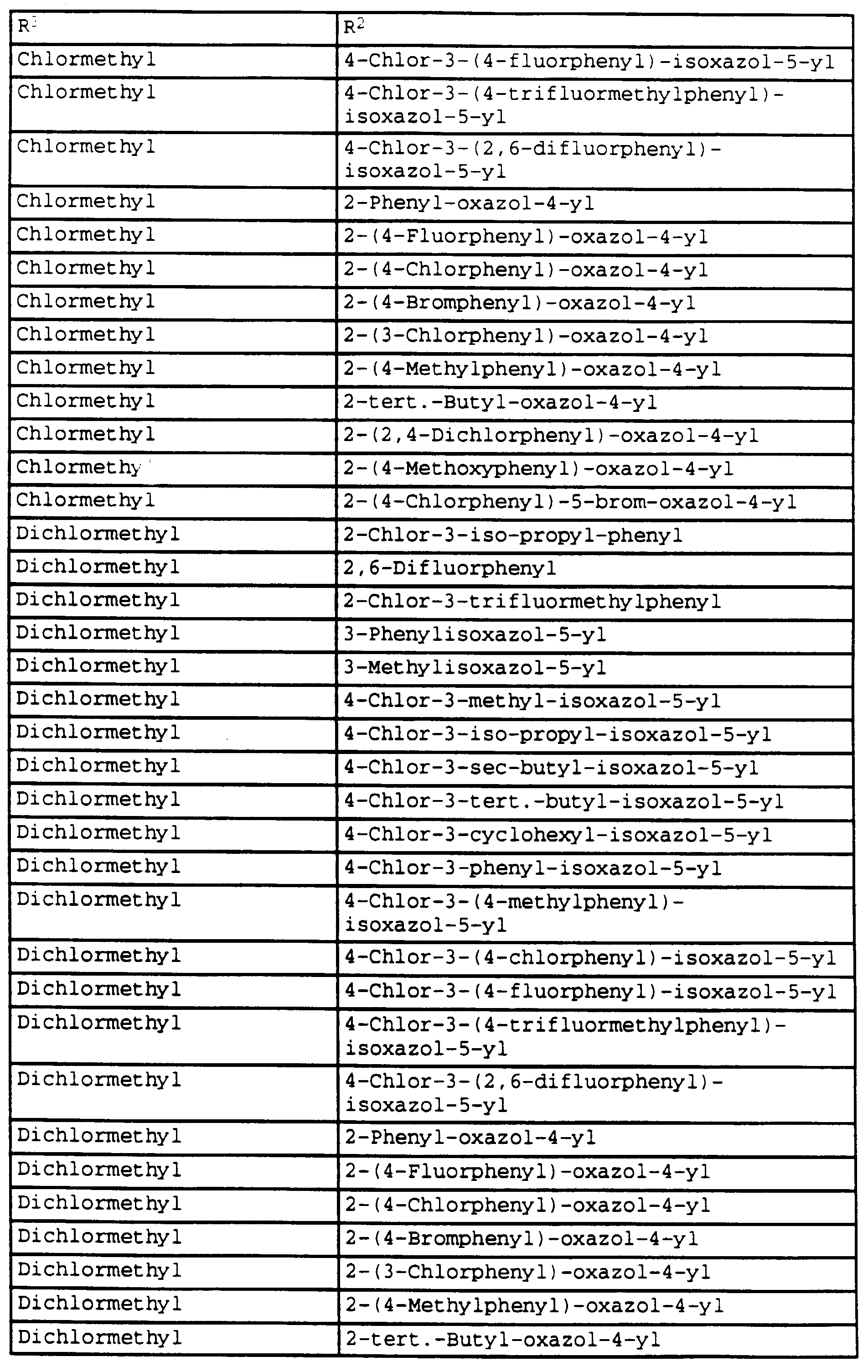

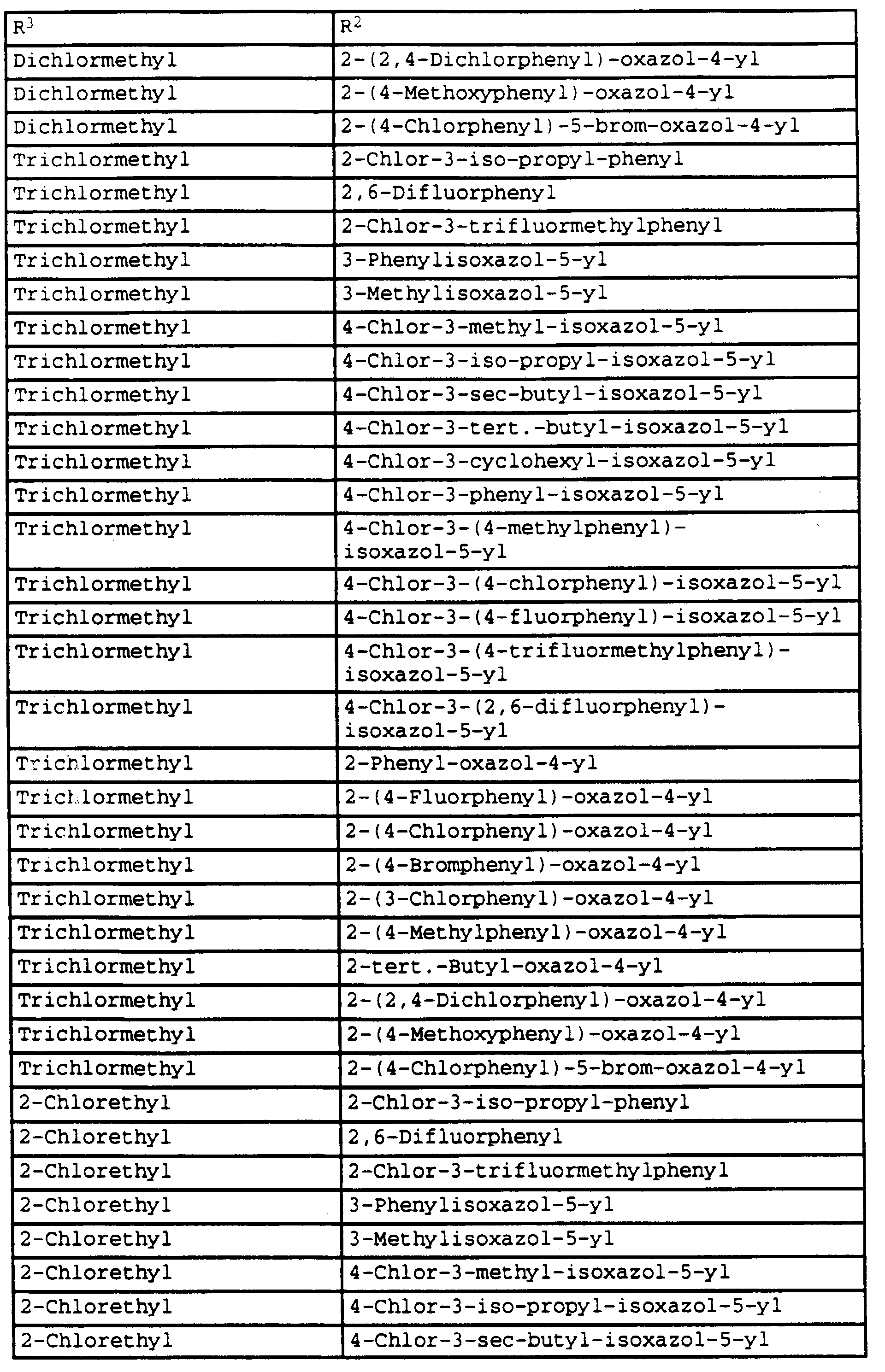

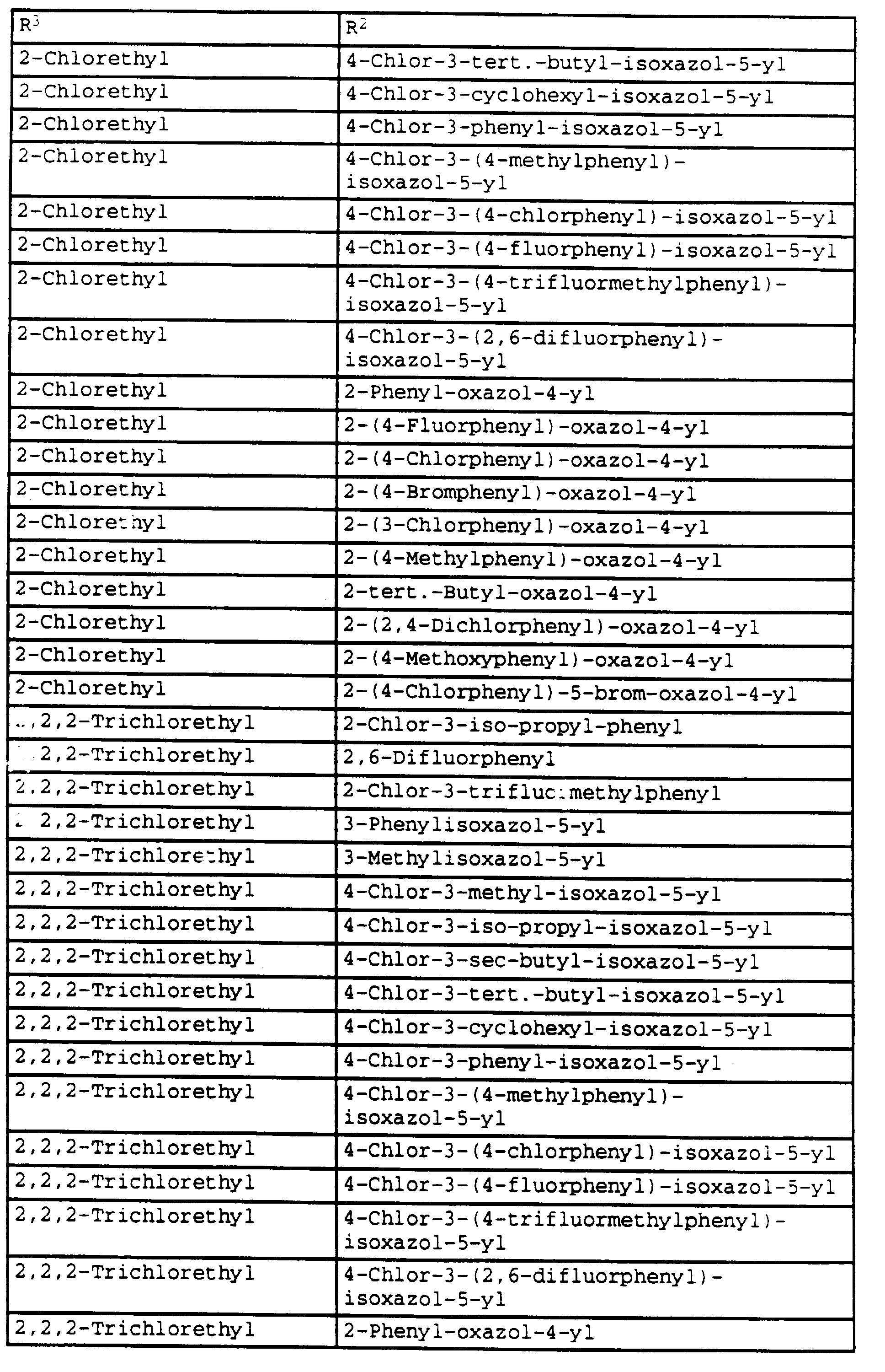

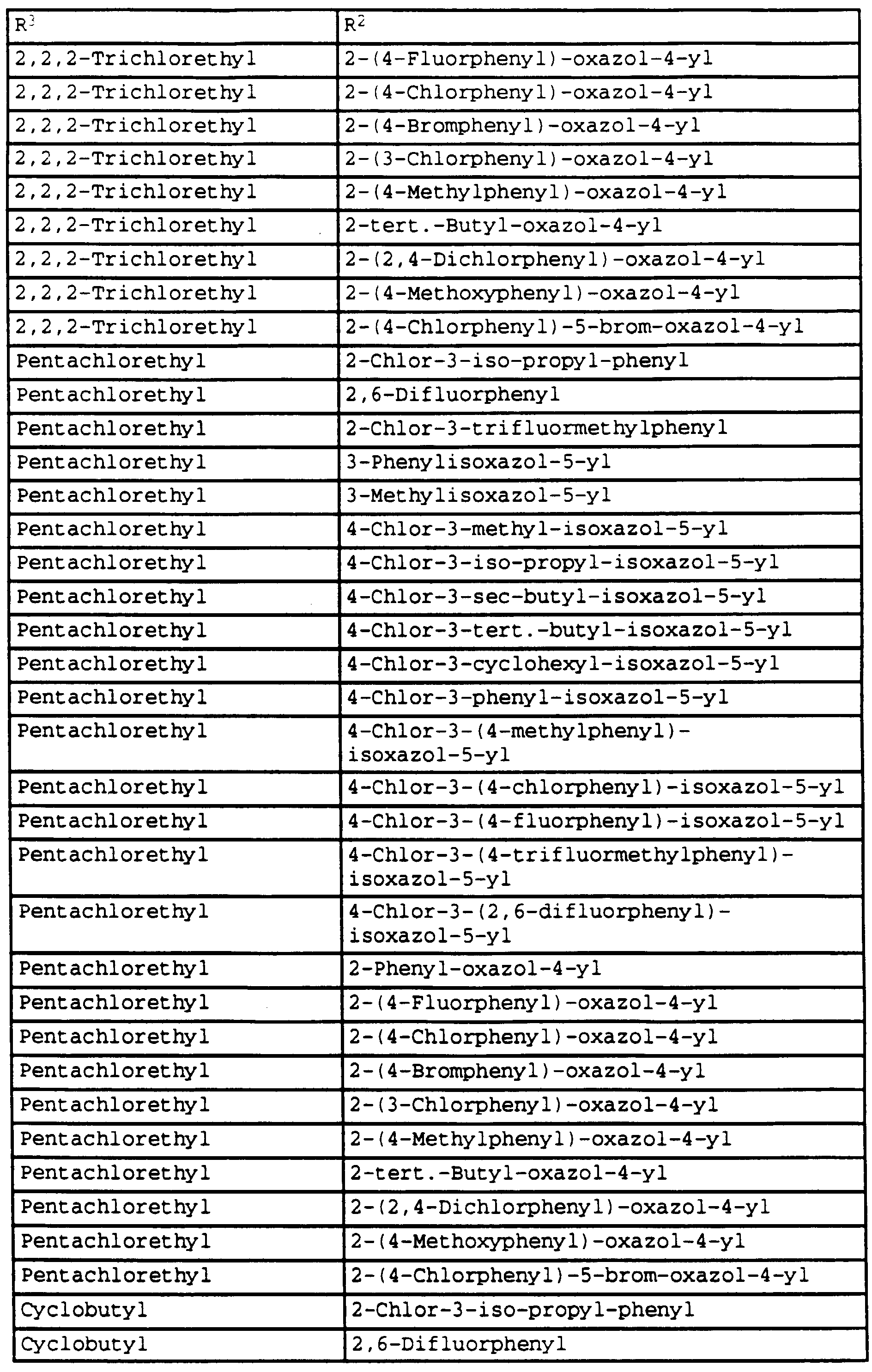

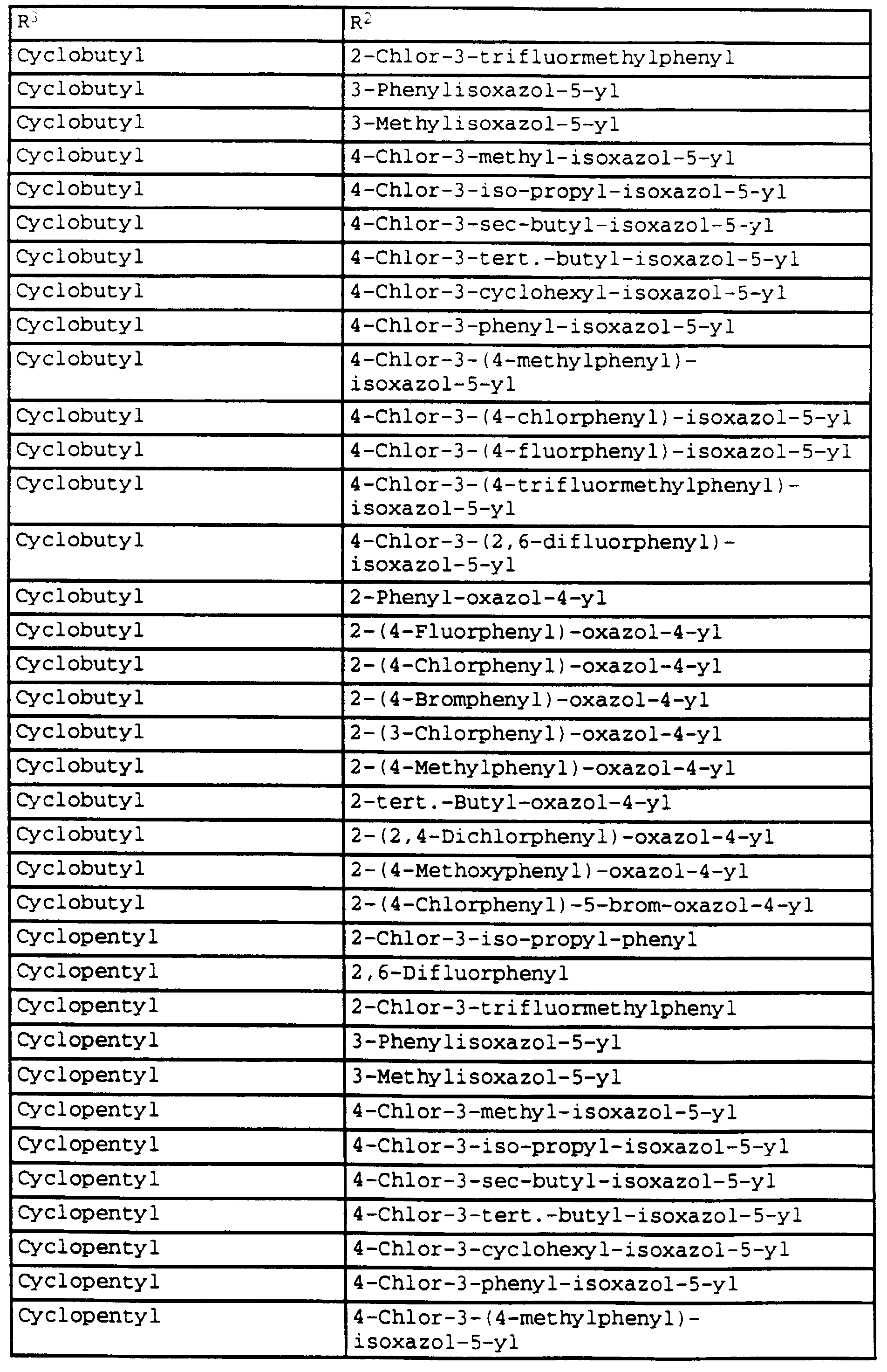

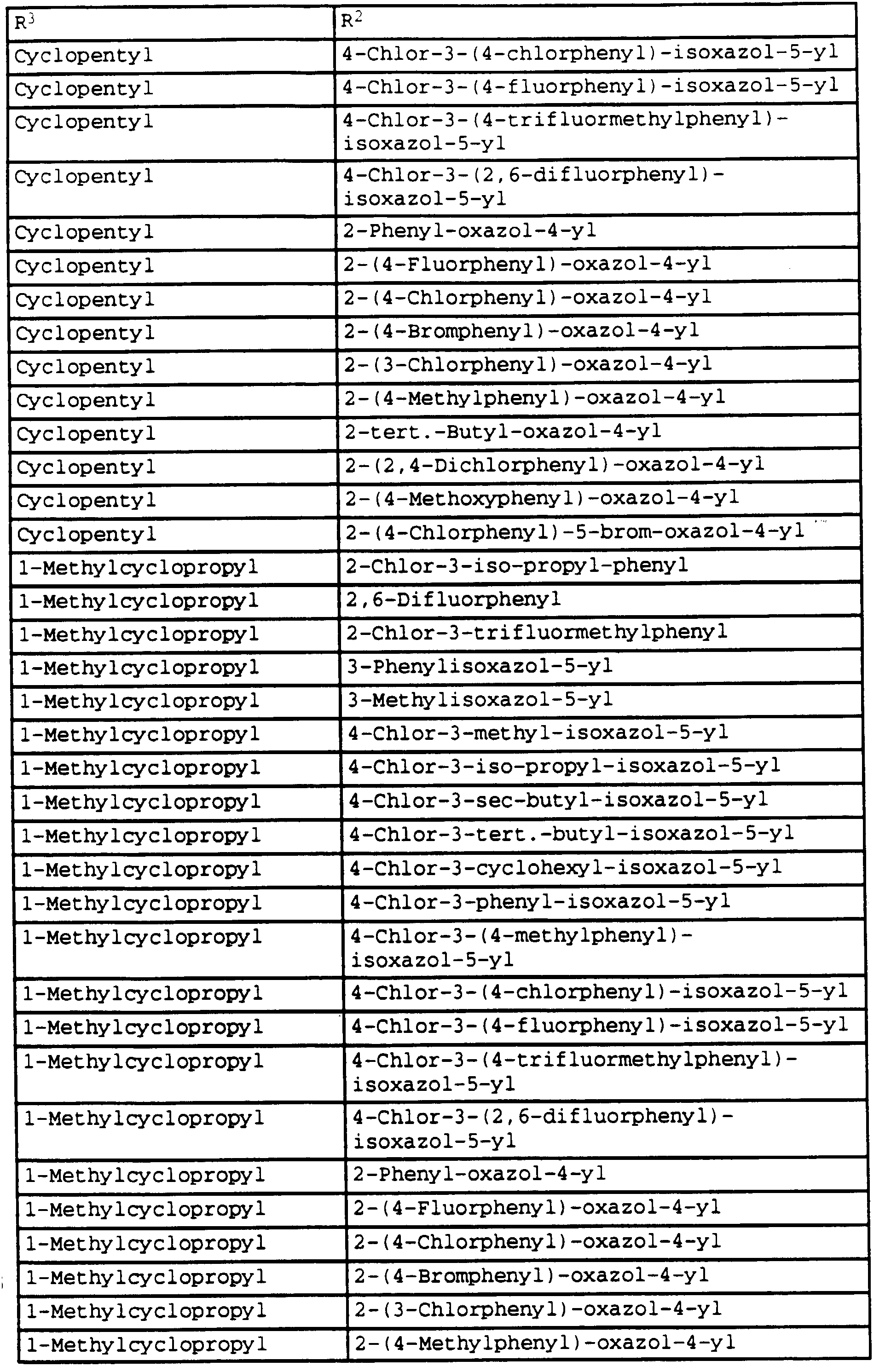

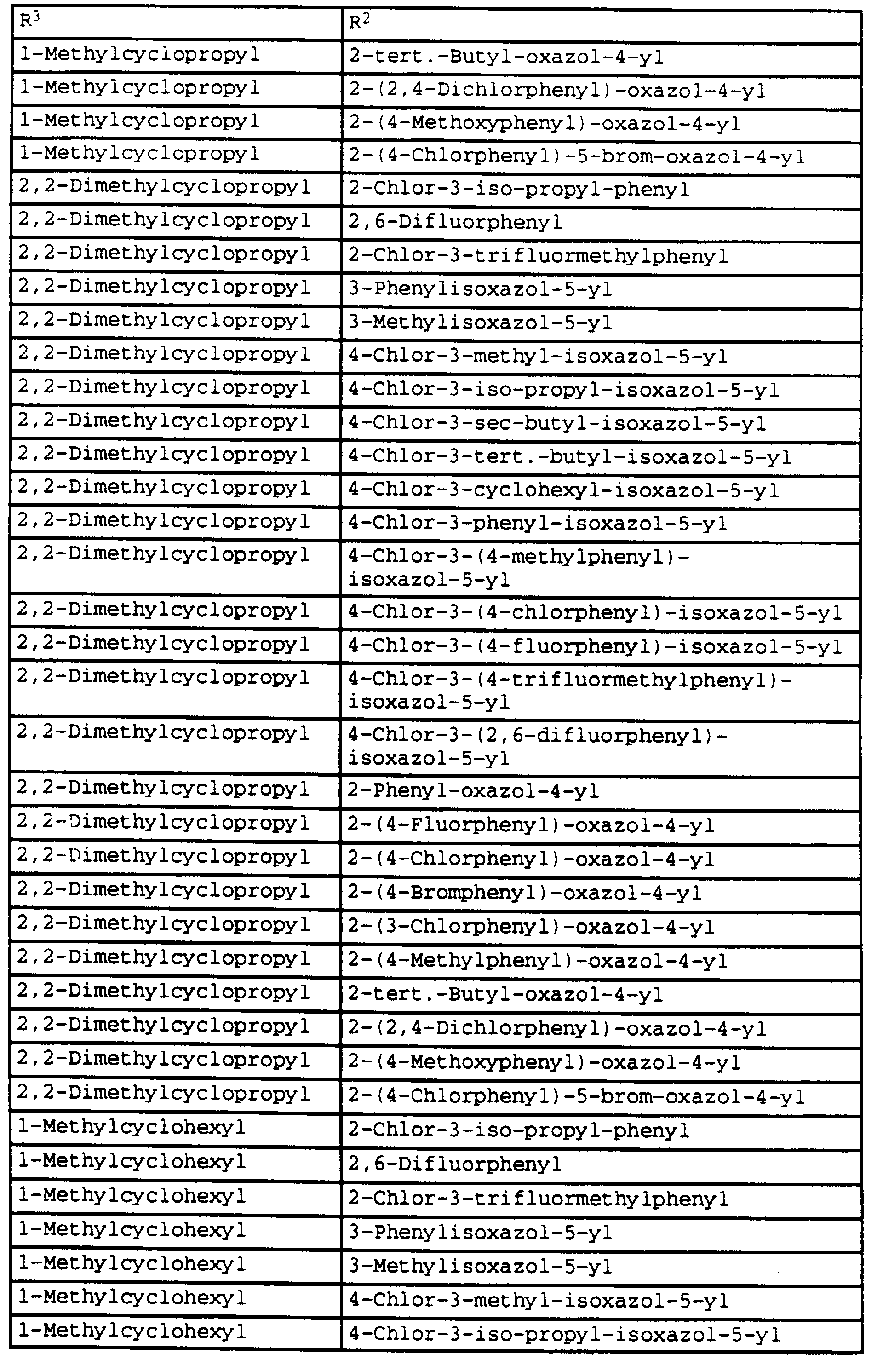

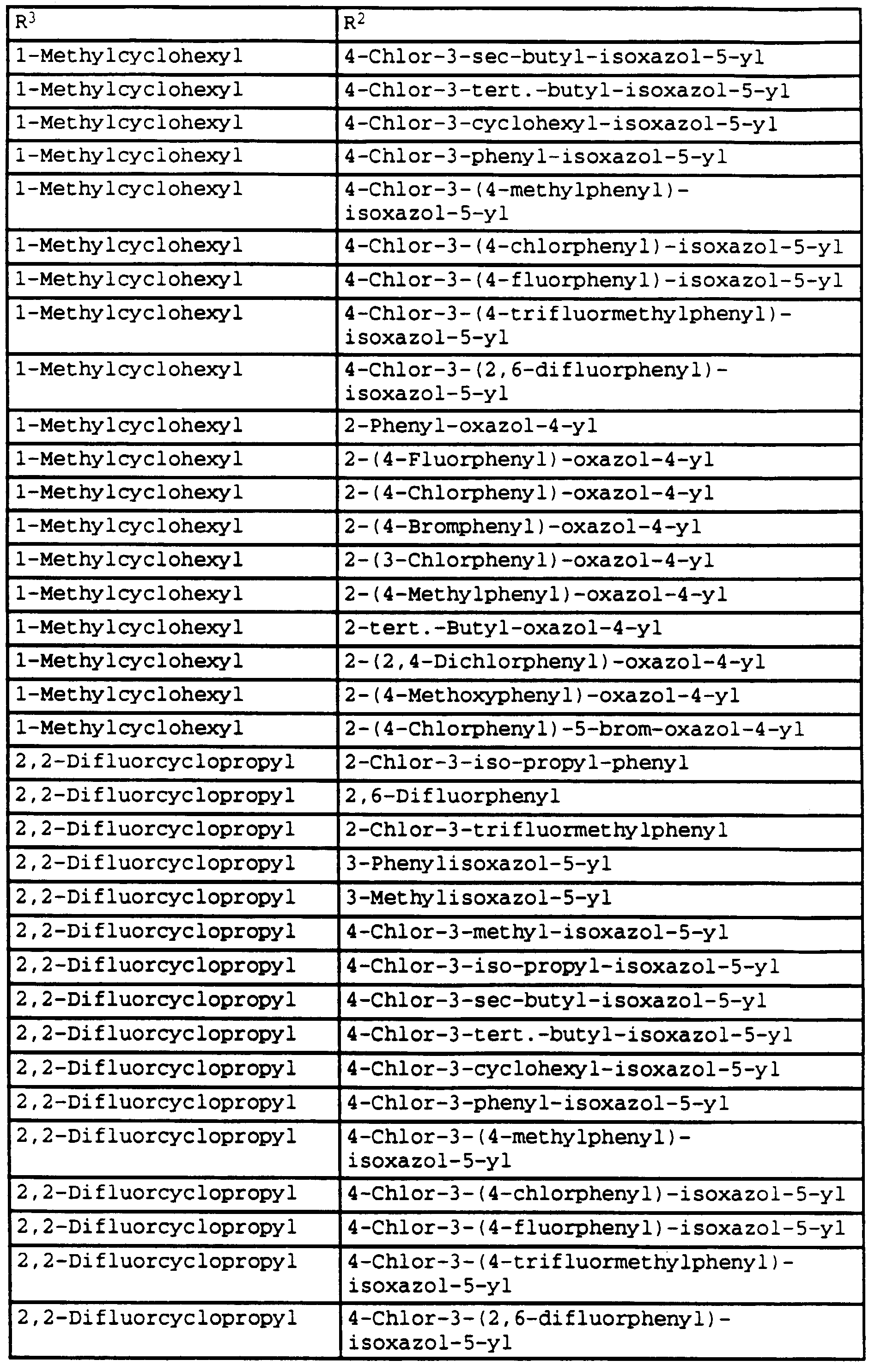

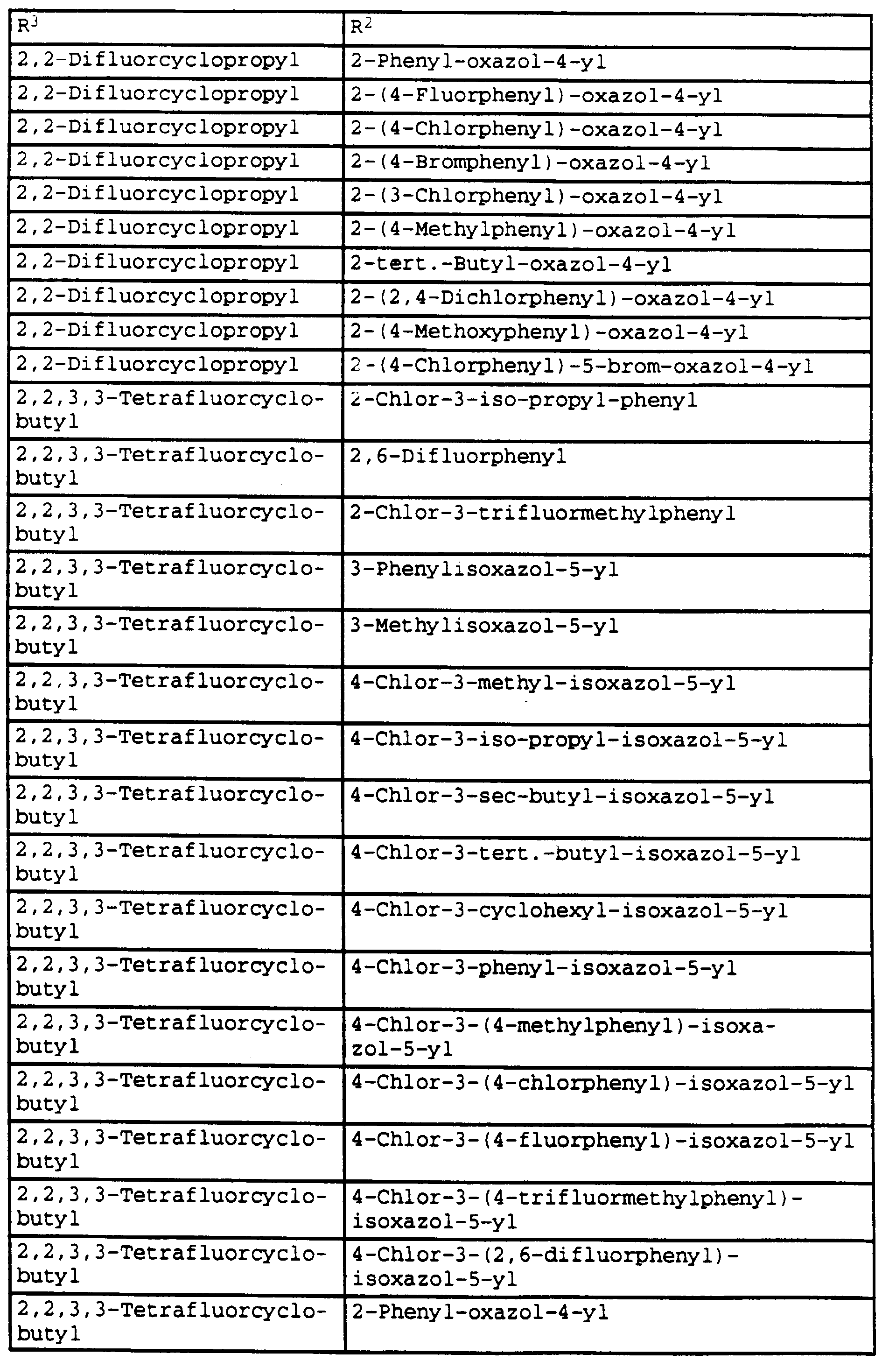

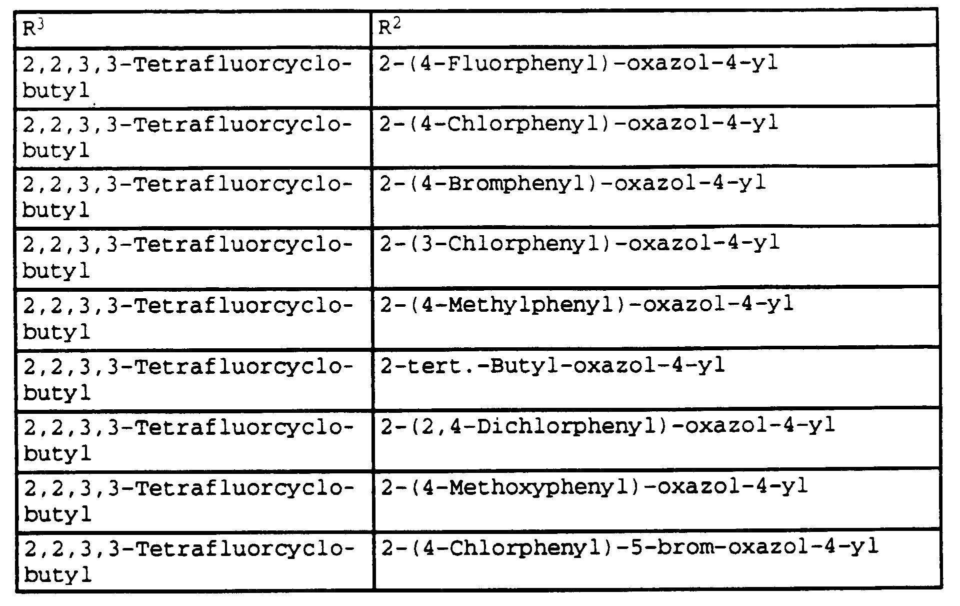

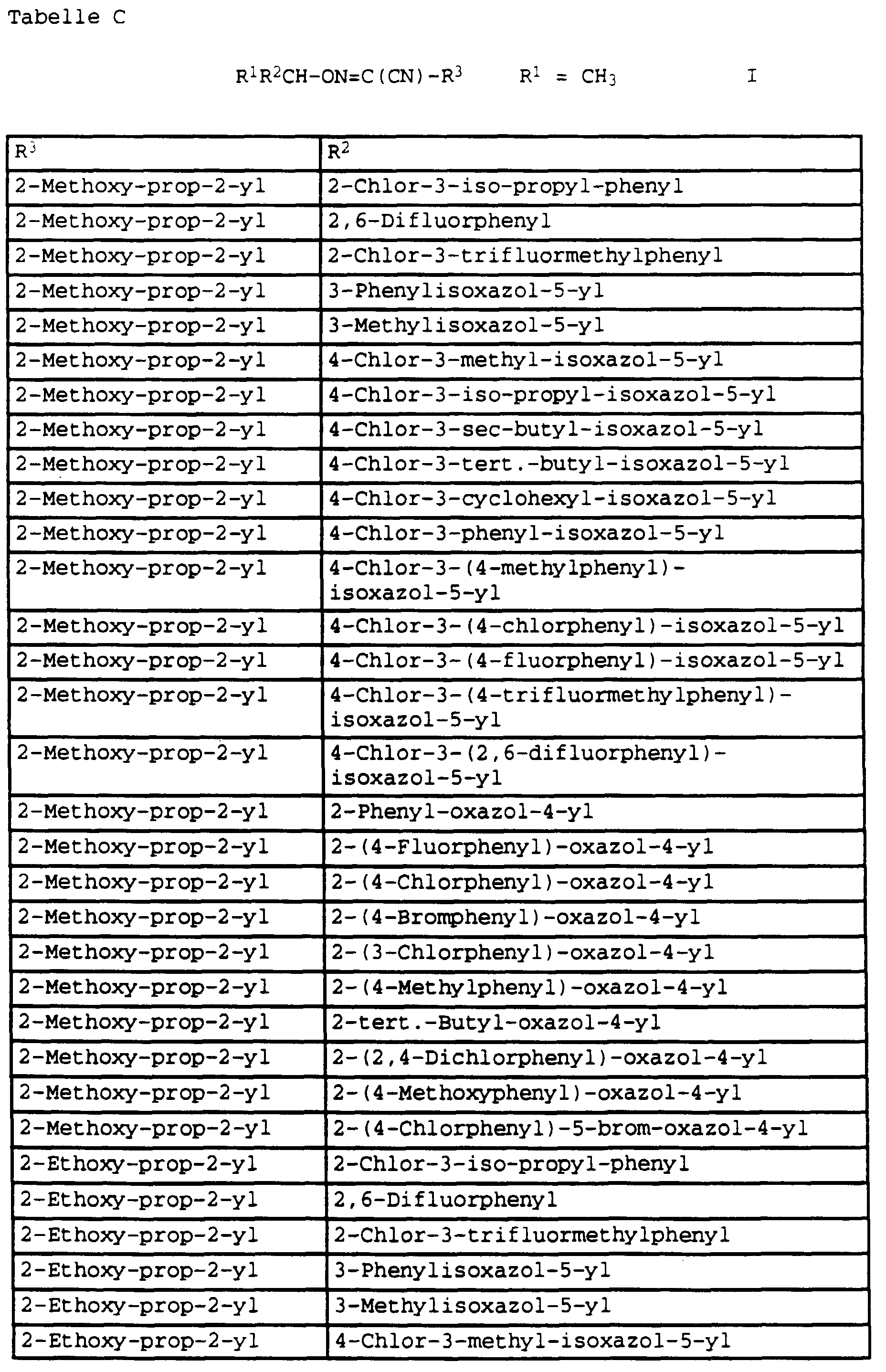

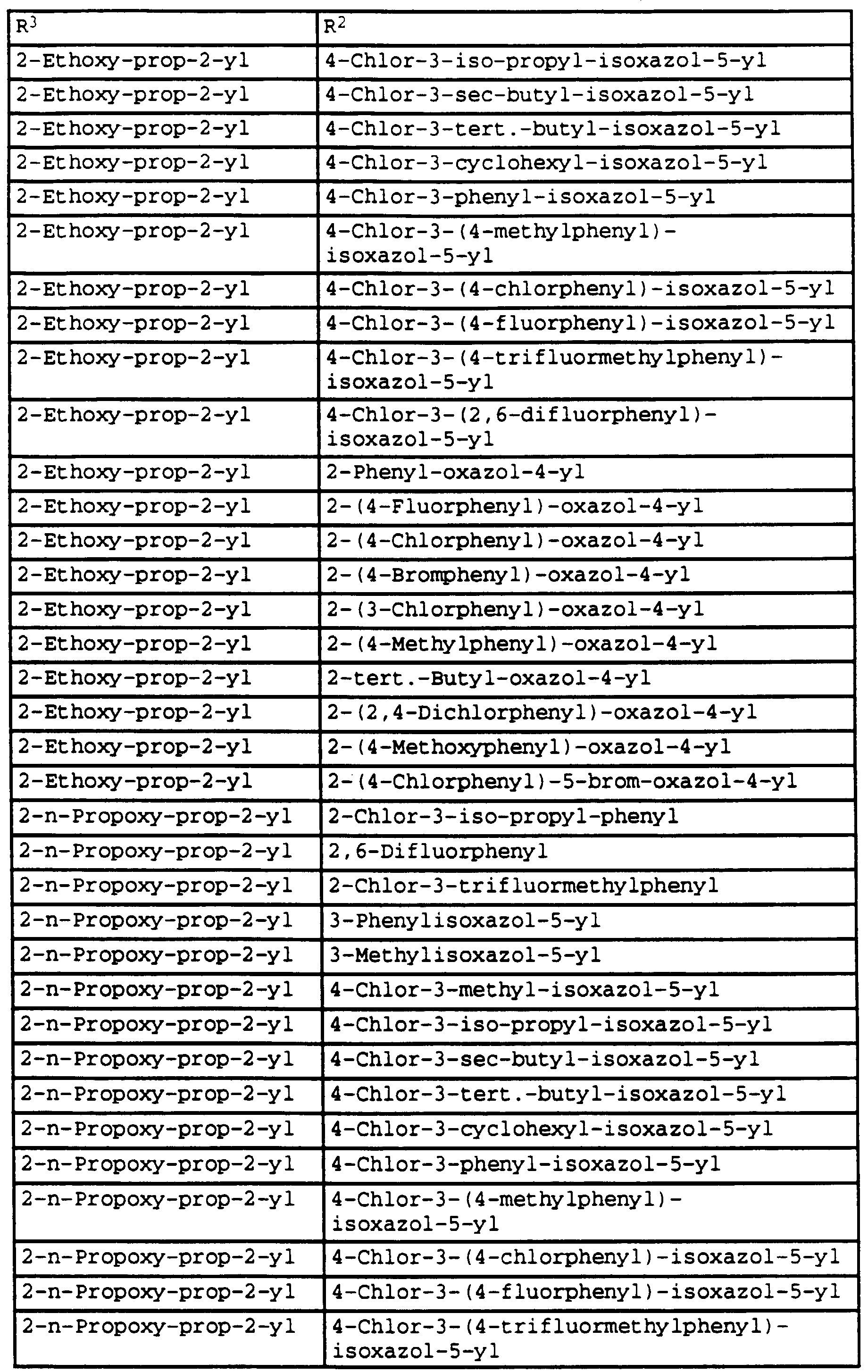

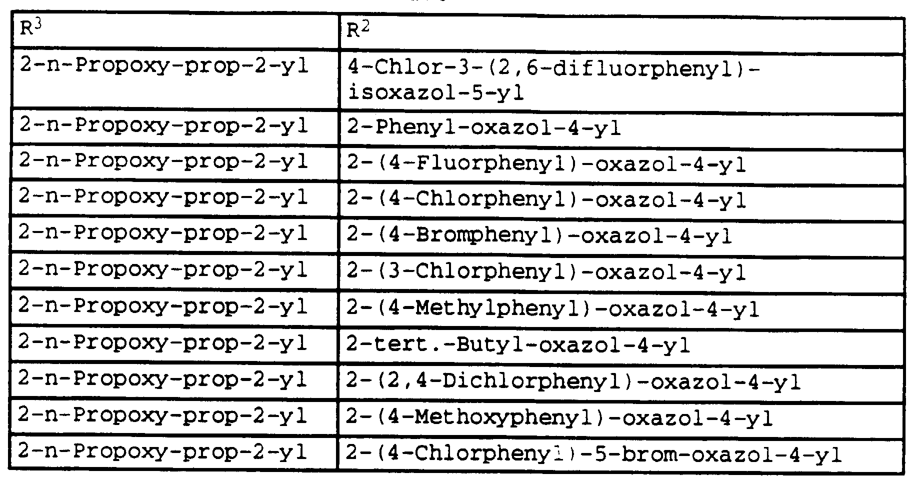

Die Verbindungen der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Hetrodera triflolii, Stockund Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Tragerstoff hergestellt werden.

Beispiele für Formulierungen sind:
I. 5 Gew.-Teile der Verbindung Nr. 1 werden mit 95 Gew.-Teilen feinteiligem Kaolin innig vermischt. Man erhalt auf diese Weise ein Stäubemittel, das 3 Gew.-% des Wirkstoffs enthält.
II. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
III.10 Gew.-Teile der Verbindung Nr. 4 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.
IV. 20 Gew.-Teile der Verbindung Nr. 13 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.
V. 80 Gew.-Teile der Verbindung Nr. 19 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,2 bis 10, vorzugsweise 0,5 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachfolgenden Synthesebeispielen wiedergegebenen Vorschriften wurden unter entsprechender Abwandlung der Ausgangsstoffe zur Gewinnung weiterer Verbindungen I benutzt. Die so erhaltenen Verbindungen sind in den anschließenden Tabellen mit physikalischen Angaben aufgeführt.

Die folgenden Beispiele und Vorschriften sollen die Herstellung der neuen Wirkstoffe und Zwischenprodukte erläutern:
Vorschrift 1:
   3-Methoxy-3-methyl-2-oximino-butyronitril
   53,6 g (0,46 mol) 2-Methoxy-2-methyl-propionaldehydoxim werden in Diethylether (ca. 1 M) bei -5 bis 10°C vorgelegt. Nach Eingasen von 35,8 g (0,5 mol) Chlor wird 1 h bei dieser Temperatur gerührt, dann bei 10°C eingeengt und der Rückstand in Diethylether aufgenommen. 24,7 (0,5 mol) Natriumcyanid werden bei 10°C in 375 ml Methanol/Wasser 20:1 vorgelegt und die obige etherische Lösung zügig zugetropft. Nach 4 h bei Raumtemperatur werden feste Bestandteile aus der Mischung entfernt und die Lösung mit 2 x 100 ml Methanol gewaschen. Die vereinigten Lösungen engt man ein und unterwirft sie einer Verteilung zwischen Methyl-tert.-Butylether und Wasser. Trocknen der organischen Phase (Natriumsulfat), einengen und kristallisieren (Dichlormethan/n-Hexan) führt zu 41,1 g (63 % d. Th.) eines weißen Pulvers mit dem Schmelzpunkt 102 - 104 °C.
Vorschrift 2:
   4-Chlormethyl-2-(4-fluorphenyl)oxazol
   139 g (1 mol) 4-Fluorbenzamid und 127 g (1 mol) 1,3-Dichloraceton werden in einem Reaktionsgefäß mit angeschlossenem Waschturm zusammengegeben. Man bringt die Mischung langsam durch Erwärmen zum Schmelzen und steigert die Temperatur schließlich auf 150°C. Dabei hält man unter Rühren bis die Gasentwicklung beendet ist (ca. 1,5 h).
   Die dunkle Reaktionsmischung wird auf Raumtemperatur abgekühlt, es werden vorsichtig 500ml konz. Schwefelsäure zugetropft und die entstandene Suspension 1,5 h gerührt. Dann gießt man unter kräftigem Rühren in Eiswasser, saugt den Niederschlag ab und wäscht diesen gut mit Wasser nach. Der Niederschlag wird getrocknet und dann aus Ethanol umkristallisiert.
   Ausbeute: 64%; Schmelzpunkt: 71 - 74°C.
Beispiel 1:
   3-Methoxy-3-methyl-2-[[(2',6'-difluorphenyl)methoxy]imino]-butyronitril
   3,55 g (25 mmol) 3-Methoxy-3-methyl-2-oximino-butyronitril, 5,18 g Kaliumcarbonat und 4,06 g (25 mmol) 2,6-Difluorbenzylchlorid werden in 50 ml trockenem Acetonitril für 5 Stunden bei 70°C und anschließend für 12 Stunden bei Raumtemperatur gerührt. Anschließend wird das Lösungsmittel bei vermindertem Druck entfernt. Der Rückstand wird in 200 ml Methyl-tert.-butylether aufgenommen. Es wird drei mal mit Natronlauge und dreimal mit Wasser gewaschen. Die vereinigten organischen Phasen werden über Natriumsulfat getrocknet und das Lösungsmittel wird bei vermindertem Druck abdestilliert. Das Rohprodukt wird an Kieselgel mit Cyclohexan/Dichlorethan 2:1 chromatographiert. Man erhält 6,2 g (92 % d. Th.) der Titelverbindung als farbloses viskoses Öl. IR (cm⁻¹): 1595,1474,1368,1273,1238,1180,1064,1010.
Beispiel 2:
   3-Methoxy-3-methyl-2-[[(3-methylisoxazol-5-yl)methoxy]imino]-butyronitril
   Analog zu der in Beispiel 1 gegebenen Vorschrift erhält man aus 4,26 g (30 mmol) 3-Methoxy-3-methyl-2-oximino-butyronitril, 4,14 g Kaliumcarbonat und 3,95 g (30 mmol) 5-Chlormethyl-3-methylisoxazol in 50 ml trockenem Acetonitril als Lösungsmittel nach Chromatographie des Rohprodukts an Kieselgel mit Cyclohexan/Dichlorethan 1:1 6,0 g (84 % d. Theorie) der Titelverbindung als farbloses viskoses Öl. IR (cm⁻¹): 1445,1367,1360,1273,1180,1122,1062,1022,1000.
Beispiel 3:
   3-Methoxy-3-methyl-2-[[[2-(4-fluorphenyl)oxazol-4-yl]methoxy] imin o]-butyronitril
   2,55 g (18 mmol) 3-Methoxy-3-methyl-2-oximino-butyronitril, 3,72 g Kaliumcarbonat und 3,8 g (18 mmol) 4-Chlormethyl-2-(4-fluorphenyl)-oxazol werden in 36 ml trockenem Aceton/Dimethylformamid 2:1 für 1 Stunde bei 45°C und anschließend für 12 Stunden bei Raumtemperatur gerührt. Nach der Aufarbeitung analog zu der in Beispiel 1 gegebenen Vorschrift und Chromatographie des Rohprodukts an Kieselgel mit Cyclohexan/Methyl-tert.-butylether 5:1 erhalt man 5,4 g (95 % d.Theorie) der Titelverbindung als kristallinen Feststoff.
   - Schmelzpunkt:: 75 - 77°C
   - IR (cm⁻¹):: 1499,1227,1070,1017,1011,965,843.

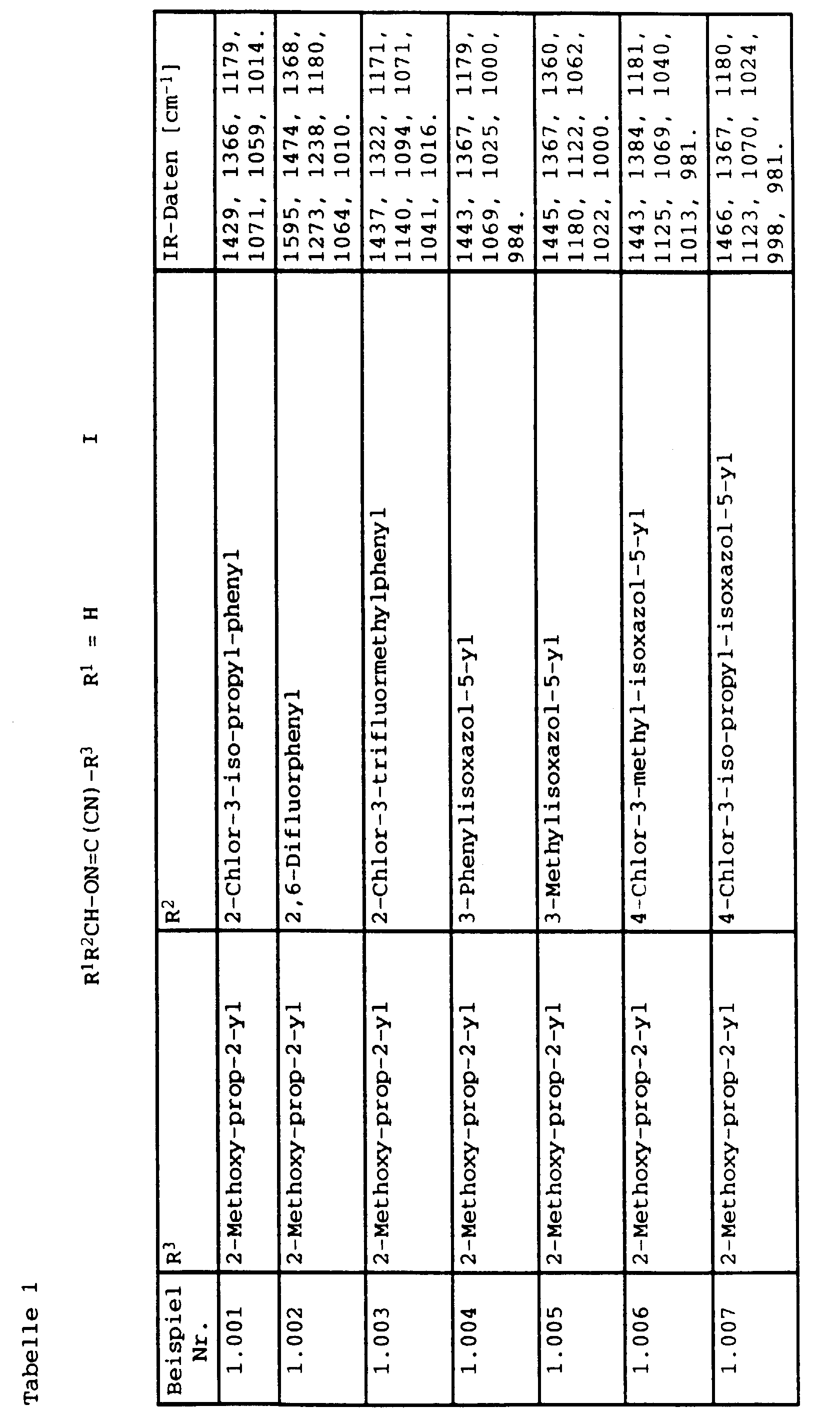

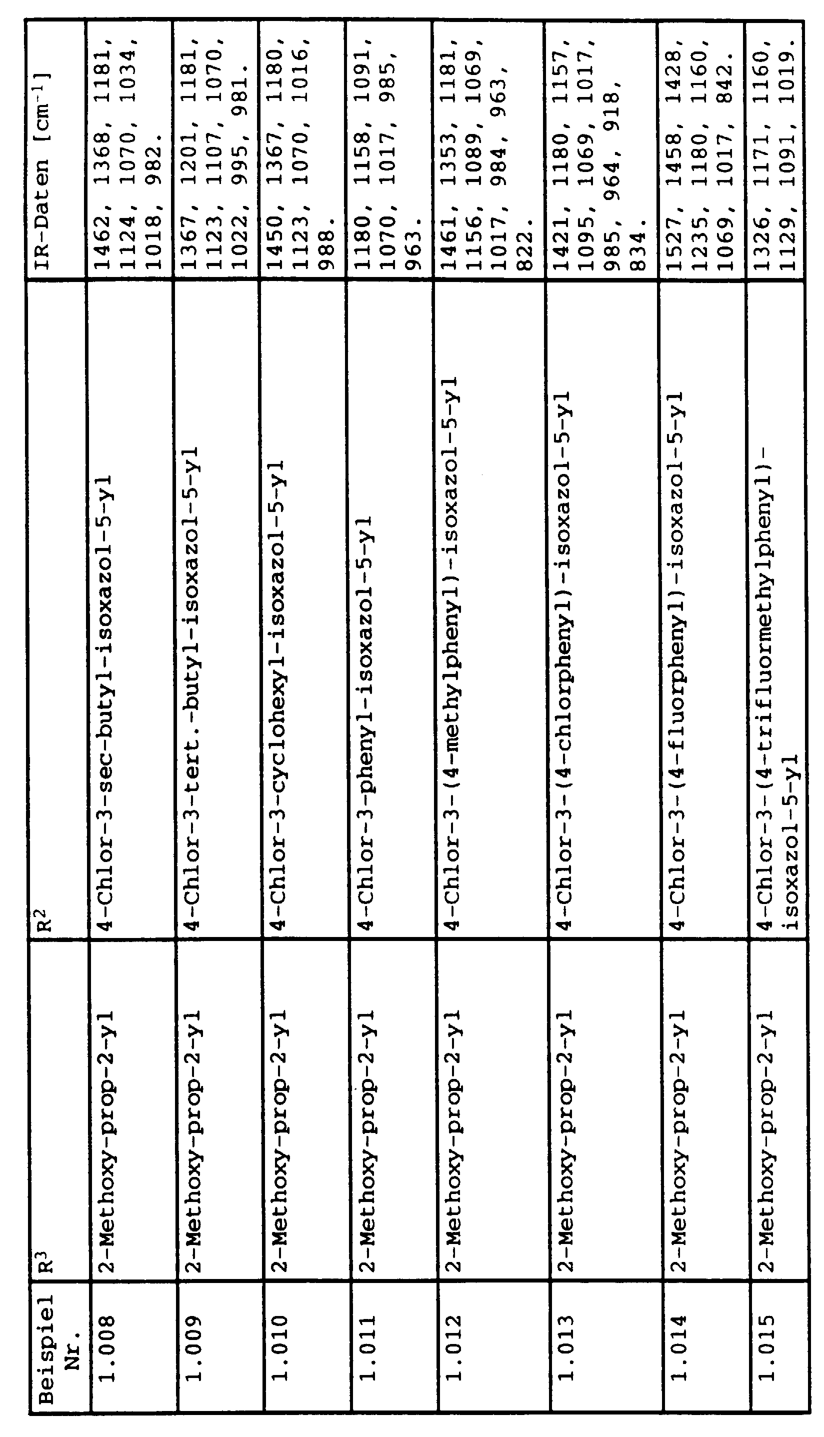

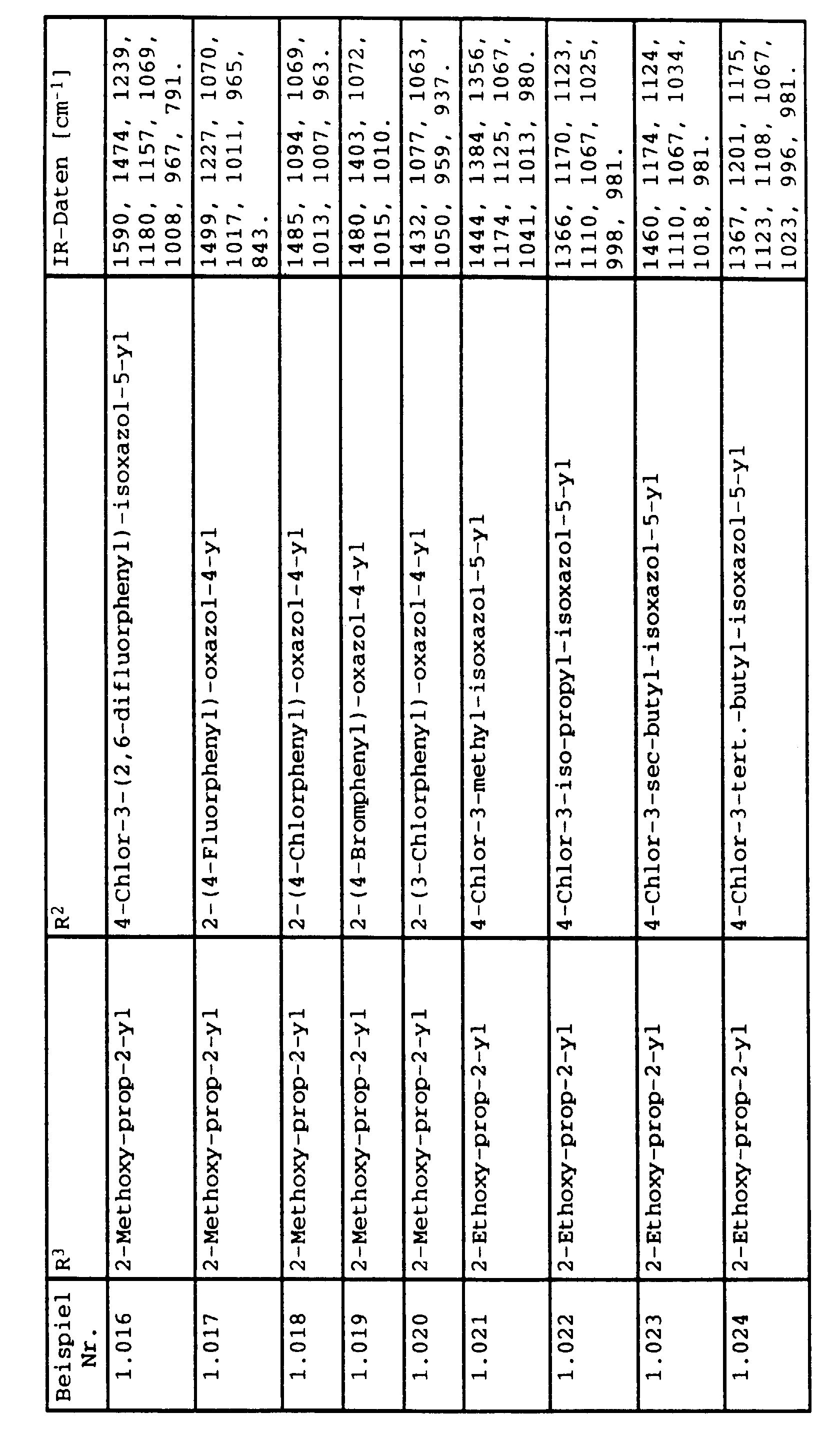

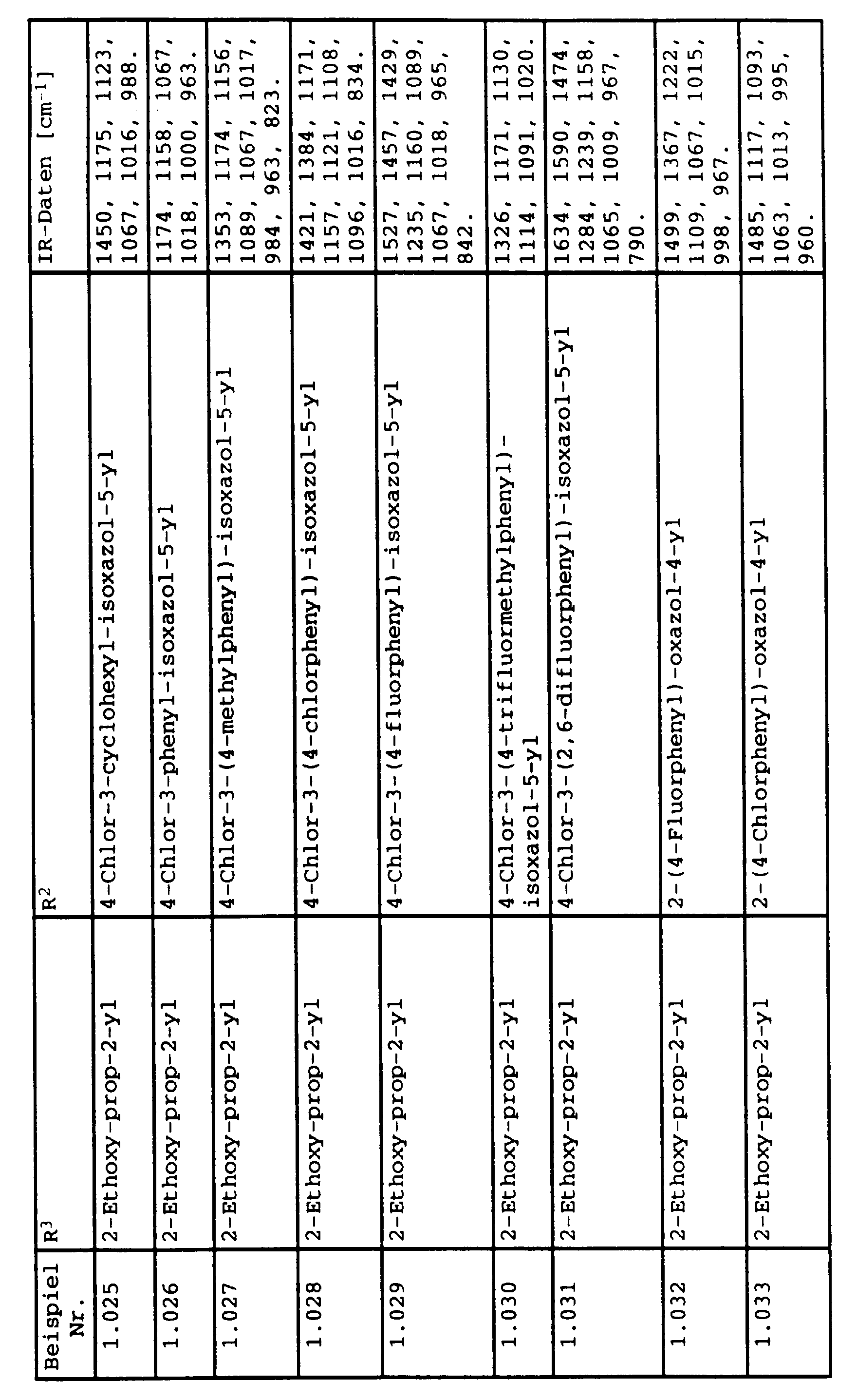

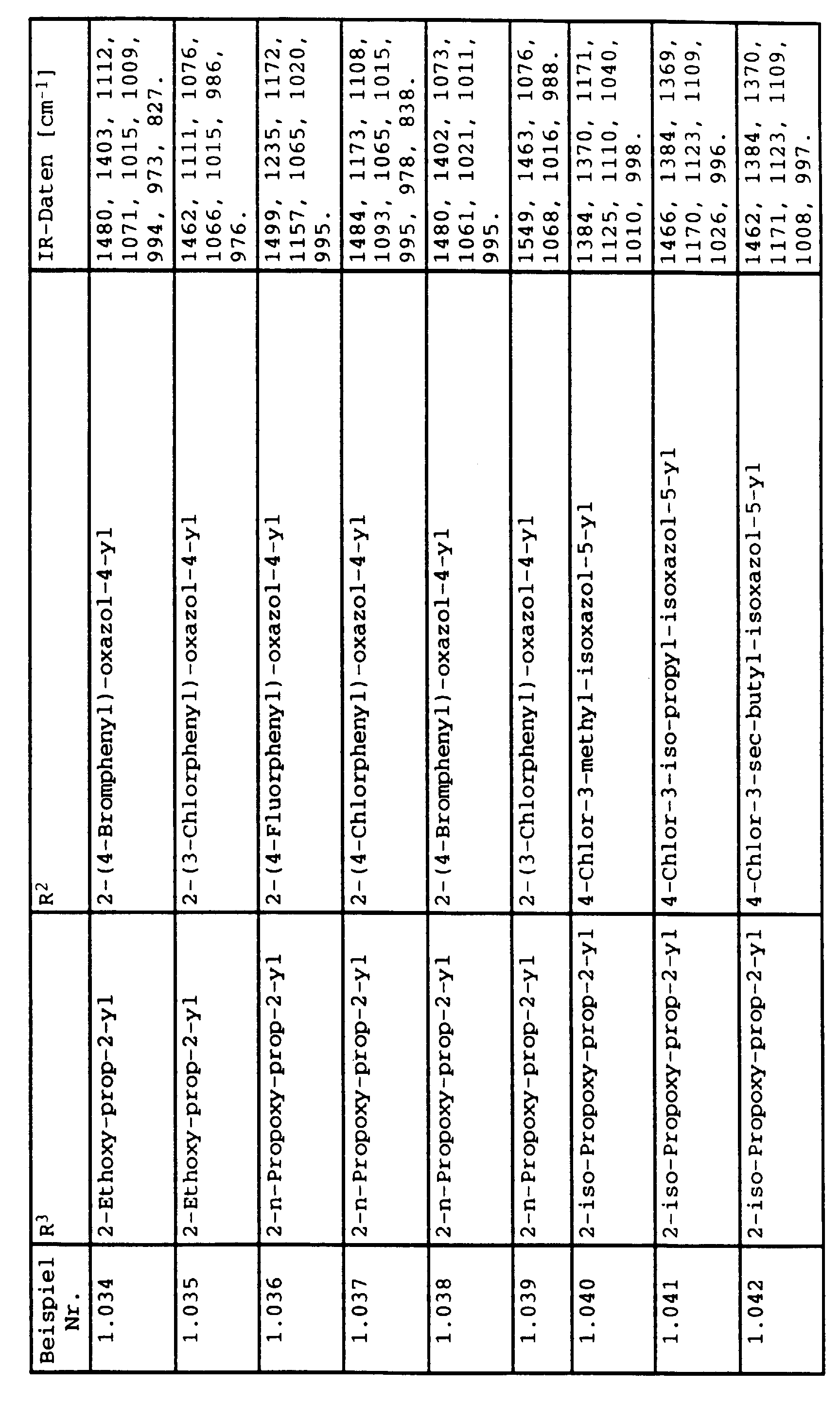

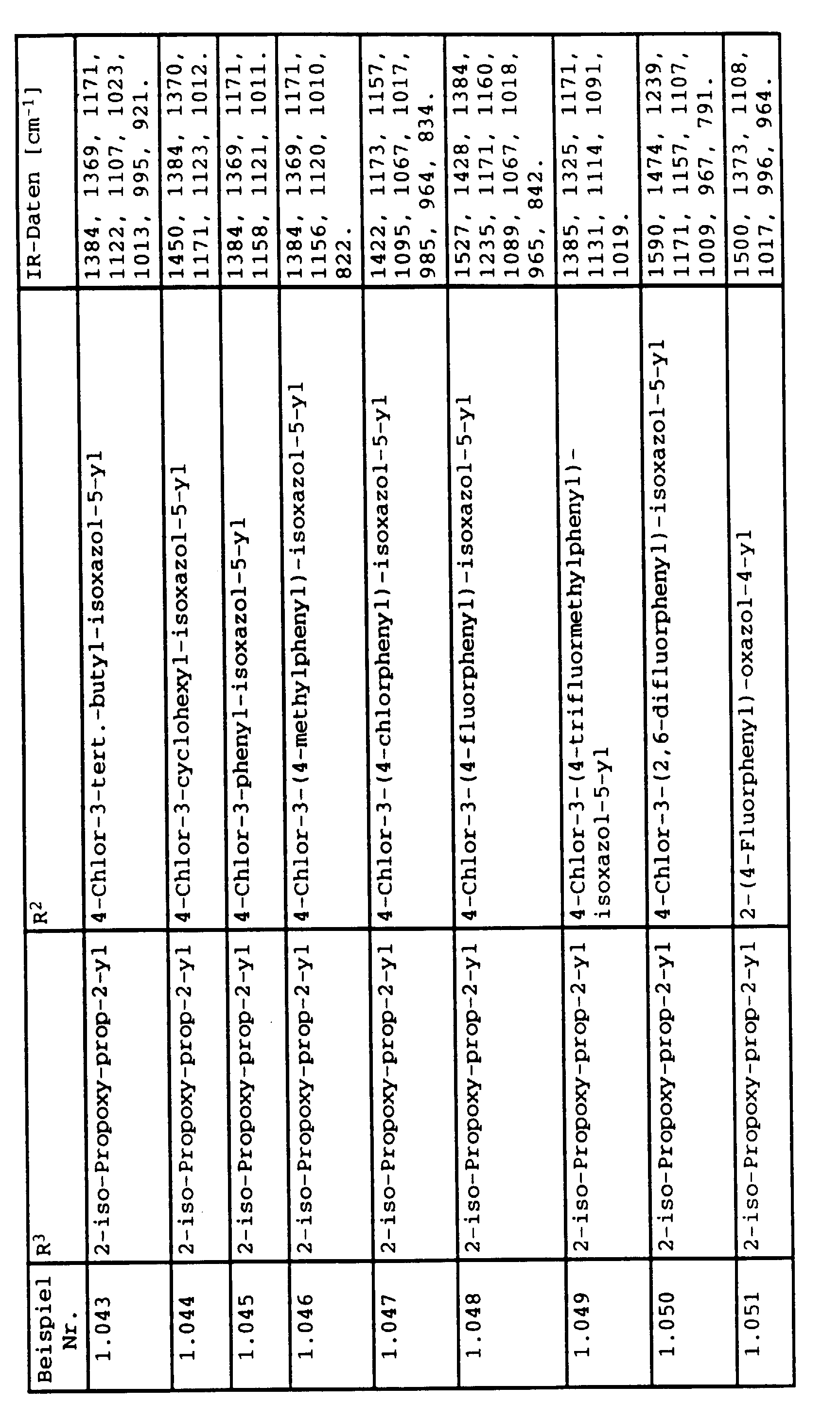

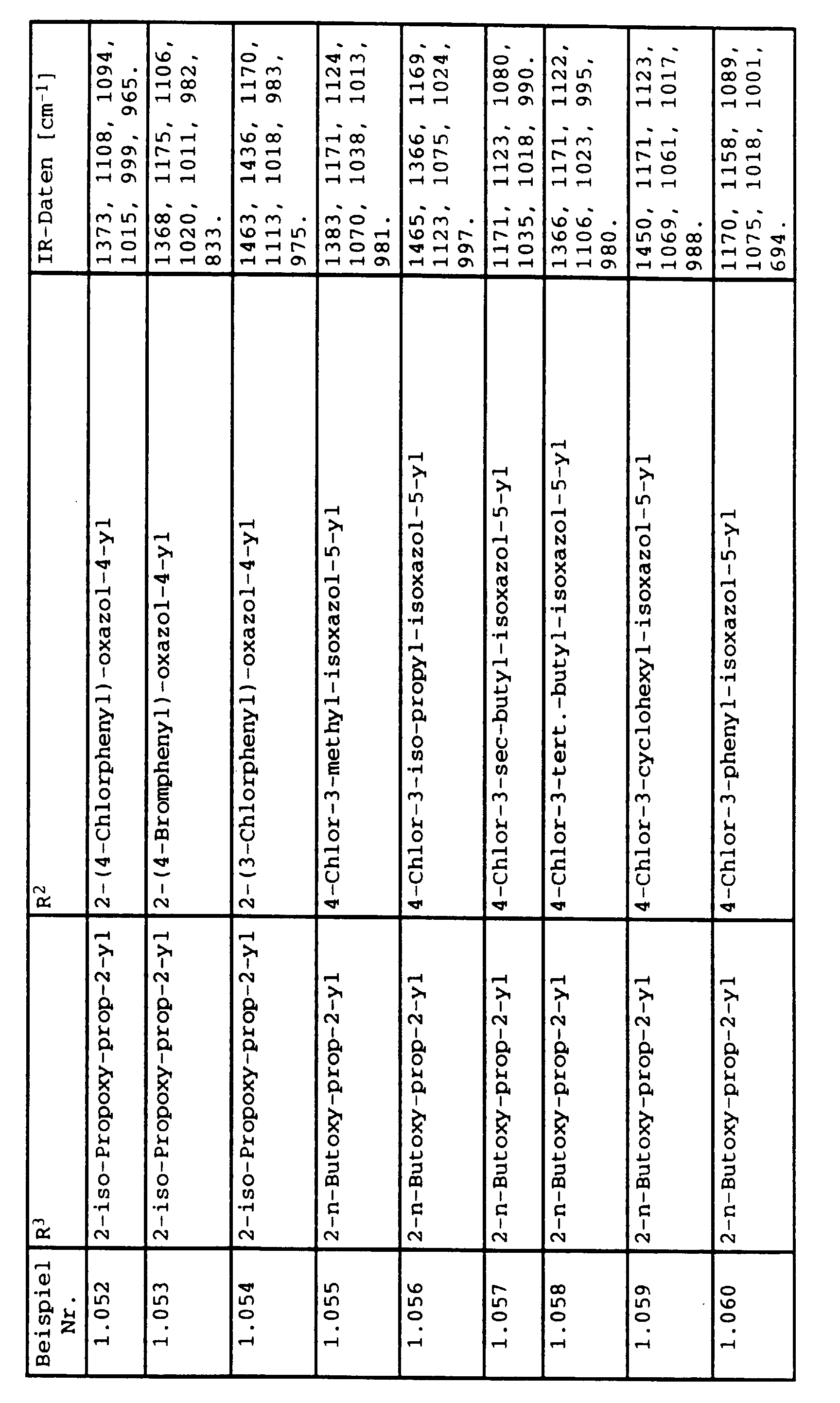

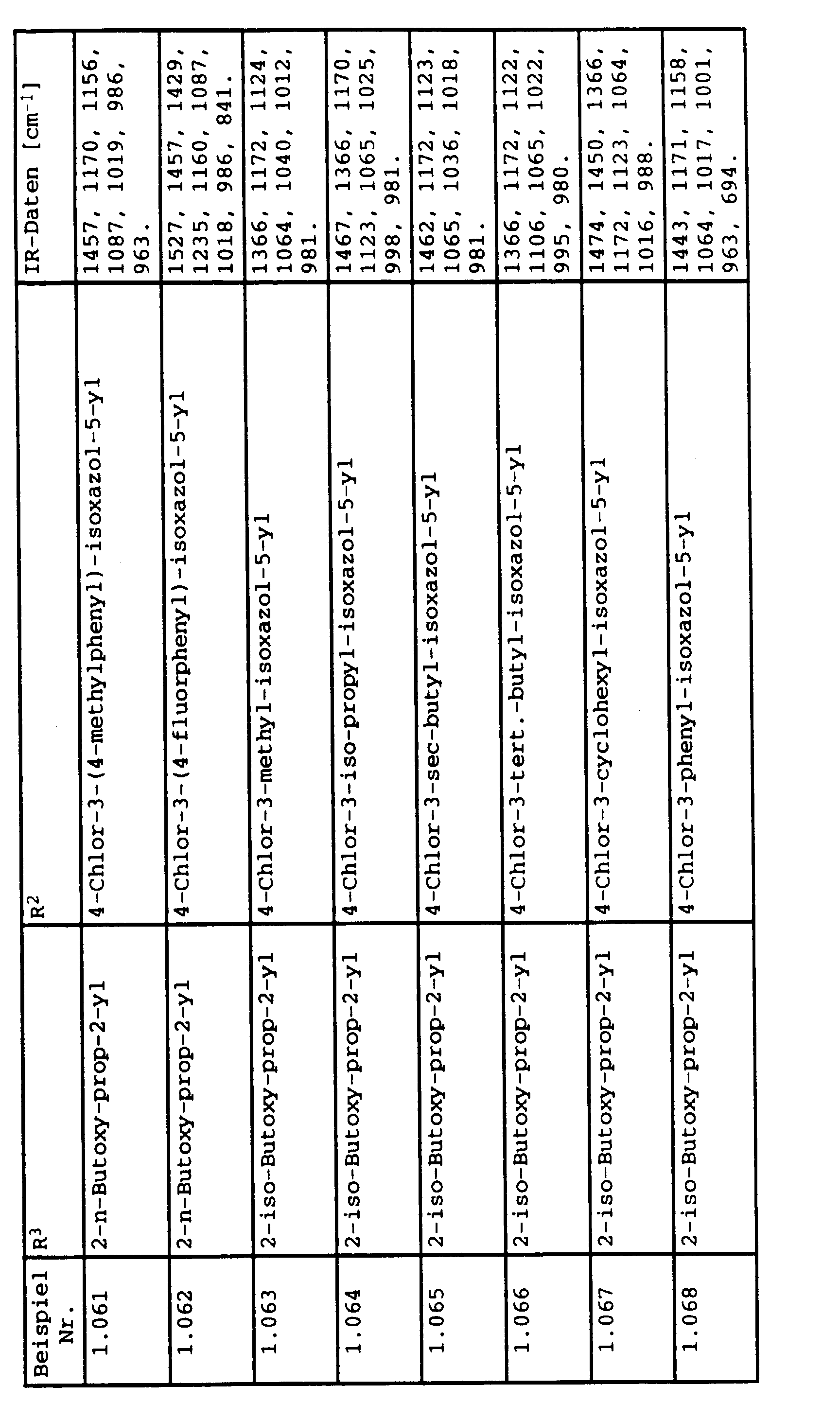

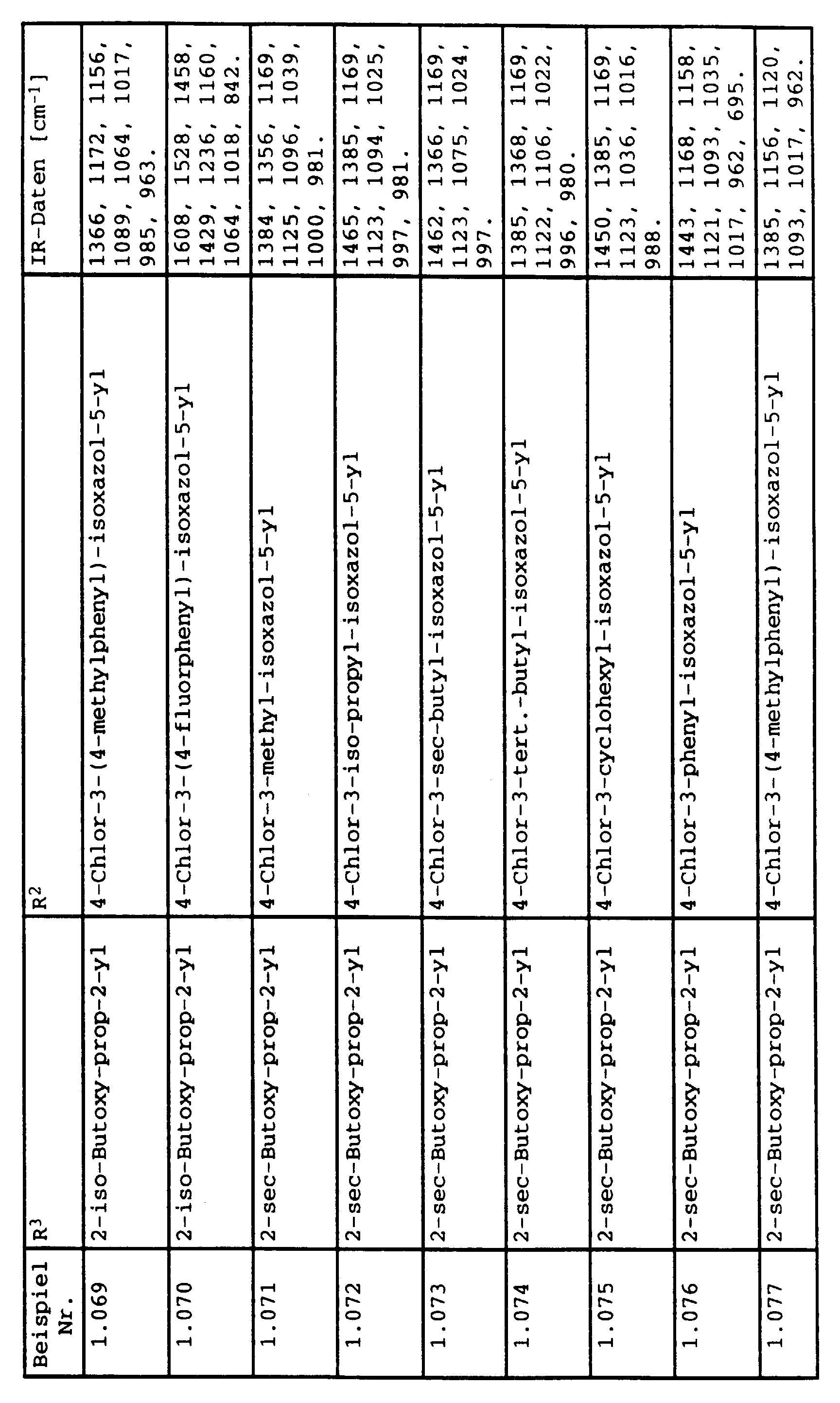

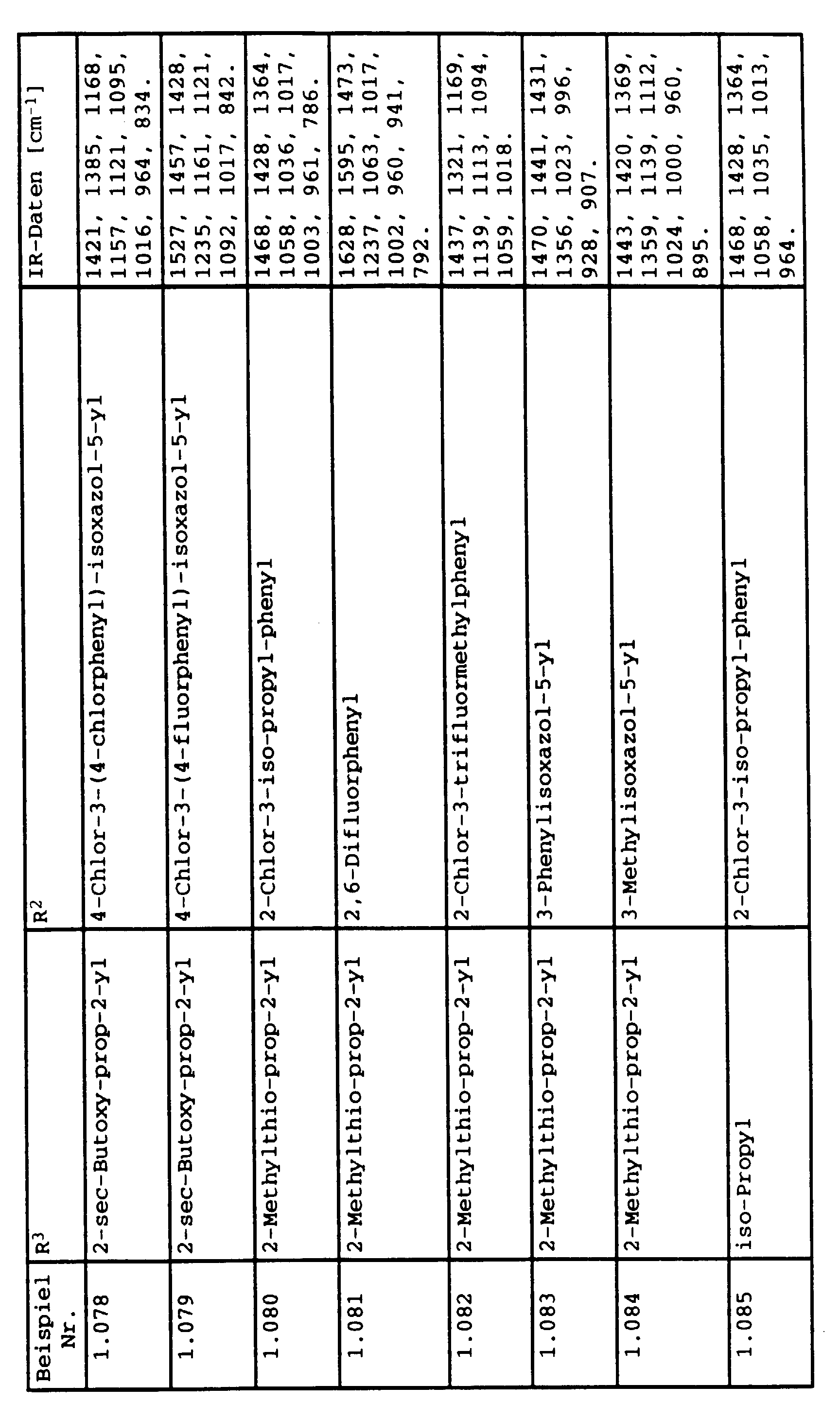

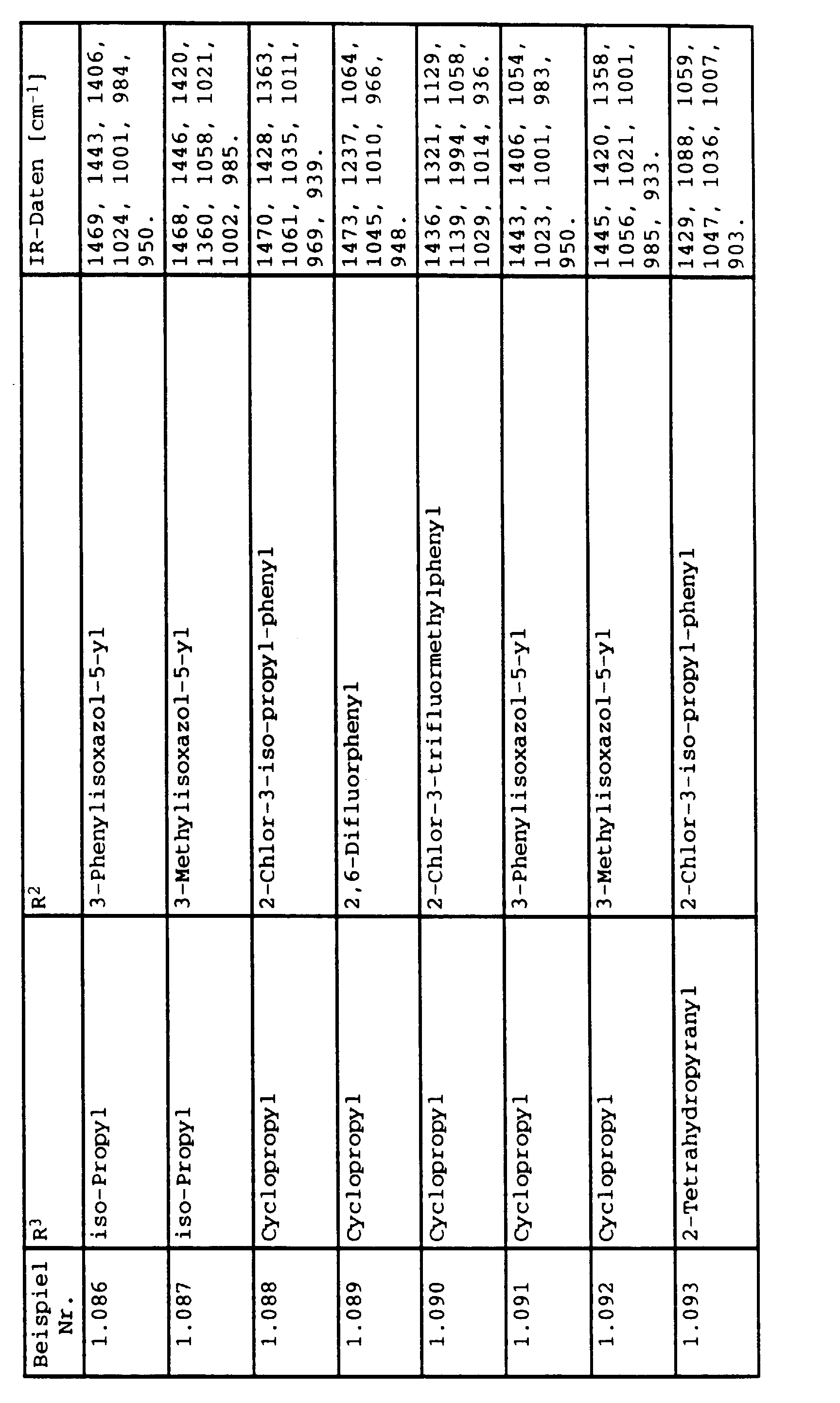

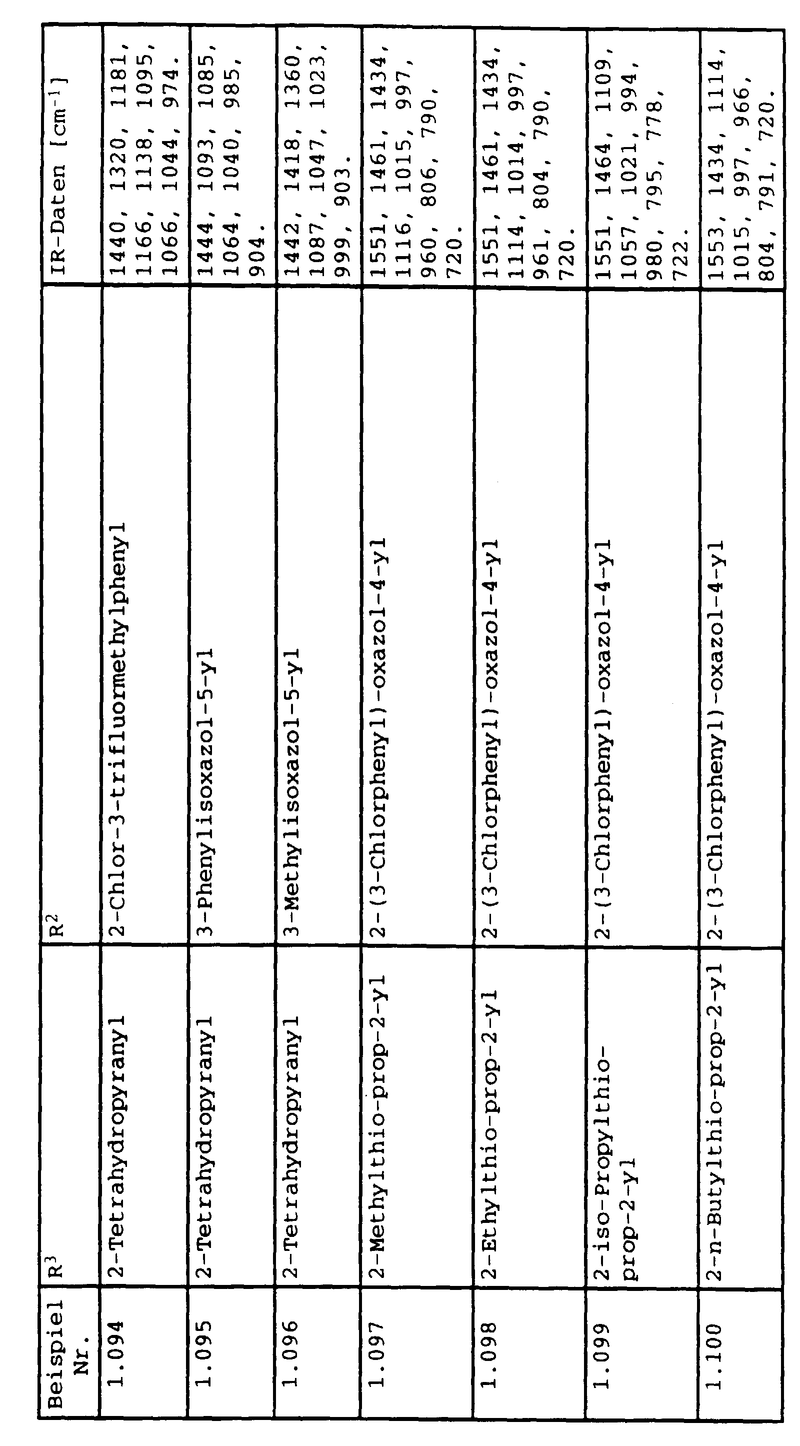

### Anwendungsbeispiele

Die insektizide Wirkung der Verbindungen der allgemeinen Formel I ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).
A. Aphis fabae (Schwarze Laus), Kontaktwirkung
   Stark befallene Buschbohnen (Vicia faba) wurden mit der wäßrigen Wirkstoffaufbereitung behandelt.
   Nach 24 h wurde die Mortalitätsrate bestimmt.
   In diesem Test zeigten die Verbindungen 1.006, 1.024, 1.040, 1.056, 1.065, 1.078, 1.082 und 1.088 Wirkschwellen von 1000 bis 200 ppm.
B. Caenorhabditis elegans (Freilebende Nematoden), Kontaktwirkung
   Der Boden eines Versuchsgefäßes wurde mit der acetonischen Lösung des Wirkstoffs benetzt und nach dem Abdampfen des Lösungsmittels mit E.-Coli-Bakteriensuspension als Nährmedium bedeckt und mit 50 µl Nematodensuspension infiziert.
   Nach 48 h wurde die Mortalitätsrate bestimmt.
   In diesem Test zeigten die Verbindungen 1.003, 1.004, 1.005, 1.032, 1.034 1.084 und 1.083 Wirkschwellen von 100 ppm.
C. Dysdercus intermedius (Baumwollwanze), Kontaktwirkung
   200 g steriler Quarzsand wurde mit 25 ml der wäßrigen Wirkstoffaufbereitung benetzt und anschließend mit ca. 20 Larven des 3. Larvenstadiums besetzt.
   Nach dem Schlüpfen der Larven in einem Kontrollexperiment wurde die Mortalität bestimmt.
   In diesem Test zeigten die Verbindungen 1.008, 1.009, 1.010, 1.012, 1.024, 1.025, 1.027, 1.028, 1.040, 1.041, 1.043, 1.044, 1.046, 1.047, 1.056, 1.072 und 1.078 Wirkschwellen von 1000 ppm.
D. Heliothis virescens (Baumwolleule), Kontakt-/Fraßwirkung
   Ca. 10 cm große Tabakpflanzen wurden mit der wäßrigen Wirkstoffaufbereitung behandelt. Nach dem Abtrocknen wurden die Pflanzen mit jeweils 10 Larven des 3. Entwicklungsstadiums belegt.
   Nach 48 h wurden Mortalität und Fraßverhinderung beurteilt.
   In diesem Test zeigten die Verbindungen 1.008, 1.009, 1.018, 1.021, 1.022, 1.033 und 1.044 Wirkschwellen von 1000 bis 400 ppm.
E. Meloidogyne incognita (Wurzelgallennematoden), Kontaktwirkung
   Tomatensetzlinge wurden 3 Wochen in stark mit Nematoden infizierter Komposterde inkubiert. Die Pflanzen wurden entnommen, von Erdresten befreit und für 1 h in die wäßrige Wirkstoffaufbereitung gesetzt. Anschließend wurden die Setzlinge einzeln in sterilisierter Erde angepflanzt.
   Nach 6 - 8 Wochen wurde der Wuchs der Pflanzen und der Befall der Wurzeln beurteilt.
   In diesem Test zeigten die Verbindungen 1.002, 1.081, 1.082, 1.084, 1.087, 1.089, 1.091, 1.092 und 1.096 Wirkschwellen von 100 ppm.
F. Musca domestica (Stubenfliege), Kontaktwirkung
   Der Boden eines Versuchsgefäßes wurde mit der acetonischen Lösung des Wirkstoffs benetzt und nach dem Abdampfen des Lösungsmittels mit 10 Fliegen besetzt.
   Nach 4 h wurde die Mortalitätsrate bestimmt.
   In diesem Test zeigten die Verbindungen 1.002, 1.003, 1.006, 1.007, 1.011, 1.021, 1.026, 1.040, 1.043, 1.045, 1.056, 1.060, 1.063, 1.080, 1.081, 1.083, 1.084, 1.085, 1.086, 1.089, 1.090 und 1.092 Wirkschwellen von 1,0 bis 100 mg.
G. Ornithodorus moubata (Zecke), Kontaktwirkung
   Je 5 Zecken (Durchmesser 1,5 - 2 mm; nach einer Blutmahlzeit) wurden für ca. 5 sec. in die wäßrige Wirkstoffaufbereitung getaucht.
   Nach 48 h wurde die Mortalitätsrate bestimmt.
   In diesem Test zeigten die Verbindungen 1.084 und 1.086, Wirkschwellen von 1000 ppm.
H. Plutella maculipennis (Kohlschaben - Raupe), Kontaktwirkung
   Blätter junger Kohlpflanzen wurden mit der wäßrigen Wirkstoffaufbereitung benetzt und anschließend auf einen angefeuchteten Filter gelegt. Die präparierten Blätter wurden anschließend mit jeweils 10 Raupen des 4. Entwicklungsstadiums belegt.
   Nach 48 h wurde die Mortalitätsrate bestimmt.
   In diesem Test zeigten die Verbindungen 1.008, 1.022, 1.023, 1.040, 1.056, 1.057, 1.071 und 1.073 Wirkschwellen von 1000 ppm.
I. Prodenia litura (Agypt. Baumwollwurm), Zuchtversuch
   Fünf Raupen des Entwicklungsstadiums L3 (10 - 12 mm), die keine feststellbare Schädigung im Kontaktversuch erlitten hatten, wurden auf Standardnährboden (3,1 1 Wasser, 80 g Agar, 137 g Bierhefe, 515 g Maismehl, 130 g Weizenkeime sowie übliche Zusatzstoffe und Vitamine (20 g Wessonsalz, 5 g Nipagin, 5 g Sorbin, 10 g Zellulose, 18 g Ascorbinsäure, 1 g Lutavit® blend (Vitamin), 5 ml alkoholische Biotin-Lösung)) aufgebracht, der zuvor mit der wäßrigen Wirkstoffaufbereitung benetzt worden war.
   Die Beobachtung erstreckte sich bis zum Schlüpfen der Falter in einem Kontrollversuch ohne Wirkstoff.
   In diesem Test zeigten die Verbindungen 1.009, 1.013, 1.022, 1.023, 1.024, 1.025, 1.044, 1.045, 1.061, 1.071, 1.075, 1.091 und 1.092 Wirkschwellen von 1,0 mg.
K. Tetranychus telarius (Rote Spinne), Kontaktwirkung
   Stark befallene getopfte Buschbohnen, die das zweite Folgeblattpaar zeigten, wurden mit wäßrigen Wirkstoffaufbereitung behandelt.
   Nach 5 Tagen im Gewächshaus wurde der Bekämpfungserfolg mittels Binokular bestimmt.
   In diesem Test zeigten die Verbindungen 1.009, 1.016, 1.062 und 1.074, Wirkschwellen von 400 bis 1000 ppm.

## Patentansprüche

1. Cyanooximether der Formel I
R¹R²CH-ON=C (CN)-R³ I
in der die Substituenten die folgende Bedeutung haben:
R¹ Wasserstoff oder C₁-C₄-Alkyl;
R² ein über ein Kohlenstoffatom gebundenes ein- bis dreikerniges aliphatisches oder aromatisches Ringsystem, das neben Kohlenstoffatomen ein bis vier Stickstoffatome oder ein bis drei Heteroatome aus der Gruppe Sauerstoff, Schwefel und Stickstoff enthalten kann, wobei dieses Ringsystem ein bis fünf Halogenatome und ein bis vier der folgenden Reste tragen kann:
- Cyano, Cyanato, Thiocyanato, Nitro, Amino, Hydroxy, Carboxyl, C₁-C₆-Alkyl, C₁-C₄-Halogenalkyl, C₁-C₆-Alkoxy, C₁-C₄-Halogenalkoxy, C₁-C₄-Alkoxy-C₁-C₄-alkyl, C₁-C₆-Alkylthio, C₁-C₄-Halogenalkylthio, C₁-C₄-Alkylthio-C₁-C₄-alkyl, C₃-C₁₅-Alkenyl, C₃-C₁₅-Alkenyloxy, C₃-C₈-Cycloalkyl, C₅-C₈-Cycloalkenyl, C₃-C₈-Cycloalkoxy, C₅-C₈-Cycloalkenyloxy, C₁-C₆-Alkylamino, Di-C₁-C₆-alkylamino, C₁-C₆-Alkylcarbonyl, C₁-C₆-Alkoxycarbonyl, C₁-C₆-Alkylaminocarbonyl, DiC₁-C₆-alkylaminocarbonyl, C₁-C₆-Alkylcarboxyl,
- Phenyl, Phenoxy, Phenylthio, Phenylamino, Phenyl-C₁-C₄-alkyl, Phenoxy-C₁-C₄-alkyl, PhenylthioC₁-C₄-alkyl, Phenylamino-C₁-C₄-alkyl, wobei die aromatischen Ringe ihrerseits ein bis fünf Halogenatome und ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, oder wobei die aromatischen Ringe ihrerseits ein bis fünf Halogenatome oder ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
- ein über ein Kohlenstoffatom gebundenes fünf- oder sechsgliedriges aromatisches Ringsystem, das neben Kohlenstoffatomen ein bis drei Stickstoffatome oder ein oder zwei Stickstoffatome und ein Sauerstoffoder ein Schwefelatom oder ein Sauerstoff- oder ein Schwefelatom enthalten kann, wobei dieses Ringsystem ein bis vier Halogenatome und ein bis drei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, oder wobei die aromatischen Ringe ihrerseits ein bis vier Halogenatome oder ein bis drei der folgenden Reste tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
oder wobei dieses Ringsystem ein bis fünf Halogenatome oder ein bis vier der folgenden Reste tragen kann:
- Cyano, Cyanato, Thiocyanato, Nitro, Amino, Hydroxy, Carboxyl, C₁-C₆-Alkyl, C₁-C₄-Halogenalkyl, C₁-C₆-Alkoxy, C₁-C₄-Halogenalkoxy, C₁-C₄-Alkoxy-C₁-C₄-alkyl, C₁-C₆-Alkylthio, C₁-C₄-Halogenalkylthio, C₁-C₄-Alkylthio-C₁-C₄-alkyl, C₃-C₁₅-Alkenyl, C₃-C₁₅-Alkenyloxy, C₃-C₈-Cycloalkyl, C₅-C₈-Cycloalkenyl, C₃-C₈-Cycloalkoxy, C₅-C₈-Cycloalkenyloxy, C₁-C₆-Alkylamino, Di-C₁-C₆-alkylamino, C₁-C₆-Alkylcarbonyl, C₁-C₆-Alkoxycarbonyl, C₁-C₆-Alkylaminocarbonyl, Di-C₁-C₆-alkylaminocarbonyl, C₁-C₆-Alkylcarboxyl,
- Phenyl, Phenoxy, Phenylthio, Phenylamino, Phenyl-C₁-C₄-alkyl, Phenoxy-C₁-C₄-alkyl, Phenylthio-C₁-C₄-alkyl, Phenylamino-C₁-C₄-alkyl, wobei die aromatischen Ringe ihrerseits ein bis fünf Halogenatome und ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, oder wobei die aromatischen Ringe ihrerseits ein bis fünf Halogenatome oder ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
- ein über ein Kohlenstoffatom gebundenes fünf- oder sechsgliedriges aromatisches Ringsystem, das neben Kohlenstoffatomen ein bis drei Stickstoffatome oder ein oder zwei Stickstoffatome und ein Sauerstoffoder ein Schwefelatom oder ein Sauerstoff- oder ein Schwefelatom enthalten kann, wobei dieses Ringsystem ein bis vier Halogenatome und ein bis drei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, oder wobei die aromatischen Ringe ihrerseits ein bis vier Halogenatome oder ein bis drei der folgenden Reste tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
R³ ggf. subst. Alkyl oder ein ggf. subst., über ein Kohlenstoffatom gebundenes, aliphatisches Ringsystem, das neben Kohlenstoffatomen ein oder zwei Heteroatome aus der Gruppe Sauerstoff, Schwefel und Stickstoff enthalten kann.

2. Cyanooximether der Formel I gemäß Anspruch 1, in der R³ die folgende Bedeutung hat:
C₁-C₈-Alkyl, welches ein bis neun Halogenatome und einen der folgenden Reste tragen kann: Cyano, Nitro, Amino, C₁-C₆-Alkoxy, C₁-C₄-Halogenalkoxy, C₁-C₆-Alkylthio, C₁-C₄-Halogenalkylthio, C₃-C₈-Cycloalkyl, C₃-C₈-Cycloalkoxy, Di-C₁-C₆-alkylamino, C₁-C₆-Alkylcarbonyl, C₁-C₆-Alkoxycarbonyl, C₁-C₆-Alkylaminocarbonyl, Di-C₁-C₆-alkylaminocarbonyl, C₁-C₆-Alkylcarboxyl, Phenyl, Phenoxy, Phenylthio, Phenyl-C₁-C₄-alkyl, Phenoxy-C₁-C₄-alkyl, Phenylthio-C₁-C₄-alkyl, Phenylamino-C₁-C₄-alkyl, wobei die aromatischen Ringe ihrerseits ein bis fünf Halogenatome und ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, oder wobei die aromatischen Ringe ihrerseits ein bis fünf Halogenatome oder ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
oder C₁-C₈-Alkyl welches ein bis neun Halogenatome oder einen der folgenden Reste tragen kann: Cyano, Nitro, Amino, C₁-C₆-Alkoxy, C₁-C₄-Halogenalkoxy, C₁-C₆-Alkylthio, C₁-C₄-Halogenalkylthio,
C₃-C₈-Cycloalkyl, C₃-C₈-Cycloalkoxy, Di-C₁-C₆-alkylamino, C₁-C₆-Alkylcarbonyl, C₁-C₆-Alkoxycarbonyl, C₁-C₆-Alkylaminocarbonyl, Di-C₁-C₆-alkylaminocarbonyl, C₁-C₆-Alkylcarboxyl, Phenyl, Phenoxy, Phenylthio, Phenyl-C₁-C₄-alkyl, Phenoxy-C₁-C₄-alkyl, Phenylthio-C₁-C₄-alkyl, Phenylamino-C₁-C₄-alkyl, wobei die aromatischen Ringe ihrerseits ein bis fünf Halogenatome und ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, oder wobei die aromatischen Ringe ihrerseits ein bis fünf Halogenatome oder ein bis drei der folgenden Reste tragen können: Cyano, Nitro, C₁-C₄-Alkyl- C₁-C₄-Halogenalkyl, C₁-C₄-Alkoxy, C₁-C₄-Halogenalkoxy, C₁-C₄-Alkylthio und C₁-C₄-Halogenalkylthio;
oder ein über ein Kohlenstoffatom gebundenes aliphatisches Ringsystem, das neben Kohlenstoffatomen ein oder zwei Heteroatome aus der Gruppe Sauerstoff, Schwefel und Stickstoff enthalten kann,
wobei dieses Ringsystem ein bis fünf Halogenatome und ein bis vier der folgenden Reste tragen kann: Cyano, Nitro, Amino, C₁-C₆-Alkyl, C₁-C₄-Halogenalkyl, C₁-C₆-Alkoxy, C₁-C₄-Halogenalkoxy, C₁-C₄-Alkoxy-C₁-C₄-alkyl, C₁-C₆-Alkylthio, C₁-C₄-Halogenalkylthio, C₁-C₄-AlkylthioC₁-C₄-alkyl, C₃-C₈-Cycloalkyl, Di-C₁-C₆-alkylamino, C₁-C₆-Alkylcarboxyl, Phenyl, Phenoxy, Phenylthio, Phenyl-C₁-C₄-alkyl, Phenoxy-C₁-C₄-alkyl, PhenylthioC₁-C₄-alkyl, wobei die aromatischen Ringe ihrerseits ein bis fünf Halogenatome und ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, oder wobei die aromatischen Ringe ihrerseits ein bis fünf Halogenatome oder ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio,
oder wobei dieses Ringsystem ein bis fünf Halogenatome oder ein bis vier der folgenden Reste tragen kann: Cyano, Nitro, Amino, C₁-C₆-Alkyl, C₁-C₄-Halogenalkyl, C₁-C₆-Alkoxy, C₁-C₄-Halogenalkoxy, C₁-C₄-Alkoxy-C₁-C₄-alkyl, C₁-C₆-Alkylthio, C₁-C₄-Halogenalkylthio, C₁-C₄-Alkylthio-C₁-C₄-alkyl, C₃-C₈-Cycloalkyl, Di-C₁-C₆-alkylamino,
C₁-C₆-Alkylcarboxyl, Phenyl, Phenoxy, Phenylthio, Phenyl-C₁-C₄-alkyl, Phenoxy-C₁-C₄-alkyl, PhenylthioC₁-C₄-alkyl, wobei die aromatischen Ringe ihrerseits ein bis fünf Halogenatome und ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, oder wobei die aromatischen Ringe ihrerseits ein bis fünf Halogenatome oder ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio.

3. Cyanooximether der Formel I gemäß Anspruch 1, in der R² Phenyl, 4-Oxazolyl oder 5-Isoxazolyl bedeutet, wobei diese Ringe ein bis fünf Halogenatome und ein bis drei der folgenden Reste tragen kann:
- Cyano, Cyanato, Thiocyanato, Nitro, Amino, Hydroxy, Carboxyl, C₁-C₆-Alkyl, C₁-C₄-Halogenalkyl, C₁-C₆-Alkoxy, C₁-C₄-Halogenalkoxy, C₁-C₄-Alkoxy-C₁-C₄-alkyl, C₁-C₆-Alkylthio, C₁-C₄-Halogenalkylthio, C₁-C₄-Alkylthio-C₁-C₄-alkyl, C₃-C₁₅-Alkenyl, C₃-C₁₅-Alkenyloxy, C₃-C₈-Cycloalkyl, C₅-C₈-Cycloalkenyl, C₃-C₈-Cycloalkoxy, C₅-C₈-Cycloalkenyloxy, C₁-C₆-Alkylamino, Di-C₁-C₆-alkylamino, C₁-C₆-Alkylcarbonyl, C₁-C₆-Alkoxycarbonyl, C₁-C₆-Alkylaminocarbonyl, DiC₁-C₆-alkylaminocarbonyl, C₁-C₆-Alkylcarboxyl,
- Phenyl, Phenoxy, Phenylthio, Phenylamino, Phenyl-C₁-C₄-alkyl, Phenoxy-C₁-C₄-alkyl, PhenylthioC₁-C₄-alkyl, Phenylamino-C₁-C₄-alkyl, wobei die aromatischen Ringe ihrerseits ein bis fünf Halogenatome und/oder ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, oder wobei die aromatischen Ringe ihrerseits ein bis fünf Halogenatome oder ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio,
- ein über ein Kohlenstoffatom gebundenes fünf- oder sechsgliedriges aromatisches Ringsystem, das neben Kohlenstoffatomen ein bis drei Stickstoffatome oder ein oder zwei Stickstoffatome und ein Sauerstoff- oder ein Schwefelatom oder ein Sauerstoff- oder ein Schwefelatom enthalten kann, wobei dieses Ringsystem ein bis vier Halogenatome und ein bis drei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, oder wobei die aromatischen Ringe ihrerseits ein bis vier Halogenatome oder ein bis drei der folgenden Reste tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
oder wobei diese Ringe ein bis fünf Halogenatome oder ein bis drei der folgenden Reste tragen kann:
- Cyano, Cyanato, Thiocyanato, Nitro, Amino, Hydroxy, Carboxyl, C₁-C₆-Alkyl, C₁-C₄-Halogenalkyl, C₁-C₆-Alkoxy, C₁-C₄-Halogenalkoxy, C₁-C₄-Alkoxy-C₁-C₄-alkyl, C₁-C₆-Alkylthio, C₁-C₄-Halogenalkylthio, C₁-C₄-Alkylthio-C₁-C₄-alkyl, C₃-C₁₅-Alkenyl, C₃-C₁₅-Alkenyloxy, C₃-C₈-Cycloalkyl, C₅-C₈-Cycloalkenyl, C₃-C₈-Cycloalkoxy, C₅-C₈-Cycloalkenyloxy, C₁-C₆-Alkylamino, Di-C₁-C₆-alkylamino, C₁-C₆-Alkylcarbonyl, C₁-C₆-Alkoxycarbonyl, C₁-C₆-Alkylaminocarbonyl, DiC₁-C₆-alkylaminocarbonyl, C₁-C₆-Alkylcarboxyl,
- Phenyl, Phenoxy, Phenylthio, Phenylamino, Phenyl-C₁-C₄-alkyl, Phenoxy-C₁-C₄-alkyl, PhenylthioC₁-C₄-alkyl, Phenylamino-C₁-C₄-alkyl, wobei die aromatischen Ringe ihrerseits ein bis fünf Halogenatome und/oder ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, oder wobei die aromatischen Ringe ihrerseits ein bis fünf Halogenatome oder ein bis drei der folgenden Reste tragen können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio,
- ein über ein Kohlenstoffatom gebundenes fünf- oder sechsgliedriges aromatisches Ringsystem, das neben Kohlenstoffatomen ein bis drei Stickstoffatome oder ein oder zwei Stickstoffatome und ein Sauerstoff- oder ein Schwefelatom oder ein Sauerstoff- oder ein Schwefelatom enthalten kann, wobei dieses Ringsystem ein bis vier Halogenatome und ein bis drei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, oder wobei die aromatischen Ringe ihrerseits ein bis vier Halogenatome oder ein bis drei der folgenden Reste tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio.

4. Cyanooximether der Formel I gemäß Anspruch 1, in der R³ die folgende Bedeutung hat: C₁-C₄-Alkyl, welches ein bis neun Halogenatome und einen der folgenden Reste tragen kann: C₁-C₄-Alkoxy, C₁-C₄-Halogenalkoxy, C₁-C₄-Alkylthio, C₃-C₈-Cycloalkyl, C₃-C₈-Cycloalkoxy, Phenyl, Phenoxy, Phenylthio, Phenyl-C₁-C₄-alkyl, Phenoxy-C₁-C₄-alkyl, Phenylthio-C₁-C₄-alkyl, wobei die aromatischen Ringe ihrerseits ein bis fünf Halogenatome und ein bis drei der folgenden Reste tragen können: C₁-C₄-Alkyl, C₁-C₄-Halogenalkyl, C₁-C₄-Alkoxy, C₁-C₄-Halogenalkoxy, C₁-C₄-Alkylthio oder wobei die aromatischen Ringe ihrerseits ein bis fünf Halogenatome oder ein bis drei der folgenden Reste tragen können: C₁-C₄-Alkyl, C₁-C₄-Halogenalkyl, C₁-C₄-Alkoxy, C₁-C₄-Halogenalkoxy und C₁-C₄-Alkylthio;
oder C₁-C₄-Alkyl, welches ein bis neun Halogenatome oder einen der folgenden Reste tragen kann: C₁-C₄-Alkoxy, C₁-C₄-Halogenalkoxy, C₁-C₄-Alkylthio, C₃-C₈-Cycloalkyl, C₃-C₈-Cycloalkoxy, Phenyl, Phenoxy, Phenylthio, Phenyl-C₁-C₄-alkyl, Phenoxy-C₁-C₄-alkyl, PhenylthioC₁-C₄-alkyl, wobei die aromatischen Ringe ihrerseits ein bis fünf Halogenatome und ein bis drei der folgenden Reste tragen können: C₁-C₄-Alkyl, C₁-C₄-Halogenalkyl, C₁-C₄-Alkoxy, C₁-C₄-Halogenalkoxy und C₁-C₄-Alkylthio, oder wobei die aromatischen Ringe ihrerseits ein bis fünf Halogenatome oder ein bis drei der folgenden Reste tragen können: C₁-C₄-Alkyl, C₁-C₄-Halogenalkyl, C₁-C₄-Alkoxy, C₁-C₄-Halogenalkoxy und C₁-C₄-Alkylthio;
oder ein über ein Kohlenstoffatom gebundenes gesättigtes oder einfach ungesättigtes 3- bis 7-gliedriges Ringsystem, das neben Kohlenstoffatomen ein oder zwei Heteroatome aus der Gruppe Sauerstoff, Schwefel und Stickstoff enthalten kann, wobei dieses Ringsystem ein bis drei der folgenden Reste tragen kann: C₁-C₄-Alkyl, C₁-C₄-Alkoxy, C₁-C₄-Alkoxy-C₁-C₄-alkyl, C₁-C₄-Alkylthio, C₁-C₄-Alkylthio-C₁-C₄-alkyl, Phenyl, Phenoxy, Phenylthio, Phenyl-C₁-C₄-alkyl, Phenoxy-C₁-C₄-alkyl, Phenylthio-C₁-C₄-alkyl, wobei die aromatischen Ringe ihrerseits ein bis fünf Halogenatome und ein bis drei der folgenden Reste tragen können: C₁-C₄-Alkyl, C₁-C₄-Halogenalkyl, C₁-C₄-Alkoxy, C₁-C₄-Halogenalkoxy und C₁-C₄-Alkylthio;
oder wobei die aromatischen Ringe ihrerseits ein bis fünf Halogenatome oder ein bis drei der folgenden Reste tragen können: C₁-C₄-Alkyl, C₁-C₄-Halogenalkyl, C₁-C₄-Alkoxy, C₁-C₄-Halogenalkoxy und C₁-C₄-Alkylthio.

5. Verfahren zur Herstellung der Verbindungen der Formel I gemäß Anspruch 1, dadurch gekennzeichnet, daß man ein Cyanoxim der Formel II
HON=C(CN)-R³ II
in an sich bekannter Weise in einem inerten organischen Lösungsmittel mit einer Verbindung III
R¹R²CH-X III
in der X für eine nukleofuge Abgangsgruppe steht, verethert.

6. Mittel zur Bekämpfung von Schädlingen, enthaltend eine wirksame Menge mindestens einer Verbindung der Formel I gemäß Anspruch 1 sowie inerte Zusatzstoffe.

7. Verfahren zur Bekämpfung von Schädlingen, dadurch gekennzeichnet, daß man die Schädlinge und/oder ihren Lebensraum mit einer wirksamen Menge mindestens einer Verbindung der Formel I gemäß Anspruch 1 behandelt.

8. Verwendung der Verbindungen der Formel I gemäß Anspruch 1 zur Bekämpfung von Schädlingen.

## Claims

1. A cyanooxime ether of the formula I
R¹R²CH-ON=C(CN)-R³ I
where
R¹ is hydrogen or C₁-C₄-alkyl;
R² is a mononuclear to trinuclear aliphatic or aromatic ring system which is bonded via a carbon atom and, in addition to carbon atoms, may contain from one to four nitrogen atoms or from one to three hetero atoms selected from the group consisting of oxygen, sulfur and nitrogen, where this ring system may carry from one to five halogen atoms and/or from one to four of the following radicals:
- cyano, cyanato, thiocyanato, nitro, amino, hydroxyl, carboxyl, C₁-C₆-alkyl, C₁-C₄-haloalkyl, C₁-C₆-alkoxy, C₁-C₄-haloalkoxy, C₁-C₄-alkoxy-C₁-C₄-alkyl, C₁-C₆-alkylthio, C₁-C₄-haloalkylthio, C₁-C₄-alkylthio-C₁-C₄-alkyl, C₃-C₁₅-alkenyl, C₃-C₁₅-alkenyloxy, C₃-C₈-cycloalkyl, C₅-C₈-cycloalkenyl, C₃-C₈-cycloalkoxy, C₅-C₈-cycloalkenyloxy, C₁-C₆-alkylamino, di-C₁-C₆-alkylamino, C₁-C₆-alkylcarbonyl, C₁-C₆-alkoxycarbonyl, C₁-C₆-alkylaminocarbonyl, di-C₁-C₆-alkylaminocarbonyl, C₁-C₆-alkylcarboxyl,
- phenyl, phenoxy, phenylthio, phenylamino, phenyl-C₁-C₄-alkyl, phenoxy-C₁-C₄-alkyl, phenylthio-C₁-C₄-alkyl or phenylamino-C₁-C₄-alkyl, where the aromatic rings in turn may carry from one to five halogen atoms and from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio, or where the aromatic rings in turn may carry from one to five halogen atoms or from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio;
- a five-membered or six-membered aromatic ring system which is bonded via a carbon atom and, in addition to carbon atoms, may contain from one to three nitrogen atoms or one or two nitrogen atoms and one oxygen or one sulfur atom or one oxygen or one sulfur atom, where this ring system may carry from one to four halogen atoms and from one to three of the following radicals: C₁-C₄-alkyl, C₁-C₄-haloalkyl, C₁-C₄-alkoxy, C₁-C₄-haloalkoxy, C₁-C₄-alkylthio and C₁-C₄-haloalkylthio, or where the aromatic rings in turn may carry from one to four halogen atoms or from one to three of the following radicals: C₁-C₄-alkyl, C₁-C₄-haloalkyl, C₁-C₄-alkoxy, C₁-C₄-haloalkoxy, C₁-C₄-alkylthio and C₁-C₄-haloalkylthio;
or where this ring system may carry from one to five halogen atoms or from one to four of the following radicals:
- cyano, cyanato, thiocyanato, nitro, amino, hydroxyl, carboxyl, C₁-C₆-alkyl, C₁-C₄-haloalkyl, C₁-C₆-alkoxy, C₁-C₄-haloalkoxy, C₁-C₄-alkoxy-C₁-C₄-alkyl, C₁-C₆-alkylthio, C₁-C₄-haloalkylthio, C₁-C₄-alkylthio-C₁-C₄-alkyl, C₃-C₁₅-alkenyl, C₃-C₁₅-alkenyloxy, C₃-C₈-cycloalkyl, C₅-C₈-cycloalkenyl, C₃-C₈-cycloalkoxy, C₅-C₈-cycloalkenyloxy, C₁-C₆-alkylamino, di-C₁-C₆-alkylamino, C₁-C₆-alkylcarbonyl, C₁-C₆-alkoxycarbonyl, C₁-C₆-alkylaminocarbonyl, di-C₁-C₆-alkylaminocarbonyl, C₁-C₆-alkylcarboxyl,
- phenyl, phenoxy, phenylthio, phenylamino, phenyl-C₁-C₄-alkyl, phenoxy-C₁-C₄-alkyl, phenylthio-C₁-C₄-alkyl or phenylamino-C₁-C₄-alkyl, where the aromatic rings in turn may carry from one to five halogen atoms and from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio, or where the aromatic rings in turn may carry from one to five halogen atoms or from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio;
- a five-membered or six-membered aromatic ring system which is bonded via a carbon atom and, in addition to carbon atoms, may contain from one to three nitrogen atoms or one or two nitrogen atoms and one oxygen or one sulfur atom or one oxygen or one sulfur atom, where this ring system may carry from one to four halogen atoms and from one to three of the following radicals: C₁-C₄-alkyl, C₁-C₄-haloalkyl, C₁-C₄-alkoxy, C₁-C₄-haloalkoxy, C₁-C₄-alkylthio and C₁-C₄-haloalkylthio, or where the aromatic rings in turn may carry from one to four halogen atoms or from one to three of the following radicals: C₁-C₄-alkyl, C₁-C₄-haloalkyl, C₁-C₄-alkoxy, C₁-C₄-haloalkoxy, C₁-C₄-alkylthio and C₁-C₄-haloalkylthio, and
R³ is unsubstituted or substituted alkyl or an unsubstituted or substituted aliphatic ring system which is bonded via a carbon atom and, in addition to carbon atoms, may contain one or two hetero atoms selected from the group consisting of oxygen, sulfur and nitrogen.

2. A cyanooxime ether of the formula I as claimed in claim 1, wherein R³ is:
C₁-C₈-alkyl which may carry from one to nine halogen atoms and one of the following radicals: cyano, nitro, amino, C₁-C₆-alkoxy, C₁-C₄-haloalkoxy, C₁-C₆-alkylthio, C₁-C₄-haloalkylthio, C₃-C₈-cycloalkyl, C₃-C₈-cycloalkoxy, di-C₁-C₆-alkylamino, C₁-C₆-alkylcarbonyl, C₁-C₆-alkoxycarbonyl, C₁-C₆-alkylaminocarbonyl, di-C₁-C₆-alkylaminocarbonyl, C₁-C₆-alkylcarboxyl, phenyl, phenoxy, phenylthio, phenyl-C₁-C₄-alkyl, phenoxy-C₁-C₄-alkyl, phenylthio-C₁-C₄-alkyl or phenylamino-C₁-C₄-alkyl, where the aromatic rings in turn may carry from one to five halogen atoms and from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio, or where the aromatic rings in turn may carry from one to five halogen atoms or from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio;
or C₁-C₈-alkyl which may carry from one to nine halogen atoms or one of the following radicals: cyano, nitro, amino, C₁-C₆-alkoxy, C₁-C₄-haloalkoxy, C₁-C_{C}-alkylthio, C₁-C₄-haloalkylthio,
C₃-C₈-cycloalkyl, C₃-C₈-cycloalkoxy, di-C₁-C₆-alkylamino, C₁-C₆-alkylcarbonyl, C₁-C₆-alkoxycarbonyl, C₁-C₆-alkylaminocarbonyl, di-C₁-C₆-alkylaminocarbonyl, C₁-C₆-alkylcarboxyl, phenyl, phenoxy, phenylthio, phenyl-C₁-C₄-alkyl, phenoxy-C₁-C₄-alkyl, phenylthio-C₁-C₄-alkyl or phenylamino-C₁-C₄-alkyl, where the aromatic rings in turn may carry from one to five halogen atoms and from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio, or where the aromatic rings in turn may carry from one to five halogen atoms or from one to three of the following radicals: cyano, nitro, C₁-C₄-alkyl, C₁-C₄-haloalkyl, C₁-C₄alkoxy, C₁-C₄-haloalkoxy, C₁-C₄-alkylthio and C₁-C₄-haloalkylthio;
or an aliphatic ring system which is bonded via a carbon atom and, in addition to carbon atoms, may contain one or two hetero atoms selected from the group consisting of oxygen, sulfur and nitrogen,
where this ring system may carry from one to five halogen atoms and from one to four of the following radicals: cyano, nitro, amino, C₁-C₆-alkyl, C₁-C₄-haloalkyl, C₁-C₆-alkoxy, C₁-C₄-haloalkoxy, C₁-C₄-alkoxy-C₁-C₄-alkyl, C₁-C₆-alkylthio, C₁-C₄-haloalkylthio, C₁-C₄-alkylthio-C₁-C₄-alkyl, C₃-C₈-cycloalkyl, di-C₁-C₆-alkylamino, C₁-C₆-alkylcarboxyl, phenyl, phenoxy, phenylthio, phenyl-C₁-C₄-alkyl, phenoxy-C₁-C₄-alkyl, phenylthio-C₁-C₄-alkyl, where the aromatic rings in turn may carry from one to five halogen atoms and from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio, or where the aromatic rings in turn may carry from one to five halogen atoms or from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio,
or where this ring system may carry from one to five halogen atoms or from one to four of the following radicals: cyano, nitro, amino, C₁-C₆-alkyl, C₁-C₄-haloalkyl, C₁-C₆-alkoxy, C₁-C₄-haloalkoxy, C₁-C₄-alkoxy-C₁-C₄-alkyl, C₁-C₆-alkylthio, C₁-C₄-haloalkylthio, C₁-C₄-alkylthio-C₁-C₄-alkyl, C₃-C₈-cycloalkyl, di-C₁-C₆-alkylamino,
C₁-C₆-alkylcarboxyl, phenyl, phenoxy, phenylthio, phenyl-C₁-C₄-alkyl, phenoxy-C₁-C₄-alkyl, phenylthio-C₁-C₄-alkyl, where the aromatic rings in turn may carry from one to five halogen atoms and from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio, or where the aromatic rings in turn may carry from one to five halogen atoms or from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio.

3. A cyanooxime ether of the formula I as claimed in claim 1, wherein R² is phenyl, 4-oxazolyl or 5-oxazolyl, where these rings may carry from one to five halogen atoms and from one to three of the following radicals:
- cyano, cyanato, thiocyanato, nitro, amino, hydroxyl, carboxyl, C₁-C₆-alkyl, C₁-C₄-haloalkyl, C₁-C₆-alkoxy, C₁-C₄-haloalkoxy, C₁-C₄-alkoxy-C₁-C₄-alkyl, C₁-C₆-alkythio, C₁-C₄-haloalkylthio, C₁-C₄-alkylthio-C₁-C₄-alkyl, C₃-C₁₅-alkenyl, C₃-C₁₅-alkenyloxy, C₃-C₈-cycloalkyl, C₅-C₈-cycloalkenyl, C₃-C₈-cycloalkoxy, C₅-C₈-cycloalkenyloxy, C₁-C₆-alkylamino, di-C₁-C₆-alkylamino, C₁-C₆-alkylcarbonyl, C₁-C₆-alkoxycarbonyl, C₁-C₆-alkylaminocarbonyl, di-C₁-C₆-alkylaminocarbonyl, C₁-C₆-alkylcarboxyl,
- phenyl, phenoxy, phenylthio, phenylamino, phenyl-C₁-C₄-alkyl, phenoxy-C₁-C₄-alkyl, phenylthio-C₁-C₄-alkyl or phenylamino-C₁-C₄-alkyl, where the aromatic rings in turn may carry from one to five halogen atoms and/or from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio, or where the aromatic rings in turn may carry from one to four halogen atoms or from one to three of the following radicals: C₁-C₄-alkyl, C₁-C₄-haloalkyl, C₁-C₄-alkoxy, C₁-C₄-haloalkoxy, C₁-C₄-alkylthio and C₁-C₄-haloalkylthio
- a five-membered or six-membered aromatic ring system which is bonded via a carbon atom and, in addition to carbon atoms, may contain from one to three nitrogen atoms or one or two nitrogen atoms and one oxygen or one sulfur atom or one oxygen or one sulfur atom, where this ring system may carry from one to four halogen atoms and from one to three of the following radicals: C₁-C₄-alkyl, C₁-C₄-haloalkyl, C₁-C₄-alkoxy, C₁-C₄-haloalkoxy, C₁-C₄-alkylthio and C₁-C₄-haloalkylthio, or where the aromatic rings in turn may carry from one to five halogen atoms or from one to three of the following radicals: C₁-C₄-alkyl, C₁-C₄-haloalkyl, C₁-C₄-alkoxy, C₁-C₄-haloalkoxy, C₁-C₄-alkylthio and C₁-C₄-haloalkylthio;
or where these rings may carry from one to five halogen atoms or from one to three of the following radicals:
- cyano, cyanato, thiocyanato, nitro, amino, hydroxyl, carboxyl, C₁-C₆-alkyl, C₁-C₄-haloalkyl, C₁-C₆-alkoxy, C₁-C₄-haloalkoxy, C₁-C₄-alkoxy-C₁-C₄-alkyl, C₁-C₆-alkylthio, C₁-C₄-haloalkylthio, C₁-C₄-alkylthioC₁-C₄-alkyl, C₃-C₁₅-alkenyl, C₃-C₁₅-alkenyloxy, C₃-C₈-cycloalkyl, C₅-C₈-cycloalkenyl, C₃-C₈-cycloalkoxy, C₅-C₈-cycloalkenyloxy, C₁-C₆-alkylamino, di-C₁-C₆-alkylamino, C₁-C₆-alkylcarbonyl, C₁-C₆-alkoxycarbonyl, C₁-C₆-alkylaminocarbonyl, di-C₁-C₆-alkylaminocarbonyl, C₁-C₆-alkylcarboxyl,
- phenyl, phenoxy, phenylthio, phenylamino, phenyl-C₁-C₄-alkyl, phenoxy-C₁-C₄-alkyl, phenylthio-C₁-C₄-alkyl or phenylamino-C₁-C₄-alkyl, where the aromatic rings in turn may carry from one to five halogen atoms and/or from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio, or where the aromatic rings in turn may carry from one to five halogen atoms or from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio,
- a five-membered or six-membered aromatic ring system which is bonded via a carbon atom and, in addition to carbon atoms, may contain from one to three nitrogen atoms or one or two nitrogen atoms and one oxygen or one sulfur atom or one oxygen or one sulfur atom, where this ring system may carry from one to four halogen atoms and from one to three of the following radicals: C₁-C₄-alkyl, C₁-C₄-haloalkyl, C₁-C₄-alkoxy, C₁-C₄-haloalkoxy, C₁-C₄-alkylthio and C₁-C₄-haloalkylthio, or where the aromatic rings in turn may carry from one to four halogen atoms or from one to three of the following radicals: C₁-C₄-alkyl, C₁-C₄-haloalkyl, C₁-C₄-alkoxy, C₁-C₄-halo- alkoxy, C₁-C₄-alkylthio and C₁-C₄-haloalkylthio.

4. A cyanooxime ether of the formula I as claimed in claim 1, wherein R³ is C₁-C₄-alkyl which may carry from one to nine halogen atoms and one of the following radicals: C₁-C₄-alkoxy, C₁-C₄-haloalkoxy, C₁-C₄-alkylthio, C₃-C₈-cycloalkyl, C₃-C₈-cycloalkoxy, phenyl, phenoxy, phenylthio, phenyl-C₁-C₄alkyl, phenoxy-C₁-C₄-alkyl, phenylthio-C₁-C₄-alkyl, where the aromatic rings in turn may carry from one to five halogen atoms and from one to three of the following radicals: C₁-C₄-alkyl, C₁-C₄-haloalkyl, C₁-C₄-alkoxy, C₁-C₄-haloalkoxy and C₁-C₄-alkylthio, or where the aromatic rings in turn may carry from one to five halogen atoms or from one to three of the following radicals: C₁-C₄-alkyl, C₁-C₄-haloalkyl, C₁-C₄-alkoxy, C₁-C₄-haloalkoxy and C₁-C₄-alkylthio;
or C₁-C₄-alkyl which may carry from one to nine halogen atoms or one of the following radicals: C₁-C₄-alkoxy, C₁-C₄-haloalkoxy, C₁-C₄alkylthio, C₃-C₈-cycloalkyl, C₃-C₈-cycloalkoxy, phenyl, phenoxy, phenylthio, phenyl-C₁-C₄-alkyl, phenoxy-C₁-C₄-alkyl, phenylthio-C₁-C₄-alkyl, where the aromatic rings in turn may carry from one to five halogen atoms and from one to three of the following radicals: C₁-C₄-alkyl, C₁-C₄-haloalkyl, C₁-C₄-alkoxy, C₁-C₄-haloalkoxy and C₁-C₄-alkylthio, or where the aromatic rings in turn may carry from one to five halogen atoms or from one to three of the following radicals: C₁-C₄-alkyl, C₁-C₄-haloalkyl, C₁-C₄-alkoxy, C₁-C₄-haloalkoxy and C₁-C₄-alkylthio;
or a saturated or monounsaturated 3-membered to 7-membered ring system which is bonded via a carbon atom and, in addition to carbon atoms, may carry one or two hetero atoms selected from the group consisting of oxygen, sulfur and nitrogen, where this ring system may carry from one to three of the following radicals: C₁-C₄-alkyl, C₁-C₄-alkoxy, C₁-C₄-alkoxy-C₁-C₄-alkyl, C₁-C₄-alkylthio, C₁-C₄-alkylthio-C₁-C₄-alkyl, phenyl, phenoxy, phenylthio, phenyl-C₁-C₄-alkyl, phenoxy-C₁-C₄-alkyl or phenylthio-C₁-C₄-alkyl, where the aromatic rings in turn may carry from one to five halogen atoms and from one to three of the following radicals: C₁-C₄-alkyl, C₁-C₄-haloalkyl, C₁-C₄-alkoxy, C₁-C₄-haloalkoxy and C₁-C₄-alkylthio;
or where the aromatic rings in turn may carry from one to five halogen atoms or from one to three of the following radicals: C₁-C₄-alkyl, C₁-C₄-haloalkyl, C₁-C₄-alkoxy, C₁-C₄-haloalkoxy and C₁-C₄-alkylthio.

5. A process for the preparation of a compound of the formula I as claimed in claim 1, wherein a cyanooxime of the formula II
HON=C(CN)-R³ II
is etherified in a conventional manner, in an inert organic solvent, with a compound III
R¹R²CH-X III
where X is a nucleofugic leaving group.

6. A pesticide containing an effective amount of at least one compound of the formula I as claimed in claim 1 and inert additives.

7. A method for controlling pests, wherein the pests and/or their habitat is or are treated with an effective amount of at least one compound of the formula I as claimed in claim 1.

8. Use of a compound of the formula I as claimed in claim 1 for controlling pests.

## Revendications

1. Ethers de cyanooximes de formule I
R¹R²CH-ON=C(CN)-R³ I
dans laquelle les symboles ont les significations suivantes :
R¹ l'hydrogène ou un groupe alkyle en C1-C4 ;
R² un système cyclique aliphatique ou aromatique mono- à tri-cyclique relié par l'intermédiaire d'un atome de carbone et qui, en plus des atomes de carbone, peut contenir un à quatre atomes d'azote ou un à trois hétéroatomes choisis parmi l'oxygène, le soufre et l'azote, ce système cyclique pouvant porter un à cinq atomes d'halogènes et un à quatre des substituants suivants :
- cyano, cyanato, thiocyanato, nitro, amino, hydroxy, carboxyle, alkyle en C1-C6, halogénoalkyle en C1-C4, alcoxy en C1-C6, halogénoalcoxy en C1-C4, (alcoxy en C1-C4)-alkyle en C1-C4, alkylthio en C1-C6, halogénoalkylthio en C1-C4, (alkylthio en C1-C4)-alkyle en C1-C4, alcényle en C3-C15, alcényloxy en C3-C15, cycloalkyle en C3-C8, cycloalcényle en C5-C8, cycloalcoxy en C3-C8, cycloalcényloxy en C5-C8, alkylamino en C1-C6, di-(alkyle en C1-C6)amino, (alkyle en C1-C6)carbonyle, (alcoxy en C1-C6)carbonyle, (alkyle en C1-C6)aminocarbonyle, di-(alkyle en C1-C6)aminocarbonyle, (alkyle en C1-C6)carboxyle,
- phényle, phénoxy, phénylthio, phénylamino, phényl-alkyle en C1-C4, phénoxy-alkyle en C1-C4, phénylthio-alkyle en C1-C4, phénylamino-alkyle en C1-C4, les noyaux aromatiques pouvant eux-mêmes porter un à cinq atomes d'halogènes et un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4, ou bien les noyaux aromatiques peuvent eux-mêmes porter un à cinq atomes d'halogènes ou un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4 ;
- un système cyclique aromatique à cinq ou six chaînons relié par l'intermédiaire d'un atome de carbone et qui, en plus des atomes de carbone, peut contenir un à trois atomes d'azote ou un ou deux atomes d'azote et un atome d'oxygène ou un atome de soufre ou un atome d'oxygène ou un atome de soufre, ce système cyclique pouvant porter un à quatre atomes d'halogènes et un à trois des substituants suivants : alkyle en C1-C4, halogénoalkyle en C1-C4, alcoxy en C1-C4, halogénoalcoxy en C1-C4, alkylthio en C1-C4 et halogénoalkylthio en C1-C4, ou bien les noyaux aromatiques peuvent eux-mêmes porter un à quatre atomes d'halogènes ou un à trois des substituants suivants : alkyle en C1-C4, halogénoalkyle en C1-C4, alcoxy en C1-C4, halogénoalcoxy en C1-C4, alkylthio en C1-C4 et halogénoalkylthio en C1-C4 ;
ou bien encore ce système cyclique peut porter un à cinq atomes d'halogènes ou un à quatre des substituants suivants :
- cyano, cyanato, thiocyanato, nitro, amino, hydroxy, carboxyle, alkyle en C1-C6, halogénoalkyle en C1-C4, alcoxy en C1-C6, halogénoalcoxy en C1-C4, (alcoxy en C1-C4)-alkyle en C1-C4, alkylthio en C1-C6, halogénoalkylthio en C1-C4, (alkylthio en C1-C4)-alkyle en C1-C4, alcényle en C3-C15, alcényloxy en C3-C15, cycloalkyle en C3-C8, cycloalcényle en C5-C8, cycloalcoxy en C3-C8, cycloalcényloxy en C5-C8, alkylamino en C1-C6, di-(alkyle en C1-C6)amino, (alkyle en C1-C6)carbonyle, (alcoxy en C1-C6)carbonyle, (alkyle en C1-C6)aminocarbonyle, di-(alkyle en C1-C6)aminocarbonyle, (alkyle en C1-C6)carboxyle,
- phényle, phénoxy, phénylthio, phénylamino, phényl-alkyle en C1-C4, phénoxy-alkyle en C1-C4, phénylthio-alkyle en C1-C4, phénylamino-alkyle en C1-C4, les noyaux aromatiques pouvant eux-mêmes porter un à cinq atomes d'halogènes et un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4, ou bien les noyaux aromatiques peuvent eux-mêmes porter un à cinq atomes d'halogènes ou un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4 ;
- un système cyclique aromatique à cinq ou six chaînons relié par l'intermédiaire d'un atome de carbone et qui, en plus des atomes de carbone, peut contenir un à trois atomes d'azote ou un ou deux atomes d'azote et un atome d'oxygène ou un atome de soufre ou un atome d'oxygène ou un atome de soufre, ce système cyclique pouvant porter un à quatre atomes d'halogènes et un à trois des substituants suivants : alkyle en C1-C4, halogénoalkyle en C1-C4, alcoxy en C1-C4, halogénoalcoxy en C1-C4, alkylthio en C1-C4 et halogénoalkylthio en C1-C4, ou bien les noyaux aromatiques peuvent eux-mêmes porter un à quatre atomes d'halogènes ou un à trois des substituants suivants : alkyle en C1-C4, halogénoalkyle en C1-C4, alcoxy en C1-C4, halogénoalcoxy en C1-C4, alkylthio en C1-C4 et halogénoalkylthio en C1-C4 ;
R³ un groupe alkyle éventuellement substitué ou un système cyclique aliphatique relié par l'intermédiaire d'un atome de carbone et éventuellement substitué et qui, en plus des atomes de carbone, peut contenir un ou deux hétéroatomes choisis parmi l'oxygène, le soufre et l'azote.

2. Ethers de cyanooximes de formule I selon la revendication 1, dans laquelle R³ a l'une des significations suivantes :
alkyle en C1-C8 pouvant porter un à neuf atomes d'halogènes et un des substituants suivants : cyano, nitro, amino, alcoxy en C1-C6, halogénoalcoxy en C1-C4, alkylthio en C1-C6, halogénoalkylthio en C1-C4, cycloalkyle en C3-C8, cycloalcoxy en C3-C8, di-(alkyle en C1-C6)amino, (alkyle en C1-C6)carbonyle, (alcoxy en C1-C6)carbonyle, (alkyle en C1-C6)aminocarbonyle, di-(alkyle en C1-C6)aminocarbonyle, (alkyle en C1-C6)carboxyle, phényle, phénoxy, phénylthio, phényl-alkyle en C1-C4, phénoxy-alkyle en C1-C4, phénylthio-alkyle en C1-C4, phénylamino-alkyle en C1-C4, les noyaux aromatiques pouvant eux-mêmes porter un à cinq atomes d'halogènes et un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4, ou bien les noyaux aromatiques peuvent eux-mêmes porter un à cinq atomes d'halogènes ou un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4 ;
ou bien alkyle en C1-C8 qui peut porter un à neuf atomes d'halogènes ou un des substituants suivants : cyano, nitro, amino, alcoxy en C1-C6, halogénoalcoxy en C1-C4, alkylthio en C1-C6, halogénoalkylthio en C1-C4,
cycloalkyle en C3-C8, cycloalcoxy en C3-C8, di-(alkyle en C1-C6)amino, (alkyle en C1-C6)carbonyle, (alcoxy en C1-C6)carbonyle, (alkyle en C1-C6)aminocarbonyle, di-(alkyle en C1-C6)aminocarbonyle, (alkyle en C1-C6)carboxyle, phényle, phénoxy, phénylthio, phénylalkyle en C1-C4, phénoxy-alkyle en C1-C4, phénylthio-alkyle en C1-C4, phénylamino-alkyle en C1-C4, les noyaux aromatiques pouvant eux-mêmes porter un à cinq atomes d'halogènes et un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4, ou bien les noyaux aromatiques peuvent eux-mêmes porter un à cinq atomes d'halogènes ou un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4 ;
ou bien un système cyclique aliphatique relié par l'intermédiaire d'un atome de carbone et qui, en plus des atomes de carbone, peut contenir un ou deux hétéroatomes choisis parmi l'oxygène, le soufre et l'azote,
ce système cyclique pouvant porter un à cinq atomes d'halogènes et un à quatre des substituants suivants : cyano, nitro, amino, alkyle en C1-C6, halogénoalkyle en C1-C4, alcoxy en C1-C6, halogénoalcoxy en C1-C4, (alcoxy en C1-C4)alkyle en C1-C4, alkylthio en C1-C6, halogénoalkylthio en C1-C4, (alkyle en C1-C4)thioalkyle en C1-C4, cycloalkyle en C3-C8, di-(alkyle en C1-C6)amino, (alkyle en C1-C6)carboxyle, phényle, phénoxy, phénylthio, phényl-alkyle en C1-C4, phénoxy-alkyle en C1-C4, phénylthio-alkyle en C1-C4, les noyaux aromatiques pouvant eux-mêmes porter un à cinq atomes d'halogènes et un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4, ou bien les noyaux aromatiques peuvent eux-mêmes porter un à cinq atomes d'halogènes ou un à trois des substituants suivants : cyano, nitro, alkyle en C1-C4, halogénoalkyle en C1-C4, alcoxy en C1-C4, halogénoalcoxy en C1-C4, alkylthio en C1-C4, et halogénoalkylthio en C1-C4,
ou bien ce système cyclique peut porter un à cinq atomes d'halogènes ou un à quatre des substituants suivants : cyano, nitro, amino, alkyle en C1-C6, halogénoalkyle en C1-C4, alcoxy en C1-C6, halogénoalcoxy en C1-C4, (alcoxy en C1-C4)alkyle en C1-C4, alkylthio en C1-C6, halogénoalkylthio en C1-C4, (alkylthio en C1-C4)alkyle en C1-C4, cycloalkyle en C3-C6, di-(alkyle en C1-C6)amino,
(alkyle en C1-C6)carboxyle, phényle, phénoxy, phénylthio, phényl-alkyle en C1-C4, phénoxy-alkyle en C1-C4, phénylthio-alkyle en C1-C4, les noyaux aromatiques pouvant eux-mêmes porter un à cinq atomes d'halogènes et un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4, ou bien les noyaux aromatiques peuvent eux-mêmes porter un à cinq atomes d'halogènes ou un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4.

3. Ethers de cyanooximes de formule I selon la revendication 1, dans laquelle R² représente un groupe phényle, 4-oxazolyle ou 5-isoxazolyle,
ces cycles pouvant porter un à cinq atomes d'halogènes et un à trois des substituants suivants :
- cyano, cyanato, thiocyanato, nitro, amino, hydroxy, carboxyle, alkyle en C1-C6, halogénoalkyle en C1-C4, alcoxy en C1-C6, halogénoalcoxy en C1-C4, (alcoxy en C1-C4)alkyle en C1-C4, alkylthio en C1-C6, halogénoalkylthio en C1-C4, (alkylthio en C1-C4)alkyle en C1-C4, alcényle en C3-C15, alcényloxy en C3-C15, cycloalkyle en C3-C8, cycloalcényle en C5-C8, cycloalcoxy en C3-C8, cycloalcényloxy en C5-C8, alkylamino en C1-C6, di-(alkyle en C1-C6)amino, (alkyle en C1-C6)carbonyle, (alcoxy en C1-C6)carbonyle, (alkyle en C1-C6)aminocarbonyle, di-(alkyle en C1-C6)aminocarbonyle, (alkyle en C1-C6)carboxyle,
- phényle, phénoxy, phénylthio, phénylamino, phényl-alkyle en C1-C4, phénoxy-alkyle en C1-C4, phénylthio-alkyle en C1-C4, phénylamino-alkyle en C1-C4, les noyaux aromatiques pouvant eux-mêmes porter un à cinq atomes d'halogènes et/ou un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4, ou bien les noyaux aromatiques peuvent eux-mêmes porter un à cinq atomes d'halogènes ou un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4,
- un système cyclique aromatique à cinq ou six chaînons reliés par l'intermédiaire d'un atome de carbone et qui, en plus des atomes de carbone, peut contenir un à trois atomes d'azote ou un ou deux atomes d'azote et un atome d'oxygène ou un atome de soufre ou un atome d'oxygène ou un atome de soufre, ce système cyclique pouvant porter un à quatre atomes d'halogènes et un à trois des substituants suivants : alkyle en C1-C4, halogénoalkyle en C1-C4, alcoxy en C1-C4, halogénoalcoxy en C1-C4, alkylthio en C1-C4 et halogénoalkylthio en C1-C4, les noyaux aromatiques pouvant eux-mêmes porter un à quatre atomes d'halogènes ou un à trois des substituants suivants : alkyle en C1-C4, halogénoalkyle en C1-C4, alcoxy en C1-C4, halogénoalcoxy en C1-C4, alkylthio en C1-C4 et halogénoalkylthio en C1-C4 ;
ou bien ces cycles peuvent porter un à cinq atomes d'halogènes ou un à trois des substituants suivants :
- cyano, cyanato, thiocyanato, nitro, amino, hydroxy, carboxyle, alkyle en C1-C6, halogénoalkyle en C1-C4, alcoxy en C1-C6, halogénoalcoxy en C1-C4, (alcoxy en C1-C4)alkyle en C1-C4, alkylthio en C1-C6, halogénoalkylthio en C1-C4, (alkylthio en C1-C4)alkyle en C1-C4, alcényle en C3-C15, alcényloxy en C3-C15, cycloalkyle en C3-C8, cycloalcényle en C5-C8, cycloalcoxy en C3-C8, cycloalcényloxy en C5-C8, alkylamino en C1-C6, di-(alkyle en C1-C6)amino, (alkyle en C1-C6)carbonyle, (alcoxy en C1-C6)carbonyle, (alkyle en C1-C6)aminocarbonyle, di-(alkyle en C1-C6)aminocarbonyle, (alkyle en C1-C6)carboxyle,
- phényle, phénoxy, phénylthio, phénylamino, phényl-alkyle en C1-C4, phénoxy-alkyle en C1-C4, phénylthio-alkyle en C1-C4, phénylamino-alkyle en C1-C4, les noyaux aromatiques pouvant eux-mêmes porter un à cinq atomes d'halogènes et/ou un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4, les noyaux aromatiques pouvant eux-mêmes porter un à cinq atomes d'halogènes ou un à trois des substituants suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4,
- un système cyclique aromatique à cinq ou six chaînons relié par l'intermédiaire d'un atome de carbone et qui, en plus des atomes de carbone, peut contenir un à trois atomes d'azote ou un ou deux atomes d'azote et un atome d'oxygène ou un atome de soufre ou un atome d'oxygène ou un atome de soufre, ce système cyclique pouvant porter un à quatre atomes d'halogènes et un à trois des substituants suivants : alkyle en C1-C4, halogénoalkyle en C1-C4, alcoxy en C1-C4, halogénoalcoxy en C1-C4, alkylthio en C1-C4, et halogénoalkylthio en C1-C4, ou bien ces noyaux aromatiques peuvent eux-mêmes porter un à quatre atomes d'halogènes ou un à trois des substituants suivants : alkyle en C1-C4, halogénoalkyle en C1-C4, alcoxy en C1-C4, halogénoalcoxy en C1-C4, alkylthio en C1-C4 et halogénoalkylthio en C1-C4.

4. Ethers de cyanooximes de formule I selon la revendication 1, dans laquelle R³ a les significations suivantes : alkyle en C1-C4 qui peut porter un à neuf atomes d'halogènes et un des substituants suivants : alcoxy en C1-C4, halogénoalcoxy en C1-C4, alkylthio en C1-C4, cycloalkyle en C3-C8, cycloalcoxy en C3-C8, phényle, phénoxy, phénylthio, phényl-alkyle en C1-C4, phénoxy-alkyle en C1-C4, phénylthio-alkyle en C1-C4, les noyaux aromatiques pouvant eux-mêmes porter un à cinq atomes d'halogènes et un à trois des substituants suivants : alkyle en C1-C4, halogénoalkyle en C1-C4, alcoxy en C1-C4, halogénoalcoxy en C1-C4, alkylthio en C1-C4, ou bien les noyaux aromatiques peuvent eux-mêmes porter un à cinq atomes d'halogènes ou un à trois des substituants suivants : alkyle en C1-C4, halogénoalkyle en C1-C4, alcoxy en C1-C4, halogénoalcoxy en C1-C4 et alkylthio en C1-C4 ;
ou bien alkyle en C1-C4 qui peut porter un à neuf atomes d'halogènes ou un des substituants suivants : alcoxy en C1-C4, halogénoalcoxy en C1-C4, alkylthio en C1-C4, cycloalkyle en C3-C8, cycloalcoxy en C3-C8, phényle, phénoxy, phénylthio, phényl-alkyle en C1-C4, phénoxy-alkyle en C1-C4, phénylthio-alkyle en C1-C4, les noyaux aromatiques pouvant eux-mêmes porter un à cinq atomes d'halogènes et un à trois des substituants suivants : alkyle en C1-C4, halogénoalkyle en C1-C4, alcoxy en C1-C4, halogénoalcoxy en C1-C4 et alkylthio en C1-C4, ou bien les noyaux aromatiques peuvent eux-mêmes porter un à cinq atomes d'halogènes ou un à trois des substituants suivants : alkyle en C1-C4, halogénoalkyle en C1-C4, alcoxy en C1-C4, halogénoalcoxy en C1-C4 et alkylthio en C1-C4 ;
ou bien un système cyclique saturé ou mono-insaturé de trois à sept chaînons relié par l'intermédiaire d'un atome de carbone et qui, en plus des atomes de carbone, peut contenir un ou deux hétéroatomes choisis parmi l'oxygène, le soufre et l'azote, ce système cyclique pouvant porter un à trois des substituants suivants : alkyle en C1-C4, alcoxy en C1-C4, (alcoxy en C1-C4)alkyle en C1-C4, alkylthio en C1-C4, (alkylthio en C1-C4)alkyle en C1-C4, phényle, phénoxy, phénylthio, phényl-alkyle en C1-C4, phénoxy-alkyle en C1-C4, phénylthio-alkyle en C1-C4, les noyaux aromatiques pouvant eux-mêmes porter un à cinq atomes d'halogènes et un à trois des substituants suivants : alkyle en C1-C4, halogénoalkyle en C1-C4, alcoxy en C1-C4, halogénoalcoxy en C1-C4 et alkylthio en C1-C4 ;
ou bien les noyaux aromatiques peuvent eux-mêmes porter un à cinq atomes d'halogènes ou un à trois des substituants suivants : alkyle en C1-C4, halogénoalkyle en C1-C4, alcoxy en C1-C4, halogénoalcoxy en C1-C4 et alkylthio en C1-C4.

5. Procédé de préparation des composés de formule I selon la revendication 1, caractérisé par le fait que l'on éthérifie une cyanooxime de formule II
HON=C(CN)-R³ II
de manière connue en soi, dans un solvant organique inerte, à l'aide d'un composé de formule III
R¹R²CH-X III
dans laquelle X représente un groupe éliminable nucléofuge.

6. Produit pour combattre les parasites, qui contient une quantité efficace d'au moins un composé de formule I selon la revendication 1 et des additifs inertes.

7. Procédé pour combattre les parasites, caractérisé par le fait que l'on traite les parasites et/ou leur habitat par une quantité efficace d'au moins un composé de formule I selon la revendication 1.

8. Utilisation des composés de formule I selon la revendication 1 pour la lutte contre les parasites.
